# EUROPEAN PATENT APPLICATION

(11) **EP 4 474 015 A1**
(43) Date of publication of application: **11.12.2024**
(21) Application number: 23177325.0
(22) Date of filing: 05.06.2023
(51) Int. Cl.: A61P 31/14, C12N 15/86

(54) **NODA-LIKE RNA-VACCINE PHARMACON AND PRODUCTION AND USES THEREOF**

(71) Applicant: Veterna Srl, 20121 Milano (IT)
(72) Inventor: Seidler, Randolph Wilfried Richard, 20121 Milano (IT); Stadler, Konrad, 20121 Milano (IT); Sno-Gubbels Melani Maria Roberta, 20121 Milano (IT)
(74) Representative: Bianchetti & Minoja with Trevisan & Cuonzo IPS SRL

(57) **Abstract**

The invention discloses a ribonucleic acid (RNA)-pharmacon comprising at least a functional part of a trans-amplifying RNA (taRNA) replication element of an Alphanoda virus.

## Description

### Field of the invention

The invention relates to "active pharmaceutical ingredient(s)" (API) or pharmacon(s) and the production thereof. API is the ingredient in a pharmaceutical (drug) that is sufficiently biologically or pharmacologically active to achieve a desired therapeutic medical effect, and the term API is (not only herein) also used for regulatory purposes to indicate that a product is ready to be used in the various clinical phases in medicinal product development. Some medication products may contain more than one active pharmaceutical ingredient.

### Background

The traditional word for API is pharmacon or pharmakon. The invention relates to RNA-based pharmacons, and particularly to self-replicating pharmacons, that comprise a therapeutic oligonucleotide, in ribonucleic acid (RNA) such as messenger RNA (mRNA) designed to encode and (transiently) express a biologically or pharmacologically active nucleic acid, protein, or peptide product in a subject or host. Typically, vaccine RNA-pharmacons are used in an RNA-vaccine. Target genes-of-interest (GOI) for inclusion in a vaccine RNA-pharmacon are preferably selected from nucleic acids encoding a biologically or pharmacologically active nucleic acid, protein, or peptide. The desired or established GOI-target sequence will typically be selected, optimized, synthesized, and inserted into a DNA plasmid to use as a template for RNA-pharmacon synthesis, generally using *in vitro* transcription (IVT) that will be used to produce the RNA-pharmacon. Subsequently, the RNA-pharmacon, or parts thereof, when provided to a human or animal subject, host, or patient, replicates, preferably self-replicates, in cells of said subject to express the desired pharmacologically active or therapeutic nucleic acid, protein, or peptide product with its known therapeutic function. Preferably such a subject is a vertebrate such as a fish, a reptile, a bird, or a mammal. In another mode, such a subject may be a non-vertebrate such as a shrimp or a prawn.

The invention therewith relates to the field of self-amplifying (self-replicating) RNA (RepRNA) pharmacons (herein also identified as replicon systems) for the introduction of foreign genetic information, said genetic information preferably encoding one or more polypeptides for prophylactic and therapeutic purposes, in a cell of a subject, such as a vertebrate, such as a fish, a reptile, a bird, or a mammal, a shrimp or a prawn. RNA-based vaccines and therapeutics are generally composed of two key elements: the RNA active substance encoding a protein of interest and a delivery vehicle such as a lipid nanoparticle (LNP) structure. Typically, the protein can be a viral antigen, such as the SARS-CoV-2 spike protein, a cancer marker, a missing protein, or mutated protein. Regardless of the route of administration, the RNA enters the cytosol, likely through a receptor-mediated mechanism or pinocytosis Then, the mRNA active substance uses the host cell translation machinery. saRNA encodes its own replication machinery; in a first step the host cell derived ribosome translate the viral encoded RNA dependent RNA polymerase, which then amplifies the viral genome. This is one of the major differences between these two classes of RNA-based products. Currently, approved vaccines and most of the clinically advanced candidates are based on nonreplicating mRNA systems. However, RepRNA pharmacon could be advantageous because lower doses of RNA are potentially sufficient for enhanced and prolonged protein expression, thereby also reducing production costs and the occurrence of some adverse reactions. The RepRNA nucleotide sequence is also longer (e.g., over 10 kb for an alphavirus replicon compared with 4.5 kb for mRNA COVID-19 vaccines), implying potential manufacturing differences. The invention relates to the field of veterinary or human medicine. RepRNA may typically be engineered from genes encoding the replication machinery of naturally occurring RNA viruses or synthetic variations thereof. Importantly, it represents only part of the genome important for virus replication, not the virus itself. Of note, the presence of putative ORFs on DNA/RNA vaccine candidates implies that said genes may contribute to the translation of undesired peptides, potentially leading to unintended clinical outcomes, and that the protein-coding potential of DNA/RNA vaccines should be rigorously examined prior to administration (Beaudoin et al. Are There Hidden Genes in DNA/RNA Vaccines? Front Immunol. 2022 Feb 8;13:801915). Further API-requirements towards therapeutic oligonucleotides are in development (Oude Blenke et al., The Storage and In-Use Stability of mRNA Vaccines and Therapeutics: Not A Cold Case. J Pharm Sci. 2023 Feb;112(2):386-403.) A set RNA-based products have emerged as one of the most promising and strategic technologies for global vaccination, infectious disease control, and future therapy development The assessment of critical quality attributes (CQAs), product-process interactions, relevant process analytical technologies, and process modelling capabilities can feed into a robust Quality by Design (QbD) framework for future development, design, and control of manufacturing processes.

In the last two decades, there has been growing interest in RNA-based technology, for example for the development of prophylactic vaccines against infectious diseases. Technological advancements in RNA (ribonucleic acid) biology, chemistry, stability, and delivery systems have accelerated the development of fully synthetic messenger RNA (mRNA) vaccines. Potent, long-lasting, and safe immune responses observed in animal models, as well as encouraging data from human clinical trials and current actual use, make mRNA-based vaccination an attractive alternative to conventional vaccine approaches. Thanks to these data, together with the potential for generic, low-cost manufacturing processes and the completely synthetic nature, the prospects for mRNA vaccines are very promising. In addition, mRNA vaccines have the potential to streamline vaccine discovery and development and facilitate a rapid response to emerging infectious diseases. Maruggi et al., (Mol. Therapy 2019; 27:1-16) overview the unique attributes of mRNA vaccine approaches, review the data of mRNA vaccines against infectious diseases, discuss the current challenges, and highlight perspectives about the future of this promising technology.

The concept for the development of an mRNA vaccine is rather straightforward. Once the antigen of choice from the pathogen target is identified, the gene is sequenced, synthesized, and cloned into the DNA (desoxyribonucleic acid) template plasmid. mRNA (sometimes also identified as non-replicating (nr)RNA) is transcribed in vitro, mRNA is essentially purified, formulated and the mRNA vaccine is delivered into the subject. The mRNA vaccine utilizes the host cell machinery for in vivo translation of mRNA into the corresponding antigen to elicit potent humoral and cellular immune responses. The final location of the antigen is determined, for example, by a signal peptide and transmembrane domain. This can be intrinsic to the natural protein sequence or engineered to direct the protein to the desired cellular compartment. Due to the ability of the host's innate system to sense and respond to (detect, transcribe, translate) RNA sequences of viral origin, mRNA vaccines induce robust innate responses, including production of chemokines and cytokines such as interleukin-12 (IL-12) and tumour necrosis factor (TNF) at the injection site. These are factors for successful induction of effective adaptive responses against the encoded antigen(s).

Currently, two forms of mRNA vaccines have been developed: conventional mRNA-molecules typically encoding the antigen of interest flanked by 5' and 3' untranslated regions (UTRs), ending in a poly-adenyl/poly(A) tail, and (self)-amplifying mRNA-molecules derived from the genome of positive- or negative stranded RNA viruses.

A conventional mRNA vaccine carries at least the coding sequence of the antigen of interest flanked by regulatory regions. The major advantages of the conventional mRNA vaccine approach are the simplicity and relatively small size of the RNA molecule. In the simplest form, the stability and activity of the conventional mRNA in vivo is limited, because of propensity of cells to limit duration of expression. However, optimization of RNA structural elements, nucleoside use, and formulation can reduce RNA degradation and in turn increase antigen expression and durability. The RNA cap (cap 0, 1, or 2 structure), UTRs, and the poly(A) tail are deemed to provide for additional stability of the mRNA molecule, accessibility to ribosomes, and interaction with the translation machinery, therefore representing targets for optimization (reviewed in Ross (Microbiol. Rev. 1995;59:423-450), Lundstrom (Future Sci. OA. 2018;4:FSO300), and Sahin et al (Nat. Rev. DrugDiscov. 2014;13:759-780). Typically, the 3' untranslated region (3' UTR) of positive-sense single-stranded RNA [ssRNA(+)] viruses is highly structured. Multiple elements in the region interact with other nucleotides and proteins of viral and cellular origin to regulate various aspects of the virus life cycle such as replication, translation, and the host-cell response (Liu et al, Structures and Functions of the 3' Untranslated Regions of Positive-Sense Single-Stranded RNA Viruses Infecting Humans and Animals. Front Cell Infect Microbiol. 2020 Aug 27; 10:453). For example, in defining the minimum essential cis-acting elements required for nodavirus RNA replication, previous work predicted the presence of a conserved stem-loop structure in the 3' untranslated region (UTR) of NoV RNA2, verified its existence biochemically, and showed that it that acts as a cis-acting replication element in chimeric NoV RNA2-based replicons in transformed yeast cells (Rosskopf et al., Virus Res. 2010;150:12-21). A minimal replicon containing the 5'-terminal 17 nt and the 3'-terminal 54 nt of RNA2 (including a predicted 3'stem-loop structure (3'SL) found in the 47 nt long 3'UTR) retained the ability to replicate in yeast, suggesting to Rosskopf that this 3'- region can direct replication of a heterologous mRNA. These data foremost suggest that the 3'SL plays an essential role in replication of NoV RNA2. The conservation of the predicted 3'SL suggests that this common motif may play a role in RNA replication for the other members of the *Nodaviridae.* 5'- replication elements were not identified by Rosskopf et al or others.

Optimized codon usage can have a beneficial impact on protein translation and replacing rare codons with frequently used synonymous codons is a common practice to enhance protein expression from DNA, RNA, and viral vector vaccines. However, this approach has been recently reviewed (Mauro and Chappell, Trends Mol Med. 2014 Nov;20(11):604-13. doi: 10.1016/j.molmed.2014.09.003. Epub 2014 Sep 25), suggesting that codon optimization may not necessarily increase protein production for mRNA therapeutics. Thus, mRNA-based vaccines are safe and produced under cell-free conditions. They offer potential for rapid vaccine design. Effectively, two vaccine-pharmacons are currently approved for prophylactic use to prevent COVID-19: mRNA-1273 (Moderna), BNT-162 (BioNTech), and other RNA-vaccine-pharmacons are in development.

A recent study showed that for equivalent levels of protection, a self-amplifying (self-replicating) replicon RNA (RepRNA) required a 64-fold lower dose and resulted in prolonged and increased transgene expression (Vogel et al., Mol Ther. 2018 Feb 7; 26(2):446-455). RepRNA (herein and elsewhere also identified as replicating RNA) are more complex and larger pharmacon molecules than mRNA, encoding an RNA replication machinery as well as the desired therapeutic gene-of-interest such as a gene encoding a vaccine antigen, and thus resulting in expanded translation of encoded antigens. Generally being derived from defective virus genomes, progeny virus or viral-like particle production in the vaccinated subject is excluded (WO 2009146867, Ljungberg and Liljeström, Expert Rev Vaccines. 2015 Feb; 14(2): 177-94). The replicative nature of RepRNA discloses several rounds of RNA amplification providing more template RNA for protein translation and thus, enhancing the antigen dosage available for activating humoral and cell-mediated immunity (CMI), as well as the duration and therefore robustness of that response.

Self-amplifying mRNA (RepRNA) vaccines are commonly based on the engineered RNA genome of positive-sense single-stranded RNA viruses, such as alphaviruses, flaviviruses, bunyaviruses, and picornaviruses. Negative-sense single-stranded RNA viruses, such as rhabdoviruses and measles viruses, can also be utilized for the development of RNA-based vaccines. A typical self-amplifying mRNA-molecule encodes not only the antigen- or gene-of-interest but also the viral replication machinery required for intracellular RNA amplification leading to high levels of expression. Alphavirus vectors are being developed as gene-based vaccines for infectious and malignant diseases. Alphavirus vectors are known as replicons due to the self-amplification of the vector RNA, which occurs in the cytoplasm of the infected cell. Such replicons typically contain an RNA-molecule with the non-structural viral protein genes encoding a viral replicase, with the 5'- and 3'-end *cis*-active (self) replication sequences, and with the native subgenomic promoter, which directs expression of the heterologous gene(s) encoded by said RNA-molecule. Such alpha virus replicons typically lack genes or essential fragments of genes encoding virion structural proteins (such as viral capsid and possible viral envelope proteins) necessary for packaging and cell-to-cell spread of infectious particles. Various RepRNAs (replicons) may be packaged into virus-like particles by providing the structural proteins in *trans,* (across RNA segments) using transient RNA cotransfection systems or stable replicon packaging cell lines. Alternatively, the DNA information that is encoding the RNA replication machinery can be introduced into cells as plasmid or copy DNA to generate virus-like particles. Alphavirus replicon particle vectors have been developed using Sindbis virus (SIN), Venezuelan equine encephalitis virus (VEE), and Semliki Forest virus, and chimeras thereof (Perri et al., J Virol. 2003 Oct;77(19):10394-403.). A recent replicating RNA encoding NSP1-4 of VEE virus and the SARS-CoV-2 spike protein, encapsulated within a lipid nanoparticle (LNP) as a vaccine following such design was recently produced as well (McKay et al., Nat Commun. 2020 Jul 9;11(1):3523. doi: 10.1038/s41467-020-17409-9).

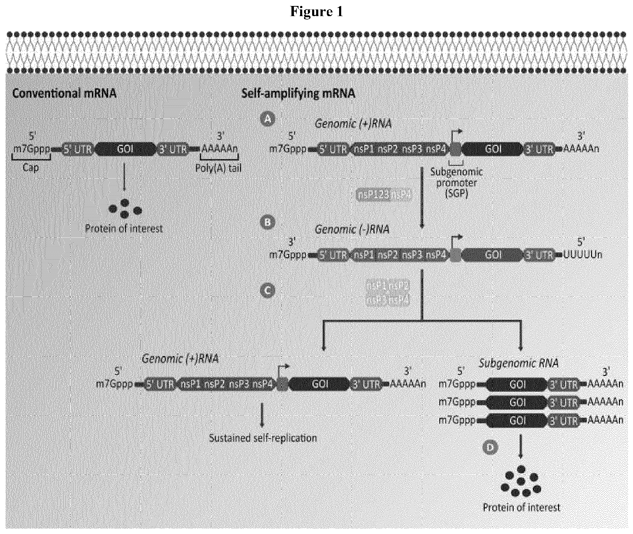

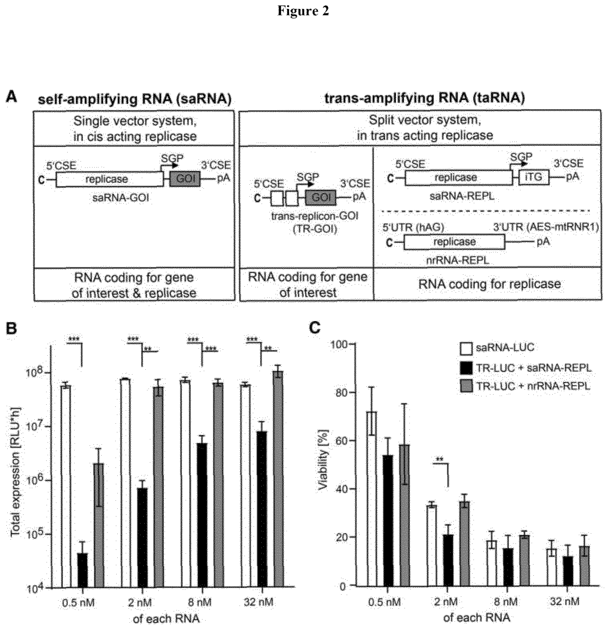

The typical replicating RNA-molecule carries the coding sequence of the replicase-machinery as well as the gene-of-interest (GOI) flanked by 5' and 3' UTRs, a terminal 5' cap structure, and a 3' polyadenylation (poly-A) tail (see also figure 1). For example, in the case of alpha-viral-like replicon vaccine systems (Blakney and Geall, Vaccines (Basel). 2021 Feb; 9(2): 97.), replicating RNA molecules are considerably larger (≈9-12 kb) than corresponding non-amplifying mRNAs solely carrying the desired vaccine antigen-of-interest. They contain the basic elements of mRNA (a cap structure at the 5' end, a 5' UTR, a 3' UTR, and a poly(A) tail of variable length) but have a large open reading frame (ORF) at the 5' end that encodes four non-structural proteins (nsP1-4) and a subgenomic promoter. Genes in the viral genome that are normally downstream of the subgenomic promoter and encode the viral structural proteins are replaced by gene(s) encoding the vaccine antigen(s). Deletion of the viral structural proteins renders the mRNA incapable of producing an infectious virus. After delivery into the cytosol of a cell by a process of transfection, the released mRNA is translationally competent, and engages with the host cell ribosome to produce the four functional components of RNA-dependent RNA polymerase (RdRp) or viral genome replication apparatus: nsP1, nsP2, nsP3 and nsP4, as required for intracellular RNA amplification and high levels of antigen expression. RNA-dependent RNA polymerase (RdRp) or RNA replicase is an enzyme that catalyses the replication of RNA from an RNA template. Specifically, it catalyses synthesis of the RNA strand complementary to a given RNA template. This contrasts with typical DNA-dependent RNA polymerases (often referred to as RNA polymerase), which all organisms use to catalyse the transcription of RNA from a DNA template and are useful in *in vitro* transcription of RNA from DNA. T7 RNA Polymerase is an example of such an RNA polymerase from the T7 bacteriophage that catalyses the formation of RNA from DNA in the 5'→ 3' direction. Only T7 DNA or DNA cloned downstream of an T7 promoter can be used. SP6 RNA Polymerase is another example of DNA-dependent RNA polymerase used for in vitro transcription. Only SP6 DNA or DNA cloned downstream of an SP6 promoter can be used. The RNA can direct its self-amplification to generate RNA intermediates and many copies of antigen-encoding subgenomic mRNA, producing high levels of the encoded antigen. Beissert et al (Mol Ther. 2020 Jan 8;28(1):119-128, see also WO2017162461 and figure 2) discuss a split replicon RNA vaccine approach based on a bipartite vector system using trans-amplifying RNA (taRNA). The vector cassette encoding the vaccine antigen originates from an alphaviral replicating RNA, from which the replicase was deleted to form a transreplicon. Replicase activity is provided in trans by a second molecule, either by a standard replicating RNA or an optimized non-replicating mRNA (nrRNA). Typically, the non-replicating RNA delivered 10- to 100-fold higher transreplicon (TR) expression than the standard RNA. Moreover, expression driven by the nrRNA-encoded replicase in this split replicon RNA system was nearly as efficient as in a conventional monopartite RNA system whereas the bipartite RNA system performed considerably less well. Beissert et al (ibid) show that the superiority of nrRNA- over replicating RNA-encoded replicase to drive expression of the transreplicon is most likely attributable to its higher translational efficiency and lack of interference with cellular translation. Beissert's data suggest that the advantage of using nrRNA for expressing replicase is governed at the level of translation within the first few hours after RNA transfer, and further studies are reportedly underway to explore the exact mechanism. Furthermore, it is conceivable that in said system replicase molecules (REPL) translated from nrRNA-REPL can more easily dissociate and relocalize to the TR, whereas replicase translated from replicating RNA-REPL is prone to bind to sequence elements in the vicinity, and thus is expected to preferentially promote *cis* replication as observed during genome replication in a number of RNA viruses (Liang et al., Virology. 2001;282:307-319; Weiland and Dreher, Proc. Natl. Acad. Sci. USA. 1993;90:6095-6099). Beissert's observation that translation of an unrelated nrRNA was strongly inhibited in cells transfected with replicating RNA-REPL, but not with nrRNA-REPL, indicates a broader interference from replicating RNA-REPL with the cellular translation machinery. Again, further investigations are deemed needed for a deeper understanding of how nrRNA-REPL outperforms replicatingRNA-REPL as a complementing element of a split-vector system.

Both conventional mRNA and RepRNA vaccines require a delivery system for cell uptake and to unload the mRNA cargo into the cytosol, where translation occurs. Once delivered in the cell, the RNA is almost immediately sensed by pattern recognition receptors (PRRs) in the endosome and in the cytoplasm. PRRs such as Toll-like receptors TLR3, TLR7, and TLR8 are localized in the endosome, and cytosolic sensors such as RIG-I, MDA5, PKR, and OAS also recognize double-stranded and single-stranded RNAs in the cytoplasm. Both conventional mRNA and replicating RNA vaccine approaches typically share essential elements of eukaryotic mRNA: a cap structure (cap 0, 1, or 2), a 5' UTR, an open reading frame (ORF), a 3' UTR, and a tail of 40-120+ adenosine residues [poly(A) tail]. Both types of RNAs are typically produced in a cell-free system of in vitro transcription, if required using modified nucleosides, by using an enzymatic transcription reaction from a linearized DNA template requiring a DNA-dependent RNA polymerase. Manufacturing of RNA vaccines against different disease targets requires the replacement of the sequence encoding the target antigen encoded in the ORF, possibly affecting the overall physicochemical and replicative characteristics of the RNA molecule. Several RNA replicon vaccines of this kind have been reported, also see the above review by Maruggi et al., and references contained therein. In particular, the longer the RNA length of the RNA-molecule comprising the replicase and/or GOI is required, the more challenging the production of high-quality RNA-molecules and subsequent RNA translated protein products is, as compared with conventional mRNA-molecules. Most replicating RNA must harbour not only the RNA-sequence of the desired fragment of the gene of interest (GOI) but must harbour its replicase as well. Often, the length of the required polyadenylation additions to such RNA molecules hampers production even further.

As reviewed by Yang et al., (Viruses. 2021 Jan 7; 13(1):73.), nodaviruses are small bisegmented RNA viruses belonging to the family *Nodaviridae.* Nodaviruses have been identified in different hosts, including insects, fishes, shrimps, prawns, pigs, dogs, and bats. A novel porcine nodavirus was first identified in the United States. RNA1 of the porcine nodavirus had the highest nucleotide identity (51.1%) to the Flock House virus, whereas its RNA2 gene shared the highest nucleotide identity (48%) with the RNA2 gene segment of caninovirus (Canine nodavirus). Genetic characterization classified porcine nodavirus as a new species under the genus *Alphanodavirus.* Within the *Nodaviridae,* traditionally the genus *Betanodavirus* is recognized as well, some also discern the genus *Gammanodavirus* as related to a bi-segmented virus of interest.

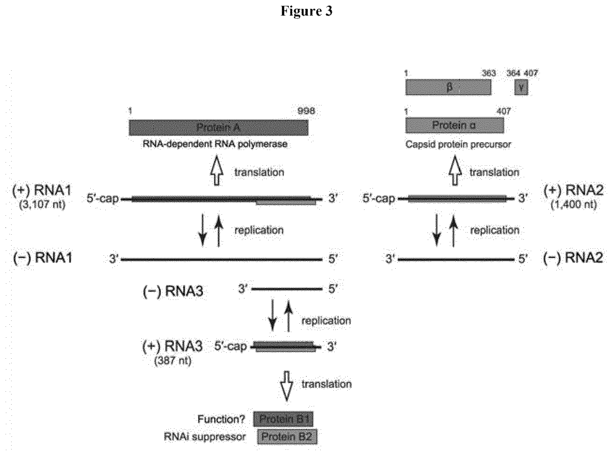

Biddlecome et al., (PLoS One. 2019; 14(6): e0215031), review the various components and stages of the related Nodamura virus (NoV) life cycle, depicting schematically the replication and translation strategies of this two-RNA-molecule/four-gene positive-sense RNA virus, see also figure 3. In *Nodaviridae* and typical other bi-segmented viruses, molecule RNA1 is translated directly by host cell ribosomes to yield "Protein A", the RNA-dependent-RNA-polymerase (RdRp or replicase) that binds the ribonucleic acid derived from the genes RNA1 and RNA2 and replicates them strongly through successive rounds of -strand and +strand synthesis. As part of the replication of the RNA1 gene, its (-) strands are "transcribed" to give not only (+) strands of the full-length RNA1, but also mRNA (subgenomic [sg] RNA3) that is translated to either a B1 protein in the same reading frame as the RdRp, or a B2 protein in an alternate reading frame. The B2 protein is known to suppress host-cell RNA interference, allowing increased accumulation of viral RNA in the host cell. The function of B1 has not yet been determined in NoV but in other members of the *Nodaviridae* B1 seems an early expression protein that has an anti-necrotic cell death function which reduces mitochondrial membrane potential loss and enhances viral host cell viability. The RNA2 gene is translated to capsid protein alpha, which assembles into a capsid in which the protein matures by undergoing autocatalytic cleavage to yield capsid protein beta and the membrane permeabilizing peptide gamma.

In an attempt to employ the Nodavirus replicase, Biddlecome et al., (in PLoS One. 2019; 14(6): e0215031 and in thesis Adam Min Biddlecome, In Vitro-Reconstituted Virus-like-particle Delivery of Self-Amplifying RNA Genes, University of California Los Angeles, 2019, see also figure 3) use a cell-free system of in vitro transcription (IVT) by using an enzymatic transcription reaction from a linearized *Nodaviridae-*derived DNA template requiring the DNA-dependent RNA1 polymerase used to *in vitro* assemble monodisperse virus-like particles (VLPs) containing reporter proteins (e.g., Luciferase or eYFP) or the tandem-repeat model antigen SIINFEKL in RNA gene form, coupled to the RNA-dependent RNA polymerase from the RNA1 of *Nodamura* insect virus. Biddlecome et al. in particular use the RNA1 gene of NoV, and typically dispenses use of its RNA2 gene or nodavirus RNA2 gene derivatives. Also, Biddlecom et al, add a reporter-gene of to the subgenomic cis-active RNA3 within said RNA1 gene, therewith disabling at least some of the functionalities of the subgenomic proteins (B1 and/or B2) derived from said RNA3. According to Biddlecome et al., (ibid) replication of a gene of interest can be coupled to the replication of the Noda viral RdRp by inserting it in the RNA1 gene in this way, but its length of GOI, is restricted to less than 1000 nt required for in vitro packaging of the ensuing replicon into a wildtype Cowpea Chlorotic Mottle Virus capsid (CCMV CP T=3), length restrictions further necessitating depriving the replicon system from some B-protein activities. Also, further to the disadvantage that its GOIs can only accommodate a length of no longer than 1000 nt, the system of Biddlecome et al., does not at all provide an actual pharmacon (i.e. a drug, medicinal, pharmaceutical, or other therapeutic preparation such as a therapeutic or preventive vaccine directed against a disease) but demonstrate its use with reporter-gene expression of renilla luciferase or eYFP for quantification of the resulting level of protein expression from the replicon or with (tandem repeats) of the SIINFEKL epitope of ovalbumin for quantification of DC maturation (marker or reporter protein expression) and antigen presentation. Such replicon systems that comprise reporter-gene(s) of interest have no actual or earlier demonstrated therapeutic use, do not result in an actual pharmacon, and the end-product, bearing (fluorescent) reporter gene functions, does not allow gaining API status for regulatory purposes in medicinal product development, even if such a system is additionally provided with a HIV gp120 gene or other. Of note, the presence of putative open-reading-frames (ORFs) encoding (parts of) reporter proteins or peptides in DNA/RNA vaccine candidates implies that said reporter ORFs may contribute to the translation of reporter (-like) proteins or peptides), potentially leading to unintended clinical outcomes.

Overall, the protein-coding potential of DNA/RNA vaccines should be rigorously examined prior to administration and the presence of a reporter gene is considered not acceptable for producing an oligonucleotide-pharmacon or API with its distinct therapeutic aim. In chapter 4 of his thesis, and following Rosskopf et al (Virus Res. 2010;150:12-21) in providing an Noda-like RNA2 sequence containing the first 17 nt 5'-terminal untranslated region UTR and the 3'-terminal 236 nt of RNA2 flanking a reporter gene and a HIV gp120 gene GOI construct Biddlecombe fails to show specific replication and/or expression of HIV envelope protein gp120 gene (ENV or Env); and clearly demonstrates less robust expression (as measured by fluorescence of eYFP) of a GOI-construct comprising a reporter gene eYFP and the gp120 gene as compared to a GOI-construct comprising a reporter gene eYFP only. In conclusion, Biddlecombe, in his chapter 4 of his theses, identifies replication and/or translation difficulties, addresses reporter gene activities only and does not provide a self-replicating RNA-pharmacon.

US 20030108863 describes the production of a nodavirus-based DNA vector that drives abundant expression of foreign genes in a wide variety of cell types. A host cell is transfected with said DNA vector (plasmid) and this DNA is initially transcribed in said host cell by a host-cell specific RNA polymerase to produce primary transcripts from which a nodaviral RNA-dependent RNA polymerase (RNA replicase) is translated. These primary transcripts are then amplified by the RNA replicase in an autonomous, cytoplasmic RNA replication. Such a vector is considered a useful addition to the current arsenal of expression vectors, and well suited to laboratory-scale and larger-scale expression of transcripts and/or proteins in eukaryotic cells. However, US 20030108863 does not relate to a cell-free system of in vitro transcription by using an enzymatic transcription reaction from a linearized *Nodaviridae-derived* DNA template requiring a DNA-dependent RNA polymerase and neither relates to essentially purified RNA(s), obtainable by such a cell free system that would be useful in an essentially pure (self-replicating) RNA vaccine but instead describes the production of a nodavirus-based DNA vector that, after introduction in cells, drives expression of foreign genes in said cells and teaches that small spherical virus capsid particles, as typically found with Noda virus, have significant potential as systems for the specific encapsidation and delivery of biological molecules, and thus addresses biological molecule delivery only and does not provide a self-replicating RNA-pharmacon.

Similarly, US 20060177819 relates to improved constructs for and methods of making recombinant alphavirus particles that are useful in immunotherapies for infectious diseases and cancer and in the delivery of genes for therapeutic purposes. However, US 20060177819 does not relate to a cell-free system of in vitro transcription by using an enzymatic transcription reaction from a linearized *Nodaviridae-derived* DNA template requiring a DNA-dependent RNA polymerase and neither to essentially purified RNA(s), obtainable by such a cell-free system that would be useful in an essentially pure RNA vaccine but instead describes a nodavirus-based DNA vector that, after introduction in cells, drives expression of alphavirus capsid genes in cells and teaches that such a nodavirus-based DNA vector can further reduce the predicted frequency for formation of replication-competent alphavirus and may optimize alphavirus particle manufacturing, again addressing biological molecule delivery only and not providing a self-replicating RNA-pharmacon.

Similarly, US6514757B1 describes the production of a nodavirus-based DNA expression vector that drives abundant expression of foreign genes in a wide variety of cell types. The DNA plasmid is initially transcribed by a host-cell RNA polymerase to produce primary transcripts from which a nodaviral RNA-dependent RNA polymerase (RNA replicase) is translated. These primary transcripts are then amplified by the RNA replicase in an autonomous, cytoplasmic RNA replication. This DNA expression vector system described depends upon the expression of two genes: the gene for a nodavirus RNA replicase (i.e., RNA-dependent RNA polymerase) and a heterologous gene that encodes a desired transcript and/or protein. For an optimal expression system, it was therefore thought necessary to design a convenient and reliable way to deliver the two genes to the same cell in a coordinated manner. In a series of experiments in US6,514,757B1 and designed to examine how far the nodaviral RNA structures could be altered without losing their ability to replicate, it was found that both RNA1 and RNA2 replicate efficiently from primary transcripts that contained tandem copies of either RNA1 or RNA2. During replication, the homodimeric transcripts were resolved into their monomeric components, with the 5' and 3' copies replicating with approximately equal efficiency. Furthermore, it was found that a heterologous gene insertion downstream from the first 3 nucleotides derived from the 5"-UTR of FHV RNA2 and upstream from the 60 nucleotides comprising the 3'-UTR allowed replication of said heterologous gene. Therefore, in US6,514,757B1 it is suggested feasible to deliver both RNA replicons from a single heterodimeric primary transcript produced from a single promoter attached to a single RNA (fusion construct) in which the 5' RNA2-derived 3-nucleotide sequence fused (covalently linked to) the 3'-end of the RNA1, followed by the heterologous transcript and the last 60 3'-nucleotides of FHV RNA2. This approach would have the advantage of a single DNA plasmid system (containing about 6 kb plus the size of the heterologous gene) which delivers equimolar amounts of the replicase gene and the heterologous gene to transfected cells. However, US6514757B1 is addressing DNA delivery only and not providing a self-replicating RNA-pharmacon.

Wild-type RNA viruses often form complex distributions of closely related but nonidentical variant genomes that are subjected to a continuous process of genetic variation, competition, and selection (Martinez et al., Quasispecies Dynamics of RNA Viruses. Viruses: Essential Agents of Life. 2012;21-42). These so-called viral quasispecies have been described in vivo through the analysis of molecular and biological clones isolated from viral populations, and more recently using ultradeep sequencing techniques. The viral quasispecies was first documented with bacteriophage Qβ, during replication in its *Escherichia coli* host; it was later confirmed for many RNA viruses.

It is recognized that the control and regulation of DNA-to-RNA transcription and RNA-to-RNA replication is an important development component for RNA-pharmacons to establish the desired therapeutic effect. Within the field of RNA-pharmacons, there exists a need to develop RNA-pharmacons with expanded transcriptional and/or replicational effects in comparison to known RNA-pharmacons such as derived from alphavirus. RNA-pharmacons derived from Nodaviridae have not materialized so far.

### The invention

In a preferred embodiment, the invention discloses a ribonucleic acid (RNA)-pharmacon comprising at least a functional part of a trans-amplifying RNA (taRNA) replication element of an Alphanoda virus. Until now such functional parts were only found in constructs with reporter gene replication and/or expression, however, as yet no actual pharmacons providing essentially therapeutic activities have been disclosed. In a preferred embodiment of the invention, it discloses a pharmacon according to the invention comprising at least a native-length 5'-untranslated region (UTR) replication-element derived from the RNA2 of said virus, wherein said replication element is 3'-linked to a ribonucleic acid encoding a (non-Alphanoda) virus open-reading-frame of a gene-of-interest (GOI). Surprisingly, the inventors identified hitherto unknown taRNA activities of a novel replication element in the most 5'-located flank of native RNA2. It is preferred that said replication element is 3'-linked to the start codon of said GOI, preferably said start codon is ATG.

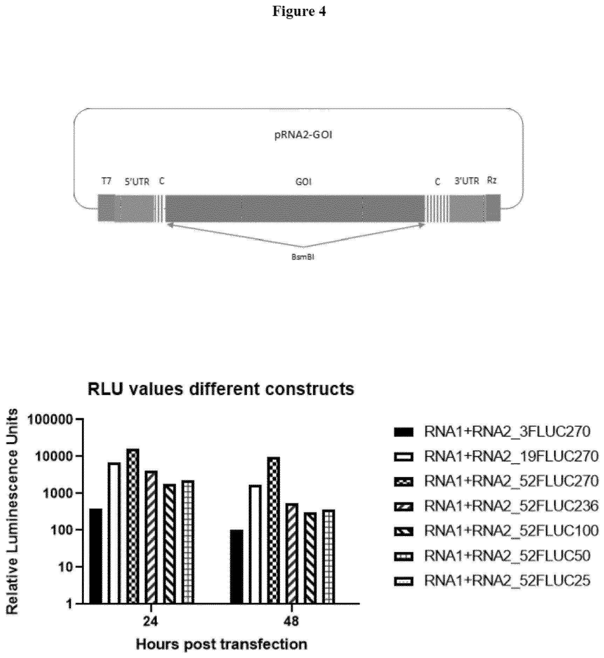

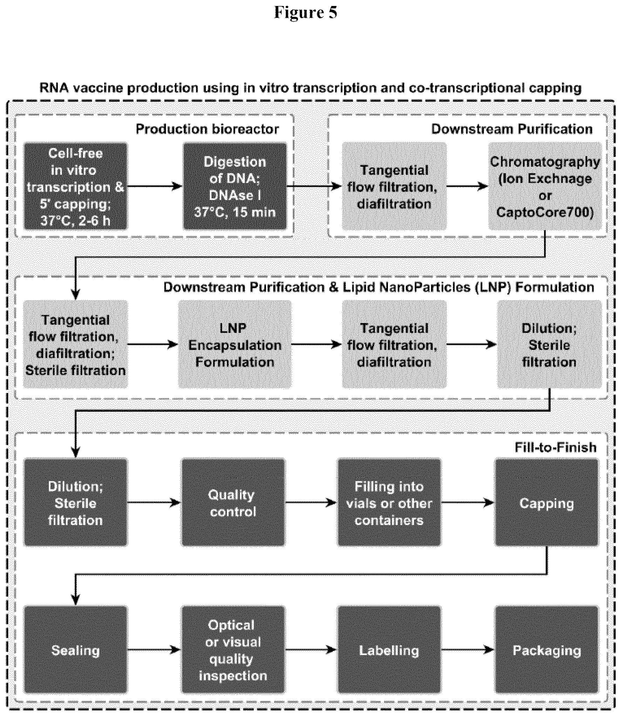

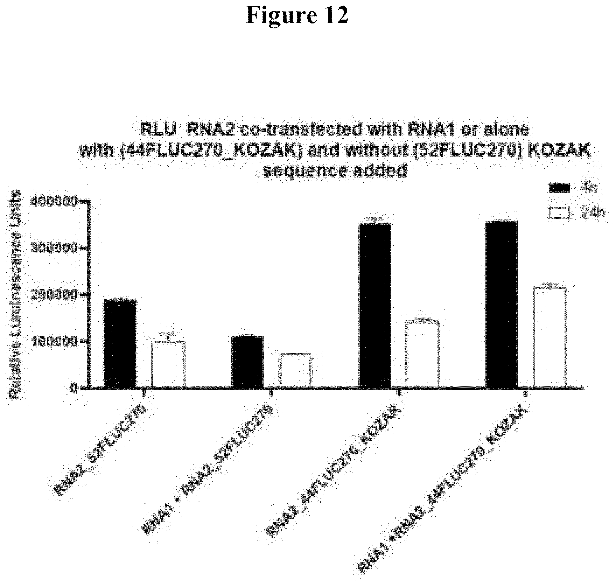

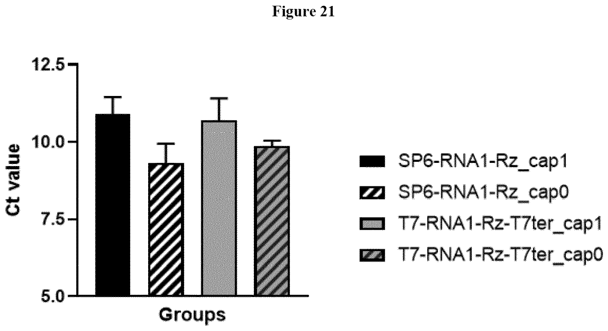

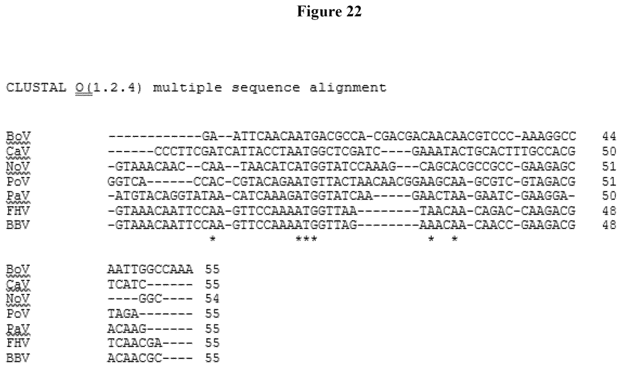

In various reporter gene RNA-replicons and pharmacons, the inventors studied requirements for improved replication and/or translation of heterogolous gene expression of a GOI (provided with 5' a start codon ATG and 3' stop codon) flanked by native 5'-UTR/CSE and 3'-UTR/CSE noda-like RNA2 replication element of varying lengths. Findings are summarized in table 1 and also depicted in figures 4, 6, 12, 19, 21 and 23, see also the respective legends therewith. Inclusion of longer 5'-UTR/CSE elements as well as longer 3'-elements improved translation and replication, whereby 3'-elements longer than ~ 850 nt's have not been assessed as yet. Also, inclusion of (improved) Kozak sequences further improved translation. Pharmacon replication benefits from capO.

| Table 1, overview of nucleotide length RNA2 replication element analyses | | | |
|---|---|---|---|
| 5'-end RNA2 replication element | **Heterologous-GOI-insertion** | | 3'-end RNA2 replication element |
| 5'-UTR/CSE | **Replication and/or translation** | | 3'-UTR/CSE |
| shortened 5'-UTR | | + | >200 and <250 nt's |
| native UTR-nt's only | | ++ | >200 and <250 nt's |
| native UTR-nt's only | | ++ | >250 and < 850 nt's |
| native UTR-nt's + AGG + >=5 | | +++ | >250 and < 850 nt's |
| native UTR-nt's + AGG + >=10 | | +++ | >250 and < 850 nt's |
| native UTR-nt's + AGG + >=20 | | ++++ | >250 and < 850 nt's |
| native UTR-nt's + AGG + >=30 | | +++++ | >250 and < 850 nt's |

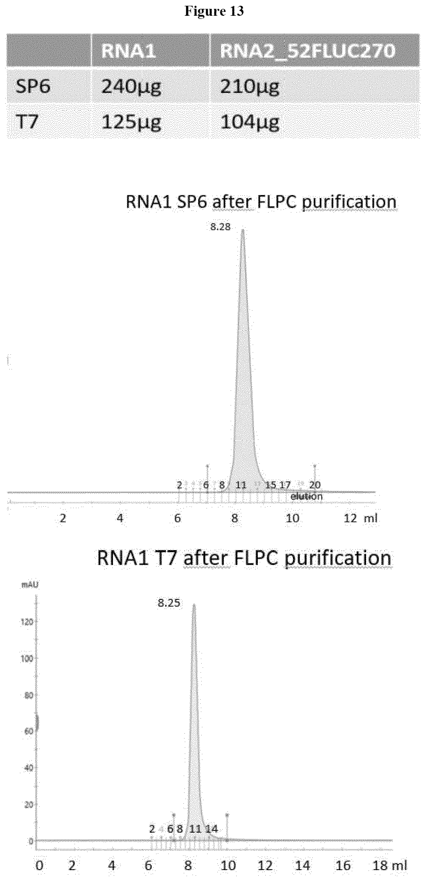

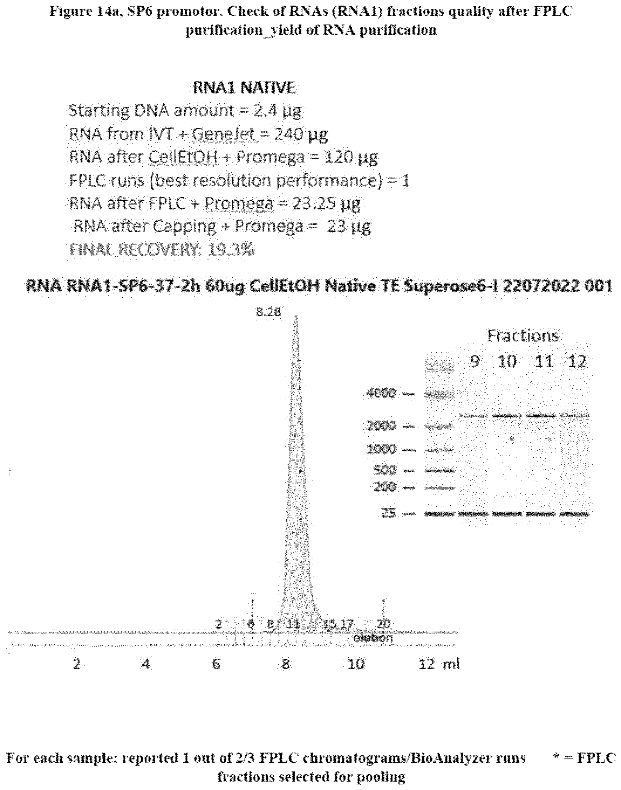

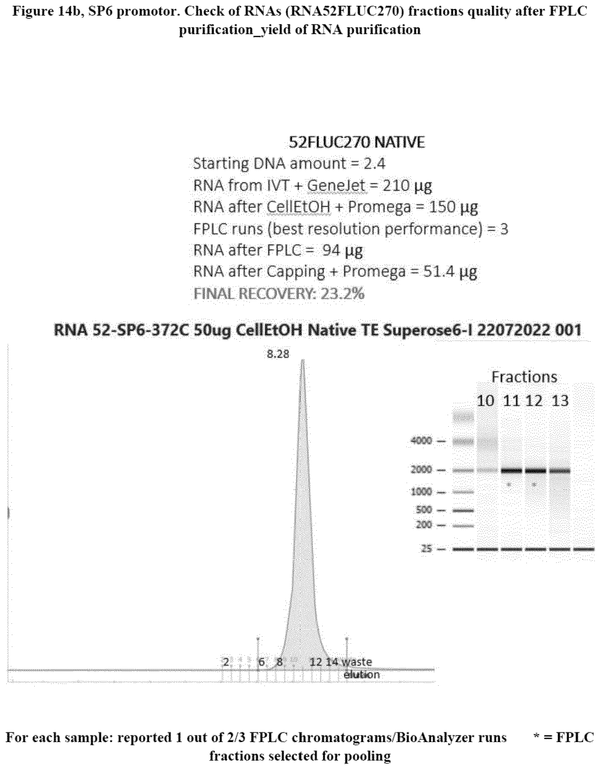

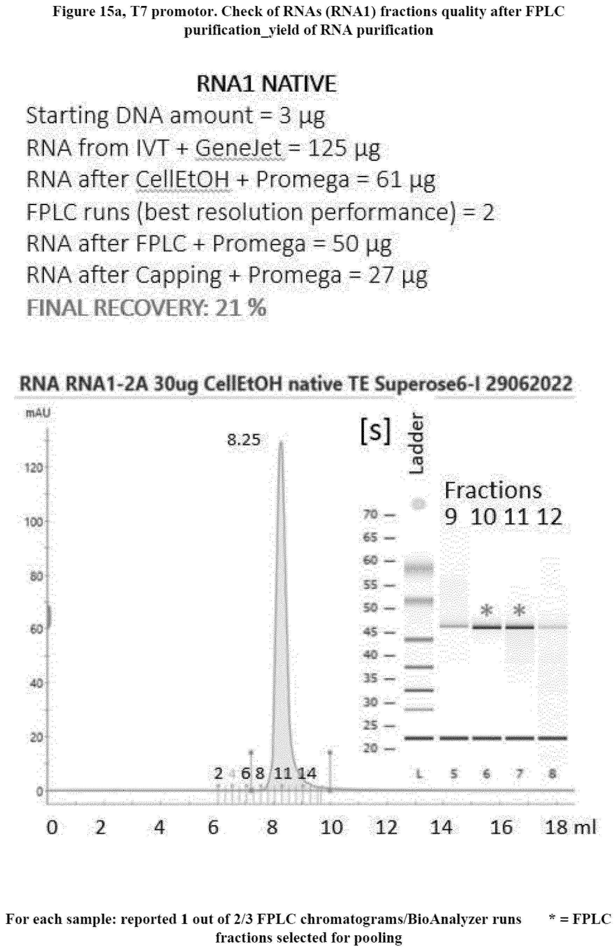

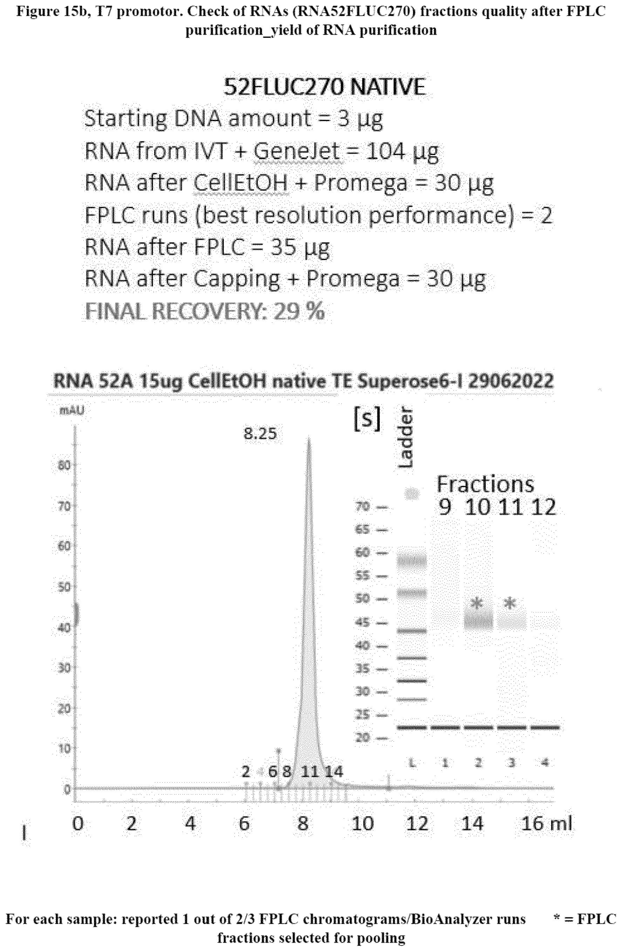

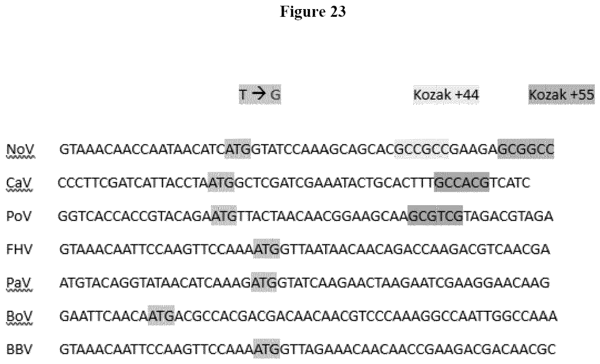

As shown in table 1 and shown in the legend of figure 23 where said 5'-replication element is further discussed, the 5'-UTR is located 5' from the native start codon ATG and the CSE is located 3' from the native start codon. For ease of reference the whole 5'-replication element is indicated as 5' UTR/CSE, whereby the start codon ATG may also be destroyed, and for example be replaced by AGG. Said destruction is preferably achieved by change of T in G, resulting in an extension of the UTR with at least 3 nucleotides AGG. Experiments (see also figure 4 and 13, and the sequences listed in this application) showed that pharmacon replication and/or translation were markedly improved after detailed sequence modifications in the native 5'UTR-sequences. Typically, said improvements comprise use of longer 5'-UTR elements than the 3- or 17-nt long 5'-stretches, as reported earlier for FHV ( Li, Y and Ball L.A. J Virol 67, 3854-3860, and followed in US6514757B1) respectively NoV (Rosskopf JJ et al. Virus Res. 2010;150:12-21), and followed in chapter 4 of the thesis of Biddlecombe) in heterologous gene expression studies. In a further embodiment, this application discloses use of the full-length native 5'-UTR followed by the (native as well as GOI) ATG-start codon to improve heterologous gene expression. To arrive at further improved 5'-UTR/CSE elements, the original nodavirus encoded start codon (ATG) is preferably destroyed (here a change of the pivotal T in G is disclosed). It is further preferred to lengthen the 5'-UTR nodavirus sequence after the destroyed start codon with at least 5, more preferably at least 10, more preferably at least 20, more preferably at least 30 nodavirus derived nucleotides to improve replicase activity (see also figure 4). Thereafter, the pharmacon construct preferably commences with a newly introduced start codon (preferably ATG, derived from the ORF of the GOI). Also, Kozak sequences may be optimized or added to further improve translation (see also figure 13).

The invention also discloses a self-replicating ribonucleic acid (RepRNA)-pharmacon comprising ribonucleic acid encoding a gene-of-interest (GOI) and ribonucleic acid encoding at least a functional part of an RNA-dependent RNA polymerase derived from a quasispecies variant nucleic acid sequence of any of the *Nodaviridae.* It is preferred that said polymerase is derived from any of the Alphanodaviridae. It is moreover preferred, that said polymerase is derived from any of the Alphanodaviridae generally capable of replicating in cells derived from a warm-blooded animal, such as Nodamuravirus, Canine nodavirus or Porcine nodavirus. It is most preferred that said polymerase is derived from any of a quasispecies of Nodamuravirus.

Therewith, the invention discloses use of a pharmacon according to the invention for use in a vaccine, herein also identified as an RNA-vaccine pharmacon. Examples of such vaccine RNA-pharmacons comprising or provided with ribonucleic acid encoding a vaccine antigen are listed as sequence here in the application under "Infectious agent vaccine RNA-pharmacons" and under "Cancer vaccine RNA-pharmacons" as described below.

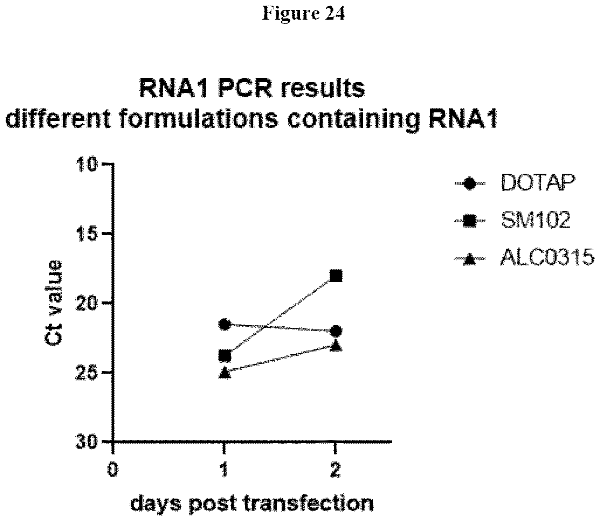

It was surprisingly discovered by the inventors, and as further shown in figures 23 and 24 and the sequences listed herein, that replicational and transcriptional effects and qualities were improved when the full-length or even better lengthened native 5'-UTR was utilized (for example when the first native start codon ATG was destroyed (here T was replaced by G) for example when a pharmacon according to the invention is equipped with at least the first five (5), preferably with at least the first 10, more preferably with at least the first 20 nucleotides, more preferably with at least the first 30 nucleotides found located downstream (3') from of a first start codon ATG of a 5' UTR/conserved sequence element (CSE) flanking the RNA2 of any of the Alphanodaviridae. preferably of any of the Alphanodaviridae generally capable of replicating in cells derived from a warm-blooded animal. A preferred RNA-vaccine pharmacon according to the invention is comprising a nucleic acid encoding at least a part of a first endogenous conserved sequence element (CSE) 5' flanking said GOI, said 5' CSE comprising the first 19 nucleotides, more preferably the first 44 nucleotides, most preferably the first 52 or 55 nucleotides of a 5' conserved sequence element (CSE) flanking the RNA2 of a quasispecies derived from Nodamuravirus. When an endogenous 5'-UTR-CSE element of any of the Alphanodoviridae is used, it is preferred to destroy the endogenous Alphanodavirus capsid start codon, preferably by changing ATG into AGG. In a preferred embodiment, the invention discloses a pharmacon wherein said replication-element is derived at by destruction of the first native start codon ATG in said RNA2, preferably by replacing T with G, and said altered replication element is 3'-linked to the start codon of said GOI, preferably said start codon is ATG. In a more preferred embodiment, wherein said replication-element is further 3'-extended with at least the first five (5), preferably at least the first 10, more preferably at least the first 20, more preferably at least the first 30 native nucleotides located downstream of a first start codon ATG of a 5' untranslated region and conserved sequence element (5'-UTR/CSE) flanking said RNA2. It is preferred that said replication element is derived from an Alphanoda virus generally capable of replicating in cells derived from a warm-blooded animal, such as from Nodamuravirus. The invention also discloses a pharmacon according to the invention wherein said ribonucleic acid encoding said GOI is 3'-linked to a ribonucleic acid derived from at least the last 50, preferably at least the last 100, more preferably at least the last 150, more preferably at least the last 200 nucleotides, more preferably at least the last 250 nucleotides of a 3'-UTR/CSE flanking said RNA2, preferably when the pharmacon according to the invention is also encoding at least a functional part of a self-amplifying RNA (saRNA) replication element of an Alphanoda virus.

In a preferred embodiment, the invention discloses a pharmacon wherein said replication-element is derived at by destruction of the first native start codon ATG in said RNA2, preferably by replacing T with G, and said altered replication element is 3'-linked to the start codon of said GOI, preferably said start codon is ATG. In a more preferred embodiment, the invention discloses a pharmacon wherein said 5'-replication-element is further 3'-extended with at least the first five (5) native nucleotides located downstream of a first start codon ATG of a 5' untranslated region and conserved sequence element (5'-UTR/CSE) flanking said RNA2 to which said GOI is 5'-linked and said GOI is 3'-linked to a ribonucleic acid derived from at least the last 50 nucleotides of a 3'-UTR/CSE flanking said RNA2, preferably when the pharmacon according to the invention is also encoding at least a functional part of a self-amplifying RNA (saRNA) replication element of an Alphanoda virus.

In a more preferred embodiment, the invention discloses a pharmacon wherein said replication-element is derived at by destruction of the first native start codon ATG in said RNA2, preferably by replacing T with G, and said altered replication element is 3'-linked to the start codon of said GOI, preferably said start codon is ATG. In a more preferred embodiment, the invention discloses a pharmacon wherein said 5'-replication-element is further 3'-extended with at least the first 10 native nucleotides located downstream of a first start codon ATG of a 5' untranslated region and conserved sequence element (5'-UTR/CSE) flanking said RNA2 to which said GOI is 5'-linked and said GOI is 3'-linked to a ribonucleic acid derived from at least the last 100 nucleotides of a 3'-UTR/CSE flanking said RNA2, preferably when the pharmacon according to the invention is also encoding at least a functional part of a self-amplifying RNA (saRNA) replication element of an Alphanoda virus.

In a more preferred embodiment, the invention discloses a pharmacon wherein said replication-element is derived at by destruction of the first native start codon ATG in said RNA2, preferably by replacing T with G, and said altered replication element is 3'-linked to the start codon of said GOI, preferably said start codon is ATG. In a more preferred embodiment, the invention discloses a pharmacon wherein said 5'-replication-element is further 3'-extended with at least the first 20 native nucleotides located downstream of a first start codon ATG of a 5' untranslated region and conserved sequence element (5'-UTR/CSE) flanking said RNA2 to which said GOI is 5'-linked and said GOI is 3'-linked to a ribonucleic acid derived from at least the last 150 nucleotides of a 3'-UTR/CSE flanking said RNA2, preferably when the pharmacon according to the invention is also encoding at least a functional part of a self-amplifying RNA (saRNA) replication element of an Alphanoda virus.

In a more preferred embodiment, the invention discloses a pharmacon wherein said replication-element is derived at by destruction of the first native start codon ATG in said RNA2, preferably by replacing T with G, and said altered replication element is 3'-linked to the start codon of said GOI, preferably said start codon is ATG. In a more preferred embodiment, the invention discloses a pharmacon wherein said 5'-replication-element is further 3'-extended with at least the first 20 native nucleotides located downstream of a first start codon ATG of a 5' untranslated region and conserved sequence element (5'-UTR/CSE) flanking said RNA2 to which said GOI is 5'-linked and said GOI is 3'-linked to a ribonucleic acid derived from at least the last 200 nucleotides of a 3'-UTR/CSE flanking said RNA2, preferably when the pharmacon according to the invention is also encoding at least a functional part of a self-amplifying RNA (saRNA) replication element of an Alphanoda virus.

In a more preferred embodiment, the invention discloses a pharmacon wherein said replication-element is derived at by destruction of the first native start codon ATG in said RNA2, preferably by replacing T with G, and said altered replication element is 3'-linked to the start codon of said GOI, preferably said start codon is ATG. In a more preferred embodiment, the invention discloses a pharmacon wherein said 5'-replication-element is further 3'-extended with at least the first 30 native nucleotides located downstream of a first start codon ATG of a 5' untranslated region and conserved sequence element (5'-UTR/CSE) flanking said RNA2 to which said GOI is 5'-linked and said GOI is 3'-linked to a ribonucleic acid derived from at least the last 200 nucleotides of a 3'-UTR/CSE flanking said RNA2, preferably when the pharmacon according to the invention is also encoding at least a functional part of a self-amplifying RNA (saRNA) replication element of an Alphanoda virus.

In a more preferred embodiment, the invention discloses a pharmacon wherein said replication-element is derived at by destruction of the first native start codon ATG in said RNA2, preferably by replacing T with G, and said altered replication element is 3'-linked to the start codon of said GOI, preferably said start codon is ATG. In a more preferred embodiment, the invention discloses a pharmacon wherein said 5'-replication-element is further 3'-extended with at least the first 20 native nucleotides located downstream of a first start codon ATG of a 5' untranslated region and conserved sequence element (5'-UTR/CSE) flanking said RNA2 to which said GOI is 5'-linked and said GOI is 3'-linked to a ribonucleic acid derived from at least the last 250 nucleotides of a 3'-UTR/CSE flanking said RNA2, preferably when the pharmacon according to the invention is also encoding at least a functional part of a self-amplifying RNA (saRNA) replication element of an Alphanoda virus.

In a preferred embodiment, the invention discloses a pharmacon wherein said replication-element is derived at by destruction of the first native start codon ATG in said RNA2, preferably by replacing T with G, and said altered replication element is 3'-linked to the start codon of said GOI, preferably said start codon is ATG. In a more preferred embodiment, the invention discloses a pharmacon wherein said 5'-replication-element is further 3'-extended with at least the first 30 native nucleotides located downstream of a first start codon ATG of a 5' untranslated region and conserved sequence element (5'-UTR/CSE) flanking said RNA2 to which said GOI is 5'-linked and said GOI is 3'-linked to a ribonucleic acid derived from at least the last 250 nucleotides of a 3'-UTR/CSE flanking said RNA2, preferably when the pharmacon according to the invention is also encoding at least a functional part of a self-amplifying RNA (saRNA) replication element of an Alphanoda virus.

It is preferred that said saRNA replication element is derived from an RNA-dependent RNA polymerase encoded by a quasispecies variant nucleic acid sequence of said virus, such as is obtainable from a ribonucleic acid sequence with accession numbers AF174533 or NC002690. In another embodiment the saRNA replication element is obtainable from ribonucleic acid sequences comprising at least one nucleotide polymorphism selected from the group of nucleotide polymorphisms identified at positions 336, 664, 702, 726, 732, 777, 837, 1218, 1219, 1956, 2589, 3129, 3179, relative to quasispecies variant sequences obtainable from the nucleic acid sequences with accession numbers AF174533 or NC_002690. The invention discloses a pharmacon according to the invention wherein the taRNA element is located on a different ribonucleic acid molecule than the molecule on which the saRNA element is located (a bisegmented RepRNA) as well as a pharmacon according to the invention wherein the taRNA element as well as the saRNA element are located on a single (monocistronic) ribonucleic acid molecule.

The invention also provides a self-replicating ribonucleic acid (RepRNA) encoding a pharmacon according to the invention and a DNA-vector encoding a RepRNA according to the invention. Also, the invention provides a ribonucleic acid according to the invention for use in the preparation of a formulation suitable for transfection, a preferred formulation is selected from any of DOTAP, SM102 and Alc0315. The invention also provides a a transfection formulation comprising ribonucleic acid according to the invention and a transfectant comprising ribonucleic acid according to the invention.

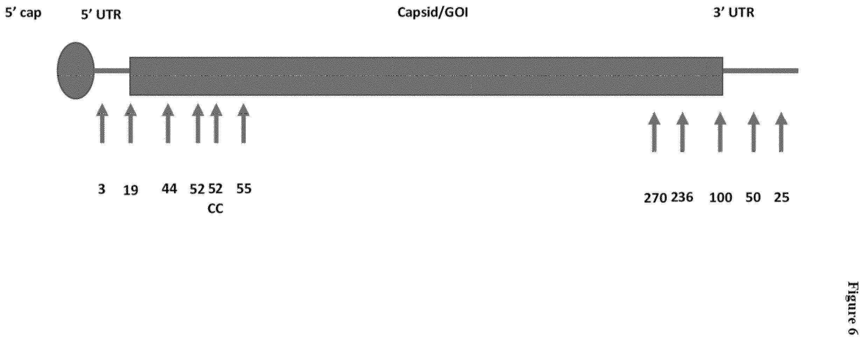

The invention so discloses a replicating RNA (RepRNA) system derived from a bi-segmented nodavirus and methods for producing a RepRNA pharmacon expressing a gene-of-interest (GOI), said pharmacon also comprising at least two (fused or non-fused) ribonucleic acid sequences, herein identified as nodavirus-like RNA1- and nodavirus-like RNA2-sequences , RNA1 at least encoding a nodavirus-like replicase functionally capable of having *cis*-activity on said RNA1 and trans-activity on nodavirus-like RNA2-sequences, therewith also capable of providing an RNA-pharmacon comprising RNA1- and/or RNA2-derived nucleic acid having said GOI. The inventors among others studied trans-acting signal requirements and, surprisingly, found that lengthening at least a part of a first conserved RNA2 sequence element (CSE) 5' flanking said GOI improves both replication and expression of said GOI-product. Said replication and expression improving 5'-UTR/CSE is disclosed herein to be preferably longer than the 3 (FHV) or 17nt (NoV) sequence used in the art (see also figures 4 and 6), a finding that greatly benefits generating therapeutically effective Noda-like RNA-pharmacons. The invention discloses a, preferably essentially purified, Noda-like self-replicating ribonucleic acid (RepRNA)-an/or RepRNA vaccine pharmacon comprising a ribonucleic acid encoding a gene-of-interest (GOI) and a ribonucleic acid encoding at least 19nt, preferably at least 44nt, most preferably at least 52nt derived from a 5'-CSE sequence of RNA2 nucleic acid of any of the Nodaviridae. In a preferred embodiment, said RepRNA and/or pharmacon contains 19 nt (nt 1 - 19) from the 5' end of RNA2, in a more preferred embodiment, said RepRNA and/or pharmacon contains 44 nt (nt 1 - 44) from the 5' end of RNA2, in a much preferred embodiment, said RepRNA and/or pharmacon contains 52 nt (nt 1 - 52) from the 5' end of RNA2, preferably derived from NoV. In another embodiment, said RepRNA and/or pharmacon contains 55 nt (nt 1 - 55) from the 5' end of RNA2, preferably derived from NoV. In a further embodiment, the invention discloses a, preferably essentially purified, Noda-like self-replicating ribonucleic acid (RNA)- vaccine pharmacon comprising a ribonucleic acid encoding a gene-of-interest (GOI) and a ribonucleic acid encoding at least the last 25nt, preferably at least the last 50nt, most preferably at least the last 100nt derived from a 3'-CSE sequence of RNA2 nucleic acid of any of the Nodaviridae. More preferably, the invention discloses a, preferably essentially purified, Noda-like self-replicating ribonucleic acid (RNA)- vaccine pharmacon comprising a ribonucleic acid encoding a gene-of-interest (GOI) and a ribonucleic acid encoding at least the last 176nt, preferably at least the last 236nt, most preferably at least the last 270nt derived from a 3'-CSE sequence of RNA2 nucleic acid of any of the Nodaviridae. INCLUDE THE LAST 650nt HERE?

The invention also discloses a preferably essentially purified, Noda-like self-replicating ribonucleic acid (RNA)- vaccine pharmacon comprising a ribonucleic acid encoding a gene-of-interest (GOI) and a ribonucleic acid encoding at least a functional part of an RNA-dependent RNA polymerase derived from a quasispecies variant nucleic acid sequence of any of the Nodaviridae. Essentially purified at least entails a near complete absence of double strand RNA in the system, preferably less than 2%, more preferably less than 1%, most preferably less than 0.5% is desired. The presence of double strand RNA can be tested with several methods known in the art (see for example J. Schönborn et al. Nucleic Acids Res.(1991)19, 2993. S. J. Richardson et al. J Clin Virol. (2010) 49(3); 180. F. Weber et al. J Virol (2006), 80(10):5059-64. K. Knoops et al. J Virol. (2012) 86(5); 2474. Or K. Karikó et al. Nucleic Acids Res. (2011) 39(21) e142), and furthermore essentially no DNA or protein contamination, preferably less than 1%, more preferably less than 0.5%, most preferably less than 0.1% of DNA or protein is desired, which can be tested with methods known in the art. Furthermore, it is desired that remnant DNA template levels are preferably < 200, more preferably < 100, most preferably < 50 ng/mg system RNA. A target system RNA purity of preferably > 80% free of impurities, more preferably > 90% free of impurities, most preferably > 95% of impurities is preferred. Throughout this application T may be used instead of U using the conventionality that T (thymidine) stands for U (uracil) in RNA. In short, essentially purified self-amplifying RNA of the invention is composed of or divided (split) into self-amplifying RNA (saRNA), where the replication machinery is encoded on the same RNA strand as the gene of interest (GOI) and trans-amplifying RNA (taRNA), where the replication machinery may be encoded alternatively on a different strand of RNA from the RNA encoding the GOI or on the same strand of RNA encoding the GOI.

In a preferred embodiment, the invention discloses a preferably essentially purified, self-replicating ribonucleic acid (RNA)-pharmacon comprising a ribonucleic acid encoding a gene-of-interest (GOI) and a ribonucleic acid encoding at least a functional part of an RNA-dependent RNA polymerase derived from a quasispecies variant nucleic acid sequence of any of the Nodaviridae, wherein said nucleic acid is provided with a ribonucleic acid encoding a functional RNA-dependent RNA polymerase comprising at least one nucleotide polymorphism selected from the group of nucleotide polymorphisms identified at positions 336, 664, 702, 726, 732, 777, 837, 1218, 1219, 1956, 2589, 3129, 3179, relative to quasispecies variant sequences obtainable from the nucleic acid sequences with accession numbers AF174533 orNC_002690 (see also Table 2).

In another preferred embodiment, the invention discloses a preferably essentially purified, self-replicating ribonucleic acid (RNA)-pharmacon comprising a ribonucleic acid encoding a gene-of-interest (GOI) and a ribonucleic acid encoding at least a functional part of an RNA-dependent RNA polymerase derived from a quasispecies variant nucleic acid sequence of any of the Nodaviridae, wherein said nucleic acid is provided with a ribonucleic acid encoding a functional RNA-dependent RNA polymerase comprising at least one nucleotide polymorphism selected from the group of nucleotide polymorphism identified at positions 664 and 1219, relative to quasispecies variant sequences obtainable from the nucleic acid sequences with accession numbers AF174533 or NC_002690 (see also Table 2).

In another preferred embodiment, the invention discloses a pharmacon according to the invention and additionally comprising a nucleic acid encoding at least a part of a first conserved sequence element (CSE) 5' flanking said GOI, said 5' CSE comprising the first 17 nucleotides, more preferably at least the first 19 nucleotides, more preferably at least the first 44 nucleotides, most preferably at least the first 52 or 55 nucleotides of a 5' conserved sequence element (CSE) flanking the RNA2 of a virus selected from the *Nodaviridae.*

In another preferred embodiment, the invention discloses a pharmacon according to the invention, comprising a nucleic acid encoding at least a part of a second CSE 3' flanking said GOI, said 3' CSE comprising the last 25 nucleotides, preferably the last 50 nucleotides, preferably the last 100 nucleotides, more preferably the last 176 nucleotides, more preferably the last 236 nucleotides. It is most preferred to include at least the last 237, more preferably at least the last 240, more preferred at least the last 250, more preferred to include the at least the last 270 nucleotides of 3' conserved sequence element (CSE) flanking the RNA2 of a virus selected from the *Nodaviridae.*

In another preferred embodiment, the invention discloses a pharmacon according to the invention wherein said ribonucleic acid encoding a functional RNA-dependent RNA polymerase is fused with a nucleic acid encoding a conserved sequence element (CSE) flanking the RNA2 of a virus selected from the *Nodaviridae.*

In another preferred embodiment, the invention discloses a pharmacon according to the invention further comprising a nucleic acid derived from a wild-type RNA1 and/or RNA2 quasispecies variant of a virus selected from the *Nodaviridae.*

In another preferred embodiment, the invention discloses a pharmacon according to the invention wherein said RNA1 is a wild-type RNA1 of a virus selected from a quasispecies variant of the Nodamura virus (NoV).

In another preferred embodiment, the invention discloses a pharmacon according to the invention provided with a ribonucleic acid encoding a functional RNA-dependent RNA polymerase comprising at least one nucleotide polymorphism selected from the group of nucleotide polymorphisms identified at positions 336, 664, 702, 726, 732, 777, 837, 1218, 1219, 1956, 2589, 3129, 3179, relative to quasispecies variant sequences obtainable from the nucleic acid sequences with accession numbers AF 174533 or NC_002690, preferably wherein said RNA1 is a wild-type RNA1 of a virus selected from a quasispecies variant of the Nodamura virus (NoV).

In another preferred embodiment, the invention discloses a pharmacon according to the invention provided with a ribonucleic acid encoding a functional RNA-dependent RNA polymerase comprising at least one nucleotide polymorphism selected from the group of nucleotide polymorphism identified at positions 664 and 1219, relative to quasispecies variant sequences obtainable from the nucleic acid sequences with accession numbers AF 174533 or NC_002690, preferably wherein said RNA1 is a wild-type RNA1 of a virus selected from a quasispecies variant of the Nodamura virus (NoV)..

In another preferred embodiment, the invention discloses a pharmacon according to the invention wherein said RNA1 encodes a viral replicase with an alanine at position 215, as shown in Table 2, preferably wherein said RNA1 is a wild-type RNA1 of a virus selected from a quasispecies variant of the Nodamura virus (NoV).

In another preferred embodiment, the invention discloses a pharmacon according to the invention wherein said RNA1 encodes a viral replicase with a proline at position 400, as shown in Table 2, preferably wherein said RNA1 is a wild-type RNA1 of a virus selected from a quasispecies variant of the Nodamura virus (NoV).

| Quasispec ies | PUBLISHED SEQUENCES | | | THIS APPLICATION | | THIS APPLICATION | |
|---|---|---|---|---|---|---|---|
| Table 2 | Accession numbers | | | SNP | coding change | SNP | coding change |
| RNA1 | | AF17453 3 | | PN04 48 | Protein A | Protein B1 | Protein B2 |
| | | NC_0026 90 | | | | | |
| position | 336 | T | | C | | | |
| | 664 | A | | G | T215A | | |
| | 702 | A | | G | _ | | |
| | 726 | C | | T | _ | | |
| | 732 | C | | T | _ | | |
| | 777 | T | | C | _ | | |
| | 837 121 | T | | C | _ | | |
| | 8 121 | C | | A | _ | | |
| | 9 195 | G | | C | A400P | | |
| | 6 258 | T | | G | _ | | |
| | 9 312 | C | | T | _ | | |
| | 9 317 | T | | A | _ | _ | L129Q |
| | 9 | A | | T | 3'UTR | | |
| RNA2 | | AF17453 4 | X159 61 | PN04 48 | CAPSID | | |
| | | NC_0026 91 | | | | | |
| position | 328 | T | G | G | _ | | |
| | 854 | G | C | G | _ | | |
| | 855 119 | C | G | C | _ | | |
| | 6 129 | G | A | G | _ | | |
| | 2 | T | C | T | _ | | |

The invention also discloses a, preferably essentially purified, self-amplifying (self-replicating) split replicon RNA system for the introduction of foreign genetic information or gene(s)-of-interest (GOI), said genetic information preferably encoding one or more (poly)peptides for prophylactic and/or therapeutic purposes, in a cell, preferably a cell of a subject, such as a vertebrate, such as a fish or a mammal. Essentially purified at least entails a near complete absence of double strand RNA in the system, preferably less than 2%, more preferably less than 1%, most preferably less than 0.5% is desired. The presence of double strand RNA can be tested with several methods known in the art (see for example J. Schönborn et al. Nucleic Acids Res. (1991)19, 2993. S. J. Richardson et al. J Clin Virol. (2010) 49(3); 180. F. Weber et al. J Virol (2006), 80(10):5059-64. K. Knoops et al. J Virol. (2012) 86(5); 2474. Or K. Karikó et al. Nucleic Acids Res. (2011) 39(21) e142), and furthermore essentially no DNA or protein contamination, preferably less than 1%, more preferably less than 0.5%, most preferably less than 0.1% of DNA or protein is desired, which can be tested with methods known in the art. Furthermore, it is desired that remnant DNA template levels are preferably < 200, more preferably < 100, most preferably < 50 ng/mg system RNA. A target system RNA purity of preferably > 80% free of impurities, more preferably > 90% free of impurities, most preferably > 95% of impurities is preferred. Throughout this application T may be used instead of U using the conventionality that T (thymidine) stands for U (uracil) in RNA. In short, essentially purified self-amplifying RNA of the invention is composed of or divided (split) into self-amplifying RNA (saRNA), where the replication machinery is encoded on the same RNA strand as the gene of interest (GOI) and trans-amplifying RNA (taRNA), where the replication machinery is encoded on a different strand of RNA from the RNA encoding the GOI. The split replicon RNA system as provided herein by the invention is obtainable by a cell-free system of in vitro transcription by using an enzymatic transcription reaction from a, preferably linearized, preferably *Nodaviridae-derived* DNA template requiring a DNA-dependent RNA polymerase, such as a T7 polymerase, more preferably a SP6 polymerase and discloses essentially purified RNA(s) that are typically required and useful for inclusion in in an essentially pure RNA vaccine composition or lipid nanoparticle formulation.

In a preferred embodiment, the invention discloses an essentially purified, isolated or synthetic, preferably single positive strand, ribonucleic acid molecule capable of trans-amplification (herein also identified as taRNA2) and derived from a bi-segmented virus, said taRNA2-molecule lacking or comprising a deletion of genetic information and coding for at least a part of the RNA2 gene encoding a structural protein of said bi-segmented virus to allow for the introduction of an insertion of genetic information derived from a gene-of-interest (GOI) not derived from said virus, preferably to allow for expressing an antigenic protein from said GOI, preferably expressing a vaccine antigen encoded by said GOI.

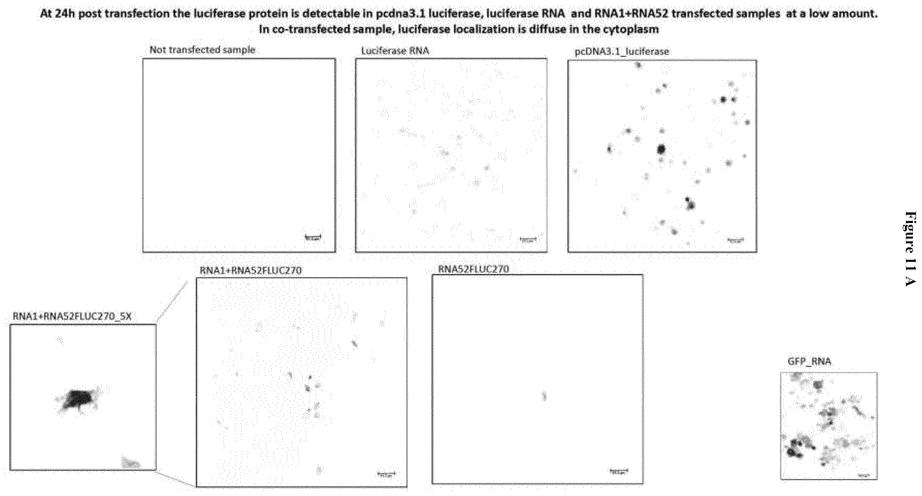

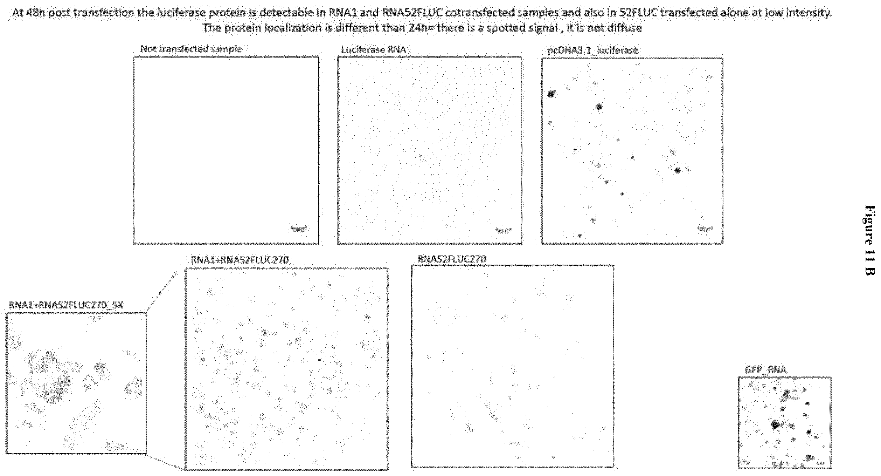

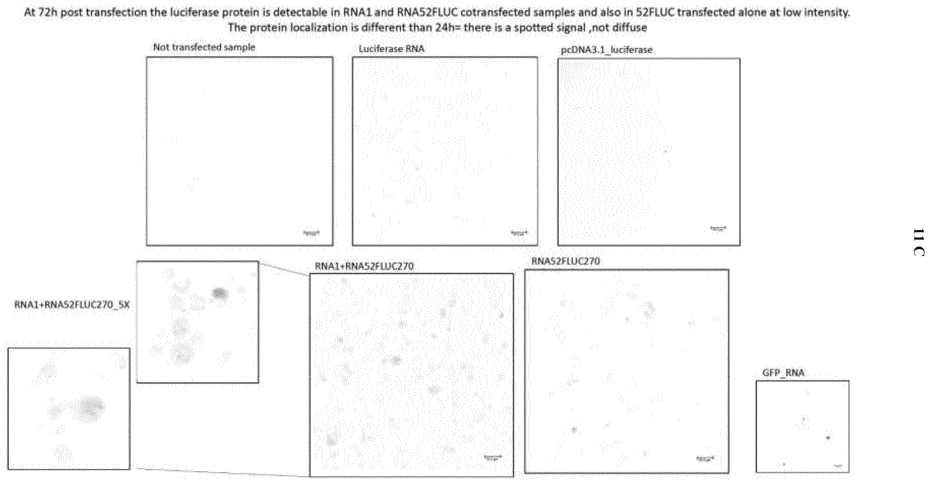

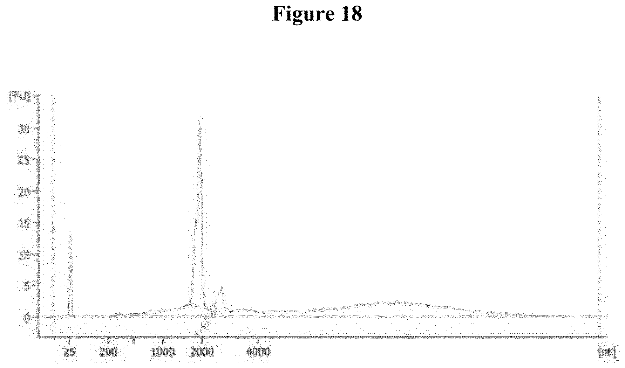

In a preferred embodiment, the invention discloses that said deletion in said taRNA2 molecule is 5' flanked by the first 19 nucleotides, more preferably the first 44 nucleotides, most preferably the first 52 nucleotides of a 5' conserved sequence element (CSE) flanking the RNA2 of a virus selected from the Nodaviridae. It is additionally preferred that said first 3, respectively 17, respectively 19, respectively 44, respectively 52, respectively 55 nucleotides do not encode for a start codon ATG. Preferably, however, 5' CSE's with 44, 52 and 55 contain a 3' Kozak sequence. In another preferred embodiment, the invention discloses that said deletion in said taRNA2 molecule is 3' flanked by the last 25 nucleotides, preferably the last 50 nucleotides, preferably the last 100 nucleotides, more preferably the last 176 nucleotides, more preferably the last 236 nucleotides, more preferably at least the last 240 nucleotides, more preferably at least the last 250 nucleotides, more preferably at least the last 270 nucleotides, most preferably at least the last 270 nucleotides of a 3' conserved sequence element (CSE) flanking the RNA2 of a virus selected from the Nodaviridae. See for examples also figures 4 and 6. Preferably, the invention discloses a composition comprising said ribonucleic acid, such as a lipid nanoparticle composition admixed with said taRNA2. Preferably said composition comprises a tRNA provided with a GOI which is 5' flanked by the first 19 nucleotides, more preferably the first 44 nucleotides, more preferably the first 52 nucleotides, most preferably the first 55 nucleotides of a 5' conserved sequence element (CSE) flanking the RNA2 of a virus selected from the Nodaviridae. It is additionally preferred that said first 3, respectively 19, respectively 44, respectively 52, respectively 55 nucleotides do not encode for a start codon ATG. Preferably, however, 5' CSE's with 44, 52 and 55 contain a 3' Kozak sequence. It is also preferred that said GOI is 3' flanked by the last 25 nucleotides, preferably the last 50 nucleotides, preferably the last 100 nucleotides, more preferably the last 176 nucleotides, more preferably the last 236 nucleotides, most preferably the last 270 nucleotides of 3' conserved sequence element (CSE) flanking the RNA2 of a virus selected from the Nodaviridae. In a further preferred embodiment, the invention discloses a taRNA2, or a taRNA2 composition according to the invention admixed with a lipid nanoparticle formulation. In a further preferred embodiment, said taRNA2 or taRNA2 composition according to the invention is admixed with an saRNA1 provided with the invention, preferably saRNA1 and taRNA2 admixed with a lipid nanoparticle formulation. Use of such taRNA2 and taRNA2+saRNA1 and compositions as provided herein in transfection of a cell is also provided. Such use is in particular provided to express an antigen or antigenic protein formulation in such a cell (see for examples fig 11 and 18), allowing for example use of such taRNA2 and taRNA2+saRNA1 and compositions as provided herein in vaccination, preferably of a vertebrate (such as a bird) , more preferably in a mammal, such as a pig or a human. The invention also discloses a method of vaccinating such a vertebrate comprising providing said vertebrate, such as intradermally, subcutaneously, or mucosally, preferably intra-muscularly, with such taRNA2 and taRNA2+saRNA1 and compositions as provided herein. The invention also discloses a lipid nanoparticle formulation provided with a taRNA2 and/or taRNA2+saRNA1 and compositions as provided herein useful in the production of a vaccine. The invention also discloses a method of vaccinating a vertebrate comprising providing said vertebrate, preferably intra-muscularly, with a lipid nanoparticle formulation as provided herein.

Therewith, in a further embodiment, the invention discloses a composition of an essentially purified, isolated or synthetic, preferably single positive strand, ribonucleic acid molecule capable of self-amplification (herein also identified as saRNA1) for expressing a replicase, said replicase herein identified as protein A derived from a bi-segmented virus capable of replicating ribonucleic acid, preferably in a cell, with an essentially purified, isolated or synthetic, preferably single strand, ribonucleic acid molecule capable of trans-amplification (herein also identified as taRNA2), said taRNA2-molecule lacking or comprising a deletion of genetic information coding for at least a part of a structural protein of said virus to allow for the introduction of genetic information not derived from said virus. Preferably, the invention discloses a composition comprising said ribonucleic acid, such as a lipid nanoparticle composition admixed with said saRNA1 and taRNA2. In the preferred embodiment, such lipid nanoparticle composition is selected from the group of nanoparticle compositions herein identified as formulation 1, formulation 2, formulation 3 and formulation 4.

Therewith the invention discloses an essentially purified split ribonucleic acid replicon system (herein also called a split RepRNA pharmacon system) comprising a composition with:
a) an essentially purified, isolated or synthetic, preferably single strand, ribonucleic acid molecule capable of self-amplification (herein also identified as saRNA1) for expressing a replicase, said replicase herein identified as protein A derived from a bi-segmented virus capable of replicating ribonucleic acid, preferably in a cell,
b) an essentially purified, isolated or synthetic, preferably single strand, ribonucleic acid molecule capable of trans-amplification (herein also identified as taRNA2), said taRNA2-molecule lacking or comprising a deletion of genetic information coding for at least a part of a structural protein of said virus to allow for the introduction of genetic information not derived from said virus, preferably to allow for expressing an antigenic protein, preferably expressing a vaccine antigen, preferably an influenza vaccine antigen such as the hemagglutin protein (HA).

In a further preferred embodiment, the invention discloses an essentially purified split ribonucleic acid replicon system (herein also called a split RepRNA pharmacon system) comprising a composition with:
a) an essentially purified, isolated or synthetic, preferably single strand, ribonucleic acid molecule capable of self-amplification (herein also identified as saRNA1) for expressing a replicase, said replicase herein identified as protein A derived from a bi-segmented virus capable of replicating ribonucleic acid, preferably in a cell,
b) an essentially purified, isolated or synthetic, preferably single strand, ribonucleic acid molecule capable of trans-amplification (herein also identified as taRNA2), said taRNA2-molecule having been provided with a GOI and said GOI preferably 5' flanked by the first 17 nucleotides, more preferably at least the first 19 nucleotides more preferably at least the first 44 nucleotides, more preferably at least the first 52 nucleotides, most preferably at least the first 55 nucleotides of a 5' conserved sequence element (CSE) flanking the RNA2 of a virus selected from the Nodaviridae. It is additionally preferred that said first 3, respectively 17, respectively 19, respectively 44, respectively 52, respectively 55 nucleotides do not encode for a start codon ATG, and/or 3'flanked by the last 25 nucleotides, preferably the last 50 nucleotides, preferably the last 100 nucleotides, more preferably the last 176 nucleotides, more preferably the last 236 nucleotides. It is most preferred to include at least the last 237, more preferably at least the last 240, more preferred at least the last 250, more preferred to include the at least the last 270 nucleotides of 3' conserved sequence element (CSE) flanking the RNA2 of a virus selected from the Nodaviridae.

In the split RepRNA system as provided herein, said saRNA1 is having cis-activity on saRNA1 and trans-activity on taRNA2 through replicase activity of said protein A expressed by said saRNA1.

In a yet further preferred embodiment, the invention discloses an essentially purified fused ribonucleic acid replicon system (herein also called a fused RepRNA pharmacon system) comprising a composition with:
a) a 5' essentially purified, isolated or synthetic, preferably single strand, ribonucleic acid molecule capable of self-amplification (herein also identified as saRNA1) for expressing a replicase, said replicase herein identified as protein A derived from a bi-segmented virus derived from the family Nodaviridae capable of replicating ribonucleic acid, preferably in a cell,
c) and fused 3' at with essentially purified, isolated or synthetic, preferably single strand, ribonucleic acid molecule capable of trans-amplification (herein also identified as taRNA2), said taRNA2-molecule having been provided with a GOI and said GOI preferably 5' flanked by the first 17 nucleotides, more preferably at least the first 19 nucleotides more preferably at least the first 44 nucleotides, more preferably at least the first 52 nucleotides, most preferably at least the first 55 nucleotides of a 5' conserved sequence element (CSE) flanking the RNA2 of a virus selected from the Nodaviridae. It is additionally preferred that said first 3, respectively 17, respectively 19, respectively 44, respectively 52, respectively 55 nucleotides do not encode for a start codon ATG, and/or 3'flanked by the last 25 nucleotides, preferably the last 50 nucleotides, preferably the last 100 nucleotides, more preferably the last 176 nucleotides, more preferably the last 236 nucleotides. It is most preferred to include at least the last 237, more preferably at least the last 240, more preferred at least the last 250, more preferred to include the at least the last 270 nucleotides of 3' conserved sequence element (CSE) flanking the RNA2 of a virus selected from the Nodaviridae.

Said split system is herein also identified as RepRNA and comprises a set of at least two RNA-molecules, saRNA1 and taRNA2, saRNA1 comprising a self-amplifying RNA construct for expressing a replicase (herein also identified as protein A) preferably derived from a bi-segmented virus and taRNA2 comprising an RNA construct that can be replicated by the replicase in trans. In a much-preferred embodiment, said taRNA2 construct comprises a GOI preferably useful in prophylaxis and therapy. In another much-preferred embodiment, said saRNA1 construct for expressing a replicase derived from a bi-segmented virus also is provided with or having a (subgenomic) coding sequence for expressing an innate immunity repressing protein (herein also identified as protein B) that can be replicated by the replicase in cis (herein said subgenomic sequence also identified as cis-amplified RNA3). Typically, addition of such a protein B prolongs expression of self- and trans-replicating RNA and/or inhibits untimely cell death and therewith having improved prophylactic and/or therapeutic activity. In another embodiment, said RNA3 is (also) provided with or having a (subgenomic) coding sequence for expressing an innate immunity repressing protein (herein also identified as protein B). It is most preferred that said saRNA1 has a coding sequence (herein also identified as RNA3) for a protein B having innate cellular ribonucleic acid (RNA) interference antagonist activity as well as innate immunity repressing activity.

In another much-preferred embodiment, saRNA1 and/or taRNA2 essentially have no or only a small 3'-poly(A) sequence. Preferably said 3'-poly(A) sequence, if present, comprises less than 20 adenosines, more preferably less than 15, more preferably less than 10, most preferably less than 5. Essentially no poly(A) sequence is required in a much-preferred embodiment of the invention, providing essential advantages in synthetic production of said RNA, for example by reducing errors in polymerase chain reactions (PCR) used to produce DNA-templates of said RNA construct. Thus, production wise a preferred system of the invention is provided wherein said saRNA1 and/or taRNA2 essentially do not require or do not have a 3'-poly(A) tail sequence or only have a small (< 20 adenosines) 3'-poly(A) tail sequence.

In another much-preferred embodiment, saRNA1 and/or taRNA2 essentially have or are provided with at least a region structurally required for transcription of taRNA2 by protein A, such region preferably comprising at least one conserved sequence element (CSE) at or close to the 5'-UTR and/or 3'-UTR end, if required close to or in the RNA encoding an ORF, allowing initiation of replication by said replicase. In a preferred embodiment according to the invention, said protein A is having RNA-dependent RNA polymerase (RdRp) activity. It is preferred that, in addition to having RdRp activity, protein A binds to and drives invagination of the outer mitochondrial membranes in a host cell to provide the compartment where RNA replication occurs. It is moreover preferred that said protein A is derived from a virus selected from the group of *Nodaviridae,* preferably from the group of *Alphanodavirus,* more preferably from a Nodamura virus (NoV) or Flock house virus (FHV). In another preferred embodiment, said protein B is having innate cellular RNA interference antagonist activity. It is moreover preferred that said protein B is derived from a virus selected from the group of *Nodaviridae,* preferably from the group of *Alphanodavirus,* more preferably from a Nodamura virus (NoV) or Flock house virus (FHV). In a further embodiment, it is preferred that the RNA construct for expressing a replicase derived from a bi-segmented virus (RNA1) comprises a conserved sequence element (CSE) at the 5'-UTR and/or 3'-UTR, preferably both, ends allowing initiation of replication by said replicase. In a further embodiment, it is also preferred that the RNA replicon that can be replicated by the replicase in trans (RNA2) comprises a conserved sequence element (CSE) at the 5'-UTR and/or 3'-UTR, preferably both, ends allowing initiation of replication by said GOI. It is moreover preferred that said CSE is derived from a virus selected from the group of *Nodaviridae,* preferably from the group of *Alphanodavirus,* more preferably from a Nodamura virus (NoV) or Flock house virus (FHV). In a further embodiment, it is preferred that the RNA construct for expressing a replicase derived from a bi-segmented virus (RNA1) comprises a 5'-cap for driving translation of the replicase. In a further preferred embodiment, the invention discloses a system, composition or lipid nanoparticle formulation according to the invention wherein said saRNA1 and/or taRNA2 is capped at the 5' nucleotide with a cap-1, and more preferably with a cap-0 structure, preferably wherein said RNA2 is additionally provided with a ribonucleic acid encoding a gene-of-interest (GOI), preferably allowing expression of an antigenic protein in a cell encoded by said GOI (see for example fig 11 and 18). In said further embodiment, it is also preferred that the RNA replicon that can be replicated by the replicase in trans (RNA2) comprises a 5'-cap for driving translation of said GOI. Such a 5'-cap is a preferable selected from a natural 5'-cap or a 5'- cap analogue. In one embodiment, a cap-0 is preferred, cap-0, being a N7-methyl guanosine connected to the 5' nucleotide through a 5' to 5' triphosphate linkage, typically also refers to as m7G cap or m7Gppp- in the literature. In the cell, the cap-0 structure is deemed essential for efficient translation of the mRNA that carries the cap. In a more preferred embodiment, cap-0 is preferred over the cap-1 commonly deemed present in Nodaviridae (Ball, J. Vir. 1995 p720). Cap-1 comprises an additional methylation over cap-0 on the 2'O position of the initiating nucleotide.ap-1, or refers to as m7GpppNm-, where Nm denotes any nucleotide with a 2'O methylation. In one embodiment, it is preferred that the RNA construct for expressing a replicase derived from a bi-segmented virus does not comprise an internal ribosomal entry site (IRES) element for driving translation of the replicase. The invention also discloses an essentially purified trans-amplifying (trans-replicating) RNA system for the introduction of foreign genetic information or gene(s)-of-interest (GOI), wherein the saRNA1 construct for expressing a replicase derived from a bi-segmented virus comprises: (1) a 5' UTR, (2) an open reading frame encoding the replicase (RdRp), and (3) a 3' UTR, preferably wherein the 5' UTR and/or 3' UTR is native to a bi-segmented virus from which the replicase is derived. In a further embodiment, the provided system according to the invention, wherein the open reading frame encoding a replicase derived from a bi-segmented virus comprises coding region(s) for non-structural protein(s) required for RNA replication, preferably wherein the saRNA1 for expressing a replicase derived from a bi-segmented virus comprises none or only a small a 3' poly(A) sequence, preferably said sequence comprises less than 20 adenosines, more preferably less than 15, more preferably less than 10, most preferably less than 5. The invention also discloses an essentially purified trans-amplifying (trans-replicating) RNA system for the introduction of foreign genetic information or gene(s)-of-interest (GOI), wherein the taRNA replicon (RNA2) construct comprises: (1 ) a bi-segmented virus 5' replication recognition sequence, and (2) an bi-segmented 3' replication recognition sequence, preferably wherein said virus 5' replication recognition sequence and said virus 3' replication recognition sequence direct replication of the RNA replicon in the presence of the replicase. It is preferred that said 5' replication recognition sequence and the said 3' replication recognition sequence are native to the bi-segmented virus or virus family from which the replicase is derived. It is moreover preferred that said 5' replication recognition sequence and the said 3' replication recognition are derived from a virus selected from the *Nodaviridae* whereas the RNA replicon comprises a heterologous nucleic acid not derived from a virus selected from the *Nodaviridae.*

It is moreover preferred that the RNA replicon comprises an open reading frame encoding a protein of interest, preferably wherein the open reading frame encoding a protein of interest is non-native to the virus from which the replicase is derived. Optionally, expression of said open reading frame encoding a protein of interest is under the control of a subgenomic promoter, preferably wherein the subgenomic promoter is native to the virus from which the replicase is derived. Preferably, the RNA2 construct and/or the RNA replicon does not comprise an open reading frame encoding an intact alphavirus structural protein or fragment thereof. The invention also discloses a vector comprising a nucleic acid sequence encoding the saRNA1 replicon construct (said construct preferably comprising a subgenomic cis-amplified RNA3 as defined herein) for expressing the virus replicase, the taRNA2 replicon construct, or both according to the invention. In a preferred embodiment, such a vector or plasmid comprises a deoxyribonucleic acid (DNA) comprising a nucleic acid sequence template encoding the saRNA1, taRNA2 or both of the system according to the invention. It is preferred that said DNA according to the invention comprises a template for in vitro transcription wherein said template is under control of a T7or a SP6 promotor to drive in vitro transcription. The invention also discloses a cell provided with a DNA according to the invention useful in a method for producing a RepRNA system according to the invention, such as under production conditions where in vitro transcription is not possible. A preferred method according to the invention for producing the split RepRNA system of the invention comprises in vitro transcription of a set of at least two ribonucleic acids, identified as saRNA1 and taRNA2, saRNA1 having cis-activity on saRNA1 and trans-activity on taRNA2, from a DNA as provided herein Preferably, the invention discloses a DNA, and a method of producing said RepRNA system of the invention, wherein said DNA encodes a nucleic acid template encoding taRNA2, said taRNA2-molecule template preferably having been provided with a GOI and said GOI preferably 5' flanked by the first 19 nucleotides, more preferably the first 44 nucleotides, more preferably the first 52 nucleotides, most preferably the first 55 nucleotides of a 5' conserved sequence element (CSE) flanking the RNA2 of a virus selected from the Nodaviridae. It is additionally preferred that said first 3, respectively 19, respectively 44, respectively 52, respectively 55 nucleotides do not encode for a start codon ATG, and/or 3' flanked by the last 25 nucleotides, preferably the last 50 nucleotides, preferably the last 100 nucleotides, more preferably the last 176 nucleotides, more preferably the last 236 nucleotides, most preferably the last 270 nucleotides of 3' conserved sequence element (CSE) flanking the RNA2 of a virus selected from the Nodaviridae.

Setting these CSE requirements at the various above identified number of nucleotides allows sufficient deletion to introduce various GOI's. The table below describes the respective lengths of the deleted wild-type capsid-related sequence fragments from RNA2 for the generation of different constructs with various 5' and 3' end; the gene of interest is preferably cloned there instead of the deleted fragment in the RNA2 vector and RNA is preferably generated by in vitro translation (IVT) thereof. The full-length RNA is 1336 nucleotides in length. For the 5' 44 construct a natural Kozak sequence (here GCC) is available upstream of the start codon. For the 5' 52CC construct 2 Cs were introduced in the sequence to generate a Kozak sequence upstream of the start codon; in consequence, the length of the RNA2 deleted fragment is the same as for the 52nt vector. For the 5' 55 construct a natural Kozak sequence (here GCC) is available upstream of the start codon of the GOI.

| Delta RNA2 | 5' 3nt | 5' 19nt | 5' 44nt | 5' 52nt | 5' 52CCnt | 5' 55 nt |
|---|---|---|---|---|---|---|
| 3' 270nt | 1063 | 1047 | 1022 | 1014 | 1014 | 1011 |
| 3' 236nt | 1097 | 1082 | 1056 | 1048 | 1048 | 1045 |
| 3' 100nt | 1233 | 1217 | 1192 | 1184 | 1184 | 1181 |
| 3' 50nt | 1283 | 1267 | 1242 | 1234 | 1234 | 1231 |
| 3' 25nt | 1308 | 1292 | 1267 | 1259 | 1259 | 1256 |

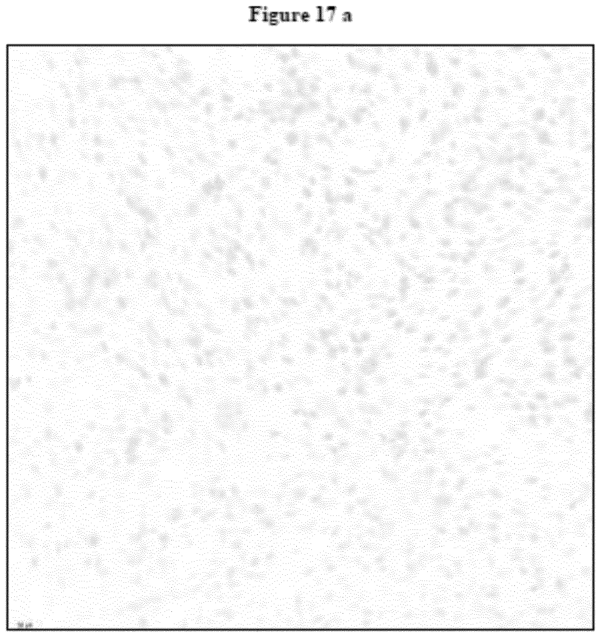

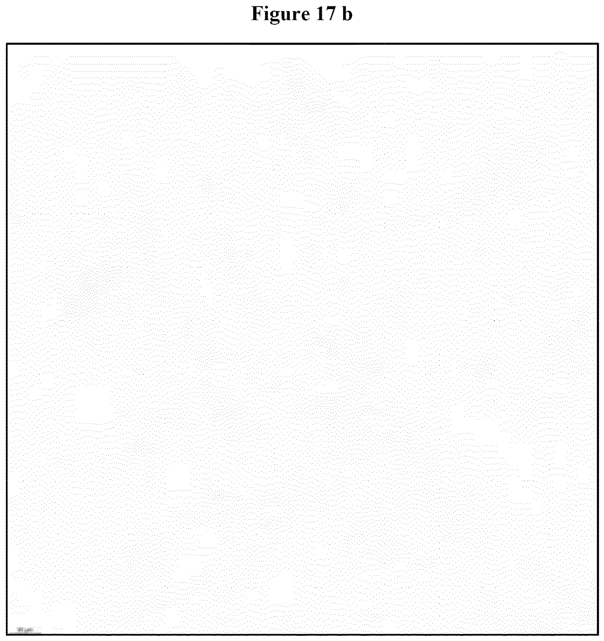

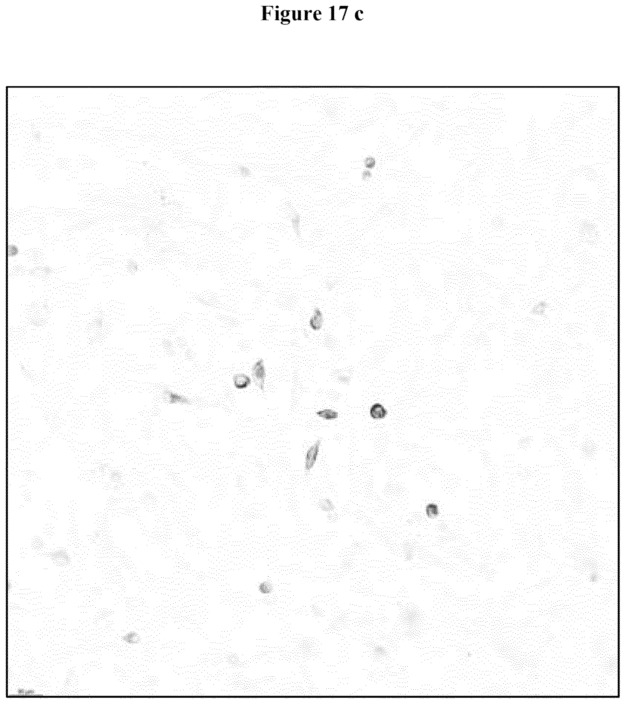

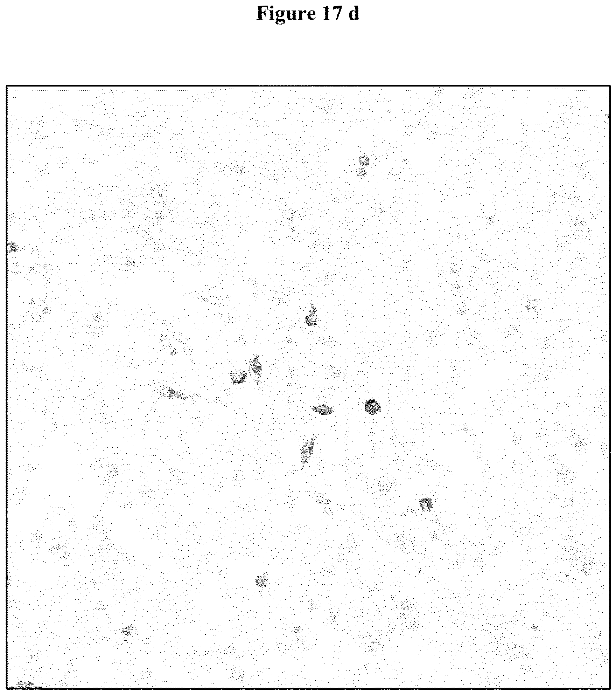

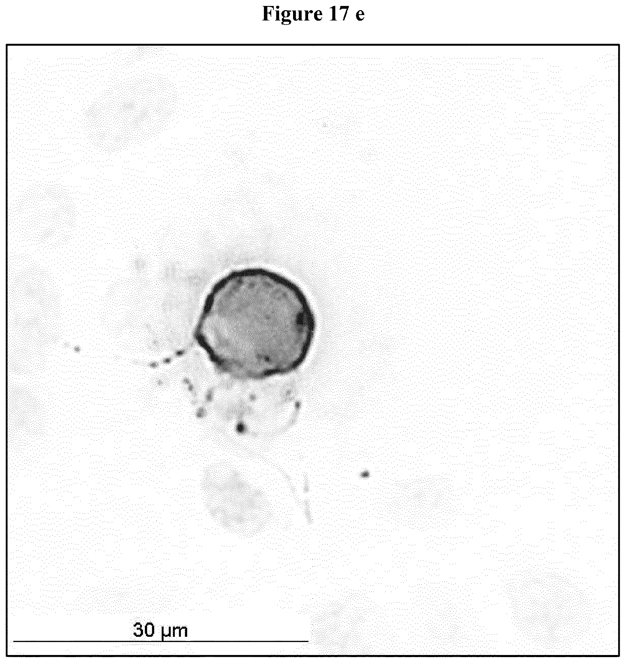

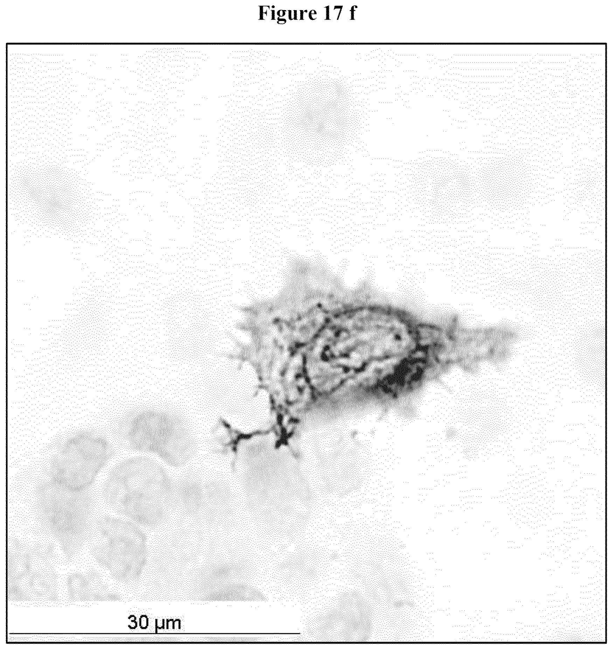

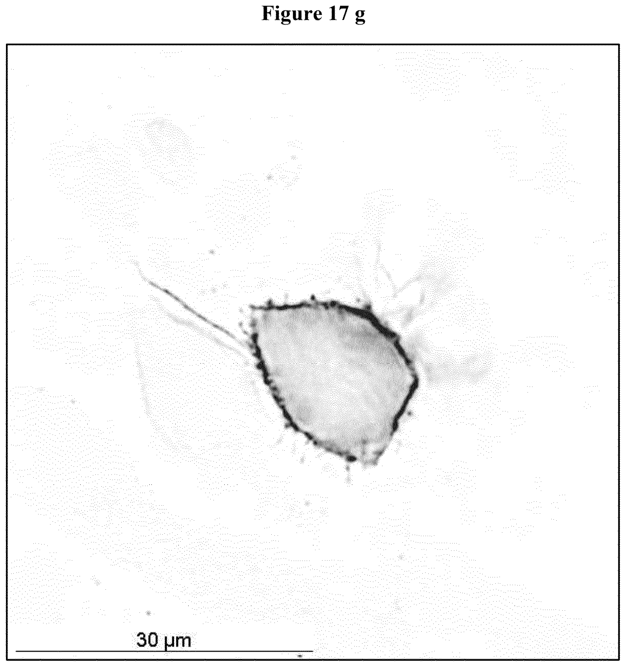

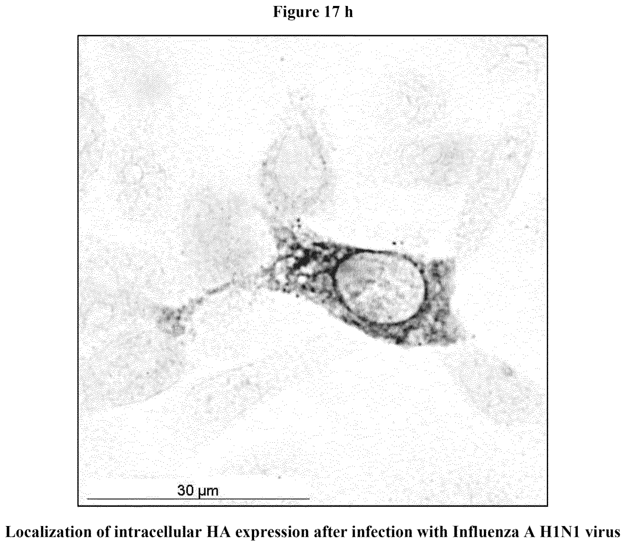

The invention also discloses a method for producing a protein in a cell comprising the steps of: (a) obtaining an essentially purified saRNA1 construct for expressing a bi-segmented virus replicase by in vitro transcription from a DNA-template, (b) obtaining an essentially purified taRNA2 replicon construct by in vitro transcription from a DNA-template that can be replicated by the replicase in trans and comprises an open reading frame encoding the protein (derived from GOI) and flanking CSE, and (c) co-inoculating the essentially purified saRNA1 construct for expressing virus replicase and the taRNA2 replicon construct into the cell, preferably wherein the saRNA1 construct for expressing virus replicase comprises a cis-amplified RNA3 construct for expressing an innate repressing protein capable of antagonizing endogenous RNA replication interference of said host cell. The invention also discloses a cell inoculated according to a method for producing a GOI-derived protein, preferably an antigenic protein (such as FLUC, see figure 11), more preferably a desirable vaccine antigen (such as HA, see figure 17), in a cell as provided herein. The invention also discloses a method for producing a protein, polypeptide or peptide in a subject comprising the steps of: (a) obtaining an essentially purified saRNA1 construct for expressing virus replicase, said virus selected from the group of *Nodaviridae,* preferably from the group of *Alphanodavirus,* more preferably from a Nodamura virus (NoV) or Flock house virus (FHV); (b) obtaining an taRNA2 replicon construct that can be replicated by the replicase in trans and comprises an open reading frame encoding the protein, and (c) administering the saRNA1 construct for expressing virus replicase and the taRNA2 replicon construct to the subject, wherein the saRNA1 construct for expressing virus replicase comprises a cis-amplified RNA3 construct for expressing an innate repressing protein capable of antagonizing RNA replication interference of said host cell.

In a further embodiment, the invention discloses a method for producing an improved split RepRNA system comprising generating and essentially purifying a set of at least two ribonucleic acids, herein identified as saRNA1 and taRNA2, the saRNA1 having *cis*-activity on saRNA1 (encoding a replicase) and optionally on cis-amplified RNA3 and *trans*-activity on taRNA2 (not encoding a replicase). Numerous transcriptional, regulatory, and responsive factors or elements, which herein are classified into *cis*-regulatory (active) factors or elements and *trans*-regulatory (active) factors or elements, regulate RNA expression (transcription and/or translation). *Cis*-active elements, such as promoters, enhancers, and silencers, are regions of non-coding RNA, which regulate the transcription and thus translation of nearby genes located on the same RNA-molecule. In contrast, *trans-*active factors regulate (or modify) the expression of distant genes located in another RNA by combining with their target sequences. In the split RepRNA system as provided herein, interactions between cis-regulatory sequences in saRNA1 and *trans*-acting factors from saRNA1 on taRNA2 control replicase and GOI expression, respectively.

It is preferred that said saRNA1 and taRNA2 are each essentially purified to therapeutic or clinical grade levels of purity generally desired for therapeutic use in animals, more preferably mammals, and preferably at least essentially purified to therapeutic or clinical grade levels of purity generally desired for use in humans. Even more preferred desired levels of essential purity for therapeutically of clinical grade purification of ribonucleic acid for use in a split RepRNA system according to the invention are selected from the group of quality analysis characteristics (QA), preferably at least selected from critical characteristics (CQA), as listed in below table (van den Berg et al., NPJ Vaccines. 2021 Apr 29;6(1):65).

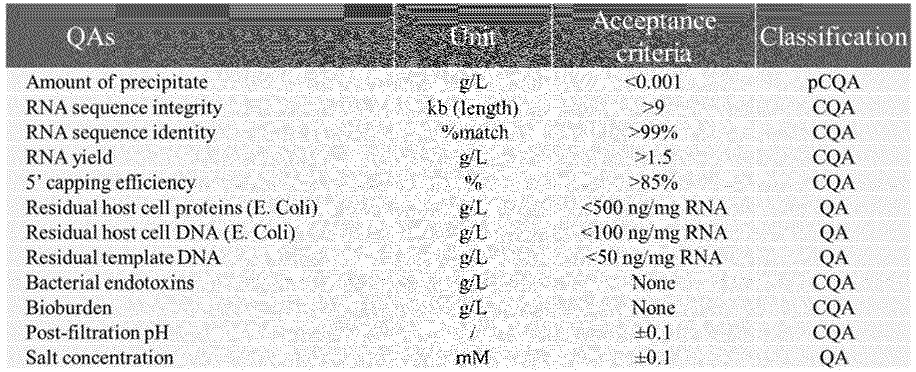

It is preferred that said saRNA1 is at least encoding a polypeptide A having RNA-replicase activity. It is furthermore preferred that said taRNA2 is at least comprising a region structurally required for transcription of taRNA2 by polypeptide A. In a much-preferred embodiment, said saRNA1 and/or taRNA2 are incapable of supporting generation of an infectious viral-like particle through transcription of RNA 1 or taRNA2. In deviance from established methods in the art, the invention discloses a method wherein the (in itself not replicative) taRNA2 additionally is provided with a ribonucleic acid encoding at least one gene-of-interest (GOI). Moreover, the invention discloses a preferred method wherein saRNA1 also comprises a subgenomic RNA3 encoding a protein-B or polypeptide-B having innate cellular RNA interference antagonist activity. For ease of production, the invention discloses a method wherein saRNA1 and/or taRNA2 have a poly A (adenosine) tail <20, preferably < 15 A, preferably <10 A, more preferably < 5 A, preferably wherein a poly A tail is absent. In a particular prefer embodiment, the invention discloses a method using at least 2 different versions of taRNA2 for inclusion in one split RepRNA system. The invention also discloses a set of at least two essentially purified ribonucleic acids, preferably obtained or obtainable with a method for producing a split RepRNA system as defined herein, said set of ribonucleic acids herein comprising at least two different nucleic acids, herein identified as saRNA1 and taRNA2, for use in and the production of a so-called "split RepRNA system". Therewith, the invention departs from the common and well-established practice in the field to construct and use one RepRNA-molecule carrying and encoding both the replicative machinery as well as the gene-of-interest (GOI). The invention discloses use of at least two RNA-constructs instead. Typically, an saRNA1 construct is provided that encodes the desired replicase (*cis*-active on saRNA1 and *trans-*active on taRNA2), whereas one or more taRNA2-constructs are provided that are employed to encode one or more GOI. Such taRNA2-constructs can also be provided with at least two genes-of-interest, said GOI preferably separated by for example an IRIS sequence or 2A "jump site" sequence (Sci Rep. 2017 May 19;7(1):2193. ).

Such a set of ribonucleic acids wherein one ribonucleic acid has cis-activity on itself as well as *trans*-activity on at least one other RNA-molecule, as provided herein, is useful in the production of a split replicon system according to the invention. Contrary to most current RepRNA systems that employ cis-activity only to express both replicase as well as GOI, production difficulties are averted through using at least two, in principle shorter, saRNA1- and taRNA2-segments for building the desired replicon system. In a preferred embodiment, said saRNA1 is at least encoding a protein A having autonomous RNA-replicase activity, preferably in a host cell. In another preferred embodiment, said RNA 2 is at least comprising a region structurally required for transcription of taRNA2 by said protein A in said cell. In a more preferred embodiment, said region is a 3' untranslated region (3' UTR). It is most preferred that said region is equipped for or even required for functional taRNA2-replicating activity by polypeptide A in said host cell. It is furthermore preferred that said saRNA1 and/or taRNA2 is or are, preferably rendered by mutation or deletion, incapable of producing an infectious virus or viral-like particle (VLP), or at least incapable of supporting intracellular generation of an infectious VLP from said saRNA1 or taRNA2 through transcription. For avoiding intracellular VLP generation, it is preferred that said RNA 1 or RNA 2 are chosen such that either are at least not encoding an essentially functional viral structural protein for particle formation that may harbor saRNA1 and taRNA2, such as a capsid protein of a virus, more preferably of a capsid protein of a virus selected from the group of *Nodaviridae,* to avoid infectious VLP-generation. Furthermore, the invention discloses a set of essentially purified saRNA1 and taRNA2 ribonucleic acids as provided herein, said taRNA2 additionally provided with a ribonucleic acid encoding a gene-of-interest (GOI), such as a *non-Nodaviridae* polypeptide. It is preferred that saRNA1 also comprises a subgenomic RNA3 encoding a protein-Bor polypeptide-B having innate cellular RNA interference antagonist activity. In a further preferred embodiment, the invention discloses a set of essentially purified saRNA1 and taRNA2 ribonucleic acids as provided herein comprising at least 2 different versions of taRNA2, for example allowing using said RepRNA for eliciting two different immune-responses, as in a dual-purpose vaccine. It is moreover preferred that said set of essentially purified saRNA1 and taRNA2 ribonucleic acids as provided herein comprises at least one ribonucleic acid that is 5'-capped, preferably saRNA1 and taRNA2 are both 5'-capped. In another embodiment, said saRNA1 and/or taRNA2 are not capped and may alternatively use internal ribosome entry sites (IRESs), preferably located in the 5'-untranslated region close to the start codon AUG to be used. Similarly, and very useful from the perspective of production and formulation of the RepRNA system provided herein, the invention discloses use or saRNA1 and/or taRNA2 that do (does) not contain a polyA tail, or only a small polyA tail of less than 20 A, preferably smaller such as less than 15 A, preferably less than 10, more preferably less than 5 A. The invention also discloses a set of essentially purified saRNA1 and taRNA2 ribonucleic acids as provided herein wherein at least one ribonucleic acid is provided with nucleoside-modified ribonucleic acid. Uridine can for example be replaced with a similar nucleoside such as pseudouridine (Ψ) or N1-methyl-pseudouridine (m1Ψ), and cytosine can be replaced by 5-methylcytosine. Some of these, such as pseudouridine and 5-methylcytosine, occur naturally in eukaryotes. Inclusion of these modified nucleosides alters the secondary structure of the mRNA, which can reduce recognition by the innate immune system while still allowing effective translation. In a much-preferred embodiment, the invention discloses a set of essentially purified saRNA1 and taRNA2 ribonucleic acids as provided herein wherein said saRNA1, or at least some of its regulatory or responsive elements in or encode by said saRNA1, is or are derived from a virus having a bi-segmented genome, such as a virus selected from the group of *Nodaviridae,* such as a porcine or canine Noda virus. Typically, also preferred in that case is a set of essentially purified saRNA1 and taRNA2 ribonucleic acids as provided herein wherein said 3' UTR, or other regulatory or responsive elements, in taRNA2 is derived from a virus having a bi-segmented genome, such as a virus selected from the group of *Nodaviridae.* In one specific embodiment as provided herein it is preferred that said virus is selected from the group of *Alphanodaviridae,* for example wherein said virus is Nodamura virus (NoV). In a preferred embodiment, the invention discloses a set of essentially purified saRNA1 and taRNA2 ribonucleic acids as provided herein wherein said taRNA2 is provided with a ribonucleic acid encoding an antigenic polypeptide or protein.

The invention also discloses a set of ribonucleic acids according to the invention for use in the preparation of a formulation suitable for transfection, preferably of said set. Transfection of nucleic acid into cells typically applies to in general chemical systems (i.e. preferably non-viral systems) that transiently open pores into the cell membrane to permit the entry of nucleic acids. Transfection not only includes chemical-based products such as calcium phosphate, cationic polymers (PEI), peptides, polymersomes, poly-lactides or liposomes, DOTAP and DDA, but also non-chemical methods such as electroporation. A cell, preferably an isolated cell or a cell in vitro or in an in vitro cell culture, that has incorporated foreign nucleic acid through a process of transfection of a set of nucleic acids according to the invention is typically called a transfectant or transfectant cell, as provided herein. In a preferred embodiment, the invention discloses a transfectant cell, more preferably the invention discloses an (in vitro) transfected cell culture or a collection of transfectant cells derived from in vitro cell culture derived from such a transfectant cell. Such an isolated transfectant cell, cell culture of collection of cells derived from such culture are typically useful for therapeutical purposes such as in oncologic therapy or for vaccination purposes. Preferably, the invention discloses at least on in vitro transfectant cell or transfectant cells in culture or transfected cells, preferably collected for therapeutic application, said at least one transfectant cell typically comprising a set of ribonucleic acids of the split RepRNA system (saRNA1 and taRNA2) according to the invention. A human provided with such a split RepRNA system is typically not identified as a transfectant herein.

A formulation suitable for transfection comprising a set of nucleic acids as provided herein is identified as a transfection formulation and, typically advantageous of the invention, transfection of RNA with such a formulation is considered always transient and does not lead to incorporation of said set of nucleic acids in the genome of said transfectant. It is herein provided to produce separate transfection formulations of each version of saRNA1 and taRNA2 contemplated, and then in a later phase mix said separate formulations into the desired transfection formulation, selecting the desired ratio of saRNA1 to taRNA2 in this second phase. Alternatively, it is also provided to mix each version of saRNA1 and taRNA2 contemplated already first, selecting the desired ratio of saRNA1 to taRNA2 first, and then produce the desired transfection formulation. As choice of transfection solution may vary with the choice of the desired animal species to be treated with the split RepRNA formulation, various transfectant solutions may be tested with in cells of various species to determine best effects. The invention also discloses a trans-amplifying-replicon (RepRNA or taRNA) system for transcription of at least one desired fragment of a gene-of-interest (GOI) in a cell, said system comprising an essentially purified ribonucleic acid encoding a replicase and an essentially purified ribonucleic acid encoding said GOI. It is preferred that the nucleic acid encoding the replicase is preferably produced separately from the nucleic acid encoding the desired protein or peptide encoded by said GOI, allowing ease of production in comparison to non-segmented replicons. It is preferred that said replicon system at least comprises a bi-segmented trans-amplifying- (ta) ribonucleic acid (RNA)-replicon system. It is furthermore preferred that said cell is a mammalian cell. It is furthermore preferred that said transcription (in said cell) is followed by translation of said GOI (in same said cell). It is more preferred that said replicase is an RNA-dependent-RNA-polymerase (RdRp). It is moreover preferred that each of nucleic acid encoding the desired replicase as well as nucleic acid encoding the at least one GOI are each located on a separate RNA-molecule. Said molecule harboring or encoding the replicase herein identified as saRNA1 and said molecule harboring the at least one GOI herein identified as taRNA2. As a preferred replicon according to the invention is at least bi-segmented with saRNA1 and taRNA2 each located at separate molecules, bi-segmented at least identifies saRNA1 and taRNA2 unambiguously as being "encoded out-of-frame". It is moreover preferred that said replicase is derived from a virus, preferably a positive-sense RNA virus, more preferably a virus from the group of *Nodaviridae,* or alternatively, selected from the group of *Alphanodavirus, Betanodavirus,* and/or *Gammanodavirus.* Instead of dispensing or discarding the taRNA2-molecule as is practiced by Biddlecome et al (ibid), the present disclosure employs the bi-segmented structure of Nodaviridae to the fullest, by using the self- and trans-amplifying facilities in an saRNA1-molecule and providing it together with at least one taRNA2-molecule with the gene-of-interest, preferably by replacing at least a substantial part of the capsid ORF in the taRNA2-molecule with the GOI instead. It is typically preferred to use the function of the subgenomic RNA3 to the fullest as well, allowing said B-protein(s) to facilitate RNA-accumulation and supporting cell viabilities to their best abilities, therewith de-risking disabling the useful functionalities of the subgenomic proteins (B1 and/or B2) derived from said RNA3 as seen with Biddlecome et al (ibid) who observed only modest T-cell responses, for the solely saRNA1-based viral-like-particle (VLP) vaccine platform.

### Figure legends

### Figure 1

(Taken from Minnaert et al Strategies for controlling the innate immune activity of conventional and self-amplifying mRNA therapeutics: Getting the message across. Adv Drug Deliv Rev. 2021 Sep;176:113900.).

Schematic representation of the structure of conventional mRNA and the structure and intracellular amplification of self-amplifying mRNA (saRNA) (this represents an alphavirus replicon). (Left) Conventional mRNA consists of five critical elements: a 5' cap structure (m7GpppN), a 5' untranslated region (5' UTR), the gene of interest (GOI), a 3' untranslated region (3' UTR) and a poly(A) tail. After conventional mRNA delivery into the cell cytosol, the encoded protein is produced directly. (Right) saRNA consists of a 5' cap structure, a 5' UTR, the sequences of viral non-structural proteins (nsP1-4), a subgenomic promoter (SGP), the GOI, a 3' UTR and a poly(A) tail. (A) When saRNA arrives in the host cell cytosol, nsP1-4 is translated and forms the early replication complex (B) that generates a complementary negative-sense RNA strand from the original saRNA. (C) In a later phase, the early replication complex is cleaved into the individual nsPs. These form the cleaved replicase that uses the negative-sense RNA strand as a template to generate new copies of the original genomic RNA and recognizes the SGP and triggers the production of an enormous amount of subgenomic RNAs, (D) which eventually leads to the production of the protein of interest.

### Figure 2

(Taken from Beissert et al. A Trans-amplifying RNA Vaccine Strategy for Induction of Potent Protective Immunity. Mol Ther. 2020 Jan 8;28(1):119-128).

Expression from Trans-amplifying RNA in conjunction with saRNA-delivered replicase activity Is found considerably less efficient as expression from self-amplifying RNA. To compare two alternative taRNA split-vector approaches, Beissert et al electroporated BHK-21 cells with increasing equimolar amounts of these vector systems encoding luciferase as reporter; a conventional saRNA single-vector-encoding luciferase was used as a control. Expression of luciferase from conventional saRNA was very high and largely dose independent. Expression levels achieved by taRNA driven by nrRNA-REPL were comparable to those of the saRNA single vector system (except at the lowest dose). In contrast, expression levels achieved by taRNA in conjunction with saRNA-REPL did not reach this benchmark.

(A) RNA vaccine platforms in this study. Self-amplifying RNA (saRNA, left) is a single-vector system encoding replicase that acts in cis to amplify the complete saRNA, and a second open reading frame with a gene of interest (GOI). Trans-amplifying RNA system (taRNA, right) is a split-vector system consisting of a transreplicon (TR), which encodes the GOI only, and a second RNA delivering alphaviral replicase. TR-GOI is amplified in trans by a replicase either encoded on saRNA (saRNA-REPL) bearing an irrelevant transgene (iTG) or on non-replicative mRNA (nrRNA-REPL). RNA structural elements for replication of saRNA or TRs are in conserved sequence elements (CSE) at the 5' and 3' ends and in the subgenomic promotor (SGP) upstream of the GOI. The UTRs (5'-human alpha globin UTR [hAG] and 3'-AES/mtRNR1-UTR) in the nrRNA-REPL lack viral CSE function and therefore do not promote replication by replicase. All RNAs are capped (C) and bear poly(A) tails (pA). (B) Expression capacity and (C) cytotoxicity of saRNA and taRNA in BHK-21 cells. Vectors bearing firefly luciferase (FLUC) as GOI were electroporated into BHK-21 cells (3 × 106 cells per sample) in equimolar amounts. (B) Total luciferase expression was measured for 72 h and approximated by calculating the area under the curve (AUC) for each sample. (C) Relative viability of cells was assessed by measuring viability after 48 h and normalization to the mock control. Mean and SD of three independent experiments are shown. Unpaired Student's t test was performed (*p < 0.05; **p < 0.01; ***p < 0.001; not annotated p > 0.05).

### Figure 3

(Taken from Sahul Hameed et al., (2019): ICTV Virus Taxonomy Profile: Nodaviridae, Journal of General Virology, 100, 3-4.). Here Flock House virus (Alphanodavirus) genome organization and strategy of replication is shown. The family Nodaviridae includes two enera, Alphanodavirus and Betanodavirus. Virions are non-enveloped and spherical in shape with icosahedral symmetry (T=3) and diameters ranging from 25-33 nm. The genome consists of two molecules of positive-sense ssRNA: RNA1 and RNA2. The virion capsid consists of 180 protein subunits arranged on a T=3 surface lattice. Alphanodaviruses generally infect insects whereas betanodaviruses are pathogens of fish. Typically, they may also replicate in yeast and mammalian cells.

**Table Nodaviridae. Characteristics of members of the family Nodaviridae.**

| Characteristic | Description |
|---|---|
| Typical member: | striped jack nervous necrosis virus (RNA1: AB056571; RNA2: AB056572) species Striped jack nervous necrosis virus, genus Betanodavirus |
| Virion | Non-enveloped spherical particles, 25-33 nm in diameter, with or without surface projections |
| Genome | Bi-partite positive-sense, ssRNA of 3.1 kb (RNA1) and 1.4 kb (RNA2) with 5'-terminal caps but without poly(A) tails |
| Replication | Cytoplasmic within virus-induced invaginations on the outer mitochondrial membrane |
| Translation | From capped genomic and subgenomic RNAs |
| Host range | Natural hosts are insects (Alphanodavirus) or fish (Betanodavirus) |
| Taxonomy | Two genera, each including four or more species |

Virion Mr is about 8-9 x 106; S20W is about 135-145. Virion buoyant density in CsCl ranges from 1.30 to 1.36 g cm-3. Virions are stable to pH values ranging from 2 to 9 and are resistant to heating at 56 oC for 30 minutes. Infectivity of virions is stable following chloroform extraction.

The genome consists of two molecules of positive-sense ssRNA: RNA1 and RNA2. Both RNA molecules are required for infectivity, and they are encapsidated in the same virus particle. Both molecules are capped at their 5'-ends and lack poly(A) tails at their 3'- ends. Both molecules typically contain conserved sequence elements (CSE) at the 5' and 3' ends for replication. The RNA content of the virion is about 16%. The capsid of virion consists of 180 protein subunits arranged on a T=3 surface lattice. Each subunit is composed of a single CP or the two products of its cleavage.

Virus replication is cytoplasmic. Infected cells contain ssRNAs corresponding to RNA1 and RNA2, as well as the subgenomic RNA3 (387 nucleotides) which derives from the 3'-terminus of RNA1 and is not packaged into virions. In addition to RNA-dependent RNA polymerase (RdRP) activity, protein A binds to and drives invagination of the outer mitochondrial membrane to provide the compartment where RNA replication occurs. RNA3 encodes either one or two proteins; protein B2 (11 kDa) is encoded by all nodaviruses in a reading frame overlapping that of protein A and is a suppressor of RNA interference. Some nodaviruses also express protein B1 (11 kDa) which corresponds to the C-terminal region of protein A and is of little-known function. In Betanodavirus, B1 is assumed an early expression protein that has an anti-necrotic cell death function which reduces the mitochondrial membrane potential loss and enhances viral host cell viability. Maturation of non-infectious provirions involves the autocatalytic cleavage of protein α into proteins β (39 kDa) and γ (4 kDa).

Nodaviruses can be classified based on genetic diversity of the RNA2 segment. Isolates with less than 80% identity at the nucleotide level of RNA2 and less than 87% identity at the amino acid level are classified as different species. The CP aa sequences of the alphanodaviruses are only about 10% identical to those of the betanodaviruses. A third group of (gamma)nodaviruses has been considered by some authors. This group includes the prawn nodaviruses Macrobrachium rosenbergii nodavirus, Penaeus vannamei nodavirus, covert mortality nodavirus and Farfantepenaeus duorarum nodavirus, infecting prawn and shrimp.

Other Noda-like viruses with a split (bi-segmented) genome are found with the tetravirus, omegatetraviruses such as Nudaurelia capensis omega virus (NCoV) and Helicoverpa armigera stunt virus (HaSV) contain bipartite positive-sense ssRNA genomes, but their RNAs are about twice the size of nodavirus RNAs and they have no 3'-terminal blockage. Tetraviruses also have larger capsids with T=4 icosahedral symmetry. Tetraviruses are a group of relatively unknown small RNA viruses with particles that display a characteristic T=4 capsid architecture. Tetraviruses are classified into three families, the Alphatetraviridae, Permutotetraviridae and Carmotetraviridae, according to the divergent characteristics of their respective viral replicases. Tetraviruses generally infect the larvae of lepidopteran insect species, many of which are important agricultural pests and, until recently, were thought to have an unusually narrow host range and tissue tropism. The development of experimental systems for studying the viral infectious life cycle in tissue culture has permitted the extension of the virus host range to mammalian cells and plants.

### Figure 4

### Top

Schematic presentation of plasmid vector pRNA2 for expressing GOI RNA2:
5' CSE requirements relating to conserved sequence elements (CSE, depicted as C in the figure): at least 3 nt, more preferably at least 19 nt, more preferably at least 44 nt, more preferably at least 52 nt, more preferably at least 55 nt.

3' CSE requirements: at least 50 nt, more preferably at least 100 nt, more preferably at least 176 nt, more preferably at least 236 nt, most preferably at least 270 nt

### Bottom

Results of transfection studies with essentially purified split RepRNA system allowing illustration of CSE length in taRNA2 for expressing GOI. It is preferred that said CSE is derived from a virus selected from the group of Nodaviridae, preferably from the group of Alphanodavirus, more preferably from a Nodamura virus (NoV) or Flock house virus (FHV).

To illustrate suitable 5' CSE length various NOD-virus derived essentially purified taRNA2 constructs carrying the GOI encoding firefly luciferase (FLUC) were prepared having a 5' CSE of 3, 19 and 52 nt (identified herein as RNA2_3FLUC270, RNA2_19FLUC270, and RNA2_52FLUC270, respectively, and transfected together with essentially purified saRNA1 in BHK21 cells, using 250ng saRNA1 and/or 250 ng taRNA2 /well.

Cationic lipid-mediated transfection, such as with lipofectamine is one of the most popular methods for introducing foreign genetic material into cells. Although first generation of lipid-based transfection reagents relied on artificial liposomes that could envelop nucleic acids and then fuse with the cell membrane to deposit their cargo inside, newer cationic lipid-based reagents spontaneously form condensed nucleic acid-cationic lipid reagent complexes via electrostatic interactions between the negatively charged nucleic acid and the positively charged head group of the synthetic lipid reagent. These complexes are believed to be taken up by the cell through endocytosis and then released in the cytoplasm. Other useful lipid nanoparticle formulations for mixing with a ribonucleic acid according to the invention are:
Formulation 1 admixed with a saRNA1+taRNA2 according to the invention.
   - ALC-0315
   - DSPC
   - Chol
   - ALC-0159

### ALC-015 (4-hydroxybutyl)azanediyl)bis(hexane-6,1-dihy)bis(2-hexyldecanoate)

### DSPC (distearoylphosphatidylcholine)

### Chol (cholesterol)

### ALC-0159 (2-[(polyethylene glycol)-2000]-N2N ditetradecylacetamide)

Formulation 2 admixed with a saRNA1+taRNA2 according to the invention.
- SM-102
- DSPC
- Chol
- DMG-PEG2k

### SM-102 (heptadecane-9-yl 8-((2-hydroxyethyl) (6-oxo6-(undecyloxy) hexyl) amino) octanoate)

### DSPC (distearoylphosphatidylcholine)

### Chol (cholesterol)

### DMG-PEG2K (1-monomethoxypolyethylneglycol-2,3-dimyristylglycerol with polyethylene glycol of average MW 2000)

Formulation 3 admixed with a saRNA1+taRNA2 according to the invention.
- DOPE
- DOTAP and
- DMG-PEG2000
- 49:49:2 M ratio

### DOPE (1,2-dioleoyl-sn-3-phosphoethanolamine), ,

### DOTAP (1,2-dioleoyl-3-trimethylammonium-propane)

### DMG-PEG2000 (1,2-dimyristoyl-sn-glycero-3-phosphoethanolamine-N-[methoxy(polyethylene glycol)-2000])

Formulation 4 admixed with a saRNA1+taRNA2 according to the invention.
- DSPC
- Chol
- PEG-DMG 2000
- DLinDMA
- 10:48:2:40 molar ratio

### DSPC (1, 2-Diastearoyl-sn-glycero-3-phosphocholine)

### Chol (cholesterol)

### PEG DMG 2000 (1,2-dimyristoyl-sn-glycero-3-phosphoethanolamine-N-[methoxy(polyethylene glycol)-2000])

### DLinDMA (1,2-dilinoleyloxy-3-dimethylaminopropane)

### An 8:1 N:P molar ratio (nitrogen on DlinDMA to phosphate on RNA) and 100 mM citrate buffer (pH 6) were used for the formulations.

Once in the cell, transfected DNA is translocated to the nucleus to be expressed by a yet unknown mechanism, while RNA or antisense oligonucleotides skip the translocation step and remain in the cytoplasm. The commercialized agent lipofectamine is composed of DOPE and 2,3-dioleyloxy-N-[2-(sperminecarboxamido)ethyl]-N,N-dimethyl-1-propanaminium trifluoroacetate (DOSPA), a cationic lipid containing quaternary ammonium and spermine. Lipofectamine protocols have been optimized to deliver mRNA in diverse cell types, including alveolar cells, cardiac muscle cells and pluripotent stem cells. 2-(((((3 S,8S,9S, 10R, 13R, 14S, 17R)-10, 13-dimethyl-17-((R)-6-methylheptan-2-yl)-2,3,4,7,8,9,10,11,12,13,14,15,16,17-tetradecahydro-1H-cyclopenta[a]phenanthren-3-yl)oxy)carbonyl)amino)-N,N-bis(2-hydroxyethyl)-N-methylethan-1-aminium bromide (BHEM-Cholesterol) was developed by modifying the head structure of 3β-[N-(N',N'-dimethylaminoethane)-carbamoyl]cholesterol (DC-Cholesterol) with hydroxyl groups to improve fusion with cellular membranes and used to produce lipid nanoparticles and lipofectamine protocols have been optimized to deliver mRNA in diverse cell types, including alveolar cells, cardiac muscle cells and pluripotent stem cells. Other lipid nanoparticle compounds for RNA delivery are known as well (Hou, X., Zaks, T., Langer, R. et al. Lipid nanoparticles for mRNA delivery. Nat Rev Mater 6, 1078-1094 (2021). Transfection was achieved by preparing a lipid nanoparticle premix A of optimem and lipofectamine and separately a lipid nanoparticle premix B of the total amount of RNA and optimem. In a next step mix A and mix B were pooling together, admixed and incubation at room temperature for 5 minutes. 30µl of this final lipid nanoparticle mixture or formulation was added to the cells, that were seeded one day prior to transfection at 30.000 cells/well, as a final step in the transfection. The cells were further incubated at 37°C for the remainder of the experiment.

Increasing the 5' UTR/CSE length increased luminescence, indicating increased expression of GOI. Similarly, to illustrate suitable 3' CSE length various Noda-virus derived essentially purified RNA2 constructs carrying the GOI encoding luciferase (FLUC) were prepared having a 3' UTR/CSE of 270, 236, 101, 50 and 25 nt (identified as RNA2_52FLUC270, RNA2_52FLUC236, RNA2_52FLUC100, RNA2_52FLUC50, and RNA2_52FLUC25, respectively, together with essentially purified RNA1 in BHK21 cells. Increasing the 3' CSE length increased luminescence, indicating increased expression of GOI. Whereas all constructs allowed functional GOI expression, RNA2_52FLUC270 showed very useful results and was selected for further use in the split RepRNA system and pharmacons herein provided herein. Nodamuravirus pharmacons (following general findings as shown in table 1) having 5' UTR/CSE length of 19 and 3'-UTR/CSE length of 236 (respectively) show useful replication and translation, those with 5'-UTR/CSE and 3'-UTR/CSE of 19 and 270 are further improved, those with 44 and 270 are more improved, those with 5'-UTR/CSE 52 or 55nt and 270 3'-UTR/CSE of show distinctly improved expression by means of adjusted Kozak sequences.

### Figure 5

Typical process flow diagram for RNA drug substance production (aka. active ingredient production, bulk production, or primary manufacturing) and drug product manufacturing (aka. fill-to-finish or secondary manufacturing). The RNA drug substance is synthesized in the production bioreactor using the in vitro transcription reaction for example with the T7 or SP6 RNA polymerase enzyme and is 5' capped co-transcriptionally. After RNA synthesis, the template DNA is digested using the DNAse I enzyme. Next, the downstream purification starts. Essential purification to clinical or therapeutically grade material preferably comprises tangential flow filtration (TFF) where the RNA molecule is retained by the filter and the other, smaller components of the reaction mix flow through the TFF filter. Next, the protein enzymes can be further removed using a chromatography unit operation, such as ion exchange chromatography, CaptoCore 700 chromatography, or hydroxyapatite chromatography. Next, the buffer is replaced for the formulation buffer in a second TFF step and then the solution is sterile filtered. This solution is then subjected to quality analyses (QA) control procedures with (critical) QA requirements as provided herein and further exemplified in van de Berg D, Kis Z, Behmer CF, et al. Quality by design modelling to support rapid RNA vaccine production against emerging infectious diseases. NPJ Vaccines. 2021; 6:65.

After QA is approved, the solution then enters the lipid nanoparticle (LNP) encapsulation unit operation for RNA drug substance production. After formulation, the LNP encapsulated RNA solution enters a third TFF for diafiltration then an optional dilution step is carried out followed by a sterile filtration operation. The sterile LNP-encapsulated RNA solution is then transferred to the fill-to-finish section. There, the solution can be further diluted and sterile filtered. Next, the formulated RNA solution again undergoes quality control.
CQAs/QAs for lipid nanoparticles (LNPs)
Identification and quantification of lipids and their degradants (chemical stability)
Separation of free drug and quantification of encapsulation efficiency
Nanoparticle morphology
Nanoparticle lamellarity
Nanoparticle size distribution
Nanoparticle surface charge (Zeta potential)
Lipid fusion (physical stability)
Nanoparticle aggregation (physical stability)
In vitro drug release
^{∗} Acceptance criteria will depend on specifics of the formulation selected as for example specified in Fan Y, Marioli M, Zhang K. Analytical characterization of liposomes and other lipid nanoparticles for drug delivery. J Pharmac Biomed Anal. 2021;192: e113642.

Then it is filled into vials or other containers. The vials are then capped, sealed, inspected, labelled, and packaged into secondary and tertiary packaging.

### Figure 6

Schematic presentations of the different deletions made in the NoV RNA2 by combinations of different 5' and 3' flanking regions, as shown. The wild type RNA2 contains a cap 0 structure at its 5'end, a 19-nucleoside long UTR, followed by the start codon of the capsid gene. Coding sequence of the capsid spans from nucleoside 20-1219 which is followed by the 3' UTR which does not contain a polyA tail. To express a gene of interest (GOI) instead of the capsid protein, different deletion mutants of RNA2 have been generated and replaced by the gene of interest. Fragments of the 5' end of RNA2 with different length have been generated: a fragment having only the first 3 nucleosides from the 5' UTR followed by the ATG start codon of the gene of interest (GOI); another fragment contains the first 19 nucleosides followed by the start codon of the GOI; another fragment containing the first 44 nucleosides of the 5' end of NoV RNA2; worthwhile to mention that nucleoside 42-44 is representing a Kozak sequence improving the protein translation from the GOI; another fragment contains the first 52 nucleotides of the 5' NoV RNA2 end, in another construct 2 Cs were included directly after nucleoside 52, generating a Kozak sequence for improved protein translation, and another fragments contains the first 55 nucleoside; like for fragment 44, the sequence immediately before the start codon represents a Kozak sequence which is beneficial for protein expression. At the 3' end we also tested an incremental increase of the nucleoside sequence derived from NoV RNA2. The length of the different sequences derived from the 3' end of NoV RNA2 were 25, 50, 100, 236, and 270 nucleosides. Combinations of different fragments derived from the 5' and 3' end from NoV RNA2 with a GOI (firefly luciferase) inserted in between the 5' and 3' fragments were generated. The following constructs are produced and identified herein as example GOI expressing RNA2 derivatives: 3Fluc270, 19Fluc270, 44Fluc270, 52Fluc270, 52ccFluc270, 55Fluc270, 52Fluc236, 52Fluc100, 52Fluc50, 52Fluc25.

Transfection was achieved by preparing a lipid nanoparticle premix A of optimem and lipofectamine and separately a lipid nanoparticle premix B of the total amount of RNA and optimem. In a next step mix A and mix B were pooled together, admixed and incubation at room temperature for 5 minutes. 30µl of this final lipid nanoparticle mixture or formulation was added to BHK-21 cells, that were seeded one day prior to transfection at 30.000 cells/well, as a final step in the transfection. The cells were further incubated at 37°C for the remainder of the experiment.

Increasing the 5' CSE length increased luminescence, indicating increased expression of GOI. Similarly, to illustrate suitable 3' CSE length various Noda-virus derived essentially purified RNA2 constructs carrying the GOI encoding luciferase (FLUC) were prepared having a 3' CSE of 270, 236, 101, 50 and 25 nt (identified as RNA2_52FLUC270, RNA2_52FLUC236, RNA2_52FLUC100, RNA2_52FLUC50, and RNA2_52FLUC25, respectively, together with essentially purified RNA1 in BHK21 cells. Increasing the 3' CSE length increased luminescence, indicating increased expression of GOI. Whereas all constructs allowed functional GOI expression, RNA2_52FLUC270 showed best results.

### Figure 7

Comparison of essentially purified split RepRNA system effects at various transfection concentrations in cell lines BHK21 (ATCC-CCL-10) (top) of hamster or PK15 (ATCC-CCL-33) (bottom) of pig origin.

In this experiment saRNA1 and taRNA2 were mixed prior to transfection or taRNA2 was transfected alone in 2 different concentrations, using two different cell lines. Transfection was achieved by preparing a mix A of optimem and lipofectamine and separately a mix B of saRNA1 and/or taRNA2 and optimem. In a next step mix A and mix B were pooled together and incubated at room temperature for 5 minutes. 30µl of this final mixture was added to the cells. And the cells were further incubated at 37°C for the remainder of the experiment.

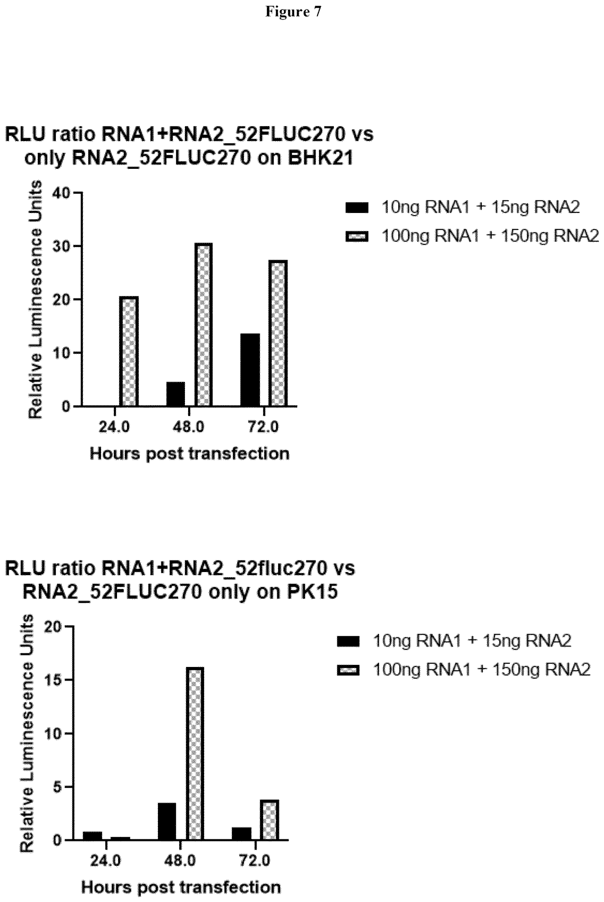

As output the amount of luminescence was measured as RLU (Relative Luminescence Units) by adding Luciferase one glow substrate to 25µl of cell lysate and measuring the signal using a Tecan plate reader. The ratio between single transfected and co-transfected taRNA2 was calculated and depicted in Figure 7.

### Figure 8

### RLU DATA FOR IVT (in vitro transcription), PERFORMED AT 16 AND 37°C with T7 PROMOTOR

The IVT reactions were performed for 2 hours according to the manufacture's protocol (MEGAscript^{®} Kit catalog number AM1330 (publication number 1330M, revision G) produced by ambion life technologies) with the exception of the incubation temperature (16°C and 37°C). Following IVT the RNA was first quantified using the quantiFluor^{®} RNA System protocol from Promega( Catalogue number E3310) and subsequently purified using the GeneJETRNA Purification Kit (K0731). After purification any dsRNA was removed by Cellulose-Ethanol treatment and RNAs were further essentially purified with the AKTA GO FPLC system using a Sepharose column. As a final step the RNA was capped using the One-Step Capping and 2'-O-Methylation (New England BioLabs; M0366).

Capped RNA from both the 16°C and 37°C IVT reaction was used for the transfection of BKH-21 cells. For the transfection 60.000 cells/well were seeded 1 day earlier to reach sufficient confluency. Transfection was achieved by preparing a mix A of optimem and lipofectamine and separately a mix B of 100ng saRNA1 and/or 150 ng taRNA2 and optimem. In a next step mix A and mix B were pooled together and incubated at room temperature for 5 minutes. 30µl of this final mixture was added to the cells. And the cells were further incubated at 37°C for the remainder of the experiment.

Samples were taken at different timepoints and the ratio between the wells with RNA2 alone or RNA1 and RNA2, produced with the IVT at its specific temperature, was calculated.

### Figure 9

### SP6 VS T7 PROMOTOR

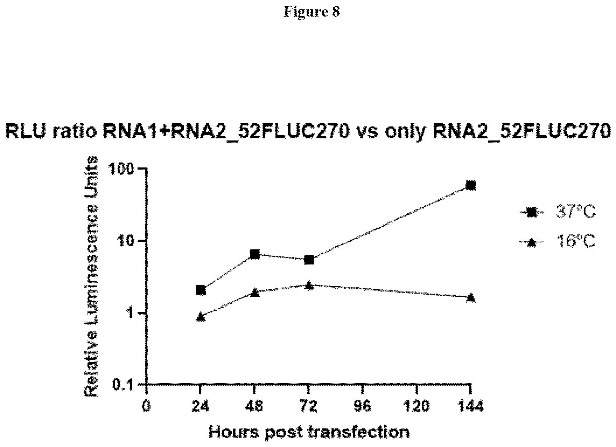

This experiment shows the direct comparison of RNA generated either by T7 or SP6 polymerase. Plasmid DNA vectors were generated, encoding the sequences of the NoV RNA1 or NoV RNA2 comprising the firefly luciferase (gene of interest) under direct control of either the T7 or the SP6 polymerase promoter. RNA was generated by IVT using either the T7 or the SP6 polymerase. The RNA was capped, purified and used for transfecting BHK-21 cells and luciferase activity was measured. Transfection was performed in a similar way as described in Figure 8.

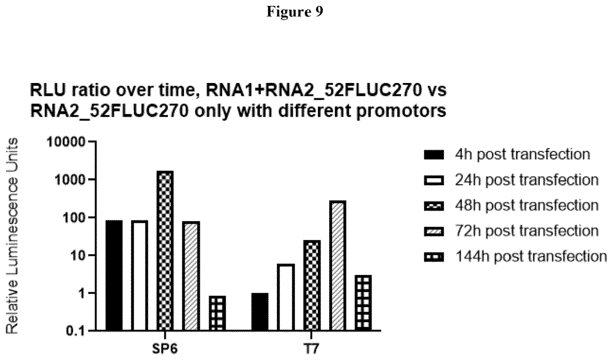

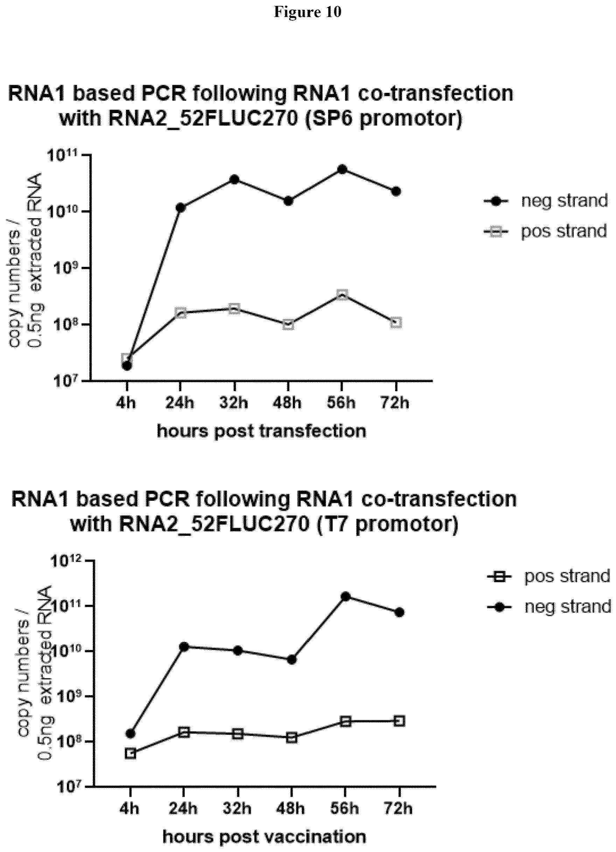

The ratio between single transfected and co-transfected taRNA2 was calculated and depicted in Figure 9. The Area under the curve shows that SP6 has 3x higher total peak area than T7 (49209 vs 15016 total peak area)

### Figure 10

### PCR RESULTS RNA1 AFTER RNA1+RNA2 CO-TRANSFECTION

For this experiment RNA1 and RNA2_52FLUC270 (encoding the luciferase gene) were prepared either by T7 or SP6 polymerase IVT as described above, capped, and purified. BHK21 cells were co-transfected with sRNA1+RNA2_52FLUC270 following the protocol described above. At the indicated time points the cells were lysed and the positive and negative strand of RNA1 was quantified by RT-PCR, and the transfection was conducted in a similar way as described for figure 8, with co-transfected sRNA1+RNA2_52FLUC270, using either SP6 or T7 as the promotor. The PCRs performed were based on RNA1.

### Top Results with SP6 produced material

Between 4-24hours post transfection a 3log10 increase in copy numbers of negative strand RNA is observed, indicating active RNA replication. The quantity of negative RNA remained stably until the last timepoint sampled. Although lower in quantity, also the positive strand PCR increases between 4-24hours post transfection, maintaining level until the end of the experiment.

### Bottom Results with T7 produced drug material

Between 4-24hours post transfection a 2log10 increase in copy numbers of extracted RNA is observed for the negative strand PCR, which is the first step, indicating active translation/transcription, which is further increasing until the last timepoint sampled. Although lower in quantity, also the positive strand PCR increases between 4-24 hours post transfection, maintaining level until the end of the experiment.

### Figure 11

Microscope pictures showing that saRNA1+RNA2 gives an FLUC-antigen/FLUC-antibody interaction at 24+48+72h post transfection.

BHK-21 cells were seeded at 60.000 cells per well 1 day prior to transfection. Transfection itself was performed, identical for all samples, independent on RNA sample/control, according to the same method as described for Figure 8.

Controls included: untransfected cells (negative control), 1 RNA (Luciferase control RNA sp6) and 1 DNA control (pcDNA3.1_luciferase_3'UTRbglobin_polyA) were used.

Samples included: RNA2_52FLUC single or RNA1+RNA2_52FLUC270 co-transfected cells.

### Protocol for intracellular staining of the cells

Collect 2^{∗}10^5 cells.
2.Centrifuge for 5 min at 1500 rpm at RT
3. Wash with 100 µl of of PBS
4.Centrifuge for 5 min at 1500 rpm at RT
5.Incubate with 100 µl FIX for 10 min at 4°C
6.Centrifuge for 5 min at 1500 rpm at RT
7.Add 100 µl of antibody diluted 1 to 100 in PER (Anti-Luc_ab185924; used 1:200) 8.Incubate for 30 minutes at 4°C
9.Centrifuge for 5 min at 1500 rpm at RT
10.Wash with 100 µl of MACS buffer
11. Add 100 µl of secondary antibody diluted 1 to 100 in MACS buffer (A32731, life technologies (goat antirabbit 488_used 1:200)
12.Incubate for 45 minutes at 4°C
13.Centrifuge for 5 min at 1500 rpm at RT
14.Wash with 100 µl of MACS buffer (twice)
15.Re-suspend in 100µl of PBS
16. spot 2µl cells on microscope slides using the Cytospin centrifuge( TS-Cytospin4manual.pdf (marshallscientific.com)) for 5 minutes at 800xg at RT
17. close the cell spot with coverslip using the specific mountant (ProLong^{™}

### Diamond Antifade Mountant (thermofisher.com))

### Fixation/PermeablizationKit (bdbiosciences.com)

### Figure 12

Incorporating the 5' KOZAK sequence improves expression of GOI. For this experiment RNA1 and RNA2 were prepared using SP6 polymerase in the IVT as described above, capped, and purified. For RNA2 either 52FLUC270, without a 5'KOZAK was used or 44FLUC270, with a KOZAK sequence. BHK21 cells were co-transfected following the protocol described above and luciferase activity was measured.

### Figure 13

The RNAs (RNA1, RNA52FLUC270) were produced with either SP6 or T7 promoter and used, after PCR purification, as template for IVT reaction at 37°C for 2hours. The RNAs were purified following protocol (GeneJet column, Cellulose treatment, FPLC running, check fraction quality, RNA concentration, capping reaction).

### Figure 14

### SP6 promoter

Check of RNAs (RNA1 and RNA52FLUC270, produced with SP6 promotor according to previously described protocol) fractions quality after FPLC purification yield of RNA purification

### Figure 15

### T7 promotor

Check of RNAs (RNA1 and RNA52FLUC270, produced with T7 promotor according to previously described protocol) fractions quality after FPLC purification yield of RNA purification

### Figure 16

Microscope pictures showing that saRNA1+RNA2_52HA270 gives an HA-antigen/HA-antibody interaction at 48h post transfection, as can be seen by the positive staining at the surface of the cells, where HA is normally expressed. The results here show A RepRNA vaccine system according to the invention to provide a RepRNA GOI-derived proteinaccording to the invention, preferably an antigenic protein (such as FLUC, see figure 11), more preferably a desirable vaccine antigen (such as HA, see figure 17), expressed in a host cell, and being recognized by the desirable antibody, in figure 17 an anti-HA-antibody. The hemagglutinin (HA) protein of influenza virus is responsible for receptor binding and membrane fusion and is found in its trimeric form at the virus surface. In an influenza virus infected cell, the HA is translated into the endoplasmatic reticulum (ER) where the protein is glycosylated and folded. Subsequently, the protein is transported along the trans-Golgi network (TGN) to the cell surface, where it assembles in larger arrays. As can be observed in figure 17, virus progeny material is budding from the cellular membrane, exposing HA-antigen to the immune system, and allowing specific antigen-antibody binding, formation, and B-cell-activation to occur. The invention here (as also shown in figure 17) discloses the HA-vaccine antigen for use in a flu vaccine, HA being the main vaccine antigen component of influenza (flu) vaccines. Indeed, current live flu vaccines foremost rely on specific induction and expression of this major surface protein in cells of a vaccinated subject and current killed vaccines foremost rely on specific presentation of this major surface protein to cells of a vaccinated subject. Both killed as well as live human vaccines are commonly standardized to induce anti-HA antibodies as the primary correlate of protection, as elevated levels of serum anti-HA antibody are correlated with elevated resistance to influenza infection.

BHK-21 cells were seeded 1 day prior to transfection. Transfection itself was performed, identical for all samples, independent on RNA sample/control, according to the same method as described for Figure 8. Untransfected cells (negative control) or RNA1+RNA2_52HA270 co-transfected cells were used and stained according to the protocol given below.
1) Fixation with 4% PFA at 2 days post transfection
2) Permeabilization of cells with 0.1% TX-100
3) Blocking with 1% milk
4) Primary - Influenza A H1N1 HA(A/California/04/2009) rabbit polyclonal antibody was added and tested @ 1:100 and 1:1,000 dilution
5) Secondary - Goat anti-Rabbit IgG, Alexa Fluor^{™} 488 @ 1:500 dilution was added as a next step
6) Images were taken on Leica Thunder.

### Figure 17

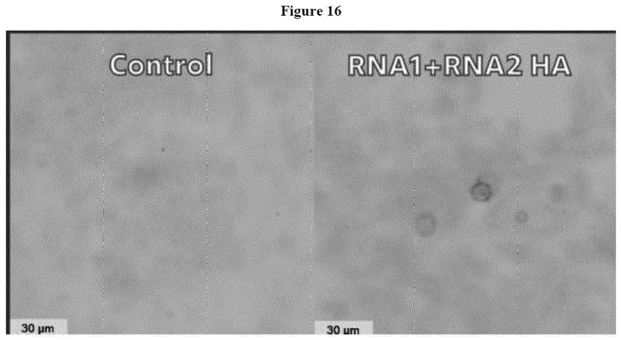

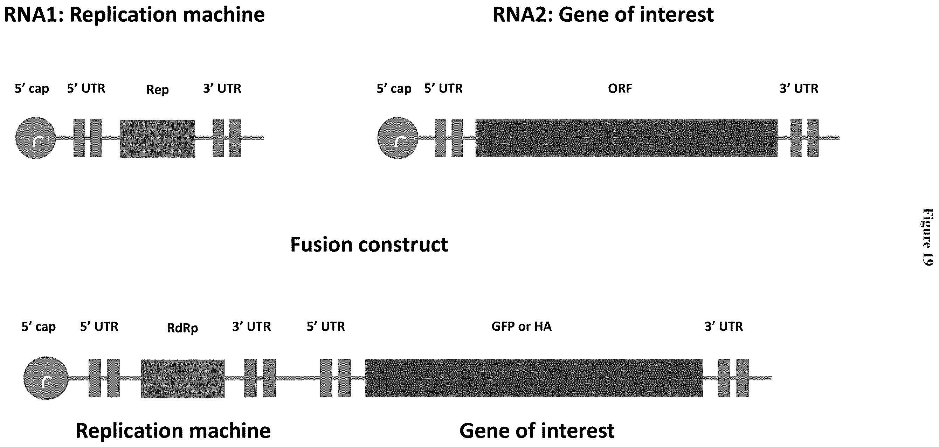

Microscope pictures from a 2^{nd} (confirmatory, figure 17) experiment showing that saRNA1+RNA2_52HA270 gives an HA-antigen/HA-antibody interaction at 48h post transfection. Experiment was performed as described for Figure 16. In the control experiments with untransfected cells no staining can be detected. In contrast, in the cells transfected with RNA1 and RNA2_52HA270 the HA is clearly detected in multiple cells, proving the expression of immune-reactive HA by the RepRNA. The identification of the cellular location of HA (Figs. 18 e-h) demonstrated that the expression of HA after RNA1+RNA2_52HA270 transfection (Figs. 18 e+f) is identical to the pattern observed after infection of cells with the influenza A virus (Fig. 19g+h) in that expression initially can be detected in the cytosol (Figs. 18 f+h) and ultimately on the cell surface (Figs. 18 e+g), showing that the HA expression of the here provided vaccine formulation after transfection with the RepRNA system encoding vaccine antigen is microscopically indiscernible from the HA expression after common influenza virus infection.
17a: Control experiment merged channel
17b: Control experiment green channel (channel 00)
17c: Transfection with RNA1 + RNA2_HA merged channel showing HA expression
17d: Transfection with RNA1 + RNA2_HA green channel (channel 00) showing HA expression
17e: Localization of HA expression on the cell membrane after transfection of RNA1+RNA2_HA
17f: Localization of intracellular HA expression after transfection of RNA1+RNA2_HA
17g: Localization of HA expression on the cell membrane after infection with Influenza A H1N1 virus
17h: Localization of intracellular HA expression after infection with Influenza A H1N1 virus.

Figure 18: production details reaction conditions:
RNA was produced according to the standard procedure by Etherna. Briefly, 5 µg of DNA linearized with XbaI was incubated with 5x eTherna IVT buffer and 2000U SP6 RNA polymerase in a total volume of 100µl at 37 degrees for 2 hours.
▪ 5x eTheRNA IVT buffer
▪ Incubation temperature 37°C
▪ Incubation time 2 hours
▪ 2000U SP6 RNA polymerase per ml reaction

### pRNA2 52Fluc270mod sp6 yield: 232µg from a 100µl reaction

### Figure 19: Details of split and fusion construct

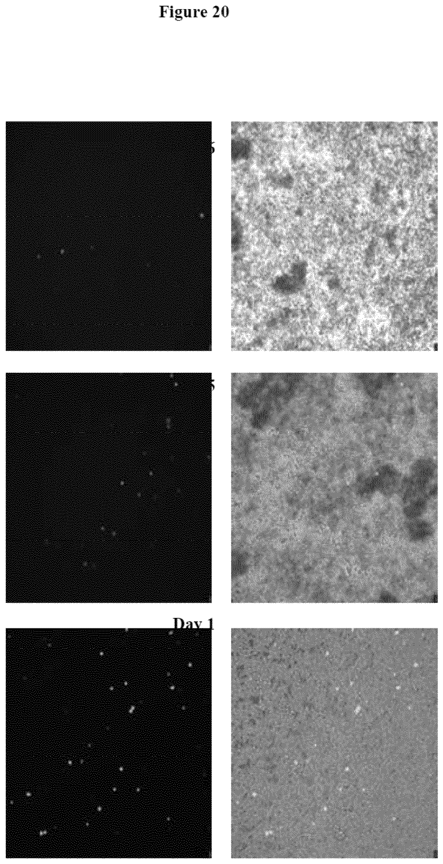

### Figure 20: Results of fusion experiment

BHK21 cells were seeded 1 day prior to transfection at 50.000 cells per well. On the day of transfection 150ng of fusion-GFP and lipofectamine 2000 was added to the cells for 4 hours before removing it again. Cells were further incubated, and images were taken on 1, 5 and 6 days post transfection using an UV/light microscope.

### Figure 21

RNA1 generated either by T7 or SP6 polymerase was capped either with a capO or a cap1 structure. 100 ug RNA was transfected into BHK-21 cells and 24 hours post transfection, the cells were lysed, RNA isolated and transcribed in cDNA. A qPCR was performed to quantitate the RNA1 copy numbers. CapO is superior to cap1 with respect to replication irrespective of material derived from processes derived by Sp6 or T7. (lower ct-value = higher number of RNA copies).

### Figure 22

Sequence alignment of the first 55 nt of the RNA2 derived from different Alphanodaviridae
Nov = Nodamuravirus (with a native-length 5'-UTR of 19 nt after which the first native start codon is found)
CaV = Canine nodavirus (with a native-length 5'-UTR of 18 nt
PoV = Porcine nodavirus (with a native-length 5'-UTR of 17 nt
PaV = Pariacotovirus (with a native-length 5'-UTR of 22 nt
FHV = Floch House Virus (with a native-length 5'-UTR of 22 nt
BBV = Black Beetle virus (with a native-length 5'-UTR of 22 nt
BoV = Boalarra virus (with native-length 5'-UTR of 10 nt
Nov, CaV and PoV are Alphanodaviridae generally capable of replicating in cells derived from a warm-blooded animal.

### Figure 23

Identification of 5'-UTR/CSE optimized replication and/or translation improving pharmacon ribonucleic acid CSE-elements in RNA2 derived from Alphanodaviridae.

In figure 23, the UTR is located 5' from the start codon ATG (indicated in blue) and the CSE is located 3' from the start codon. For ease of reference the whole replication element is indicated as 5' UTR-CSE, whereby the start codon ATG may also be destroyed. Said destruction is preferably achieved by change of T in G, resulting in an extension of the UTR with at least 3 nucleotides AGG. Experiments (see also figure 4 and 13, and the sequences listed in this application) showed that pharmacon replication and/or translation were markedly improved after detailed sequence modifications in the native 5'UTR-sequences (native sequences shown here in figure 23). Typically, said improvements comprise use of longer 5'-UTR elements than 3 or 17 nt, as reported earlier for FHV respectively NoV in heterologous gene expression studies. In a first embodiment, his application discloses use of the full-length native 5'-UTR followed by the (native as well as GOI) ATG-start codon to improve heterologous gene expression. To arrive at further improved 5'-UTR/CSE elements, the original nodavirus encoded start codon (ATG indicated in blue) is preferably destroyed (here a change of the pivotal T in G is disclosed), to lengthen the 5'-UTR nodavirus sequence after the destroyed start codon with at at least 5, more preferably at least 10, more preferably at least 20, more preferably at least 30 nodavirus derived nucleotides to improve replicase activity (see also figure 4). Thereafter, the pharmacon construct preferably commences with a newly introduced start codon (preferably ATG, derived from the GOI). Also, Kozak sequences may be optimized or added to further improve translation (see also figure 13).

For the NoV 5'-UTR/CSE construct for use in a pharmacon according to the invention; starting at position +44, +52, +52CC, and +55 the natural start codon (identified in blue) was destroyed by changing the T in position +21 to a G. The constructs with a 5' UTR of 44 nt has an almost optimal natural Kozak sequence (GCCGCC, nt 39-44, identified in yellow), as well as the construct with 55 nt at its 5' end (GCGGCC, identified in green). For the construct +52CC two C were introduced to optimize the Kozak sequence.

CaV and PoV each have a nearly optimal natural Kozak sequence (GCCAGC and GCGTCG respectively, each identified in grey, that may be optimized further.

For the other alphanoda viruses such natural Kozak sequences could not be identified but may readily be introduced or optimized in analogy with the NoV constructs here used.

Figure 24 RNA1 PCR results after transfection with different formulations containing RNA1.

BHK21 cells were transfected with different formulations containing 150ng RNA1. Samples, cell pellets, were taken at 1- and 2-days post transfection. RNA was extracted and tested by PCR for RNA1-pharmacon.
Dotap: cationic lipid; 1,2-dioleoyl-3-trimethylammonium-propane
SM102: ionizable amino lipid; 8-[(2-hydroxyethyl)[6-oxo-6-(undecyloxy)hexyl]amino]-octanoic acid, 1-octylnonyl ester
Alc0315: ionisable aminolipid ; 4-hydroxybutyl)azanediyl]di(hexane-6,1-diyl) bis(2-hexyldecanoate)

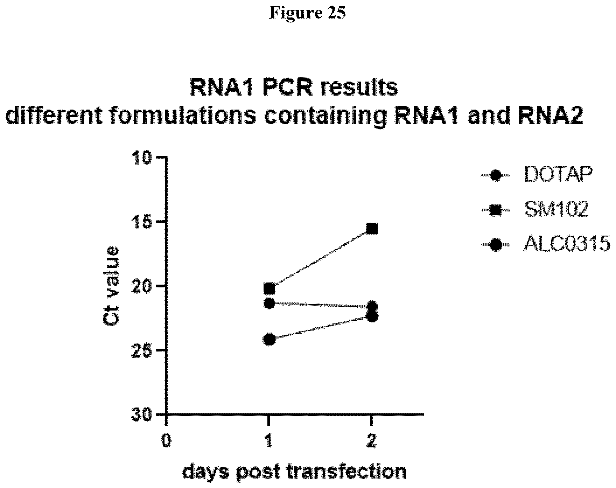

Figure 25 RNA1 PCR results after transfection with different formulations containing RNA1 -pharmacon as well as RNA2-HA-pharmacon 52HA270.

BHK21 cells were transfected with different formulations containing 150ng RNA1 with 150ng RNA2-HA. Samples, cell pellets, were taken at 1- and 2-days post transfection. RNA was extracted and tested by PCR for RNA1.
Dotap: cationic lipid; 1,2-dioleoyl-3-trimethylammonium-propane
SM102: ionizable amino lipid; 8-[(2-hydroxyethyl)[6-oxo-6-(undecyloxy)hexyl]amino]-octanoic acid, 1-octylnonyl ester
Alc0315: ionisable aminolipid ; 4-hydroxybutyl)azanediyl]di(hexane-6,1-diyl) bis(2-hexyldecanoate)

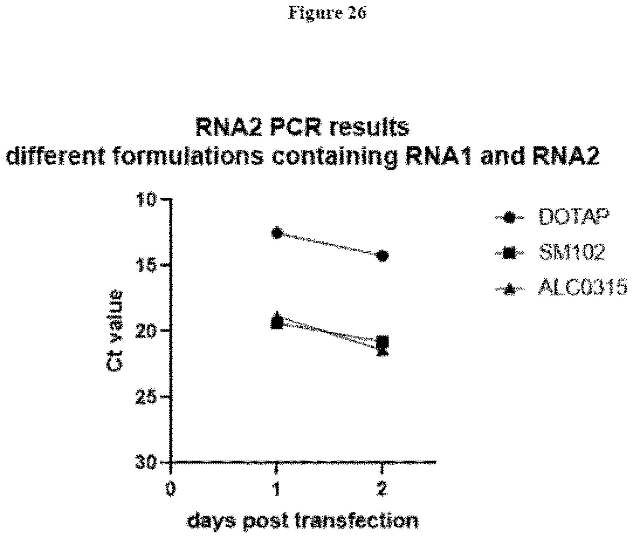

Figure 26 RNA2 PCR results after transfection with different formulations containing RNA1 -pharmacon as well as RNA2-HA-pharmacon 52HA270.
Dotap: cationic lipid; 1,2-dioleoyl-3-trimethylammonium-propane
SM102: ionizable amino lipid; 8-[(2-hydroxyethyl)[6-oxo-6-(undecyloxy)hexyl]amino]-octanoic acid, 1-octylnonyl ester
Alc0315: ionisable aminolipid ; 4-hydroxybutyl)azanediyl]di(hexane-6,1-diyl) bis(2-hexyldecanoate)
BHK21 cells were transfected with different formulations containing 150ng RNA1 with 150ng RNA2. Samples, cell pellets, were taken at 1- and 2-days post transfection. RNA was extracted and tested by PCR for RNA2 HA

### Detailed description

Administration of a synthetic and/or in vitro-generated RNA that structurally may resemble and functionally may act as natural messenger RNA to a patient or subject results in the controlled production of biologically or pharmacologically active and therapeutic proteins or peptides via the endogenous and constitutively active translation machinery (e.g., ribosomes) that exists within the subject's own cells. RNA-therapeutics is a rapidly evolving field that offers the potential for developing precise, targeted therapies for a variety of diseases. RNA molecules, including messenger RNA (mRNA), small interfering RNA (siRNA), and microRNA (miRNA), play essential roles in gene expression and regulation. By targeting these molecules, RNA-therapeutics hold great promise for treating genetic disorders, cancers, viral infections, and other diseases. Lee et al (Biomedicines. 2022 Jan 12;10(1):158), Zogg et al, (Int J Mol Sci. 2022 Mar 1;23(5):2736) and Zhu at el. (Cell Death Dis 13, 644 (2022) highlight recent advances in RNA-therapeutics, including the development of novel delivery systems, and discusses their potential applications in clinical settings. RNA-vaccines represent a promising alternative to conventional vaccine approaches because of their high potency, capacity for rapid development and potential for low-cost manufacture and safe administration. Recent technological advances have now provided multiple mRNA vaccine platforms against infectious diseases and several types of cancer, and some have demonstrated encouraging results in both animal models and humans. Pardi et al. (Nat Rev Drug Discov 17, 261-279 (2018).) discloses a detailed overview of mRNA vaccines and considers future directions and challenges in advancing this promising vaccine platform to widespread therapeutic use. Maruggi et al., (Mol. Therapy 2019; 27:1-16) overview the unique attributes of mRNA vaccine approaches, review the data of mRNA vaccines against infectious diseases, discuss the current challenges, and highlight perspectives about the future of this promising technology.

The concept for the development of an mRNA vaccine is rather straightforward. Once the antigen of choice from the pathogen target is identified, the GOI is sequenced, synthesized, and cloned into the DNA (desoxyribonucleic acid) template plasmid. mRNA (sometimes also identified as non-replicating (nr)RNA) is transcribed in vitro, mRNA is essentially purified, and the mRNA vaccine is delivered into the subject. Alternatively, instead of having been transcribed from a template, the nrRNA is synthesised directly. The mRNA vaccine utilizes the host cell machinery for in vivo translation of mRNA into the corresponding antigen to elicit potent humoral and cellular immune responses. The final location of the antigen is determined by the signal peptide and transmembrane domain. This can be intrinsic to the natural protein sequence or engineered to direct the protein to the desired cellular compartment. Due to the ability of the host's innate system to sense and respond to (detect, transcribe, translate) RNA sequences of viral origin, mRNA vaccines induce robust innate responses, including production of chemokines and cytokines such as interleukin-12 (IL-12) and tumour necrosis factor (TNF) at the injection site. These are factors deemed instrumental to successful induction of effective adaptive responses against the encoded antigen.

Nodamura virus (NoV) is a positive-strand RNA virus belonging to the family Nodaviridae. It has a small genome (∼4.3 kb) and encodes two open reading frames (ORFs) that produce the viral replicase and capsid proteins, respectively. Nodaviruses have only little been studied for their potential use in RNA-based therapies. They are known to cause diseases in fish and insects, but their unique features may make them candidates to be employed in the development of novel RNA therapies against human diseases. Nodaviruses possess a bipartite genome that codes for two separate subgenomic RNAs, identified as RNA1 and RNA2. The viral RNA1-gene encodes the viral RNA-dependent RNA polymerase (RdRp), which is responsible for replication of the viral genome; and the viral RNA2-gene encodes the capsid protein. These two independent RNA strands have independent promoters, allowing for differential regulation of gene expression. These features have been exploited to engineer Nodamura virus-based vectors that can deliver therapeutic RNAs to targeted cells. As reviewed by Yang et al., (Viruses. 2021 Jan 7;13(1):73.), nodaviruses are small bisegmented RNA viruses belonging to the family *Nodaviridae.* Nodaviruses have been identified in different hosts, including insects, fishes, shrimps, prawns, dogs, and bats.

One of the unique aspects of viruses is their lack of constraint in using different forms of nucleic acid for their genomes. They are not limited to double-stranded DNA; they can also use single-stranded DNA (for example, parvoviruses), negative or positive single-stranded RNA (for example, orthomyxoviruses and picornaviruses, respectively) or double-stranded RNA (for example, reoviruses). Viruses that use RNA as a genome have evolved novel mechanisms for replication and transcription (for example, RNA-dependent RNA polymerases) as well as translation. In addition, many RNA viruses replicate in the cytoplasm, where they cannot use cellular RNA-processing systems that are primarily located in the nucleus. Even if these viruses entered the nucleus, RNA-dependent transcription might not be compatible with cellular mRNA processing, which is highly dependent on cellular DNA-dependent RNA polymerase type II. Thus, it is not surprising that the mRNA structures and translation strategies that are used by RNA viruses vary considerably from the general model for translation of cellular and DNA virus mRNAs, which contain 7-methylGpppG caps on the 5' end, are spliced and contain polyA tails on the 3' end. This mRNA structure is the basis of cap-dependent translation, in which the 5' cap is recognized by eukaryotic initiation factor 4F (eIF4F), which identifies the mRNA for translation. Subsequently, interactions between eIF4F and polyA-binding proteins that are bound to the polyA tail further identify the mRNA and set up the structure that is needed for the 40S ribosomal subunit to attach to the 5' end of the RNA and begin scanning for an AUG initiation codon in the appropriate context for translation initiation. Although many RNA and DNA viruses use this translation-initiation mechanism, they have also evolved means to bypass it; this is especially true for the RNA viruses. Many translatable viral mRNAs are not capped and use internal ribosome entry sites (IRESs), which are most often located in the 5'-untranslated region of viral mRNAs that are close to the AUG to be used. There are no definitive structural or sequential definitions of IRESs, although many seem to have a high degree of secondary, and possibly tertiary, RNA structure. Viral IRESs can directly bind 40S ribosomal subunits and bypass the use of many initiation factors, including eIF4F. Other viral IRESs seem to use alternative mechanisms that might not require a direct interaction with the 40S subunit, but instead require additional proteins, called IRES trans-acting factors, to attract ribosomes. Viruses that use IRESs have also evolved ways to diminish cap-dependent translation such that there will be little competition from host mRNAs for ribosomes. Often, these viral mechanisms function through the destruction of the eIF4F complex, but viruses also have other tricks. For example, if ribosomes scan a viral mRNA for an AUG, a shunting mechanism can be used in which major parts of a complex mRNA leader sequence can be bypassed and are not melted by the scanning ribosomes.

Although these specialized mechanisms were discovered and have been most extensively studied in viral systems, it must be remembered that viruses rarely create entirely unique biochemical mechanisms. Instead, they mimic or capitalize on existing cellular mechanisms. Thus, IRESs have been found in cellular genes, and there is growing evidence to suggest that their use is widespread and important, especially during times of cellular stress when cap-dependent translation might be inhibited. An additional benefit of the use of IRESs by viruses is that it might provide an additional means of avoiding the inhibitory effects of cellular stress responses.

Vaccines are THE most effective tool for a sustainable prevention of infectious diseases in humans and animals. Infectious diseases can either be classified as endemic or emerging/epidemic. Especially for the latter, the time from detection to providing a solution is absolutely critical. In the past, it typically required 4 to 5 years before a vaccine could be made available for newly emerging diseases or vaccine escape strains (re-emerging diseases). Vaccines based on expressing the vaccine antigen encoded by genes directly in the host target cells, notably through mRNA, have been proven to be highly successful in providing a very effective and safe solution. One key advantage of RNA vaccines is the unprecedented speed for designing a new vaccine candidate combined with the ability to serve as a true platform accelerating development and regulatory review. This is in contrast to traditional vaccine manufacturing, which is typically highly complex, time consuming and costly. For this reason, many regulatory authorities - including the European Medicines Agency (EMA) - have put forward proposals on how platform technologies can help better assess the risk/benefit of a new vaccine and therefore accelerate development of new products and reduce development cost (EMA. Concept paper for the development of a guideline on data requirements for vaccine platform technology master files (PTMF). EMA/CVMP/IWP/582191/2020; 20 January 2021).

Vaccines based on RNA technology have thoroughly been researched over the past decades but have struggled to translate into commercial products for infectious diseases. This has fundamentally changed with the need to provide a fast, efficacious, and safe solution in the context of the COVID-19 pandemic. The mRNA-based vaccines developed for SARS-CoV-2 were not only developed and approved in record time but also show a highly favorable vaccine profile combined with a high degree of efficacy and safety.

However, the currently used mRNA vaccines have some major limitations, including: (1) the relatively high vaccine doses needed to induce a robust immune response and (2) the need for repeated application (immune response boosting) and (3) unfavorable storage conditions. The need for high doses to achieve full efficacy results in substantial production capacity requirements and limits the co-administration/combination with other vaccines due to tolerability concerns. The need for repeated administration is also a sign of insufficient immune stimulation by the administered doses; however, the tolerability profile of very high doses may limit an escalation of the doses.

To overcome these issues, the use of self-amplifying RNA (saRNA), also called replicon technology, has been suggested instead of non-replicating mRNA. The saRNA can preserve the beneficial properties of mRNA vaccines, while requiring a substantially lower dose and overcoming the need for repeated administration. Research using saRNA systems have been shown to induce a protective immune response rapidly after a single dose, thus leading to fewer and lower doses and subsequently reducing the load on manufacturing at scale (Bloom K et al., Self-amplifying RNA vaccines for infectious diseases. Gene Ther. 2021;28:117-129).

To achieve this, saRNA is engineered from genomes encoding the replication machinery of naturally occurring RNA viruses, while replacing structural viral proteins with vaccine antigen(s). Importantly, the replicon represents only part of the replication machine of the virus, not a replication competent virus itself. After administration and upon uptake of the saRNA into the cytosol of a target host cell, the replicon directs the self-amplification of the gene(s) of interest (GOI) and produces large quantities of vaccine antigen, which is subsequently presented to the humoral and the cellular arm of the immune system.

As a result, a robust immune response is attained via the administration of a single dose of saRNA that is about 30 to 100 x lower than conventional non-replicating mRNA yet leads to a higher and longer expression of the vaccine antigen.

There are several viruses that are candidates for such a replicon system, all of them are based on RNA virus replication machines, most prominently alphaviruses, flaviviruses, measles virus and rhabdoviruses (Lundstrom K. Replicon RNA Viral Vectors as Vaccines. Vaccines. 2016;4:39). These replicon systems are relatively large and all genes encoding the replication machine as well as the GOI are combined on ONE segment and comes with a major disadvantage, in that the length of the construct is correlated with production cost due to the sheer amount of material needed and the decreased efficiency of in vitro transcription (IVT) with long constructs. For example, most of the replicon systems currently used are based on alphaviruses. For example, a shortcoming of the alphavirus replicon system is its large size since the replication machinery itself already requires approx. 8 kb and, depending on the nature of the antigen-encoding gene, the entire replicon RNA including the gene of interest comprises frequently about 9-13 kb. The size and complexity of this construct makes it difficult to generate high quality RNA with good yields and often requires complex and costly purification steps.

It has now been found that the prior art problems and shortcomings may be effectively and advantageously overcome by an RNA split replicon system comprising: a first segment comprising the RNA-1 sequence of Nodamura virus (wild-type will do); a second segment comprising deletion variants of taRNA2 of Nodamura virus wherein the coding region of the capsid protein has been at least partially deleted and substituted by a sequence encoding for one or more gene of interest. Nodamura virus (NoV) is a small, non-enveloped positive stranded RNA virus which was originally isolated from mosquitoes but has demonstrated to infect and replicate also in fish and some mammals (Murphy FA et al. Characterization of Nodamura virus, an arthropod transmissible picornavirus. Virology. 1970;40:1008-1021). The NoV saRNA possesses the beneficial characteristics of having only approximately 1/3 of its size of the alphavirus saRNA. Its genome is composed of two positive-sense RNA segments: RNA1 is approx. 3.1 kb in length and encodes the RNA-dependent RNA polymerase (RdRp) necessary for the replication of the genome and the B2 protein that suppresses host-cell RNA interference; RNA2 is encoding the capsid protein.

The RNA1 carries all information required for RNA transcription and replication and encodes at least two polypeptides - protein A and B2. Protein A is an RNA-dependent RNA polymerase (RdRp) that is required for virus replication. Protein B2 is translated from the subgenomic RNA3/ORF1b which is located at the 3' end of RNA1. Protein B2 suppresses host-mediated RNA silencing (Howley PM, Knipe DM. Fields Virology. 6th ed. 2013. Philadephia: Wolters Kluwer.) and transactivates RNA2 replication. A schematic presentation of the segmented Nodavirus genome with key gene locations is reported in Figure 3.

In the split replicon system of the invention, the gene encoding the capsid protein is largely deleted and replaced by the GOI. Previous use inserted the gene of interest on RNA1, which required partial deletion of the B2 protein resulting in a diminished ability to fend off the innate system hampering RNA replication in the cell and thus, limiting the antigen expression. For the purposes of the invention, replicon systems from wild-type Nodamura viruses may be used. Alternatively, Flock house virus, Black beetle virus, Boolarra virus, Pariacoto virus can also be used. The gene(s) of interest may be any immunogenic or immune-modulatory peptide, protein, or proteinaceous substance (herein identified as antigen), for example a bacterial vaccine antigen as provided herein (such as a Streptococcus suis vaccine antigen) , or viral vaccine antigen as provided herein (such as an influenza virus vaccine antigen or ORF2d from porcine circovirus type 2 (PCV2)) or tumor vaccine antigen. Within tumor vaccine antigens, specifically preferred are tumor neoantigens, including antigens produced by tumor viruses integrated into the genome and antigens produced by mutant proteins, which may be abundantly expressed only in tumor cells and have strong immunogenicity and tumor heterogeneity. A growing number of studies have highlighted the relationship between neoantigens and T cells' recognition of cancer cells. In a preferred embodiment the invention discloses a transfectant, such as a human T-cell or dendritic cell provided with a replication system of the invention provided with a tumor antigen.

The gene of interest is may preferably be inserted between the two 5'- and 3' UTR regions in a minimal replicon containing the at least 3 nucleoside (ns, nt is also used) CSE, more preferably the at least 19 ns CSE, more preferably at least 52 ns CSE the 5'-end and the 25 ns, more preferably at least 50 ns, CSE more preferably the at least 100 ns CSE, more preferably the at least 236 ns CSE on the 3'-end of RNA2 from NoV or FHV. The RNA split replicon system of the invention may suitably be formulated in vaccine compositions for human or veterinary use.

DNA plasmids are generated, containing the information for wild-type (wt) saRNA1 and taRNA2 under the control of a DNA-dependent RNA polymerase promoter (typically T7 and SP6, also T3 is used). Moreover, the plasmids are equipped with a ribozyme derived from Hepatitis Delta virus at the 3' end of RNA 1 and taRNA2 to achieve homogeneous 3'ending of the RNA molecules. Linearized plasmids are used as templates for transcription and generation of wild-type saRNA1 and taRNA2. The RNA is essentially purified, 5' capped and tested *in vitro* for functionality by co-transfecting insect and mammalian cell lines (e.g. BHK-21) to rescue wt Nodamura virus. For the split replicon system, the wt essentially purified RNA1 may be used without the need for any further modification.

Deletion mutants meeting sequence requirements of taRNA2 for replication and translation are isolated and tested for their ability to replicate in mammalian cell lines (*e.g*.*,*BHK-21).

A schematic representation of a construct according to the invention is reported in Figure 4.

RNA stability and consequently antigen expression, critical to induce a robust immune response and vaccine efficacy, may be improved by including capping structures of the RNA at its 5' end, and modulating, preferably shortening the length of the poly(A) tail at its 3' end to <20 A, more preferably <15 A, more preferably <10 A, more preferably <5 A, more preferably to essentially no added poly(A) sequence. In addition, replacement of one or both pyrimidines (cytidine and uridine) with non-natural analogues (*i.e.,* 5-methylcytidine-5'-triphosphate (5mC) and pseudouridine-5'-triphosphate, respectively) reduces the stimulation of Toll-like receptors (TLR), protein kinase R (PKR) and retinoic acid-inducible protein I (RIG-I), resulting in a muted innate immune response *in vivo*. The use of modified nucleotides hides the exogenous RNA and can therefore significantly reduce this pattern recognition interaction and thus improve protein translation. Of note, RNA1 and RNA2 of NoV and FHV are 7-methylGpppN capped (cap-0 type) but are essentially not 3' polyadenylated which simplifies RNA manufacturing, allowing advantageous and straightforward in vivo, in vitro, as well as chemical, synthesis, and transcription of a vaccine according to the invention.

As discussed by Rosskopf et al, the 3' UTR of RNA2 may contain CSE or other tertiary structures forming essential elements for RNA replication (Rosskopf JJ et al. A 3' terminal stem-loop structure in Nodamura virus RNA2 forms an essential cis-acting signal for RNA replication. Virus Res. 2010;150:12-21). Structural and sequence determinants for initiation of RNA replication were investigated. For the nodavirus Flock House virus (FHV) undefined sequences within the 3'-terminal 50 nucleotides (nt) of FHV RNA2 were found essential for its replication. It was also shown that a conserved stem-loop structure (3'SL) is predicted to form near the 3' end of the RNA2 segments of seven nodaviruses, including NoV. Rosskopf et al hypothesized that the 3'SL structure from NoV RNA2 is an essential cis-acting element for RNA replication. To determine whether the structure can actually form within RNA2, they analyzed the secondary structure of NoV RNA2 in vitro transcripts using nuclease mapping. The resulting nuclease maps were 86% consistent with the predicted 3'SL structure, suggesting that it can form in solution. They used a reverse genetic system for launch of NoV replication in yeast cells to test the function of the 3'SL in the viral life cycle. Deletion of the nucleotides that comprise the 3'SL from a NoV2-GFP chimeric replicon resulted in a severe defect in RNA2 replication. A minimal replicon containing the 5'-terminal 17 nt and the 3'-terminal 54 nt of RNA2 (including the predicted 3'SL) retained the ability to replicate in yeast, suggesting that this region may direct replication of a heterologous mRNA. Rosskopf's data in particular data suggest that the 3'SL plays an essential role in replication of NoV RNA2. The conservation of the predicted 3'SL suggests that this common motif plays a role in RNA replication for the other members of the Nodaviridae as well.

US 20030108863 does not relate to a cell-free system of in vitro transcription by using an enzymatic transcription reaction from a linearized *Nodaviridae*-derived DNA template requiring a DNA-dependent RNA polymerase and neither relates to essentially purified RNA(s), obtainable by such a cell free system that would be useful in an essentially pure RNA vaccine but instead describes the production of a nodavirus-based DNA vector that, after introduction in cells, drives expression of foreign genes in said cells and teaches that small spherical virus capsid particles, as typically found with Noda virus, have significant potential as systems for the specific encapsidation and delivery of biological molecules. Similarly, US 20060177819 does not relate to a cell-free system of in vitro transcription by using an enzymatic transcription reaction from a linearized *Nodaviridae-*derived DNA template requiring a DNA-dependent RNA polymerase and neither to essentially purified RNA(s), obtainable by such a cell-free system that would be useful in an essentially pure RNA vaccine but instead describes a nodavirus-based DNA vector that, after introduction in cells, drives expression of alpha virus capsid genes in cells and teaches that such a nodavirus-based DNA vector can further reduce the predicted frequency for formation of replication-competent alpha virus and may optimize alpha virus particle manufacturing. Biddlecome et al., relates to RNA useful in mRNA vaccines (PLoS One. 2019; 14(6): e0215031) but uses only the RNA1 gene of nodavirus, and typically dispenses its RNA2 gene or nodavirus RNA2 gene derivatives. Biddlecom et., add a gene of interest (GOI) to the subgenomic cis-active RNA3 within said RNA1 gene, therewith disabling at least some the functionalities of the subgenomic proteins (B1 and/or B2) derived from said RNA3. According to Biddlecome et al., (ibid) replication of a gene of interest can be coupled to the replication of the Noda viral RdRp by inserting it in the RNA1 gene in this way, but its length, and thus length of GOI, is restricted to guarantee in vitro packaging of the ensuing replicon into a wildtype Cowpea Chlorotic Mottle Virus capsid (CCMV CP T=3), length restrictions necessitating depriving the replicon system from some B-protein activities.

Similarly, US 20060177819 does not relate to a cell-free system of in vitro transcription by using an enzymatic transcription reaction from a linearized *Nodaviridae-*derived DNA template requiring a DNA-dependent RNA polymerase and neither to essentially purified RNA(s), obtainable by such a cell-free system that would be useful in an essentially pure RNA vaccine. US 20060177819 instead describes a nodavirus-based DNA vector that, after introduction in cells, drives expression of alpha virus capsid genes in cells and teaches that such a nodavirus-based DNA vector can further reduce the predicted frequency for formation of replication-competent alpha virus and may optimize alpha virus particle manufacturing.

US20060177819 suggests at paragraphs 0071 to 0073 to use a non-alpha virus (such as Nodavirus based) viral RNA replication machinery to help express one or more alpha virus structural proteins but those paragraphs are silent as to how that machinery is supposed to enter cells needed to express those proteins. Only at paragraphs 0133 to 0137 it becomes apparent that said machinery is to be introduced via commonplace (copy)DNA technology, by amplification of alphavirus capsid genes by polymerase chain reaction (PCR, a typical DNA technology) using primers (referring to Table 2 at page 12) that introduce restriction sites (again solely DNA technology, see also paragraph 0133).

In general, restriction enzymes cannot be used on RNA, and certainly the MluI, NcoI, or BssHHII restrictions enzymes cannot be used on RNA to obtain RNA fragments. Subsequently, US20060177819 suggests to directly clone the PCR product directly in the RNA2 (generated from FHV or NoV), which again are digested with compatible DNA restriction enzymes. Again, this is typical DNA technology and not a topic of the present invention.

As is widely known in the art, a DNA fragment cannot be cloned into an RNA fragment.

Paragraph 0134 in US20060177819 further elaborates on the proposed cloning: US20060177819 again suggests using primers for the amplification of alphavirus capsid genes containing a MluI restriction site in the fwd (SEQ 19) and rev (SEQ 20) primer. PCR product is assumedly digested with MluI and cloned in the MluI digested FHV RNA2 vector and the BssHHII digested NoV RNA2 vector. These vectors must be DNA, otherwise there would be no such cloning possible. BssHII produces compatible cohesive ends with MluI. Currently there are only 3 NoV RNA2 segments known in the art and they don't contain a BssHII site, raising the question how the US20060177819 can suggest using these enzymes at all (see the table below) if the art shows that these sites are not present and the US20060177819 is not enabling.

In addition, US20060177819 suggests an alternative method for the generation of Nodavirus helper RNA by cloning into the NcoI and MluI site of FHV RNA2 and the NcoI and BssHII site of NoV RNA2. US20060177819 suggests that the NcoI site is 5' to the MluI and BssHII site in the FHV and NoV RNA2, respectively, separated by 1 nucleotide. In the currently published NoV RNA2 sequences (see table below) there is neither a BssHII nor a NcoI site. Some, but not all the FHV RNA2 sequence contain a MluI and a NcoI site, again illustrating that US20060177819 is not enabling anything for all Nodaviridae, and also the location is different than assumed or postulated in the US20060177819.

Furthermore, there is an additional problem with the putative method of US20060177819 as suggested; the start codon for the capsid gene is located at position 22 and thus, upstream of the MluI site which can be found in some isolates at position 60 or 61, respectively. When proteins are translated from RNA, always the first ATG (AUG) is used and if the original start codon for the capsid protein is not mutated (which is indeed not mentioned in the US20060177819), the alphavirus capsid genes and subsequent GOI cannot be translated, again identifying a serious lack of enablement in the US20060177819.

In conclusion, none of the suggested RNA technology of US20060177819 is sufficiently or enabling disclosed in the context of Nodavirus and the provision of Nodavirus RNA replication machinery. Again, we conclude that US 20060177819 does not relate to a cell-free system of in vitro transcription by using an enzymatic transcription reaction from a linearized *Nodaviridae*-derived DNA template requiring a DNA-dependent RNA polymerase (no such polymerases are mentioned in respect to Nodavirus DNA in the US20060177819) and neither does US 20060177819 relates to essentially purified RNA(s), obtainable by a cell-free system that would be useful in an essentially pure RNA vaccine according to the present invention.

| **Virus isolate/accession number** | **BssHII** | **NcoI** |
|---|---|---|
| | g/cgcgc | c/catgg |
| NoV RNA2/X15961.1 | - | - |
| NoV RNA2/NC_002691.1 | - | - |
| NoV RNA2/**AF174534.1** | - | - |

| **Virus isolate/accession number** | **NcoI** | **MluI** |
|---|---|---|
| | c/catgg | a/cgcgt |
| FHV RNA2/ JF461542.1 | - | 60 |
| FHV RNA2/ EF690538.1 | - | - |
| FHV RNA2/ X15959.1 | 852 | 61 |
| FHV RNA2/ GQ342966.1 | - | - |
| FHV RNA2/ NC_004144.1 | 852 | 61 |
| | | |

### GOI examples

Examples of GOI include the HA gene from the pandemic swine influenza virus or Ide_{Ssuis} which are inserted in the optimized constructs shown. For the *S. suis* construct, Ide_{Ssuis} from serotype 2 may be used by optimizing the sequence for expression in mammalian cells, i.e. by predicting and deleting potential glycosylation sites using an appropriate prediction algorithm and eventually equipping the protein with an appropriate signal peptide to target it to the cellular secretory pathway. Expression of the protein may be quantified *in vitro* by transfecting BHK-21 cells with the replicon and analyzing the cell supernatants by ELISA and/or on their capability to cleave swine IgMs. It has been shown that intradermal injection of pigs in the ear leads to a robust immune response with a substantially reduced dose (Saade F, Petrovsky N. Technologies for enhanced efficacy of DNA vaccines. Expert Rev Vaccines. 2012;11:189-209).

The vaccine compositions comprising the split RNA replicons according to the invention include infectious disease vaccines, cancer vaccines, fish vaccines, poultry vaccines, swine vaccines (e.g. against pandemic swine influenza), cattle vaccines, dog vaccines, cat vaccines, equine vaccines. Combined vaccines, i.e. polyvalent vaccines, including the replicons of the invention coding for different GOI, may also be developed. Lipid nanoparticles (LNP) may be conveniently used instead of capsid packaging. When using e.g. 2 different taRNA2 molecules, taRNA2¹ and taRNA2² to express different proteins together with saRNA1, the vaccine can be formulated in a way that taRNA2¹ and taRNA2² are expressed within the same cell or from different cells. taRNA2¹ and taRNA2² may be comprised in same nanoparticles or in different nanoparticles. The vaccine compositions will be administered by any suitable route (intramuscular, intradermal, subcutaneous, oral, transmucosal). The dosage will be determined by the skilled practitioner according to known methods, starting from pre-clinical and clinical tests. For instance, Minimum immunizing dose (MID), Onset of immunity (OOI), Duration of immunity (DOI), Maximum titre safety study will be evaluated in accordance with the US and European Pharmacopoeia (USP, EP) by immunizing animals, in particular pigs, for example with 0.01, 0.03, 0.1, 0.3, 1, 3, 10, 30, or 100 µg (or a selected range thereof as deemed required and corresponding to approximately 0.0004 to 4 µg/kg) of formulated split-replicon vaccine RNA; the animals are subsequently challenged with the relevant pathogen evaluating the clinically relevant endpoints so as to provide the relevant vaccine dose needed. This method will allow an approximation of the dose needed to immunize humans.

### Workflow

In a first step towards the generation of a replicon system based on the Nodamura virus (NoV) genome, a reverse genetic system for the virus is generated. To this end, two in vitro transcription plasmids are designed, pRNA1 and pRNA2, in which the genetic information from saRNA1 and taRNA2, respectively, is placed in an in vitro transcription plasmid under the control of a T7 or SP6 promoter. The 3'-UTR from saRNA1 and taRNA2 is immediately followed by a ribosomal cleavage site from Hepatitis Delta virus to achieve homogeneous 3'-ends of the generated RNA constructs; the HDV ribozyme site may be followed by a T7 termination sequence which is (preferably immediately) followed by a unique restriction site for plasmid linearization. The resulting DNA plasmids are introduced into host bacteria (typically E. coli) via transformation, usually by electroporation. The bacteria replicate during fermentation, making copies of the plasmids and producing high yields of pRNA1 and pRNA2. Once the bacteria have reached the desired density, they are harvested, and pRNA1 and pRNA2 are extracted (typically by alkaline lysis) and the pRNA1 and pRNA2 are purified by chromatography and subjected to quality control.

To generate a replicon system for the expression of heterologous proteins, plasmid pRNA2 containing the genetic information from taRNA2 from NoV is modified accordingly. Different deletion mutants of pRNA2 may generated to identify the optimal size of the deletion for a GOI at hand. taRNA2 molecules having nt 1-3, 1-19, and 1-52 at its 5'end and the last 270, 236, 100, 50, and 25nt at their 3'end are generated. The gene of interest is placed between the 5' and 3' end of the taRNA2 molecule. Examples for HA derived from an influenza virus H1N1 and the ORF2 from PCV2 strain GDYZ are provided. Furthermore, the tyrosine residue in position 21 in the 5'-end of taRNA2 is changed to a guanine or an appropriate analogue to remove the internal start codon at that position. Plasmids containing the gene of interest (GOI) are processed as described above.

The plasmid DNAs are linearized by enzymatic digestion, preferably with a unique DNA restriction enzyme preferably situated immediately downstream of the HDV ribozyme or alternatively downstream of the T7 termination site if present. mRNA is synthesized from the linear DNA templates, which is incubated with enzymes and nucleotides or analogues thereof in a process called in vitro transcription (IVT). In a next step, a 5' cap 0 structure is added enzymatically to the RNA to stabilize the RNA. Alternatively, a cap 1 or cap 2 structure can be added already co-transcriptionally to the mRNA. The mRNA molecule is isolated by removing impurities such as DNA, nucleotides, and enzymes. Finally, the concentration of mRNA is adjusted and subjected to quality control before it is ready for formulation e.g., by encapsulating it into LNPs.

### Cancer vaccines

Cancer is a group of diseases characterized by uncontrolled cell growth and invasion. It is a major cause of death worldwide, accounting for an estimated 9.6 million deaths in 2018. Although there have been significant advances in cancer treatment over the past few decades, many patients still face poor outcomes, particularly those with advanced disease. In addition, current cancer therapies such as chemotherapy and radiation therapy can cause significant side effects and often fail to eradicate the disease, resulting in relapse and progression of the disease.

Cancer vaccines represent a new and promising approach to cancer treatment which aims at activating the immune system to recognize and destroy cancer cells. Cancer cells can sometimes evade detection by the immune system, or even actively suppress immune responses against them. Cancer vaccines aim to overcome these obstacles by introducing antigens that are (ideally) uniquely expressed by cancer cells, into the body to stimulate an immune response. This response can include the production of specific antibodies and the activation of immune cells such as T-cells. The goal is to stimulate immunity to recognize, attack and destroy cancer cells, similar to infectious pathogens. The modern era of cancer vaccines began with the identification of tumor-associated antigens (TAAs) and the development of techniques to stimulate immune responses against them.

Cancer vaccines typically consist of a relevant antigen such as a TAA and a vaccine platform which delivers the vaccine antigen to the relevant cells or tissues and often contributes to the simulation of the immune response. Vaccine platforms can consist of peptides, nucleic acids or viral vectors and often comprise formulations such as lipid nanoparticles (LNPs) and/or adjuvants.

TAAs are proteins that are expressed on the surface of tumor cells and can be recognized by the immune system as foreign. These antigens are derived from normal proteins that are overexpressed, mutated, or abnormally expressed in cancer cells.

There are different types of TAAs, including:
- Shared antigens: These are antigens that are expressed on both tumor cells and normal cells but are overexpressed on tumor cells. Examples include human epidermal growth factor receptor 2 (HER2/neu), a protein that is overexpressed in some breast cancers, and EGFR, a protein overexpressed in some lung and colon cancers.
- Cancer-testis antigens (CTAs): These are antigens that are normally expressed in the testes but are also expressed in some tumors. Examples include MAGE-A3, NY-ESO-1, and SSX.
- Differentiation antigens: These are proteins that are expressed during embryonic development but are not normally expressed in adult tissues. They can be re-expressed in some types of cancer cells. Examples include alpha-fetoprotein (AFP) in liver cancer and melanoma antigen recognized by T cells (MART-1) in melanoma.
- Mutated antigens/neoantigens: These are antigens that are derived from mutations in the tumor cell's DNA. Because these mutations are unique to the tumor, these antigens can trigger a strong immune response and can be targeted without affecting normal cells.
- Viral antigens: Some cancers are associated with viral infections, such as human papillomavirus (HPV) in cervical cancer and hepatitis B virus (HBV) in liver cancer. Viral antigens expressed by the tumor cells can be targeted with a vaccine.

TAAs are the basis for several cancer immunotherapy approaches, including cancer vaccines, adoptive T-cell therapy, and checkpoint inhibitors. However, not all patients respond to immunotherapy, and tumors can develop mechanisms to evade immune detection. Ongoing research is focused on identifying new TAAs and developing more effective immunotherapies.

In the examples various sequences identifying certain embodiments of the invention are listed.

### Examples with List of sequences

### Preferred 5'-UTR/CSE RNA2-derived sequences.

Several preferred 5'-UTR/CSE alphanodavirus RNA2-derived sequences for use in a noda-like RNA-pharmacon, preferably located in said pharmacon directly 5' from the start codon ATG of the preferred GOI, to improve in vitro and in vivo protein-A replicase activity derived from (the preferably corresponding) virus RNA1 and acting on the RNA2 derived 5'-UTR material. Preferably, NoV RNA1 is combined with 5'-UTR NoV RNA2-derived sequences; CaV RNA1 is combined with 5'-UTR CaV RNA2-derived sequences; and so on. More preferably, NoV, CaV, PoV, or other mammalian-derived viral sequences are used in a pharmacon according to the invention, as they demonstrably have a replicase with the ability to replicate at typical (warm-blooded) mammalian body temperatures.
Nov = Nodamuravirus
CaV = Canine nodavirus
PoV = Porcine nodavirus
PaV = Pariacotovirus
FHV = Floch House Virus
BBV = Black Beetle virus
BoV = Boalarra virus

Nov, CaV and PoV are Alphanodaviridae generally capable of replicating in cells derived from a warm-blooded animal.

Below sets of preferred 5'-UTR/CSE RNA2-derived sequences are derived from the 5'-UTR/CSE elements of RNA2 and identical per virus identified up to codon AGG. AGG denotes the destroyed ATG-start codon of the capsid-encoding-sequences of the respective Alphanodavirus. Preferred are 5'-UTR/CSE elements with at least 5 nucleotides added 3' from said AGG codon, more preferred at least 10, more preferably at least 20, more preferably at least 30 nucleotides added 3' from said AGG codon. In some embodiments used herein even longer 5'-UTR/CSE elements are used. Preferred 5'-UTR/CSE RNA2-derived sequences thus comprise at least the first 5 nucleotides, more preferred at least the first 10, more preferably at least the first 20, more preferably at least the first 30 nucleotides located downstream (3') of a first start codon ATG located in a 5' conserved sequence element (CSE) flanking the endogenous RNA2 of any of the Alphanodaviridae, preferably of any of the Alphanodaviridae generally capable of replicating in cells derived from a warm-blooded animal. At present, up to and at around 60 nucleotides in said desired 5'-UTR/CSE elements are foreseen to improve. This increased length is desired when one or more Kozak sequences are included to better induce translation of the GOI.

Preferred 5'-UTR/CSE RNA2-derived sequences comprise:
NoV GTAAACAACCAATAACATCAGGGTATC
NoV GTAAACAACCAATAACATCAGGGTATCCAAAG
NoV GTAAACAACCAATAACATCAGGGTATCCAAAGCAGCA
NoV GTAAACAACCAATAACATCAGGGTATCCAAAGCAGCACGCCG
NoV
   GTAAACAACCAATAACATCAGGGTATCCAAAGCAGCACGCCGCC
NoV
   GTAAACAACCAATAACATCAGGGTATCCAAAGCAGCACGCCGCCGAA
NoV 
GTAAACAACCAATAACATCAGGGTATCCAAAGCAGCACGCCGCCGAA
GAGCG
NoV 
GTAAACAACCAATAACATCAGGGTATCCAAAGCAGCACGCCGCCGAA
GAGCGGCC
CaV CCCTTCGATCATTACCTAAGGGCTCG
CaV CCCTTCGATCATTACCTAAGGGCTCGATCGA
CaV CCCTTCGATCATTACCTAAGGGCTCGATCGAAATAC
CaV CCCTTCGATCATTACCTAAGGGCTCGATCGAAATACTGCAC
CaV
   CCCTTCGATCATTACCTAAGGGCTCGATCGAAATACTGCACTTTGC
CaV
   CCCTTCGATCATTACCTAAGGGCTCGATCGAAATACTGCACTTTGCCACG
PoV GGTCACCACCGTACAGAAGGTTACT
PoV GGTCACCACCGTACAGAAGGTTACTAACAA
PoV GGTCACCACCGTACAGAAGGTTACTAACAACGGAA
PoV GGTCACCACCGTACAGAAGGTTACTAACAACGGAAGCAAGCGTC
PoV GGTCACCACCGTACAGAAGGTTACTAACAACGGAAGCAAGCGTCG
FHV GTAAACAATTCCAAGTTCCAAAAGGGTTAA
FHV GTAAACAATTCCAAGTTCCAAAAGGGTTAATAACA
FHV GTAAACAATTCCAAGTTCCAAAAGGGTTAATAACAACAGA
FHV GTAAACAATTCCAAGTTCCAAAAGGGTTAATAACAACAGACCAAG
FHV 
GTAAACAATTCCAAGTTCCAAAAGGTTAATAACAACAGACCAAGAC
GTC
PaV ATGTACAGGTATAACATCAAAGAGGGTATC
PaV ATGTACAGGTATAACATCAAAGAGGGTATCAAGA
PaV ATGTACAGGTATAACATCAAAGAGGGTATCAAGAACTAA
PaV
   ATGTACAGGTATAACATCAAAGAGGGTATCAAGAACTAAGAATC
PaV 
ATGTACAGGTATAACATCAAAGAGGGTATCAAGAACTAAGAATCGAA
GG
BoV GAATTCAACAAGGACGCC
BoV GAATTCAACAAGGACGCCACGAC
BoV GAATTCAACAAGGACGCCACGACGACAA
BoV GAATTCAACAAGGACGCCACGACGACAACAACG
BoV GAATTCAACAAGGACGCCACGACGACAACAACGTCCCA
BBV GTAAACAATTCCAAGTTCCAAAAGGGTTAG
BBV GTAAACAATTCCAAGTTCCAAAAGGGTTAGAAACA
BBV GTAAACAATTCCAAGTTCCAAAAGGGTTAGAAACAACAAC
BBV
   GTAAACAATTCCAAGTTCCAAAAGGGTTAGAAACAACAACCGAAG
BBV

GTAAACAATTCCAAGTTCCAAAAGGGTTAGAAACAACAACCGAAGAC
GAC

### Infectious agent vaccine RNA-pharmacons

Examples for bi-segmented pharmacons cloned in the RNA2 of Nodamura virus (NoV) and replacing partially the capsid protein. The sequences are equipped at their 5' end with up to 55 nt derived from the 5' end of RNA2 followed by the gene of interest and the last 270 nt from the 3' end of RNA2 of NoV. At position 21 the T was replaced by G to mutate the natural occurring ATG start codon. Replication is facilitated in a bi-segmented fashion by RNA1.

The examples are taken from human and animal health.

Moreover, pharmacons in form of monocistronic replicons are created. Those replicons are designed as follows: RNA1 wildtype derived from NoV is directly fused at its 3'end with the first 55 nt derived from the 5' end of RNA2; the T in position 21 was replaced by G to mutate the natural occurring ATG start codon. This stretch of 55 nt is followed by the gene of interest and the terminal 270 nt from the 3' end of RNA2. All pharmacons (both RNA1 and RNA2) derived are capped with capO and to be applied together with RNA1 as replicase provided and RNA2 as GOI-provider, bi-segmented of mono-cistronic.
NoV RNA1 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
 
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
 
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGT
Hepatitis B: HBsAg
   55_HBsAg_270 
GTAAACAACCAATAACATCAGGGTATCCAAAGCAGCACGCCGCCGAA
GAGCGGCCATGGAGAACACAGCATCAGGATTCCTAGGACCCCTGCTCGTGTT
ACAGGCGGGGTTTTTCTTGTTGACAAGAATCCTCACAATACCACAGAGTCTAG
ACTCGTGGTGGACTTCTCTCAATTTTCTAGGGGGAGCACCCACGTGTCCTGGC
CAAAATTCGCAGTCCCCAACCTCCAATCACTCACCAACCTCTTGTCCTCCAAT
TTGTCCTGGCTATCGCTGGATGTGTCTGCGGCGTTTTATCATATTCCTCTTCAT
CCTGCTGCTATGCCTCATCTTCTTGTTGGTTCTTCTGGACTATCACGGTATGTT
GCCCGTTTGTCCTCTACTTCCAGGAACATCAACCACCAGCACGGGGCCATGCA
AGACCTGCACGATTCCTGCTCAAGGAACCTCTATGTTTCCCTCTTGTTGCTGTA
CAAAACCTTCGGACGGAAACTGCACTTGTATTCCCATCCCATCATCCTGGGCT
TTCGCAAGATTCCTATGGGAGTGGGCCTCAGTCCGTTTCTCCTGGCTCAGTTT
GCTAGTGCCATTTGTTCAGTGGTCCGTAGGGCTTTCCCCCACTGTTTGGCTTTC
AGTTATATGGATGATGTGGTATTGGGGGCCAAGTCTGTACAACATCTTGAGTC
CCTTTTTACCTCTATTACCAATTTTCTTTTGTCTTTGGGTATACATTTGAGTCGC
TTGCAAAGACAACGCCACATTTTGGGAACGCGTCCGATCCATCCTGAAATCTG
GGCTCAACTTTGCTTCGACCATACCTGGCCCCGTAGGAGTGGCAGCGACAGG
GATCAAAGGCATCATTGAAGCCATTGGTTCCTTGTGGGTTTGATTCCACCCAC
AGAAGCGTTGACGACGCAAAACGTCCTTAAAGCGTTGACGACGCAAAACGTC
CCCAAGCTCGTAGCACCGACCCTATACCCATCTCTAGGGTCTTCAACCTCTTG
GT
Fusion construct 55_HBsAg_270 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
 
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
 
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
 
ATGCCAGTGGTGTAAACAACCAATAACATCAGGGTATCCAAAGCAGCACGCC
GCCGAAGAGCGGCCATGGAGAACACAGCATCAGGATTCCTAGGACCCCTGCT
CGTGTTACAGGCGGGGTTTTTCTTGTTGACAAGAATCCTCACAATACCACAGA
GTCTAGACTCGTGGTGGACTTCTCTCAATTTTCTAGGGGGAGCACCCACGTGT
CCTGGCCAAAATTCGCAGTCCCCAACCTCCAATCACTCACCAACCTCTTGTCC
TCCAATTTGTCCTGGCTATCGCTGGATGTGTCTGCGGCGTTTTATCATATTCCT
CTTCATCCTGCTGCTATGCCTCATCTTCTTGTTGGTTCTTCTGGACTATCACGG
TATGTTGCCCGTTTGTCCTCTACTTCCAGGAACATCAACCACCAGCACGGGGC
CATGCAAGACCTGCACGATTCCTGCTCAAGGAACCTCTATGTTTCCCTCTTGT
TGCTGTACAAAACCTTCGGACGGAAACTGCACTTGTATTCCCATCCCATCATC
CTGGGCTTTCGCAAGATTCCTATGGGAGTGGGCCTCAGTCCGTTTCTCCTGGC
TCAGTTTGCTAGTGCCATTTGTTCAGTGGTCCGTAGGGCTTTCCCCCACTGTTT
GGCTTTCAGTTATATGGATGATGTGGTATTGGGGGCCAAGTCTGTACAACATC
TTGAGTCCCTTTTTACCTCTATTACCAATTTTCTTTTGTCTTTGGGTATACATTT
GAGTCGCTTGCAAAGACAACGCCACATTTGGGAACGCGTCCGATCCATCCT
GAAATCTGGGCTCAACTTTGCTTCGACCATACCTGGCCCCGTAGGAGTGGCAG
CGACAGGGATCAAAGGCATCATTGAAGCCATTGGTTCCTTGTGGGTTTGATTC
CACCCACAGAAGCGTTGACGACGCAAAACGTCCTTAAAGCGTTGACGACGCA
AAACGTCCCCAAGCTCGTAGCACCGACCCTATACCCATCTCTAGGGTCTTCAA
CCTCTTGGT
44_HBsAg_270 
GTAAACAACCAATAACATCAGGGTATCCAAAGCAGCACGCCGCCATG
GAGAACACAGCATCAGGATTCCTAGGACCCCTGCTCGTGTTACAGGCGGGGT
TTTTCTTGTTGACAAGAATCCTCACAATACCACAGAGTCTAGACTCGTGGTGG
ACTTCTCTCAATTTTCTAGGGGGAGCACCCACGTGTCCTGGCCAAAATTCGCA
GTCCCCAACCTCCAATCACTCACCAACCTCTTGTCCTCCAATTTGTCCTGGCTA
TCGCTGGATGTGTCTGCGGCGTTTTATCATATTCCTCTTCATCCTGCTGCTATG
CCTCATCTTCTTGTTGGTTCTTCTGGACTATCACGGTATGTTGCCCGTTTGTCC
 
TCTACTTCCAGGAACATCAACCACCAGCACGGGGCCATGCAAGACCTGCACG
ATTCCTGCTCAAGGAACCTCTATGTTTCCCTCTTGTTGCTGTACAAAACCTTCG
GACGGAAACTGCACTTGTATTCCCATCCCATCATCCTGGGCTTTCGCAAGATT
CCTATGGGAGTGGGCCTCAGTCCGTTTCTCCTGGCTCAGTTTGCTAGTGCCAT
TTGTTCAGTGGTCCGTAGGGCTTTCCCCCACTGTTTGGCTTTCAGTTATATGGA
TGATGTGGTATTGGGGGCCAAGTCTGTACAACATCTTGAGTCCCTTTTTACCT
CTATTACCAATTTTCTTTTGTCTTTGGGTATACATTTGAGTCGCTTGCAAAGAC
AACGCCACATTTTGGGAACGCGTCCGATCCATCCTGAAATCTGGGCTCAACTT
TGCTTCGACCATACCTGGCCCCGTAGGAGTGGCAGCGACAGGATCAAAGGC
ATCATTGAAGCCATTGGTTCCTTGTGGGTTTGATTCCACCCACAGAAGCGTTG
ACGACGCAAAACGTCCTTAAAGCGTTGACGACGCAAAACGTCCCCAAGCTCG
TAGCACCGACCCTATACCCATCTCTAGGGTCTTCAACCTCTTGGT
Fusion construct 44_HBsAg_270 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
 
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
 
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCATGGAGAACACAGCATCAGGATTCCTAGGACCCCTGCTCGTGTTACAGG
CGGGGTTTTTCTTGTTGACAAGAATCCTCACAATACCACAGAGTCTAGACTCG
TGGTGGACTTCTCTCAATTTTCTAGGGGGAGCACCCACGTGTCCTGGCCAAAA
TTCGCAGTCCCCAACCTCCAATCACTCACCAACCTCTTGTCCTCCAATTTGTCC
TGGCTATCGCTGGATGTGTCTGCGGCGTTTTATCATATTCCTCTTCATCCTGCT
GCTATGCCTCATCTTCTTGTTGGTTCTTCTGGACTATCACGGTATGTTGCCCGT
TTGTCCTCTACTTCCAGGAACATCAACCACCAGCACGGGGCCATGCAAGACCT
GCACGATTCCTGCTCAAGGAACCTCTATGTTTCCCTCTTGTTGCTGTACAAAA
CCTTCGGACGGAAACTGCACTTGTATTCCCATCCCATCATCCTGGGCTTTCGC
 
AAGATTCCTATGGGAGTGGGCCTCAGTCCGTTTCTCCTGGCTCAGTTTGCTAG

TGCCATTTGTTCAGTGGTCCGTAGGGCTTTCCCCCACTGTTTGGCTTTCAGTTA

TATGGATGATGTGGTATTGGGGGCCAAGTCTGTACAACATCTTGAGTCCCTTT

TTACCTCTATTACCAATTTTCTTTTGTCTTTGGGTATACATTTGAGTCGCTTGC

AAAGACAACGCCACATTTTGGGAACGCGTCCGATCCATCCTGAAATCTGGGC

TCAACTTTGCTTCGACCATACCTGGCCCCGTAGGAGTGGCAGCGACAGGGAT

CAAAGGCATCATTGAAGCCATTGGTTCCTTGTGGGTTTGATTCCACCCACAGA

AGCGTTGACGACGCAAAACGTCCTTAAAGCGTTGACGACGCAAAACGTCCCC

AAGCTCGTAGCACCGACCCTATACCCATCTCTAGGGTCTTCAACCTCTTGGT
19_HBsAg_270 
GTAAACAACCAATAACATCATGGAGAACACAGCATCAGGATTCCTAGG
ACCCCTGCTCGTGTTACAGGCGGGGTTTTTCTTGTTGACAAGAATCCTCACAA
TACCACAGAGTCTAGACTCGTGGTGGACTTCTCTCAATTTTCTAGGGGGAGCA
CCCACGTGTCCTGGCCAAAATTCGCAGTCCCCAACCTCCAATCACTCACCAAC
CTCTTGTCCTCCAATTTGTCCTGGCTATCGCTGGATGTGTCTGCGGCGTTTTAT
CATATTCCTCTTCATCCTGCTGCTATGCCTCATCTTCTTGTTGGTTCTTCTGGAC
TATCACGGTATGTTGCCCGTTTGTCCTCTACTTCCAGGAACATCAACCACCAG
CACGGGGCCATGCAAGACCTGCACGATTCCTGCTCAAGGAACCTCTATGTTTC
CCTCTTGTTGCTGTACAAAACCTTCGGACGGAAACTGCACTTGTATTCCCATC
CCATCATCCTGGGCTTTCGCAAGATTCCTATGGGAGTGGGCCTCAGTCCGTTT
CTCCTGGCTCAGTTTGCTAGTGCCATTTGTTCAGTGGTCCGTAGGGCTTTCCCC
CACTGTTTGGCTTTCAGTTATATGGATGATGTGGTATTGGGGGCCAAGTCTGT
ACAACATCTTGAGTCCCTTTTTACCTCTATTACCAATTTTCTTTTGTCTTTGGGT
ATACATTTGAGTCGCTTGCAAAGACAACGCCACATTTGGGAACGCGTCCGAT
CCATCCTGAAATCTGGGCTCAACTTTGCTTCGACCATACCTGGCCCCGTAGGA
GTGGCAGCGACAGGGATCAAAGGCATCATTGAAGCCATTGGTTCCTTGTGGG
TTTGATTCCACCCACAGAAGCGTTGACGACGCAAAACGTCCTTAAAGCGTTG
ACGACGCAAAACGTCCCCAAGCTCGTAGCACCGACCCTATACCCATCTCTAG
 GGTCTTCAACCTCTTGGT
Fusion construct 19_HBsAg_270 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
 
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
 
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCATGGAGAACACAGCATCAGGAT
TCCTAGGACCCCTGCTCGTGTTACAGGCGGGGTTTTTCTTGTTGACAAGAATC
CTCACAATACCACAGAGTCTAGACTCGTGGTGGACTTCTCTCAATTTTCTAGG
GGGAGCACCCACGTGTCCTGGCCAAAATTCGCAGTCCCCAACCTCCAATCACT
CACCAACCTCTTGTCCTCCAATTTGTCCTGGCTATCGCTGGATGTGTCTGCGGC
GTTTTATCATATTCCTCTTCATCCTGCTGCTATGCCTCATCTTCTTGTTGGTTCT
TCTGGACTATCACGGTATGTTGCCCGTTTGTCCTCTACTTCCAGGAACATCAA
CCACCAGCACGGGGCCATGCAAGACCTGCACGATTCCTGCTCAAGGAACCTC
TATGTTTCCCTCTTGTTGCTGTACAAAACCTTCGGACGGAAACTGCACTTGTA
TTCCCATCCCATCATCCTGGGCTTTCGCAAGATTCCTATGGGAGTGGGCCTCA
GTCCGTTTCTCCTGGCTCAGTTTGCTAGTGCCATTTGTTCAGTGGTCCGTAGGG
CTTTCCCCCACTGTTTGGCTTTCAGTTATATGGATGATGTGGTATTGGGGGCC
AAGTCTGTACAACATCTTGAGTCCCTTTTTACCTCTATTACCAATTTTCTTTTG
TCTTTGGGTATACATTTGAGTCGCTTGCAAAGACAACGCCACATTTTGGGAAC
GCGTCCGATCCATCCTGAAATCTGGGCTCAACTTTGCTTCGACCATACCTGGC
CCCGTAGGAGTGGCAGCGACAGGGATCAAAGGCATCATTGAAGCCATTGGTT
CCTTGTGGGTTTGATTCCACCCACAGAAGCGTTGACGACGCAAAACGTCCTTA
AAGCGTTGACGACGCAAAACGTCCCCAAGCTCGTAGCACCGACCCTATACCC
ATCTCTAGGGTCTTCAACCTCTTGGT
PCV2d: ORF2d
   55_ORF2d_270 
GTAAACAACCAATAACATCAGGGTATCCAAAGCAGCACGCCGCCGAA
GAGCGGCCATGACGTATCCAAGGAGGCGTTTCCGCAGACGAAGACACCGCCC
CCGCAGCCATCTTGGCCAGATCCTCCGCCGCCGCCCCTGGCTCGTCCACCCCC
GCCACCGTTACCGCTGGAGAAGGAAAAATGGCATCTTCAACACCCGCCTCTC
CCGCACCATCGGTTATACTGTCAAGAAAACCACAGTCAGAACGCCCTCCTGG
AATGTGGACATGATGAGATTTAATATTAATGATTTCTTCCCCCAGGAGGGGG
CTCAAACCCCCTCACTGTGCCCTTTGAATACTACAGAATAAGGAAGGTTAAG
GTTGAACTCTGGCCCTGCTCCCCAATCACCCAGGGTGACAGGGGAGTGGGCT
CCACTGCTGTTATTCTAGATGATAACTTTGTAACAAAGGCCAATGCCCTAACC
TATGACCCCTATGTAAACTACTCCTCCCGCCATACCATAACCCAGCCCTTCTC
CTACCACTCCCGGTACTTTACCCCGAAACCTGTCCTTGATGGGACAATCGATT
ACTTCCAACCCAATAACAAAAGAAATCAACTCTGGCTGAGACTACACACTAC
TGGAAATGTAGACCATGTAGGCCTCGGCACTGCGTTCGAAAACAGTATATAC
GACCAGGACTACAATATCCGTATAACCATGTATGTACAATTCAGAGAATTTA
ATCTTAAAGACCCCCCACTTAACCCTAAGTGAGTCGCTTGCAAAGACAACGC
CACATTTTGGGAACGCGTCCGATCCATCCTGAAATCTGGGCTCAACTTTGCTT
CGACCATACCTGGCCCCGTAGGAGTGGCAGCGACAGGGATCAAAGGCATCAT
TGAAGCCATTGGTTCCTTGTGGGTTTGATTCCACCCACAGAAGCGTTGACGAC
GCAAAACGTCCTTAAAGCGTTGACGACGCAAAACGTCCCCAAGCTCGTAGCA
CCGACCCTATACCCATCTCTAGGGTCTTCAACCTCTTGGT
Fusion construct 55_ORF2d_270 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
 
ACACGAGAAGACCGTGAGATCAGCCTGTTTCCGCAAAATTCCATCGTTTCCGC
AGAACACGCGCGGAAAGCGGATAATGGGCATCCGATCTCCGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTCCAGCTATACGCTGG
CATTCCGTGATCAGGTGCTGCATGCAGAGCACAACCGGCATTGGTTTTGCCCG
 
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
 
ATGCCAGTGGTGTAAACAACCAATAACATCAGGGTATCCAAAGCAGCACGCC
GCCGAAGAGCGGCCATGACGTATCCAAGGAGGCGTTTCCGCAGACGAAGACA
CCGCCCCCGCAGCCATCTTGGCCAGATCCTCCGCCGCCGCCCCTGGCTCGTCC
ACCCCCGCCACCGTTACCGCTGGAGAAGGAAAAATGGCATCTTCAACACCCG
CCTCTCCCGCACCATCGGTTATACTGTCAAGAAAACCACAGTCAGAACGCCCT
CCTGGAATGTGGACATGATGAGATTTAATATTAATGATTTTCTTCCCCCAGGA
GGGGGCTCAAACCCCCTCACTGTGCCCTTTGAATACTACAGAATAAGGAAGG
TTAAGGTTGAACTCTGGCCCTGCTCCCCAATCACCCAGGGTGACAGGGGAGT
GGGCTCCACTGCTGTTATTCTAGATGATAACTTTGTAACAAAGGCCAATGCCC
TAACCTATGACCCCTATGTAAACTACTCCTCCCGCCATACCATAACCCAGCCC
TTCTCCTACCACTCCCGGTACTTTACCCCGAAACCTGTCCTTGATGGGACAAT
CGATTACTTCCAACCCAATAACAAAAGAAATCAACTCTGGCTGAGACTACAC
ACTACTGGAAATGTAGACCATGTAGGCCTCGGCACTGCGTTCGAAAACAGTA
TATACGACCAGGACTACAATATCCGTATAACCATGTATGTACAATTCAGAGA
ATTTAATCTTAAAGACCCCCCACTTAACCCTAAGTGAGTCGCTTGCAAAGACA
ACGCCACATTTTGGGAACGCGTCCGATCCATCCTGAAATCTGGGCTCAACTTT
GCTTCGACCATACCTGGCCCCGTAGGAGTGGCAGCGACAGGGATCAAAGGCA
TCATTGAAGCCATTGGTTCCTTGTGGGTTTGATTCCACCCACAGAAGCGTTGA
CGACGCAAAACGTCCTTAAAGCGTTGACGACGCAAAACGTCCCCAAGCTCGT
AGCACCGACCCTATACCCATCTCTAGGGTCTTCAACCTCTTGGT
44_ORF2d_270 
GTAAACAACCAATAACATCAGGGTATCCAAAGCAGCACGCCGCCATG
ACGTATCCAAGGAGGCGTTTCCGCAGACGAAGACACCGCCCCGCAGCCATC
TTGGCCAGATCCTCCGCCGCCGCCCCTGGCTCGTCCACCCCCGCCACCGTTAC
CGCTGGAGAAGGAAAAATGGCATCTTCAACACCCGCCTCTCCCGCACCATCG
GTTATACTGTCAAGAAAACCACAGTCAGAACGCCCTCCTGGAATGTGGACAT
GATGAGATTTAATATTAATGATTTCTTCCCCCAGGAGGGGGCTCAAACCCCC
TCACTGTGCCCTTTGAATACTACAGAATAAGGAAGGTTAAGGTTGAACTCTGG
 
CCCTGCTCCCCAATCACCCAGGGTGACAGGGGAGTGGCTCCACTGCTGTTAT
TCTAGATGATAACTTTGTAACAAAGGCCAATGCCCTAACCTATGACCCCTATG
TAAACTACTCCTCCCGCCATACCATAACCCAGCCCTTCTCCTACCACTCCCGG
TACTTTACCCCGAAACCTGTCCTTGATGGGACAATCGATTACTTCCAACCCAA
TAACAAAAGAAATCAACTCTGGCTGAGACTACACACTACTGGAAATGTAGAC
CATGTAGGCCTCGGCACTGCGTTCGAAAACAGTATATACGACCAGGACTACA
ATATCCGTATAACCATGTATGTACAATTCAGAGAATTTAATCTTAAAGACCCC
CCACTTAACCCTAAGTGAGTCGCTTGCAAAGACAACGCCACATTTTGGGAAC
GCGTCCGATCCATCCTGAAATCTGGGCTCAACTTTGCTTCGACCATACCTGGC
CCCGTAGGAGTGGCAGCGACAGGGATCAAAGGCATCATTGAAGCCATTGGTT
CCTTGTGGGTTTGATTCCACCCACAGAAGCGTTGACGACGCAAAACGTCCTTA
AAGCGTTGACGACGCAAAACGTCCCCAAGCTCGTAGCACCGACCCTATACCC
ATCTCTAGGGTCTTCAACCTCTTGGT
Fusion construct 44_ORF2d_270 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
 
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
 
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCATGACGTATCCAAGGAGGCGTTTCCGCAGACGAAGACACCGCCCCCGCA
GCCATCTTGGCCAGATCCTCCGCCGCCGCCCCTGGCTCGTCCACCCCCGCCAC
CGTTACCGCTGGAGAAGGAAAAATGGCATCTTCAACACCCGCCTCTCCCGCA
CCATCGGTTATACTGTCAAGAAAACCACAGTCAGAACGCCCTCCTGGAATGT
GGACATGATGAGATTTAATATTAATGATTTCTTCCCCCAGGAGGGGGCTCAA
ACCCCCTCACTGTGCCCTTTGAATACTACAGAATAAGGAAGGTTAAGGTTGA
ACTCTGGCCCTGCTCCCCAATCACCCAGGGTGACAGGGGAGTGGGCTCCACT
GCTGTTATTCTAGATGATAACTTTGTAACAAAGGCCAATGCCCTAACCTATGA
 
CCCCTATGTAAACTACTCCTCCCGCCATACCATAACCCAGCCCTTCTCCTACC
ACTCCCGGTACTTTACCCCGAAACCTGTCCTTGATGGACAATCGATTACTTC
CAACCCAATAACAAAAGAAATCAACTCTGGCTGAGACTACACACTACTGGAA
ATGTAGACCATGTAGGCCTCGGCACTGCGTTCGAAAACAGTATATACGACCA
GGACTACAATATCCGTATAACCATGTATGTACAATTCAGAGAATTTAATCTTA
AAGACCCCCCACTTAACCCTAAGTGAGTCGCTTGCAAAGACAACGCCACATT
TTGGGAACGCGTCCGATCCATCCTGAAATCTGGGCTCAACTTTGCTTCGACCA
TACCTGGCCCCGTAGGAGTGGCAGCGACAGGGATCAAAGGCATCATTGAAGC
CATTGGTTCCTTGTGGGTTTGATTCCACCCACAGAAGCGTTGACGACGCAAAA
CGTCCTTAAAGCGTTGACGACGCAAAACGTCCCCAAGCTCGTAGCACCGACC
CTATACCCATCTCTAGGGTCTTCAACCTCTTGGT
19_ORF2d_270 
GTAAACAACCAATAACATCATGACGTATCCAAGGAGGCGTTTCCGCAG
ACGAAGACACCGCCCCCGCAGCCATCTTGGCCAGATCCTCCGCCGCCCCT
GGCTCGTCCACCCCCGCCACCGTTACCGCTGGAGAAGGAAAATGGCATCTT
CAACACCCGCCTCTCCCGCACCATCGGTTATACTGTCAAGAAAACCACAGTCA
GAACGCCCTCCTGGAATGTGGACATGATGAGATTTAATATTAATGATTTTCTT
CCCCCAGGAGGGGGCTCAAACCCCCTCACTGTGCCCTTTGAATACTACAGAAT
AAGGAAGGTTAAGGTTGAACTCTGGCCCTGCTCCCCAATCACCCAGGGTGAC
AGGGGAGTGGGCTCCACTGCTGTTATTCTAGATGATAACTTTGTAACAAAGGC
CAATGCCCTAACCTATGACCCCTATGTAAACTACTCCTCCCGCCATACCATAA
CCCAGCCCTTCTCCTACCACTCCCGGTACTTTACCCCGAAACCTGTCCTTGATG
GGACAATCGATTACTTCCAACCCAATAACAAAAGAAATCAACTCTGGCTGAG
ACTACACACTACTGGAAATGTAGACCATGTAGGCCTCGGCACTGCGTTCGAA
AACAGTATATACGACCAGGACTACAATATCCGTATAACCATGTATGTACAATT
CAGAGAATTTAATCTTAAAGACCCCCCACTTAACCCTAAGTGAGTCGCTTGCA
AAGACAACGCCACATTTGGGAACGCGTCCGATCCATCCTGAAATCTGGGCT
CAACTTTGCTTCGACCATACCTGGCCCCGTAGGAGTGGCAGCGACAGGATC
 
AAAGGCATCATTGAAGCCATTGGTTCCTTGTGGGTTTGATTCCACCCACAGAA
GCGTTGACGACGCAAAACGTCCTTAAAGCGTTGACGACGCAAAACGTCCCCA
AGCTCGTAGCACCGACCCTATACCCATCTCTAGGGTCTTCAACCTCTTGGT
Fusion construct 19_ORF2d_270 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
 
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
 
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCATGACGTATCCAAGGAGGCGTT
TCCGCAGACGAAGACACCGCCCCCGCAGCCATCTTGGCCAGATCCTCCGCCG
CCGCCCCTGGCTCGTCCACCCCCGCCACCGTTACCGCTGGAGAAGGAAAAT
GGCATCTTCAACACCCGCCTCTCCCGCACCATCGGTTATACTGTCAAGAAAAC
CACAGTCAGAACGCCCTCCTGGAATGTGGACATGATGAGATTTAATATTAAT
GATTTCTTCCCCCAGGAGGGGGCTCAAACCCCCTCACTGTGCCCTTTGAATA
CTACAGAATAAGGAAGGTTAAGGTTGAACTCTGGCCCTGCTCCCAATCACC
CAGGGTGACAGGGGAGTGGGCTCCACTGCTGTTATTCTAGATGATAACTTTGT
AACAAAGGCCAATGCCCTAACCTATGACCCCTATGTAAACTACTCCTCCCGCC
ATACCATAACCCAGCCCTTCTCCTACCACTCCCGGTACTTTACCCCGAAACCT
GTCCTTGATGGGACAATCGATTACTTCCAACCCAATAACAAAAGAAATCAAC
TCTGGCTGAGACTACACACTACTGGAAATGTAGACCATGTAGGCCTCGGCAC
TGCGTTCGAAAACAGTATATACGACCAGGACTACAATATCCGTATAACCATG
TATGTACAATTCAGAGAATTTAATCTTAAAGACCCCCCACTTAACCCTAAGTG
AGTCGCTTGCAAAGACAACGCCACATTTTGGGAACGCGTCCGATCCATCCTG
AAATCTGGGCTCAACTTTGCTTCGACCATACCTGGCCCCGTAGGAGTGGCAGC
GACAGGGATCAAAGGCATCATTGAAGCCATTGGTTCCTTGTGGGTTTGATTCC
ACCCACAGAAGCGTTGACGACGCAAAACGTCCTTAAAGCGTTGACGACGCAA
AACGTCCCCAAGCTCGTAGCACCGACCCTATACCCATCTCTAGGGTCTTCAAC
 CTCTTGGT
Pharmacon L1 from human papilloma virus 16 to be applied together with RNA1 
GTAAACAACCAATAACATCAGGGTATCCAAAGCAGCACGCCGCCGAA
GAGCGGCCATGTCTCTTTGGCTGCCTAGTGAGGCCACTGTCTACTTGCCTCCT
GTCCCAGTATCTAAGGTTGTAAGCACGGATGAATATGTTGCACGCACAAACA
TATATTATCATGCAGGAACATCCAGACTACTTGCAGTTGGACATCCCTATTTT
CCTATTAAAAACCTAACAATAACAAAATATTAGTTCCTAAAGTATCAGGATT
ACAATACAGGGTATTTAGAATACATTTACCTGACCCCAATAAGTTTGGTTTTC
CTGACACCTCATTTTATAATCCAGATACACAGCGGCTGGTTTGGGCCTGTGTA
GGTGTTGAGGTAGGCCGTGGTCAGCCATTAGGTGTGGGCATTAGTGGCCATC
CTTTATTAAATAAATTGGATGACACAGAAAATGCTAGTGCTTATGCAGCAAAT
GCAGGTGTGGATAATAGAGAATGTATATCTATGGATTACAAACAAACACAAT
TGTGTTTAATTGGTTGCAAACCACCTATAGGGGAACACTGGGGCAAAGGATC
CCCATGTACCAATGTTGCAGTAAATCCAGGTGATTGTCCACCATTAGAGTTAA
TAAACACAGTTATTCAGGATGGTGATATGGTTGATACTGGCTTTGGTGCTATG
GACTTTACTACATTACAGGCTAACAAAAGTGAAGTTCCACTGGATATTTGTAC
ATCTATTTGCAAATATCCAGATTATATTAAAATGGTGTCAGAACCATATGGCG
ACAGCTTATTTTTTATTTACGAAGGGAACAAATGTTTGTTAGACATTTATTTA
ATAGGGCTGGTGCTGTTGGTGAAAATGTACCAGACGATTTATACATTAAAGG
CTCTGGGTCTACTGCAAATTTAGCCAGTTCAAATTATTTTCCTACACCTAGTG
GTTCTATGGTTACCTCTGATGCCCAAATATTCAATAAACCTTATTGGTTACAA
CGAGCACAGGGCCACAATAATGGCATTTGTTGGGGTAACCAACTATTTGTTAC
TGTTGTTGATACTACACGCAGTACAAATATGTCATTATGTGCTGCCATATCTA
CTTCAGAAACTACATATAAAAATACTAACTTTAAGGAGTACCTACGACATGG
GGAGGAATATGATTTACAGTTTATTTTTCAACTGTGCAAAATAACCTTAACTG
CAGACGTTATGACATACATACATTCTATGAATTCCACTATTTTGGAGGACTGG
AATTTTGGTCTACAACCTCCCCCAGGAGGCACACTAGAAGATACTTATAGGTT
TGTAACATCCCAGGCAATTGCTTGTCAAAAACATACACCTCCAGCACCTAAA
 
GAAGATCCCCTTAAAAAATACACTTTTTGGGAAGTAAATTTAAAGGAAAAGT
TTTCTGCAGACCTAGATCAGTTTCCTTTAGGACGCAAATTTTACTACAAGCA
GGATTGAAGGCCAAACCAAAATTTACATTAGGAAAACGAAAAGCTACACCCA
CCACCTCATCTACCTCTACAACTGCTAAACGCAAAAAACGTAAGCTGTAAGTC
GCTTGCAAAGACAACGCCACATTTTGGGAACGCGTCCGATCCATCCTGAAAT
CTGGGCTCAACTTTGCTTCGACCATACCTGGCCCCGTAGGAGTGGCAGCGACA
GGGATCAAAGGCATCATTGAAGCCATTGGTTCCTTGTGGGTTTGATTCCACCC
ACAGAAGCGTTGACGACGCAAAACGTCCTTAAAGCGTTGACGACGCAAAACG
TCCCCAAGCTCGTAGCACCGACCCTATACCCATCTCTAGGGTCTTCAACCTCT
TGGT
Fusion construct Pharmacon L1 from human papilloma virus 16 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
 
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
 
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGTCTCTTTGGCTGCCTAGTGAGGCCACTGTCTACTTG
CCTCCTGTCCCAGTATCTAAGGTTGTAAGCACGGATGAATATGTTGCACGCAC
AAACATATATTATCATGCAGGAACATCCAGACTACTTGCAGTTGGACATCCCT
ATTTTCCTATTAAAAAACCTAACAATAACAAAATATTAGTTCCTAAAGTATCA
GGATTACAATACAGGGTATTTAGAATACATTTACCTGACCCCAATAAGTTTGG
TTTTCCTGACACCTCATTTTATAATCCAGATACACAGCGGCTGGTTTGGGCCT
GTGTAGGTGTTGAGGTAGGCCGTGGTCAGCCATTAGGTGTGGGCATTAGTGG
CCATCCTTTATTAAATAAATTGGATGACACAGAAAATGCTAGTGCTTATGCAG
CAAATGCAGGTGTGGATAATAGAGAATGTATATCTATGGATTACAAACAAAC
ACAATTGTGTTTAATTGGTTGCAAACCACCTATAGGGGAACACTGGGGCAAA
GGATCCCCATGTACCAATGTTGCAGTAAATCCAGGTGATTGTCCACCATTAGA
 
GTTAATAAACACAGTTATTCAGGATGGTGATATGGTTGATACTGGCTTTGGTG
CTATGGACTTTACTACATTACAGGCTAACAAAGTGAAGTTCCACTGGATATT
TGTACATCTATTTGCAAATATCCAGATTATATTAAAATGGTGTCAGAACCATA
TGGCGACAGCTTATTTTTTATTTACGAAGGGAACAAATGTTTGTTAGACATT
TATTTAATAGGGCTGGTGCTGTTGGTGAAAATGTACCAGACGATTTATACATT
AAAGGCTCTGGGTCTACTGCAAATTTAGCCAGTTCAAATTATTTCCTACACC
TAGTGGTTCTATGGTTACCTCTGATGCCCAAATATTCAATAAACCTTATTGGTT
ACAACGAGCACAGGGCCACAATAATGGCATTTGTTGGGGTAACCAACTATTT
GTTACTGTTGTTGATACTACACGCAGTACAAATATGTCATTATGTGCTGCCAT
ATCTACTTCAGAAACTACATATAAAAATACTAACTTTAAGGAGTACCTACGAC
ATGGGGAGGAATATGATTTACAGTTTATTTTTCAACTGTGCAAAATAACCTTA
ACTGCAGACGTTATGACATACATACATTCTATGAATTCCACTATTTTGGAGGA
CTGGAATTTTGGTCTACAACCTCCCCCAGGAGGCACACTAGAAGATACTTATA
GGTTTGTAACATCCCAGGCAATTGCTTGTCAAAAACATACACCTCCAGCACCT
AAAGAAGATCCCCTTAAAAAATACACTTTTTGGGAAGTAAATTTAAAGGAAA
AGTTTTCTGCAGACCTAGATCAGTTTCCTTTAGGACGCAAATTTTTACTACAA
GCAGGATTGAAGGCCAAACCAAAATTTACATTAGGAAAACGAAAAGCTACAC
CCACCACCTCATCTACCTCTACAACTGCTAAACGCAAAAAACGTAAGCTGTA
AGTCGCTTGCAAAGACAACGCCACATTTTGGAACGCGTCCGATCCATCCTG
AAATCTGGGCTCAACTTTGCTTCGACCATACCTGGCCCCGTAGGAGTGGCAGC
GACAGGGATCAAAGGCATCATTGAAGCCATTGGTTCCTTGTGGGTTTGATTCC
ACCCACAGAAGCGTTGACGACGCAAAACGTCCTTAAAGCGTTGACGACGCAA
AACGTCCCCAAGCTCGTAGCACCGACCCTATACCCATCTCTAGGGTCTTCAAC
CTCTTGGT
SARS-CoV2 spike Wuhan Hu-1 to be applied together with RNA1 
GTAAACAACCAATAACATCAGGGTATCCAAAGCAGCACGCCGCCGAA
GAGCGGCCATGTTTGTTTTTCTTGTTTATTGCCACTAGTCTCTAGTCAGTGTG
TTAATCTTACAACCAGAACTCAATTACCCCCTGCATACACTAATTCTTTCACA
 
CGTGGTGTTTATTACCCTGACAAAGTTTTCAGATCCTCAGTTTACATTCAACT
CAGGACTTGTTCTTACCTTTCTTTTCCAATGTTACTTGGTTCCATGCTATACAT
GTCTCTGGGACCAATGGTACTAAGAGGTTTGATAACCCTGTCCTACCATTTAA
TGATGGTGTTTATTTTGCTTCCACTGAGAAGTCTAACATAATAAGAGGCTGGA
TTTTTGGTACTACTTTAGATTCGAAGACCCAGTCCCTACTTATTGTTAATAACG
CTACTAATGTTGTTATTAAAGTCTGTGAATTTCAATTTTGTAATGATCCATTTT
TGGGTGTTTATTACCACAAAAACAACAAAAGTTGGATGGAAAGTGAGTTCAG
AGTTTATTCTAGTGCGAATAATTGCACTTTTGAATATGTCTCTCAGCCTTTTCT
TATGGACCTTGAAGGAAAACAGGGTAATTTCAAAAATCTTAGGGAATTTGTG
TTTAAGAATATTGATGGTTATTTTAAAATATATTCTAAGCACACGCCTATTAA
TTTAGTGCGTGATCTCCCTCAGGGTTTTTCGGCTTTAGAACCATTGGTAGATTT
GCCAATAGGTATTAACATCACTAGGTTTCAAACTTTACTTGCTTTACATAGAA
GTTATTTGACTCCTGGTGATTCTTCTTCAGGTTGGACAGCTGGTGCTGCAGCTT
ATTATGTGGGTTATCTTCAACCTAGGACTTTTCTATTAAAATATAATGAAAAT
GGAACCATTACAGATGCTGTAGACTGTGCACTTGACCCTCTCTCAGAAACAA
AGTGTACGTTGAAATCCTTCACTGTAGAAAAAGGAATCTATCAAACTTCTAAC
TTTAGAGTCCAACCAACAGAATCTATTGTTAGATTTCCTAATATTACAAACTT
GTGCCCTTTTGGTGAAGTTTTTAACGCCACCAGATTTGCATCTGTTTATGCTTG
GAACAGGAAGAGAATCAGCAACTGTGTTGCTGATTATTCTGTCCTATATAATT
CCGCATCATTTTCCACTTTTAAGTGTTATGGAGTGTCTCCTACTAAATTAAATG
ATCTCTGCTTTACTAATGTCTATGCAGATTCATTTGTAATTAGAGGTGATGAA
GTCAGACAAATCGCTCCAGGGCAAACTGGAAAGATTGCTGATTATAATTATA
AATTACCAGATGATTTTACAGGCTGCGTTATAGCTTGGAATTCTAACAATCTT
GATTCTAAGGTTGGTGGTAATTATAATTACCTGTATAGATTGTTTAGGAAGTC
TAATCTCAAACCTTTTGAGAGAGATATTTCAACTGAAATCTATCAGGCCGGTA
GCACACCTTGTAATGGTGTTGAAGGTTTTAATTGTTACTTTCCTTTACAATCAT
ATGGTTTCCAACCCACTAATGGTGTTGGTTACCAACCATACAGAGTAGTAGTA
CTTTCTTTTGAACTTCTACATGCACCAGCAACTGTTTGTGGACCTAAAAAGTC
 
TACTAATTTGGTTAAAAACAAATGTGTCAATTTCAACTTCAATGGTTTAACAG
GCACAGGTGTTCTTACTGAGTCTAACAAAAAGTTTCTGCCTTTCCAACAATTT
GGCAGAGACATTGCTGACACTACTGATGCTGTCCGTGATCCACAGACACTTG
AGATTCTTGACATTACACCATGTTCTTTTGGTGGTGTCAGTGTTATAACACCA
GGAACAAATACTTCTAACCAGGTTGCTGTTCTTTATCAGGATGTTAACTGCAC
AGAAGTCCCTGTTGCTATTCATGCAGATCAACTTACTCCTACTTGGCGTGTTT
ATTCTACAGGTTCTAATGTTTTTCAAACACGTGCAGGCTGTTTAATAGGGGCT
GAACATGTCAACAACTCATATGAGTGTGACATACCCATTGGTGCAGGTATAT
GCGCTAGTTATCAGACTCAGACTAATTCTCCTCGGCGGGCACGTAGTGTAGCT
AGTCAATCCATCATTGCCTACACTATGTCACTTGGTGCAGAAAATTCAGTTGC
TTACTCTAATAACTCTATTGCCATACCCACAAATTTTACTATTAGTGTTACCAC
AGAAATTCTACCAGTGTCTATGACCAAGACATCAGTAGATTGTACAATGTAC
ATTTGTGGTGATTCAACTGAATGCAGCAATCTTTTGTTGCAATATGGCAGTTT
TTGTACACAATTAAACCGTGCTTTAACTGGAATAGCTGTTGAACAAGACAAA
AACACCCAAGAAGTTTTTGCACAAGTCAAACAAATTTACAAAACACCACCAA
TTAAAGATTTTGGTGGTTTTAATTTTTCACAAATATTACCAGATCCATCAAAA
CCAAGCAAGAGGTCATTTATTGAAGATCTACTTTTCAACAAAGTGACACTTGC
AGATGCTGGCTTCATCAAACAATATGGTGATTGCCTTGGTGATATTGCTGCTA
GAGACCTCATTTGTGCACAAAAGTTTAACGGCCTTACTGTTTTGCCACCTTTG
CTCACAGATGAAATGATTGCTCAATACACTTCTGCACTGTTAGCGGGTACAAT
CACTTCTGGTTGGACCTTTGGTGCAGGTGCTGCATTACAAATACCATTTGCTA
TGCAAATGGCTTATAGGTTTAATGGTATTGGAGTTACACAGAATGTTCTCTAT
GAGAACCAAAAATTGATTGCCAACCAATTTAATAGTGCTATTGGCAAAATTC
AAGACTCACTTTCTTCCACAGCAAGTGCACTTGGAAAACTTCAAGATGTGGTC
AACCAAAATGCACAAGCTTTAAACACGCTTGTTAAACAACTTAGCTCCAATTT
TGGTGCAATTTCAAGTGTTTTAAATGATATCCTTTCACGTCTTGACAAAGTTG
AGGCTGAAGTGCAAATTGATAGGTTGATCACAGGCAGACTTCAAAGTTTGCA
GACATATGTGACTCAACAATTAATTAGAGCTGCAGAAATCAGAGCTTCTGCT
 
AATCTTGCTGCTACTAAAATGTCAGAGTGTGTACTTGGACAATCAAAAAGAG
TTGATTTTTGTGGAAAGGCTATCATCTTATGTCCTTCCCTCAGTCAGCACCTC
ATGGTGTAGTCTTCTTGCATGTGACTTATGTCCCTGCACAAGAAAAGAACTTC
ACAACTGCTCCTGCCATTTGTCATGATGGAAAAGCACACTTTCCTCGTGAAGG
TGTCTTTGTTTCAAATGGCACACACTGGTTTGTAACACAAAGGAATTTTTATG
AACCACAAATCATTACTACAGACAACACATTTGTGTCTGGTAACTGTGATGTT
GTAATAGGAATTGTCAACAACACAGTTTATGATCCTTTGCAACCTGAATTAGA
CTCATTCAAGGAGGAGTTAGATAAATATTTTAAGAATCATACATCACCAGAT
GTTGATTTAGGTGACATCTCTGGCATTAATGCTTCAGTTGTAAACATTCAAAA
AGAAATTGACCGCCTCAATGAGGTTGCCAAGAATTTAAATGAATCTCTCATCG
ATCTCCAAGAACTTGGAAAGTATGAGCAGTATATAAAATGGCCATGGTACAT
TTGGCTAGGTTTTATAGCTGGCTTGATTGCCATAGTAATGGTGACAATTATGC
TTTGCTGTATGACCAGTTGCTGTAGTTGTCTCAAGGGCTGTTGTTCTTGTGGAT
CCTGCTGCAAATTTGATGAAGACGACTCTGAGCCAGTGCTCAAAGGAGTCAA
ATTACATTACACATAAGTCGCTTGCAAAGACAACGCCACATTTTGGGAACGC
GTCCGATCCATCCTGAAATCTGGGCTCAACTTTGCTTCGACCATACCTGGCCC
CGTAGGAGTGGCAGCGACAGGGATCAAAGGCATCATTGAAGCCATTGGTTCC
TTGTGGGTTTGATTCCACCCACAGAAGCGTTGACGACGCAAAACGTCCTTAAA
GCGTTGACGACGCAAAACGTCCCCAAGCTCGTAGCACCGACCCTATACCCAT
CTCTAGGGTCTTCAACCTCTTGGT
Fusion construct SARS-CoV2 spike Wuhan Hu-1 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
 
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
 
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGTTTGTTTTTCTTGTTTTATTGCCACTAGTCTCTAGTC
 
AGTGTGTTAATCTTACAACCAGAACTCAATTACCCCCTGCATACACTAATTCT
TTCACACGTGGTGTTTATTACCCTGACAAAGTTTTCAGATCCTCAGTTTACAT
TCAACTCAGGACTTGTTCTTACCTTTCTTTTCCAATGTTACTTGGTTCCATGCT
ATACATGTCTCTGGGACCAATGGTACTAAGAGGTTTGATAACCCTGTCCTACC
ATTTAATGATGGTGTTTATTTTGCTTCCACTGAGAAGTCTAACATAATAAGAG
GCTGGATTTTTGGTACTACTTTAGATTCGAAGACCCAGTCCCTACTTATTGTTA
ATAACGCTACTAATGTTGTTATTAAAGTCTGTGAATTTCAATTTTGTAATGATC
CATTTTTGGGTGTTTATTACCACAAAAACAACAAAAGTTGGATGGAAAGTGA
GTTCAGAGTTTATTCTAGTGCGAATAATTGCACTTTTGAATATGTCTCTCAGCC
TTTTCTTATGGACCTTGAAGGAAAACAGGGTAATTTCAAAAATCTTAGGGAAT
TTGTGTTTAAGAATATTGATGGTTATTTTAAAATATATTCTAAGCACACGCCT
ATTAATTTAGTGCGTGATCTCCCTCAGGGTTTTTCGGCTTTAGAACCATTGGTA
GATTTGCCAATAGGTATTAACATCACTAGGTTTCAAACTTTACTTGCTTTACAT
AGAAGTTATTTGACTCCTGGTGATTCTTCTTCAGGTTGGACAGCTGGTGCTGC
AGCTTATTATGTGGGTTATCTTCAACCTAGGACTTTTCTATTAAAATATAATG
AAAATGGAACCATTACAGATGCTGTAGACTGTGCACTTGACCCTCTCTCAGAA
ACAAAGTGTACGTTGAAATCCTTCACTGTAGAAAAAGGAATCTATCAAACTT
CTAACTTTAGAGTCCAACCAACAGAATCTATTGTTAGATTTCCTAATATTACA
AACTTGTGCCCTTTTGGTGAAGTTTTTAACGCCACCAGATTTGCATCTGTTTAT
GCTTGGAACAGGAAGAGAATCAGCAACTGTGTTGCTGATTATTCTGTCCTATA
TAATTCCGCATCATTTTCCACTTTTAAGTGTTATGGAGTGTCTCCTACTAAATT
AAATGATCTCTGCTTTACTAATGTCTATGCAGATTCATTTGTAATTAGAGGTG
ATGAAGTCAGACAAATCGCTCCAGGGCAAACTGGAAAGATTGCTGATTATAA
TTATAAATTACCAGATGATTTTACAGGCTGCGTTATAGCTTGGAATTCTAACA
ATCTTGATTCTAAGGTTGGTGGTAATTATAATTACCTGTATAGATTGTTTAGG
AAGTCTAATCTCAAACCTTTTGAGAGAGATATTTCAACTGAAATCTATCAGGC
CGGTAGCACACCTTGTAATGGTGTTGAAGGTTTTAATTGTTACTTTCCTTTACA
ATCATATGGTTTCCAACCCACTAATGGTGTTGGTTACCAACCATACAGAGTAG
 
TAGTACTTTCTTTTGAACTTCTACATGCACCAGCAACTGTTTGTGGACCTAAA
AAGTCTACTAATTTGGTTAAAAACAAATGTGTCAATTTCAACTTCAATGGTTT
AACAGGCACAGGTGTTCTTACTGAGTCTAACAAAAAGTTTCTGCCTTTCCAAC
AATTTGGCAGAGACATTGCTGACACTACTGATGCTGTCCGTGATCCACAGACA
CTTGAGATTCTTGACATTACACCATGTTCTTTTGGTGGTGTCAGTGTTATAACA
CCAGGAACAAATACTTCTAACCAGGTTGCTGTTCTTTATCAGGATGTTAACTG
CACAGAAGTCCCTGTTGCTATTCATGCAGATCAACTTACTCCTACTTGGCGTG
TTTATTCTACAGGTTCTAATGTTTTTCAAACACGTGCAGGCTGTTTAATAGGG
GCTGAACATGTCAACAACTCATATGAGTGTGACATACCCATTGGTGCAGGTAT
ATGCGCTAGTTATCAGACTCAGACTAATTCTCCTCGGCGGGCACGTAGTGTAG
CTAGTCAATCCATCATTGCCTACACTATGTCACTTGGTGCAGAAAATTCAGTT
GCTTACTCTAATAACTCTATTGCCATACCCACAAATTTTACTATTAGTGTTACC
ACAGAAATTCTACCAGTGTCTATGACCAAGACATCAGTAGATTGTACAATGT
ACATTTGTGGTGATTCAACTGAATGCAGCAATCTTTTGTTGCAATATGGCAGT
TTTTGTACACAATTAAACCGTGCTTTAACTGGAATAGCTGTTGAACAAGACAA
AAACACCCAAGAAGTTTTTGCACAAGTCAAACAAATTTACAAAACACCACCA
ATTAAAGATTTTGGTGGTTTTAATTTTTCACAAATATTACCAGATCCATCAAA
ACCAAGCAAGAGGTCATTTATTGAAGATCTACTTTTCAACAAAGTGACACTTG
CAGATGCTGGCTTCATCAAACAATATGGTGATTGCCTTGGTGATATTGCTGCT
AGAGACCTCATTTGTGCACAAAAGTTTAACGGCCTTACTGTTTTGCCACCTTT
GCTCACAGATGAAATGATTGCTCAATACACTTCTGCACTGTTAGCGGGTACAA
TCACTTCTGGTTGGACCTTTGGTGCAGGTGCTGCATTACAAATACCATTTGCT
ATGCAAATGGCTTATAGGTTTAATGGTATTGGAGTTACACAGAATGTTCTCTA
TGAGAACCAAAAATTGATTGCCAACCAATTTAATAGTGCTATTGGCAAAATTC
AAGACTCACTTTCTTCCACAGCAAGTGCACTTGGAAAACTTCAAGATGTGGTC
AACCAAAATGCACAAGCTTTAAACACGCTTGTTAAACAACTTAGCTCCAATTT
TGGTGCAATTTCAAGTGTTTTAAATGATATCCTTTCACGTCTTGACAAAGTTG
AGGCTGAAGTGCAAATTGATAGGTTGATCACAGGCAGACTTCAAAGTTTGCA
 
GACATATGTGACTCAACAATTAATTAGAGCTGCAGAAATCAGAGCTTCTGCT
AATCTTGCTGCTACTAAAATGTCAGAGTGTGTACTTGGACAATCAAAAAGAG
TTGATTTTTGTGGAAAGGGCTATCATCTTATGTCCTTCCCTCAGTCAGCACCTC
ATGGTGTAGTCTTCTTGCATGTGACTTATGTCCCTGCACAAGAAAAGAACTTC
ACAACTGCTCCTGCCATTTGTCATGATGGAAAAGCACACTTTCCTCGTGAAGG
TGTCTTTGTTTCAAATGGCACACACTGGTTTGTAACACAAAGGAATTTTTATG
AACCACAAATCATTACTACAGACAACACATTTGTGTCTGGTAACTGTGATGTT
GTAATAGGAATTGTCAACAACACAGTTTATGATCCTTTGCAACCTGAATTAGA
CTCATTCAAGGAGGAGTTAGATAAATATTTTAAGAATCATACATCACCAGAT
GTTGATTTAGGTGACATCTCTGGCATTAATGCTTCAGTTGTAAACATTCAAAA
AGAAATTGACCGCCTCAATGAGGTTGCCAAGAATTTAAATGAATCTCTCATCG
ATCTCCAAGAACTTGGAAAGTATGAGCAGTATATAAAATGGCCATGGTACAT
TTGGCTAGGTTTTATAGCTGGCTTGATTGCCATAGTAATGGTGACAATTATGC
TTTGCTGTATGACCAGTTGCTGTAGTTGTCTCAAGGGCTGTTGTTCTTGTGGAT
CCTGCTGCAAATTTGATGAAGACGACTCTGAGCCAGTGCTCAAAGGAGTCAA
ATTACATTACACATAAGTCGCTTGCAAAGACAACGCCACATTTGGGAACGC
GTCCGATCCATCCTGAAATCTGGGCTCAACTTTGCTTCGACCATACCTGGCCC
CGTAGGAGTGGCAGCGACAGGGATCAAAGGCATCATTGAAGCCATTGGTTCC
TTGTGGGTTTGATTCCACCCACAGAAGCGTTGACGACGCAAAACGTCCTTAAA
GCGTTGACGACGCAAAACGTCCCCAAGCTCGTAGCACCGACCCTATACCCAT
CTCTAGGGTCTTCAACCTCTTGGT
Receptor binding domain RBD from SARS-CoV2 spike strain Wuhan Hu-1 to be applied together with RNA1 
GTAAACAACCAATAACATCAGGGTATCCAAAGCAGCACGCCGCCGAA
GAGCGGCCACAAACTTGTGCCCTTTTGGTGAAGTTTTAACGCCACCAGATTT
GCATCTGTTTATGCTTGGAACAGGAAGAGAATCAGCAACTGTGTTGCTGATTA
TTCTGTCCTATATAATTCCGCATCATTTTCCACTTTTAAGTGTTATGGAGTGTC
TCCTACTAAATTAAATGATCTCTGCTTTACTAATGTCTATGCAGATTCATTTGT
 
AATTAGAGGTGATGAAGTCAGACAAATCGCTCCAGGGCAAACTGGAAAGATT
GCTGATTATAATTATAAATTACCAGATGATTTACAGGCTGCGTTATAGCTTG
GAATTCTAACAATCTTGATTCTAAGGTTGGTGGTAATTATAATTACCTGTATA
GATTGTTTAGGAAGTCTAATCTCAAACCTTTTGAGAGAGATATTTCAACTGAA
ATCTATCAGGCCGGTAGCACACCTTGTAATGGTGTTGAAGGTTTTAATTGTTA
CTTTCCTTTACAATCATATGGTTTCCAACCCACTAATGGTGTTGGTTACCAACC
ATACAGAGTAGTAGTACTTTCTTTTGAACTTCTACATGCACCAGCAACTGTTT
GTGGAGTCGCTTGCAAAGACAACGCCACATTTGGGAACGCGTCCGATCCAT
CCTGAAATCTGGGCTCAACTTTGCTTCGACCATACCTGGCCCCGTAGGAGTGG
CAGCGACAGGGATCAAAGGCATCATTGAAGCCATTGGTTCCTTGTGGGTTTG
ATTCCACCCACAGAAGCGTTGACGACGCAAAACGTCCTTAAAGCGTTGACGA
CGCAAAACGTCCCCAAGCTCGTAGCACCGACCCTATACCCATCTCTAGGGTCT
TCAACCTCTTGGT
Fusion construct RBD domain SARS-CoV2 spike strain Wuhan Hu-1 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
 
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
 
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCACAAACTTGTGCCCTTTTGGTGAAGTTTTAACGCCACC
AGATTTGCATCTGTTTATGCTTGGAACAGGAAGAGAATCAGCAACTGTGTTGC
TGATTATTCTGTCCTATATAATTCCGCATCATTTCCACTTTTAAGTGTTATGG
AGTGTCTCCTACTAAATTAAATGATCTCTGCTTTACTAATGTCTATGCAGATTC
ATTTGTAATTAGAGGTGATGAAGTCAGACAAATCGCTCCAGGGCAAACTGGA
AAGATTGCTGATTATAATTATAAATTACCAGATGATTTTACAGGCTGCGTTAT
AGCTTGGAATTCTAACAATCTTGATTCTAAGGTTGGTGGTAATTATAATTACC
TGTATAGATTGTTTAGGAAGTCTAATCTCAAACCTTTTGAGAGAGATATTTCA
 
ACTGAAATCTATCAGGCCGGTAGCACACCTTGTAATGGTGTTGAAGGTTTTAA
TTGTTACTTTCCTTTACAATCATATGGTTTCCAACCCACTAATGGTGTTGGTTA
CCAACCATACAGAGTAGTAGTACTTTCTTTTGAACTTCTACATGCACCAGCAA
CTGTTTGTGGAGTCGCTTGCAAAGACAACGCCACATTTTGGGAACGCGTCCGA
TCCATCCTGAAATCTGGGCTCAACTTTGCTTCGACCATACCTGGCCCCGTAGG
AGTGGCAGCGACAGGGATCAAAGGCATCATTGAAGCCATTGGTTCCTTGTGG
GTTTGATTCCACCCACAGAAGCGTTGACGACGCAAAACGTCCTTAAAGCGTT
GACGACGCAAAACGTCCCCAAGCTCGTAGCACCGACCCTATACCCATCTCTA
GGGTCTTCAACCTCTTGGT
Rabies virus G to be applied together with RNA1 
GTAAACAACCAATAACATCAGGGTATCCAAAGCAGCACGCCGCCGAA
GAGCGGCCATGGTTCCTCAGGCTCTCCTGTTTGTACCCCTTCTGGTTTTCCAT
TGTGTTTTGGGAAATTCCCTATTTACACGATACCAGACAAGCTTGGTCCCTGG
AGCCCGATTGACATACATCACCTCAGCTGCCCAAACAATTTGGTATTGGAGG
ACGAAGGATGCACCAACCTGTCAGGGTTCTCCTACATGGAACTTAAAGTTGG
ATACATCTCAGCCATAAAAATGAACGGGTTCACTTGCACAGGCGTTGTGACG
GAGGCTGAAACCTACACTAACTTCGTTGGTTATGTCACAACCACGTTCAAAAG
AAAGCATTTCCGCCCAACACCAGATGCATGTAGAGCCGCGTACAACTGGAAG
ATGGCCGGTGACCCCAGATATGAAGAGTCTCTACACAATCCGTACCCTGACT
ACCACTGGCTTCGAACTGTAAAAACCACCAAGGAGTCTCTCGTTATCATATCT
CCAAGTGTGGCAGATTTGGACCCATATGACAGATCCCTTCACTCGAGGGTCTT
CCCTGGCGGGAATTGCTCAGGAGTAGCGGTGTCTTCTACCTACTGCTCCACTA
ACCACGATTACACCATTTGGATGCCCGAGAATCCGAGACTAGGGATGTCTTGT
GACATTTTTACCAATAGTAGAGGGAAGAGAGCATCCAAAGGGAGTGAGACTT
GCGGCTTTGTAGATGAAAGAGGCCTATATAAGTCTTTAAAAGGAGCATGCAA
ACTCAAGTTATGTGGAGTTCTCGGACTTAGACTTATGGATGGAACATGGGTCG
CGATGCAAACATCAAATGAAACCAAATGGTGCCCTCCCGGTCAGTTGGTGAA
TTTGCACGACTTTCGCTCAGACGAAATTGAGCACCTTGTTGTAGAGGAGTTGG
 
TCAAGAAGAGAGAGGAGTGTCTGGATGCACTAGAGTCCATCATGACCACCAA
GTCAGTGAGTTTCAGACGTCTCAGTCATTTAAGAAAACTTGTCCCTGGGTTTG
GAAAAGCATATACCATATTCAACAAGACCTTGATGGAAGCCGATGCTCACTA
CAAGTCAGTCAGAACTTGGAATGAGATCATCCCTTCAAAAGGGTGTTTAAGA
GTTGGGGGGAGGTGTCATCCTCATGTAAACGGGGTATTTTTCAATGGTATAAT
ATTAGGACCTGACGGCAATGTCTTAATCCCAGAGATGCAATCATCCCTCCTCC
AGCAACATATGGAGTTGTTGGTATCCTCGGTTATCCCCCTTATGCACCCCCTG
GCAGACCCGTCTACCGTTTTCAAGAACGGTGACGAGGCTGAGGATTTTGTTGA
AGTTCACCTTCCCGATGTGCACGAACGGATCTCAGGAGTTGACTTGGGTCTCC
CGAACTGGGGGAAGTATGTATTACTGAGTGCAGGGGCCCTGACTGCCTTGAT
GTTGATAATTTTCCTGATGACATGCTGGAGAAGAGTCAATCGATCGGAACCTA
CACAACACAATCTCAGAGGGACAGGGAGGGAGGTGTCAGTCACTCCCCAAAG
CGGGAAGATCATATCTTCATGGGAATCATACAAGAGCGGGGGTGAGACCGGA
CTGTGAGTCGCTTGCAAAGACAACGCCACATTTTGGGAACGCGTCCGATCCAT
CCTGAAATCTGGGCTCAACTTTGCTTCGACCATACCTGGCCCCGTAGGAGTGG
CAGCGACAGGGATCAAAGGCATCATTGAAGCCATTGGTTCCTTGTGGGTTTG
ATTCCACCCACAGAAGCGTTGACGACGCAAAACGTCCTTAAAGCGTTGACGA
CGCAAAACGTCCCCAAGCTCGTAGCACCGACCCTATACCCATCTCTAGGGTCT
TCAACCTCTTGGT
Fusion construct rabies virus G 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
 
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
 
AGCGCAGCGAGACCTCAGGTCGTATACCGACATGCTGGTCTCATGCCCCGCG
AGGGCGAAGCGATTCGGTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGGTTCCTCAGGCTCTCCTGTTTGTACCCCTTCTGGTT
TTTCCATTGTGTTTTGGGAAATTCCCTATTTACACGATACCAGACAAGCTTGG
 
TCCCTGGAGCCCGATTGACATACATCACCTCAGCTGCCCAAACAATTTGGTAT
TGGAGGACGAAGGATGCACCAACCTGTCAGGGTTCTCCTACATGGAACTTAA
AGTTGGATACATCTCAGCCATAAAAATGAACGGGTTCACTTGCACAGGCGTT
GTGACGGAGGCTGAAACCTACACTAACTTCGTTGGTTATGTCACAACCACGTT
CAAAAGAAAGCATTTCCGCCCAACACCAGATGCATGTAGAGCCGCGTACAAC
TGGAAGATGGCCGGTGACCCCAGATATGAAGAGTCTCTACACAATCCGTACC
CTGACTACCACTGGCTTCGAACTGTAAAAACCACCAAGGAGTCTCTCGTTATC
ATATCTCCAAGTGTGGCAGATTTGGACCCATATGACAGATCCCTTCACTCGAG
GGTCTTCCCTGGCGGGAATTGCTCAGGAGTAGCGGTGTCTTCTACCTACTGCT
CCACTAACCACGATTACACCATTTGGATGCCCGAGAATCCGAGACTAGGGAT
GTCTTGTGACATTTTTACCAATAGTAGAGGGAAGAGAGCATCCAAAGGGAGT
GAGACTTGCGGCTTTGTAGATGAAAGAGGCCTATATAAGTCTTTAAAAGGAG
CATGCAAACTCAAGTTATGTGGAGTTCTCGGACTTAGACTTATGGATGGAACA
TGGGTCGCGATGCAAACATCAAATGAAACCAAATGGTGCCCTCCCGGTCAGT
TGGTGAATTTGCACGACTTTCGCTCAGACGAAATTGAGCACCTTGTTGTAGAG
GAGTTGGTCAAGAAGAGAGAGGAGTGTCTGGATGCACTAGAGTCCATCATGA
CCACCAAGTCAGTGAGTTTCAGACGTCTCAGTCATTTAAGAAAACTTGTCCCT
GGGTTTGGAAAAGCATATACCATATTCAACAAGACCTTGATGGAAGCCGATG
CTCACTACAAGTCAGTCAGAACTTGGAATGAGATCATCCCTTCAAAAGGGTG
TTTAAGAGTTGGGGGGAGGTGTCATCCTCATGTAAACGGGGTATTTTTCAATG
GTATAATATTAGGACCTGACGGCAATGTCTTAATCCCAGAGATGCAATCATCC
CTCCTCCAGCAACATATGGAGTTGTTGGTATCCTCGGTTATCCCCCTTATGCA
CCCCCTGGCAGACCCGTCTACCGTTTTCAAGAACGGTGACGAGGCTGAGGAT
TTTGTTGAAGTTCACCTTCCCGATGTGCACGAACGGATCTCAGGAGTTGACTT
GGGTCTCCCGAACTGGGGGAAGTATGTATTACTGAGTGCAGGGGCCCTGACT
GCCTTGATGTTGATAATTTTCCTGATGACATGCTGGAGAAGAGTCAATCGATC
GGAACCTACACAACACAATCTCAGAGGGACAGGGAGGGAGGTGTCAGTCACT
CCCCAAAGCGGGAAGATCATATCTTCATGGAATCATACAAGAGCGGGGGTG
 
AGACCGGACTGTGAGTCGCTTGCAAAGACAACGCCACATTTTGGGAACGCGT
CCGATCCATCCTGAAATCTGGGCTCAACTTTGCTTCGACCATACCTGGCCCCG
TAGGAGTGGCAGCGACAGGGATCAAAGGCATCATTGAAGCCATTGGTTCCTT
GTGGGTTTGATTCCACCCACAGAAGCGTTGACGACGCAAAACGTCCTTAAAG
CGTTGACGACGCAAAACGTCCCCAAGCTCGTAGCACCGACCCTATACCCATCT
CTAGGGTCTTCAACCTCTTGGT
MERS spike England 1 isolate H123990006 to be applied together with RNA1 
GTAAACAACCAATAACATCAGGGTATCCAAAGCAGCACGCCGCCGAA
GAGCGGCCATGATACACTCAGTGTTTCTACTGATGTTCTTGTTAACACCTACA
GAAAGTTACGTTGATGTAGGGCCAGATTCTGTTAAGTCTGCTTGTATTGAGGT
TGATATACAACAGACTTTCTTTGATAAAACTTGGCCTAGGCCAATTGATGTTT
CTAAGGCTGACGGTATTATATACCCTCAAGGCCGTACATATTCTAACATAACT
ATCACTTATCAAGGTCTTTTTCCCTATCAGGGAGACCATGGTGATATGTATGT
TTACTCTGCAGGACATGCTACAGGCACAACTCCACAAAAGTTGTTTGTAGCTA
ACTATTCTCAGGACGTCAAACAGTTTGCTAATGGGTTTGTCGTCCGTATAGGA
GCAGCTGCCAATTCCACTGGCACTGTTATTATTAGCCCATCTACCAGCGCTAC
TATACGAAAAATTTACCCTGCTTTTATGCTGGGTTCTTCAGTTGGTAATTTCTC
AGATGGTAAAATGGGCCGCTTCTTCAATCATACTCTAGTTCTTTTGCCCGATG
GATGTGGCACTTTACTTAGAGCTTTTTATTGTATTCTAGAGCCTCGCTCTGGAA
ATCATTGTCCTGCTGGCAATTCCTATACTTCTTTTGCCACTTATCACACTCCTG
CAACAGATTGTTCTGATGGCAATTACAATCGTAATGCCAGTCTGAACTCTTTT
AAGGAGTATTTTAATTTACGTAACTGCACCTTTATGTACACTTATAACATTAC
CGAAGATGAGATTTTAGAGTGGTTTGGCATTACACAAACTGCTCAAGGTGTTC
ACCTCTTCTCATCTCGGTATGTTGATTTGTACGGCGGCAATATGTTTCAATTTG
CCACCTTGCCTGTTTATGATACTATTAAGTATTATTCTATCATTCCTCACAGTA
TTCGTTCTATCCAAAGTGATAGAAAAGCTTGGGCTGCCTTCTACGTATATAAA
CTTCAACCGTTAACTTTCCTGTTGGATTTTTCTGTTGATGGTTATATACGCAGA
GCTATAGACTGTGGTTTTAATGATTTGTCACAACTCCACTGCTCATATGAATC
 
CTTCGATGTTGAATCTGGAGTTTATTCAGTTTCGTCTTTCGAAGCAAAACCTTC
TGGCTCAGTTGTGGAACAGGCTGAAGGTGTTGAATGTGATTTTTCACCTCTTC
TGTCTGGCACACCTCCTCAGGTTTATAATTTCAAGCGTTTGGTTTTACCAATT
GCAATTATAATCTTACCAAATTGCTTTCACTTTTTCTGTGAATGATTTTACTT
GTAGTCAAATATCTCCAGCAGCAATTGCTAGTAACTGTTATTCTTCACTGATT
TTGGATTATTTTCATACCCACTTAGTATGAAATCCGATCTCAGTGTTAGTTCT
GCTGGTCCAATATCCCAGTTTAATTATAAACAGTCCTTTTCTAATCCCACATGT
TTGATTTTAGCGACTGTTCCTCATAACCTTACTACTATTACTAAGCCTCTTAAG
TACAGCTATATTAACAAGTGCTCTCGTTTTCTTTCTGATGATCGTACTGAAGTA
CCTCAGTTAGTGAACGCTAATCAATACTCACCCTGTGTATCCATTGTCCCATC
CACTGTGTGGGAAGACGGTGATTATTATAGGAAACAACTATCTCCACTTGAA
GGTGGTGGCTGGCTTGTTGCTAGTGGCTCAACTGTTGCCATGACTGAGCAATT
ACAGATGGGCTTTGGTATTACAGTTCAATATGGTACAGACACCAATAGTGTTT
GCCCCAAGCTTGAATTTGCTAATGACACAAAAATTGCCTCTCAATTAGGCAAT
TGCGTGGAATATTCCCTCTATGGTGTTTCGGGCCGTGGTGTTTTTCAGAATTGC
ACAGCTGTAGGTGTTCGACAGCAGCGCTTTGTTTATGATGCGTACCAGAATTT
AGTTGGCTATTATTCTGATGATGGCAACTACTACTGTTTGCGTGCTTGTGTTAG
TGTTCCTGTTTCTGTCATCTATGATAAAGAAACTAAAACCCACGCTACTCTAT
TTGGTAGTGTTGCATGTGAACACATTTCCTCTACCATGTCTCAATACTCCCGTT
CTACGCGATCAATGCTTAAACGGCGAGATTCTACATATGGCCCCCTTCAGACA
CCTGTTGGTTGTGTCCTAGGACTTGTTAATTCCTCTTTGTTCGTAGAGGACTGC
AAGTTGCCTCTTGGTCAATCTCTCTGTGCTCTTCCTGACACACCTAGTACTCTC
ACACCTCGCAGTGTGCGCTCTGTTCCAGGTGAAATGCGCTTGGCATCCATTGC
TTTTAATCATCCTATTCAGGTTGATCAACTTAATAGTAGTTATTTTAAATTAAG
TATACCCACTAATTTTTCCTTTGGTGTGACTCAGGAGTACATTCAGACAACCA
TTCAGAAAGTTACTGTTGATTGTAAACAGTACGTTTGCAATGGTTTCCAGAAG
TGTGAGCAATTACTGCGCGAGTATGGCCAGTTTTGTTCCAAAATAAACCAGGC
TCTCCATGGTGCCAATTTACGCCAGGATGATTCTGTACGTAATTTGTTTGCGA
 
GCGTGAAAAGCTCTCAATCATCTCCTATCATACCAGGTTTTGGAGGTGACTTT
AATTTGACACTTCTAGAACCTGTTTCTATATCTACTGGCAGTCGTAGTGCACG
TAGTGCTATTGAGGATTTGCTATTTGACAAAGTCACTATAGCTGATCCTGGTT
ATATGCAAGGTTACGATGATTGCATGCAGCAAGGTCCAGCATCAGCTCGTGA
TCTTATTTGTGCTCAATATGTGGCTGGTTACAAAGTATTACCTCCTCTTATGGA
TGTTAATATGGAAGCCGCGTATACCTCATCTTTGCTTGGCAGCATAGCAGGTG
TTGGCTGGACTGCTGGCTTATCCTCCTTTGCTGCTATTCCATTTGCACAGAGTA
TCTTTTATAGGTTAAACGGTGTTGGCATTACTCAACAGGTTCTTTCAGAGAAC
CAAAAGCTTATTGCCAATAAGTTTAATCAGGCTCTGGGAGCTATGCAAACAG
GCTTCACTACAACTAATGAAGCTTTTCACAAGGTTCAGGATGCTGTGAACAAC
AATGCACAGGCTCTATCCAAATTAGCTAGCGAGCTATCTAATACTTTTGGTGC
TATTTCCGCCTCTATTGGAGACATCATACAACGTCTTGATGTTCTCGAACAGG
ACGCCCAAATAGACAGACTTATTAATGGCCGTTTGACAACACTAAATGCTTTT
GTTGCACAGCAGCTTGTTCGTTCCGAATCAGCTGCTCTTTCGGCTCAATTGGC
TAAAGATAAAGTCAATGAGTGTGTCAAGGCACAATCCAAGCGTTCTGGATTT
TGCGGTCAAGGCACACATATAGTGTCCTTTGTTGTAAATGCCCCTAATGGCCT
TTACTTCATGCATGTTGGTTATTACCCTAGCAACCACATTGAGGTTGTTTCTGC
TTATGGTCTTTGCGATGCAGCTAACCCTACTAATTGTATAGCCCCTGTTAATG
GCTACTTTATTAAAACTAATAACACTAGGATTGTTGATGAGTGGTCATATACT
GGCTCGTCCTTCTATGCACCTGAGCCCATCACCTCTCTTAATACTAAGTATGTT
GCACCACAGGTGACATACCAAAACATTTCTACTAACCTCCCTCCTCCTCTTCT
CGGCAATTCCACCGGGATTGACTTCCAAGATGAGTTGGATGAGTTTTTCAAAA
ATGTTAGCACCAGTATACCTAATTTTGGTTCTCTAACACAGATTAATACTACA
TTACTCGATCTTACCTACGAGATGTTGTCTCTTCAACAAGTTGTTAAAGCCCTT
AATGAGTCTTACATAGACCTTAAAGAGCTTGGCAATTATACTTATTACAACAA
ATGGCCGTGGTACATTTGGCTTGGTTTCATTGCTGGGCTTGTTGCCTTAGCTCT
ATGCGTCTTCTTCATACTGTGCTGCACTGGTTGTGGCACAAACTGTATGGGAA
AACTTAAGTGTAATCGTTGTTGTGATAGATACGAGGAATACGACCTCGAGCC
 
GCATAAGGTTCATGTTCACTAAGTCGCTTGCAAAGACAACGCCACATTTTGGG
AACGCGTCCGATCCATCCTGAAATCTGGGCTCAACTTTGCTTCGACCATACCT
GGCCCCGTAGGAGTGGCAGCGACAGGGATCAAAGGCATCATTGAAGCCATTG
GTTCCTTGTGGGTTTGATTCCACCCACAGAAGCGTTGACGACGCAAAACGTCC
TTAAAGCGTTGACGACGCAAAACGTCCCCAAGCTCGTAGCACCGACCCTATA
CCCATCTCTAGGGTCTTCAACCTCTTGGT
Fusion construct MERS spike England 1 isolate H123990006 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
 
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
 
GCACGTACCGGAGTAGACCTCGAGACACTTCGGTCTCTGGATCAGCGTCTAG
GAGAAATCACTGACGTCTGGGCGGATATTACCATCAACCGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGATACACTCAGTGTTTCTACTGATGTTCTTGTTAACA
CCTACAGAAAGTTACGTTGATGTAGGGCCAGATTCTGTTAAGTCTGCTTGTAT
TGAGGTTGATATACAACAGACTTTCTTTGATAAAACTTGGCCTAGGCCAATTG
ATGTTTCTAAGGCTGACGGTATTATATACCCTCAAGGCCGTACATATTCTAAC
ATAACTATCACTTATCAAGGTCTTTTTCCCTATCAGGGAGACCATGGTGATAT
GTATGTTTACTCTGCAGGACATGCTACAGGCACAACTCCACAAAAGTTGTTTG
TAGCTAACTATTCTCAGGACGTCAAACAGTTTGCTAATGGGTTTGTCGTCCGT
ATAGGAGCAGCTGCCAATTCCACTGGCACTGTTATTATTAGCCCATCTACCAG
CGCTACTATACGAAAAATTTACCCTGCTTTTATGCTGGGTTCTTCAGTTGGTA
ATTTCTCAGATGGTAAAATGGGCCGCTTCTTCAATCATACTCTAGTTCTTTTGC
CCGATGGATGTGGCACTTTACTTAGAGCTTTTTATTGTATTCTAGAGCCTCGCT
CTGGAAATCATTGTCCTGCTGGCAATTCCTATACTTCTTTTGCCACTTATCACA
CTCCTGCAACAGATTGTTCTGATGGCAATTACAATCGTAATGCCAGTCTGAAC
TCTTTTAAGGAGTATTTTAATTTACGTAACTGCACCTTTATGTACACTTATAAC
ATTACCGAAGATGAGATTTTAGAGTGGTTTGGCATTACACAAACTGCTCAAG
 
GTGTTCACCTCTTCTCATCTCGGTATGTTGATTTGTACGGCGGCAATATGTTTC
AATTTGCCACCTTGCCTGTTTATGATACTATTAAGTATTATTCTATCATTCCTC
ACAGTATTCGTTCTATCCAAAGTGATAGAAAAGCTTGGGCTGCCTTCTACGTA
TATAAACTTCAACCGTTAACTTTCCTGTTGGATTTTCTGTTGATGGTTATATA
CGCAGAGCTATAGACTGTGGTTTAATGATTTGTCACAACTCCACTGCTCATA
TGAATCCTTCGATGTTGAATCTGGAGTTTATTCAGTTTCGTCTTTCGAAGCAA
AACCTTCTGGCTCAGTTGTGGAACAGGCTGAAGGTGTTGAATGTGATTTTTCA
CCTCTTCTGTCTGGCACACCTCCTCAGGTTTATAATTTCAAGCGTTTGGTTTTT
ACCAATTGCAATTATAATCTTACCAAATTGCTTTCACTTTTTCTGTGAATGAT
TTTACTTGTAGTCAAATATCTCCAGCAGCAATTGCTAGTAACTGTTATTCTTCA
CTGATTTTGGATTATTTTCATACCCACTTAGTATGAAATCCGATCTCAGTGTT
AGTTCTGCTGGTCCAATATCCCAGTTTAATTATAAACAGTCCTTTTCTAATCCC
ACATGTTTGATTTTAGCGACTGTTCCTCATAACCTTACTACTATTACTAAGCCT
CTTAAGTACAGCTATATTAACAAGTGCTCTCGTTTTCTTTCTGATGATCGTACT
GAAGTACCTCAGTTAGTGAACGCTAATCAATACTCACCCTGTGTATCCATTGT
CCCATCCACTGTGTGGGAAGACGGTGATTATTATAGGAAACAACTATCTCCAC
TTGAAGGTGGTGGCTGGCTTGTTGCTAGTGGCTCAACTGTTGCCATGACTGAG
CAATTACAGATGGGCTTTGGTATTACAGTTCAATATGGTACAGACACCAATAG
TGTTTGCCCCAAGCTTGAATTTGCTAATGACACAAAAATTGCCTCTCAATTAG
GCAATTGCGTGGAATATTCCCTCTATGGTGTTTCGGGCCGTGGTGTTTTTCAG
AATTGCACAGCTGTAGGTGTTCGACAGCAGCGCTTTGTTTATGATGCGTACCA
GAATTTAGTTGGCTATTATTCTGATGATGGCAACTACTACTGTTTGCGTGCTTG
TGTTAGTGTTCCTGTTTCTGTCATCTATGATAAAGAAACTAAAACCCACGCTA
CTCTATTTGGTAGTGTTGCATGTGAACACATTTCCTCTACCATGTCTCAATACT
CCCGTTCTACGCGATCAATGCTTAAACGGCGAGATTCTACATATGGCCCCCTT
CAGACACCTGTTGGTTGTGTCCTAGGACTTGTTAATTCCTCTTTGTTCGTAGAG
GACTGCAAGTTGCCTCTTGGTCAATCTCTCTGTGCTCTTCCTGACACACCTAGT
ACTCTCACACCTCGCAGTGTGCGCTCTGTTCCAGGTGAAATGCGCTTGGCATC
 
CATTGCTTTTAATCATCCTATTCAGGTTGATCAACTTAATAGTAGTTATTTTAA
ATTAAGTATACCCACTAATTTTTCCTTTGGTGTGACTCAGGAGTACATTCAGA
CAACCATTCAGAAAGTTACTGTTGATTGTAAACAGTACGTTTGCAATGGTTTC
CAGAAGTGTGAGCAATTACTGCGCGAGTATGGCCAGTTTTGTTCCAAAATAA
ACCAGGCTCTCCATGGTGCCAATTTACGCCAGGATGATTCTGTACGTAATTTG
TTTGCGAGCGTGAAAAGCTCTCAATCATCTCCTATCATACCAGGTTTTGGAGG
TGACTTTAATTTGACACTTCTAGAACCTGTTTCTATATCTACTGGCAGTCGTAG
TGCACGTAGTGCTATTGAGGATTTGCTATTTGACAAAGTCACTATAGCTGATC
CTGGTTATATGCAAGGTTACGATGATTGCATGCAGCAAGGTCCAGCATCAGCT
CGTGATCTTATTTGTGCTCAATATGTGGCTGGTTACAAAGTATTACCTCCTCTT
ATGGATGTTAATATGGAAGCCGCGTATACCTCATCTTTGCTTGGCAGCATAGC
AGGTGTTGGCTGGACTGCTGGCTTATCCTCCTTTGCTGCTATTCCATTTGCACA
GAGTATCTTTTATAGGTTAAACGGTGTTGGCATTACTCAACAGGTTCTTTCAG
AGAACCAAAAGCTTATTGCCAATAAGTTTAATCAGGCTCTGGGAGCTATGCA
AACAGGCTTCACTACAACTAATGAAGCTTTTCACAAGGTTCAGGATGCTGTGA
ACAACAATGCACAGGCTCTATCCAAATTAGCTAGCGAGCTATCTAATACTTTT
GGTGCTATTTCCGCCTCTATTGGAGACATCATACAACGTCTTGATGTTCTCGA
ACAGGACGCCCAAATAGACAGACTTATTAATGGCCGTTTGACAACACTAAAT
GCTTTTGTTGCACAGCAGCTTGTTCGTTCCGAATCAGCTGCTCTTTCGGCTCAA
TTGGCTAAAGATAAAGTCAATGAGTGTGTCAAGGCACAATCCAAGCGTTCTG
GATTTTGCGGTCAAGGCACACATATAGTGTCCTTTGTTGTAAATGCCCCTAAT
GGCCTTTACTTCATGCATGTTGGTTATTACCCTAGCAACCACATTGAGGTTGTT
TCTGCTTATGGTCTTTGCGATGCAGCTAACCCTACTAATTGTATAGCCCCTGTT
AATGGCTACTTTATTAAAACTAATAACACTAGGATTGTTGATGAGTGGTCATA
TACTGGCTCGTCCTTCTATGCACCTGAGCCCATCACCTCTCTTAATACTAAGTA
TGTTGCACCACAGGTGACATACCAAAACATTTCTACTAACCTCCCTCCTCCTC
TTCTCGGCAATTCCACCGGGATTGACTTCCAAGATGAGTTGGATGAGTTTTTC
AAAAATGTTAGCACCAGTATACCTAATTTTGGTTCTCTAACACAGATTAATAC
 
TACATTACTCGATCTTACCTACGAGATGTTGTCTCTTCAACAAGTTGTTAAAG
CCCTTAATGAGTCTTACATAGACCTTAAAGAGCTTGGCAATTATACTTATTAC
AACAAATGGCCGTGGTACATTTGGCTTGGTTTCATTGCTGGGCTTGTTGCCTT
AGCTCTATGCGTCTTCTTCATACTGTGCTGCACTGGTTGTGGCACAAACTGTA
TGGGAAAACTTAAGTGTAATCGTTGTTGTGATAGATACGAGGAATACGACCT
CGAGCCGCATAAGGTTCATGTTCACTAAGTCGCTTGCAAAGACAACGCCACA
TTTTGGGAACGCGTCCGATCCATCCTGAAATCTGGGCTCAACTTTGCTTCGAC
CATACCTGGCCCCGTAGGAGTGGCAGCGACAGGGATCAAAGGCATCATTGAA
GCCATTGGTTCCTTGTGGGTTTGATTCCACCCACAGAAGCGTTGACGACGCAA
AACGTCCTTAAAGCGTTGACGACGCAAAACGTCCCCAAGCTCGTAGCACCGA
CCCTATACCCATCTCTAGGGTCTTCAACCTCTTGGT
Spike protein from Porcine epidemic diarrhea virus - isolate PEDV YZ, GenBank: MK841495.1 to be applied together with RNA1 
GTAAACAACCAATAACATCAGGGTATCCAAAGCAGCACGCCGCCGAA
GAGCGGCCATGAAGTCTTTAACCTACTTCTGGTTGTTCTTACCAGTACTTTCA
ACACTTAGCCTACCACAAGATGTCACCAGGTGCTCAGCTAACACTAATTTTAG
GCGGTTCTTTTCAAAATTTAATGTTCAGGCGCCTGCAGTTGTTGTACTGGGCG
GTTATCTACCTATTGGTGAAAACCAGGGTGTCAATTCAACTTGGTACTGTGCT
GGCCAACATCCAACTGCTAGTGGCGTTCATGGTATCTTTGTTAGCCATATTAG
AGGTGGTCATGGCTTTGAGATTGGCATTTCGCAAGAGCCTTTTGACCCTAGTG
GTTACCAGCTTTATTTACATAAGGCTACTAACGGTAACACTAATGCTACTGCG
CGACTGCGCATTTGCCAGTTTCCTAGCATTAAAACATTGGGCCCCACTGCTAA
TAATGATGTTACAACAGGTCGTAATTGCCTATTTAACAAAGCCATCCCAGCTC
ATATGAGTGAACATAGTGTTGTCGGCATAACATGGGATAATGATCGTGTCACT
GTCTTTTCTGACAAGATCTATTATTTTTATTTTAAAAATGATTGGTCCCGTGTT
GCGACAAAGTGTTACAACAGTGGAGGTTGTGCTATGCAATATGTTTACGAAC
CCACCTATTACATGCTTAATGTTACTAGTGCTGGTGAGGATGGTATTTCTTATC
AACCCTGTACAGCTAATTGCATTGGTTATGCTGCCAATGTATTTGCTACTGAG
 
CCCAATGGCCACATACCAGAAGGTTTAGTTTAATAATTGGTTCTTTGTCC
AATGATTCCACTTTGGTGCATGGTAAGGTGGTTTCCAACCAACCATTGTTGGT
CAATTGTCTTTGGCCATTCCTAAGATTTATGGACTAGGCCAATTTTCTCCTT
TAATCAAACGATCGATGGTGTTTGTAATGGAGCTGCTGTGCAGCGTGCACCA
GAGGCCCTGAGGTTTAATATTAATGACACCTCTGTCATTCTTGCTGAAGGCTC
AATTGTACTTCATACTGCTTTAGGAACAAATTTTCTTTTGTTGCAGTAATTC
CTCAGATCCTCACTTAGCCACCTTCGCCACACCTCTGGGTGCTACCCAAGTAC
CCTATTATTGTTTCTTAAAGTGGATACTTACAACTCCACTGTTTATAAATTCC
TGGCTATTTTACCTCCTACCGTCAGGGAAATTGTCATCACCAAGTATGGTGAT
GTTTATGTCAATGGGTTTGGCTACTTGCATCTCGGTTTGTTGGATGCTGTCACA
ATTAATTTCACTGGTCATGGCACTGACGATGACGTTTCTGGTTTTTGGACCAT
AGCATCGACTAATTTTGTTGACGCACTTATCGAAGTTCAAGGAACTGCCATTC
AGCGTATTCTTTATTGTGATGATCCTGTTAGCCAACTCAAGTGTTCTCAGGTTT
CTTTTGACCTTGACGATGGTTTTACCCTATTTCCTCTAGAAACCTTCTGAGTC
ATGAACAGCCAATTTCTTTTGTTACTCTGCCATCATTTAATGATCATTCTTTTG
TTAACATTACTGTCTCTGCTTCCTTTGGTGGTCATAGTGGTGCCAACCTTATTG
CATCTGACACTACTATCAATGGGTTTAGTTCTTTCTGTGTTGAAACTAGACAA
TTTACCATTTCACTGTTTATAATGTTACAAACAGTTATGGTTATGTGTCTAAA
TCACAGGACAGTAATTGCCCTTTCACCTTGCAATCTGTTAATGATTACCTGTCT
TTTAGCAAATTTTGTGTTTCTACCAGCCTTTTGGCTAGTGCCTGTACCATAGAT
CTTTTTGGTTACCCTGAGTTTGGTAGTGGTGTTAAGTTCACGTCCCTTTACTTT
CAATTCACAAAGGGTGAGTTGATTACTGGCACGCCTAAACCACTTGTAGGTGT
TACGGACGTTTCTTTTATGACTCTGGATGTGTGTACCAAGTATACTATCTATG
GCTTTAAAGGTGAGGGTATCATTACCCTTACAAATTCTAGCTTTTTGGCAGGT
ATTTATTATACATCTGATTCTGGACAGTTGTTAGCTTTTAAGAATGTCACTAGT
GGTGCTGTTTATTCTGTTACGCCATGTTCTTTTTCAGAGCAGGCTGCATATGTT
GATGATGATATAGTGGGTGTTATTTCTAGTTTGTCTAACTCCACTTTTAACAGT
ACTAGGGAGTTGCCTGGTTTCTTCTACCATTCTAATGATGGCTCTAATTGTAC
 
AGAGCCTGTGTTGGTGTATAGTAACATAGGTGTTTGTAAATCTGGCAGTATTG
GCTATGTTCTATCTAAGTCTGGCCAAGTCAAGATTGCACCCACGGTTACTGGG
AATATTAGTATTCCCACCAACTTTAGTATGAGTATTAGGACAGAATATTTACA
GCTTTACAACACGCCTGTTAGTGTTGATTGTGCTACATATGTTTGCAGTGGTA
ACTCTCGTTGTAAACAATTACTCACCCAGTACATTGCAGCATGTAAGACCATA
GAGTCAGCGTTACAACTCAGCGCTAGGCTTGAGTCTGCTGAAGTCAACTCTAT
GCTTACTATTTCTGAAGAGGCTCTACAGTTAGCTACCATCAGTTCGTTTAATG
GTGATGGATATAATTTTACTAATGTGCTGGGTGTTTCCGTTCGTGATCCTGCA
AGTGGCAGGGTGGTACAAAAAGGTCTTTATTGAAGACCTGCTTTTTAATAA
AGTGGTTACTAATGGCCTTGGTACTGTTGATGAAGACTATAAGCGCTGTTCTA
ATGGCCGTTCTGTGGCAGATTTAGTCTGTGCGCAGTATTACTCTGGTGTCATG
GTACTACCTGGTGTTGTTGACGCTGAGAAGCTTCATATGTATAGTGCGTCTCT
CATCGGTGGTATGGTGCTAGGAGGTTTTACTGCAGCAGCGGCATTGCCTTTTA
GCTATGCTGTTCAAGCGAGACTCAATTATCTTGCTCTACAGACGGATGTTCTA
CAGCGGAACCAGCAATTGCTTGCTGAGTCTTTTAACTCTGCTATTGGTGATAT
AACTTCAGCCTTTGAGAGTGTTAAAGAGGCTATTAGTCAAACTTCTAAGGGTT
TGAACACTGTGGCTCATGCGCTTACTAAGGTTCAAGAGGTTGTTAATTCGCAG
GGTGCAGCTTTGACCCAACTCACCGTACAGCTGCAACACAACTTCCAAGCCAT
TTCTAGTTCTATTGATGACATTTATTCCCGACTGGACATTCTTTCAGCCGATGT
TCAGGTTGACCGTCTCATCACCGGCAGATTATCAGCACTTAATGCTTTTGTTG
CTCAAACCCTCACTAAGTATACTGAGGTTCAGGCTAGCAGGAAGCTAGCACA
GCAAAAGGTTAATGAGTGCGTTAAATCGCAATCTCAGCGTTATGGTTTTTGCG
GTGGTGATGGCGAGCACATTTTCTCTCTGGTACAGGCAGCACCTCAGGGCCTG
CTGTTTTTACATACAGTACTTGTACCGGGTGATTTTGTAGATGTTATTGCCATC
GCTGGCTTATGCGTTAACGATGAAATTGCCTTGACTCTACGTGAGCCTGGCTT
AGTCTTGTTTACGCATGAACTTCAAAATCACACTGCGACGGAATATTTGTTT
CATCGCGACGTATGTTTGAACCTAGAAAACCTACCGTTAGTGATTTTGTTCAA
ATTGAGAGTTGTGTGGTCACCTATGTCAATTTGACTAGAGACCAACTACCAGA
 
TGTAATCCCGGATTACATCGATGTTAACAAAACACTTTATGAGATTTTAGCTT
CTCTGCCCAATAGAACTGGTCCAAGTCTTCCTTTAGATGTTTTAATGCCACTT
ATCTTAATCTCACTGGTGAAATTGCAGATTTAGAGCAGCGTTCAGAGTCTCTC
CGTAATACTACAGAAGAGCTCCAAAGTCTTATATATAATATCAACAACACAC
TAGTTGACCTTGAGTGGCTCAACCGAGTTGAGACATATATCAAGTGGCCGTG
GTGGGTTTGGTTGATTATTTTTATTGTTCTCATCTTTGTTGTGTCATTATTAGTG
TTTTGCTGCATTTCCACGGGTTGTTGTGGATGCTGCGGCTGCTGTGGTGCTTGT
TTTGCAGGTTGTTGTAGGGGTCCTAGACTTCAACCTTACGAAGCTTTTGAAAA
GGTCCACGTGCAGTGAGTCGCTTGCAAAGACAACGCCACATTTGGGAACGC
GTCCGATCCATCCTGAAATCTGGGCTCAACTTTGCTTCGACCATACCTGGCCC
CGTAGGAGTGGCAGCGACAGGGATCAAAGGCATCATTGAAGCCATTGGTTCC
TTGTGGGTTTGATTCCACCCACAGAAGCGTTGACGACGCAAAACGTCCTTAAA
GCGTTGACGACGCAAAACGTCCCCAAGCTCGTAGCACCGACCCTATACCCAT
CTCTAGGGTCTTCAACCTCTTGGT
Fusion construct Spike protein from Porcine epidemic diarrhea virus - isolate PEDV YZ 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
 
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
 
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGAAGTCTTTAACCTACTTCTGGTTGTTCTTACCAGTA
CTTTCAACACTTAGCCTACCACAAGATGTCACCAGGTGCTCAGCTAACACTAA
TTTTAGGCGGTTCTTTTCAAAATTTAATGTTCAGGCGCCTGCAGTTGTTGTACT
GGGCGGTTATCTACCTATTGGTGAAAACCAGGGTGTCAATTCAACTTGGTACT
GTGCTGGCCAACATCCAACTGCTAGTGGCGTTCATGGTATCTTTGTTAGCCAT
ATTAGAGGTGGTCATGGCTTTGAGATTGGCATTTCGCAAGAGCCTTTTGACCC
 
TAGTGGTTACCAGCTTTATTTACATAAGGCTACTAACGGTAACACTAATGCTA
CTGCGCGACTGCGCATTTGCCAGTTTCCTAGCATTAAAACATTGGGCCCACT
GCTAATAATGATGTTACAACAGGTCGTAATTGCCTATTTAACAAAGCCATCCC
AGCTCATATGAGTGAACATAGTGTTGTCGGCATAACATGGGATAATGATCGT
GTCACTGTCTTTTCTGACAAGATCTATTATTTTATTTTAAAAATGATTGGTCC
CGTGTTGCGACAAAGTGTTACAACAGTGGAGGTTGTGCTATGCAATATGTTTA
CGAACCCACCTATTACATGCTTAATGTTACTAGTGCTGGTGAGGATGGTATTT
CTTATCAACCCTGTACAGCTAATTGCATTGGTTATGCTGCCAATGTATTTGCTA
CTGAGCCCAATGGCCACATACCAGAAGGTTTTAGTTTTAATAATTGGTTTCTT
TTGTCCAATGATTCCACTTTGGTGCATGGTAAGGTGGTTTCCAACCAACCATT
GTTGGTCAATTGTCTTTTGGCCATTCCTAAGATTTATGGACTAGGCCAATTTTT
CTCCTTTAATCAAACGATCGATGGTGTTTGTAATGGAGCTGCTGTGCAGCGTG
CACCAGAGGCCCTGAGGTTTAATATTAATGACACCTCTGTCATTCTTGCTGAA
GGCTCAATTGTACTTCATACTGCTTTAGGAACAAATTTTTCTTTTGTTTGCAGT
AATTCCTCAGATCCTCACTTAGCCACCTTCGCCACACCTCTGGGTGCTACCCA
AGTACCCTATTATTGTTTTCTTAAAGTGGATACTTACAACTCCACTGTTTATAA
ATTCCTGGCTATTTTACCTCCTACCGTCAGGGAAATTGTCATCACCAAGTATG
GTGATGTTTATGTCAATGGGTTTGGCTACTTGCATCTCGGTTTGTTGGATGCTG
TCACAATTAATTTCACTGGTCATGGCACTGACGATGACGTTTCTGGTTTTTGG
ACCATAGCATCGACTAATTTTGTTGACGCACTTATCGAAGTTCAAGGAACTGC
CATTCAGCGTATTCTTTATTGTGATGATCCTGTTAGCCAACTCAAGTGTTCTCA
GGTTTCTTTTGACCTTGACGATGGTTTTTACCCTATTTCCTCTAGAAACCTTCT
GAGTCATGAACAGCCAATTTCTTTTGTTACTCTGCCATCATTTAATGATCATTC
TTTTGTTAACATTACTGTCTCTGCTTCCTTTGGTGGTCATAGTGGTGCCAACCT
TATTGCATCTGACACTACTATCAATGGGTTTAGTTCTTTCTGTGTTGAAACTAG
ACAATTTACCATTTCACTGTTTATAATGTTACAAACAGTTATGGTTATGTGTC
TAAATCACAGGACAGTAATTGCCCTTTCACCTTGCAATCTGTTAATGATTACC
TGTCTTTTAGCAAATTTTGTGTTTCTACCAGCCTTTTGGCTAGTGCCTGTACCA
 
TAGATCTTTTTGGTTACCCTGAGTTTGGTAGTGGTGTTAAGTTCACGTCCCTTT
ACTTTCAATTCACAAAGGGTGAGTTGATTACTGGCACGCCTAAACCACTTGTA
GGTGTTACGGACGTTTCTTTTATGACTCTGGATGTGTGTACCAAGTATACTAT
CTATGGCTTTAAAGGTGAGGGTATCATTACCCTTACAAATTCTAGCTTTTTGG
CAGGTATTTATTATACATCTGATTCTGGACAGTTGTTAGCTTTTAAGAATGTC
ACTAGTGGTGCTGTTTATTCTGTTACGCCATGTTCTTTTTCAGAGCAGGCTGCA
TATGTTGATGATGATATAGTGGGTGTTATTTCTAGTTTGTCTAACTCCACTTTT
AACAGTACTAGGGAGTTGCCTGGTTTCTTCTACCATTCTAATGATGGCTCTAA
TTGTACAGAGCCTGTGTTGGTGTATAGTAACATAGGTGTTTGTAAATCTGGCA
GTATTGGCTATGTTCTATCTAAGTCTGGCCAAGTCAAGATTGCACCCACGGTT
ACTGGGAATATTAGTATTCCCACCAACTTTAGTATGAGTATTAGGACAGAATA
TTTACAGCTTTACAACACGCCTGTTAGTGTTGATTGTGCTACATATGTTTGCAG
TGGTAACTCTCGTTGTAAACAATTACTCACCCAGTACATTGCAGCATGTAAGA
CCATAGAGTCAGCGTTACAACTCAGCGCTAGGCTTGAGTCTGCTGAAGTCAA
CTCTATGCTTACTATTTCTGAAGAGGCTCTACAGTTAGCTACCATCAGTTCGTT
TAATGGTGATGGATATAATTTTACTAATGTGCTGGGTGTTTCCGTTCGTGATC
CTGCAAGTGGCAGGGTGGTACAAAAAGGTCTTTTATTGAAGACCTGCTTTTT
AATAAAGTGGTTACTAATGGCCTTGGTACTGTTGATGAAGACTATAAGCGCTG
TTCTAATGGCCGTTCTGTGGCAGATTTAGTCTGTGCGCAGTATTACTCTGGTGT
CATGGTACTACCTGGTGTTGTTGACGCTGAGAAGCTTCATATGTATAGTGCGT
CTCTCATCGGTGGTATGGTGCTAGGAGGTTTTACTGCAGCAGCGGCATTGCCT
TTTAGCTATGCTGTTCAAGCGAGACTCAATTATCTTGCTCTACAGACGGATGT
TCTACAGCGGAACCAGCAATTGCTTGCTGAGTCTTTTAACTCTGCTATTGGTG
ATATAACTTCAGCCTTTGAGAGTGTTAAAGAGGCTATTAGTCAAACTTCTAAG
GGTTTGAACACTGTGGCTCATGCGCTTACTAAGGTTCAAGAGGTTGTTAATTC
GCAGGGTGCAGCTTTGACCCAACTCACCGTACAGCTGCAACACAACTTCCAA
GCCATTTCTAGTTCTATTGATGACATTTATTCCCGACTGGACATTCTTTCAGCC
GATGTTCAGGTTGACCGTCTCATCACCGGCAGATTATCAGCACTTAATGCTTT
 
TGTTGCTCAAACCCTCACTAAGTATACTGAGGTTCAGGCTAGCAGGAAGCTA
GCACAGCAAAAGGTTAATGAGTGCGTTAAATCGCAATCTCAGCGTTATGGTTT
TTGCGGTGGTGATGGCGAGCACATTTTCTCTCTGGTACAGGCAGCACCTCAGG
GCCTGCTGTTTTTACATACAGTACTTGTACCGGGTGATTTTGTAGATGTTATTG
CCATCGCTGGCTTATGCGTTAACGATGAAATTGCCTTGACTCTACGTGAGCCT
GGCTTAGTCTTGTTTACGCATGAACTTCAAAATCACACTGCGACGGAATATTT
TGTTTCATCGCGACGTATGTTTGAACCTAGAAAACCTACCGTTAGTGATTTTG
TTCAAATTGAGAGTTGTGTGGTCACCTATGTCAATTTGACTAGAGACCAACTA
CCAGATGTAATCCCGGATTACATCGATGTTAACAAAACACTTTATGAGATTTT
AGCTTCTCTGCCCAATAGAACTGGTCCAAGTCTTCCTTTAGATGTTTTTAATGC
CACTTATCTTAATCTCACTGGTGAAATTGCAGATTTAGAGCAGCGTTCAGAGT
CTCTCCGTAATACTACAGAAGAGCTCCAAAGTCTTATATATAATATCAACAAC
ACACTAGTTGACCTTGAGTGGCTCAACCGAGTTGAGACATATATCAAGTGGC
CGTGGTGGGTTTGGTTGATTATTTTTATTGTTCTCATCTTTGTTGTGTCATTATT
AGTGTTTTGCTGCATTTCCACGGGTTGTTGTGGATGCTGCGGCTGCTGTGGTG
CTTGTTTTGCAGGTTGTTGTAGGGGTCCTAGACTTCAACCTTACGAAGCTTTTG
AAAAGGTCCACGTGCAGTGAGTCGCTTGCAAAGACAACGCCACATTTTGGGA
ACGCGTCCGATCCATCCTGAAATCTGGGCTCAACTTTGCTTCGACCATACCTG
GCCCCGTAGGAGTGGCAGCGACAGGGATCAAAGGCATCATTGAAGCCATTGG
TTCCTTGTGGGTTTGATTCCACCCACAGAAGCGTTGACGACGCAAAACGTCCT
TAAAGCGTTGACGACGCAAAACGTCCCCAAGCTCGTAGCACCGACCCTATAC
CCATCTCTAGGGTCTTCAACCTCTTGGT
Spike gene from Transmissible gastroenteritis virus (TGEV) AJ271965.2 to be applied together with RNA1 
GTAAACAACCAATAACATCAGGGTATCCAAAGCAGCACGCCGCCGAA
GAGCGGCCATGAAAAACTATTTGTGGTTTTGGTCGTAATGCCATTGATTTAT
GGAGACAATTTTCCTTGTTCTAAATTGACTAATAGAACTATAGGCAACCAGTG
GAATCTCATTGAAACCTTCCTTCTAAACTATAGTAGTAGGTTACCACCTAATT
 
CAGATGTGGTGTTAGGTGATTATTTCCTACTGTACAACCTTGGTTTAATTGCA
TTCGCAATGATAGTAATGACCTTTATGTTACACTGGAAAATCTTAAAGCATTG
TATTGGGATTATGCTACAGAAAATATCACTTGGAATCACAGACAACGGTTAA
ACGTAGTCGTTAATGGATACCCATACTCCATCACAGTTACAACAACCCGCAAT
TTTAATTCTGCTGAAGGTGCTATTATATGCATTTGTAAGGGCTCACCACCTAC
TACCACCACAGAATCTAGTTTGACTTGCAATTGGGGTAGTGAGTGCAGGTTAA
ACCATAAGTTCCCTATATGTCCTTCTAATTCAGAGGCAAATTGTGGTAATATG
CTGTATGGCCTACAATGGTTTGCAGATGAGGTTGTTGCTTATTTACATGGTGC
TAGTTACCGTATTAGTTTTGAAAATCAATGGTCTGGCACTGTCACATTTGGTG
ATATGCGTGCGACAACATTAGAAGTCGCTGGCACGCTTGTAGACCTTTGGTGG
TTTAATCCTGTTTATGATGTCAGTTATTATAGGGTTAATAATAAAAATGGTAC
TACCGTAGTTTCCAATTGCACTGATCAATGTGCTAGTTATGTGGCTAATGTTTT
TACTACACAGCCAGGAGGTTTTATACCATCAGATTTTAGTTTTAATAATTGGT
TCCTTCTAACTAATAGCTCCACGTTGGTTAGTGGTAAATTAGTTACCAAACAG
CCGTTATTAGTTAATTGCTTATGGCCAGTCCCTAGCTTTGAAGAAGCAGCTTC
TACATTTTGTTTTGAGGGTGCTGGCTTTGATCAATGTAATGGTGCTGTTTTAAA
TAATACTGTAGACGTCATTAGGTTCAACCTTAATTTTACTACAAATGTACAAT
CAGGTAAGGGTGCCACAGTGTTTTCATTGAACACAACGGGTGGTGTCACTCTT
GAAATTTCATGTTATACAGTGAGTGACTCGAGCTTTTTCAGTTACGGTGAAAT
TCCGTTCGGCGTAACTGATGGACCACGGTACTGTTACGTACACTATAATGGCA
CAGCTCTTAAGTATTTAGGAACATTACCACCTAGTGTCAAGGAGATTGCTATT
AGTAAGTGGGGCCATTTTTATATTAATGGTTACAATTTCTTTAGCACATTTCCT
ATTGATTGTATATCTTTTAATTTGACCACTGGTGATAGTGACGTTTTCTGGACA
ATAGCTTACACATCGTACACTGAAGCATTAGTACAAGTTGAAAACACAGCTA
TTACAAAGGTGACGTATTGTAATAGTCACGTTAATAACATTAAATGCTCTCAA
ATTACTGCTAATTTGAATAATGGATTTTATCCTGTTTCTTCAAGTGAAGTTGGT
CTTGTCAATAAGAGTGTTGTGTTACTACCTAGCTTTTACACACATACCATTGTT
AACATAACTATTGGTCTTGGTATGAAGCGTAGTGGTTATGGTCAACCCATAGC
 
CTCAACATTAAGTAACATCACACTACCAATGCAGGATCACAACACCGATGTG
TACTGTATTCGTTCTGACCAATTTTCAGTTTATGTTCATTCTACTTGCAAAAGT
GCTTTATGGGACAATATTTTAAGCGAAACTGCACGGACGTTTAGATGCCAC
AGCTGTTATAAAAACTGGTACTTGTCCTTTCTCATTTGATAAATTGAACAATT
ACTTAACTTTTAACAAGTTCTGTTTGTCGTTGAGTCCTGTTGGTGCTAATTGTA
AGTTTGATGTAGCTGCCCGTACAAGAACCAATGAGCAGGTTGTTAGAAGTTT
GTATGTAATATATGAAGAAGGAGACAACATAGTGGGTGTACCGTCTGATAAT
AGTGGTGTGCACGATTTGTCAGTGCTACACCTAGATTCCTGCACAGATTACAA
TATATATGGTAGAACTGGTGTTGGTATTATTAGACAAACTAACAGGACGCTAC
TTAGTGGCTTATATTACACATCACTATCAGGTGATTTGTTAGGTTTTAAAAAT
GTTAGTGATGGTGTCATCTACTCTGTAACGCCATGTGATGTAAGCGCACAAGC
AGCTGTTATTGATGGTACCATAGTTGGGGCTATCACTTCCATTAACAGTGAAC
TGTTAGGTCTAACACATTGGACAACAACACCTAATTTTTATTACTACTCTATA
TATAATTACACAAATGATAGGACTCGTGGCACTGCAATTGACAGTAATGATG
TTGATTGTGAACCTGTCATAACCTATTCTAACATAGGTGTTTGTAAAAATGGT
GCTTTTGTTTTTATTAACGTCACACATTCTGATGGAGACGTGCAACCAATTAG
CACTGGTAATGTCACGATACCTACAAACTTTACCATATCCGTGCAAGTCGAAT
ATATTCAGGTTTACACTACACCAGTGTCAATAGACTGTTCAAGATATGTTTGT
AATGGTAACCCTAGGTGTAACAAATTGTTAACACAATACGTTTCTGCATGTCA
AACTATTGAGCAAGCACTTGCAATGGGTGCCAGACTTGAAAACATGGAGGTT
GATTCCATGTTGTTTGTTTCTGAAAATGCCCTTAAATTGGCATCTGTTGAAGC
ATTCAATAGTTCAGAAACTTTAGACCCTATTTACAAAGAATGGCCTAATATAG
GTGGTTCTTGGCTAGAAGGTCTAAAATACATACTTCCGTCCCATAATAGCAAA
CGTAAGTATCGTTCAGCTATAGAGGACTTGCTTTTTGATAAGGTTGTAACATC
TGGTTTAGGTACAGTTGATGAAGATTATAAACGTTGTACAGGTGGTTATGACA
TAGCTGACTTAGTATGTGCTCAATACTATAATGGCATCATGGTGCTACCTGGT
GTGGCTAATGCTGACAAAATGACTATGTACACAGCATCCCTTGCAGGTGGTAT
AACATTAGGTGCACTTGGTGGAGGCGCCGTGGCTATACCTTTTGCAGTAGCAG
 
TTCAGGCTAGACTTAATTATGTTGCTCTACAAACTGATGTATTGAACAAAAAC
CAGCAGATTCTGGCTAGTGCTTTCAATCAAGCTATTGGTAACATTACACAGTC
ATTTGGTAAGGTTAATGATGCTATACATCAAACATCACGAGGTCTTGCTACTG
TTGCTAAAGCATTGGCAAAAGTGCAAGATGTTGTCAACATACAAGGGCAAGC
TTTAAGCCACCTAACAGTACAATTGCAAAATAATTTCCAAGCCATTAGTAGTT
CTATTAGTGACATTTATAATAGGCTTGACGAATTGAGTGCTGATGCACAAGTT
GACAGGCTGATCACAGGAAGACTTACAGCACTTAATGCATTTGTGTCTCAGA
CTCTAACCAGACAAGCGGAGGTTAGGGCTAGTAGACAACTTGCCAAAGACAA
GGTTAATGAATGCGTTAGGTCTCAGTCTCAGAGATTCGGATTCTGTGGTAATG
GTACACATTTGTTTTCACTCGCAAATGCAGCACCAAATGGCATGATTTTCTTT
CACACAGTGCTATTACCAACGGCTTATGAAACTGTGACTGCTTGGCCAGGTAT
TTGTGCTTCAGATGGTGATCGCACTTTTGGACTTGTCGTTAAAGATGTCCAGT
TGACTTTGTTTCGTAATCTAGATGACAAGTTCTATTTGACCCCCAGAACTATG
TATCAGCCTAGAGTTGCAACTAGTTCTGACTTTGTTCAAATTGAAGGGTGCGA
TGTGCTGTTTGTTAATGCAACTGTAAGTGATTTGCCTAGTATTATACCTGATTA
TATTGATATTAATCAGACTGTTCAAGACATATTAGAAAATTTAGACCAAATT
GGACTGTACCTGAGTTGACATTTGACATTTTTAACGCAACCTATTTAAACCTG
ACTGGTGAAATTGATGACTTAGAATTTAGGTCAGAAAAGCTACATAACACCA
CTGTAGAACTTGCCATTCTCATTGACAACATTAACAATACATTAGTCAATCTT
GAATGGCTCAATAGAATTGAAACCTATGTAAAATGGCCTTGGTATGTGTGGCT
ACTAATAGGCTTAGTAGTAATATTTTGCATACCATTACTGCTATTTTGCTGTTG
TAGTACAGGTTGCTGTGGATGCATAGGTTGTTTAGGAAGTTGTTGTCACTCTA
TATGTAGTAGAAGACAATTTGAAAATTACGAACCAATTGAAAAAGTGCACGT
CCATTAAGTCGCTTGCAAAGACAACGCCACATTTTGGGAACGCGTCCGATCC
ATCCTGAAATCTGGGCTCAACTTTGCTTCGACCATACCTGGCCCCGTAGGAGT
GGCAGCGACAGGGATCAAAGGCATCATTGAAGCCATTGGTTCCTTGTGGGTT
TGATTCCACCCACAGAAGCGTTGACGACGCAAAACGTCCTTAAAGCGTTGAC
GACGCAAAACGTCCCCAAGCTCGTAGCACCGACCCTATACCCATCTCTAGGG
 TCTTCAACCTCTTGGT
Fusion construct: Spike gene from Transmissible gastroenteritis virus 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
 
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
 
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGAAAAACTATTTGTGGTTTTGGTCGTAATGCCATTG
ATTTATGGAGACAATTTTCCTTGTTCTAAATTGACTAATAGAACTATAGGCAA
CCAGTGGAATCTCATTGAAACCTTCCTTCTAAACTATAGTAGTAGGTTACCAC
CTAATTCAGATGTGGTGTTAGGTGATTATTTCCTACTGTACAACCTTGGTTTA
ATTGCATTCGCAATGATAGTAATGACCTTTATGTTACACTGGAAAATCTTAAA
GCATTGTATTGGGATTATGCTACAGAAAATATCACTTGGAATCACAGACAAC
GGTTAAACGTAGTCGTTAATGGATACCCATACTCCATCACAGTTACAACAACC
CGCAATTTTAATTCTGCTGAAGGTGCTATTATATGCATTTGTAAGGGCTCACC
ACCTACTACCACCACAGAATCTAGTTTGACTTGCAATTGGGGTAGTGAGTGCA
GGTTAAACCATAAGTTCCCTATATGTCCTTCTAATTCAGAGGCAAATTGTGGT
AATATGCTGTATGGCCTACAATGGTTTGCAGATGAGGTTGTTGCTTATTTACA
TGGTGCTAGTTACCGTATTAGTTTTGAAAATCAATGGTCTGGCACTGTCACAT
TTGGTGATATGCGTGCGACAACATTAGAAGTCGCTGGCACGCTTGTAGACCTT
TGGTGGTTTAATCCTGTTTATGATGTCAGTTATTATAGGGTTAATAATAAAAA
TGGTACTACCGTAGTTTCCAATTGCACTGATCAATGTGCTAGTTATGTGGCTA
ATGTTTTTACTACACAGCCAGGAGGTTTTATACCATCAGATTTAGTTTTAATA
ATTGGTTCCTTCTAACTAATAGCTCCACGTTGGTTAGTGGTAAATTAGTTACC
AAACAGCCGTTATTAGTTAATTGCTTATGGCCAGTCCCTAGCTTTGAAGAAGC
AGCTTCTACATTTTGTTTTGAGGGTGCTGGCTTTGATCAATGTAATGGTGCTGT
TTTAAATAATACTGTAGACGTCATTAGGTTCAACCTTAATTTTACTACAAATG
 
TACAATCAGGTAAGGGTGCCACAGTGTTTCATTGAACACAACGGGTGGTGT
CACTCTTGAAATTTCATGTTATACAGTGAGTGACTCGAGCTTTTCAGTTACG
GTGAAATTCCGTTCGGCGTAACTGATGGACCACGGTACTGTTACGTACACTAT
AATGGCACAGCTCTTAAGTATTTAGGAACATTACCACCTAGTGTCAAGGAGA
TTGCTATTAGTAAGTGGGGCCATTTTATATTAATGGTTACAATTTCTTTAGCA
CATTTCCTATTGATTGTATATCTTTTAATTTGACCACTGGTGATAGTGACGTTT
TCTGGACAATAGCTTACACATCGTACACTGAAGCATTAGTACAAGTTGAAAA
CACAGCTATTACAAAGGTGACGTATTGTAATAGTCACGTTAATAACATTAAAT
GCTCTCAAATTACTGCTAATTTGAATAATGGATTTTATCCTGTTTCTTCAAGTG
AAGTTGGTCTTGTCAATAAGAGTGTTGTGTTACTACCTAGCTTTTACACACAT
ACCATTGTTAACATAACTATTGGTCTTGGTATGAAGCGTAGTGGTTATGGTCA
ACCCATAGCCTCAACATTAAGTAACATCACACTACCAATGCAGGATCACAAC
ACCGATGTGTACTGTATTCGTTCTGACCAATTTTCAGTTTATGTTCATTCTACT
TGCAAAAGTGCTTTATGGGACAATATTTTTAAGCGAAACTGCACGGACGTTTT
AGATGCCACAGCTGTTATAAAAACTGGTACTTGTCCTTTCTCATTTGATAAAT
TGAACAATTACTTAACTTTTAACAAGTTCTGTTTGTCGTTGAGTCCTGTTGGTG
CTAATTGTAAGTTTGATGTAGCTGCCCGTACAAGAACCAATGAGCAGGTTGTT
AGAAGTTTGTATGTAATATATGAAGAAGGAGACAACATAGTGGGTGTACCGT
CTGATAATAGTGGTGTGCACGATTTGTCAGTGCTACACCTAGATTCCTGCACA
GATTACAATATATATGGTAGAACTGGTGTTGGTATTATTAGACAAACTAACAG
GACGCTACTTAGTGGCTTATATTACACATCACTATCAGGTGATTTGTTAGGTT
TTAAAAATGTTAGTGATGGTGTCATCTACTCTGTAACGCCATGTGATGTAAGC
GCACAAGCAGCTGTTATTGATGGTACCATAGTTGGGGCTATCACTTCCATTAA
CAGTGAACTGTTAGGTCTAACACATTGGACAACAACACCTAATTTTTATTACT
ACTCTATATATAATTACACAAATGATAGGACTCGTGGCACTGCAATTGACAGT
AATGATGTTGATTGTGAACCTGTCATAACCTATTCTAACATAGGTGTTTGTAA
AAATGGTGCTTTTGTTTTTATTAACGTCACACATTCTGATGGAGACGTGCAAC
CAATTAGCACTGGTAATGTCACGATACCTACAAACTTTACCATATCCGTGCAA
 
GTCGAATATATTCAGGTTTACACTACACCAGTGTCAATAGACTGTTCAAGATA
TGTTTGTAATGGTAACCCTAGGTGTAACAAATTGTTAACACAATACGTTTCTG
CATGTCAAACTATTGAGCAAGCACTTGCAATGGGTGCCAGACTTGAAAACAT
GGAGGTTGATTCCATGTTGTTTGTTTCTGAAAATGCCCTTAAATTGGCATCTGT
TGAAGCATTCAATAGTTCAGAAACTTTAGACCCTATTTACAAAGAATGGCCTA
ATATAGGTGGTTCTTGGCTAGAAGGTCTAAAATACATACTTCCGTCCCATAAT
AGCAAACGTAAGTATCGTTCAGCTATAGAGGACTTGCTTTTTGATAAGGTTGT
AACATCTGGTTTAGGTACAGTTGATGAAGATTATAAACGTTGTACAGGTGGTT
ATGACATAGCTGACTTAGTATGTGCTCAATACTATAATGGCATCATGGTGCTA
CCTGGTGTGGCTAATGCTGACAAAATGACTATGTACACAGCATCCCTTGCAGG
TGGTATAACATTAGGTGCACTTGGTGGAGGCGCCGTGGCTATACCTTTTGCAG
TAGCAGTTCAGGCTAGACTTAATTATGTTGCTCTACAAACTGATGTATTGAAC
AAAAACCAGCAGATTCTGGCTAGTGCTTTCAATCAAGCTATTGGTAACATTAC
ACAGTCATTTGGTAAGGTTAATGATGCTATACATCAAACATCACGAGGTCTTG
CTACTGTTGCTAAAGCATTGGCAAAAGTGCAAGATGTTGTCAACATACAAGG
GCAAGCTTTAAGCCACCTAACAGTACAATTGCAAAATAATTTCCAAGCCATTA
GTAGTTCTATTAGTGACATTTATAATAGGCTTGACGAATTGAGTGCTGATGCA
CAAGTTGACAGGCTGATCACAGGAAGACTTACAGCACTTAATGCATTTGTGTC
TCAGACTCTAACCAGACAAGCGGAGGTTAGGGCTAGTAGACAACTTGCCAAA
GACAAGGTTAATGAATGCGTTAGGTCTCAGTCTCAGAGATTCGGATTCTGTGG
TAATGGTACACATTTGTTTTCACTCGCAAATGCAGCACCAAATGGCATGATTT
TCTTTCACACAGTGCTATTACCAACGGCTTATGAAACTGTGACTGCTTGGCCA
GGTATTTGTGCTTCAGATGGTGATCGCACTTTTGGACTTGTCGTTAAAGATGT
CCAGTTGACTTTGTTTCGTAATCTAGATGACAAGTTCTATTTGACCCCCAGAA
CTATGTATCAGCCTAGAGTTGCAACTAGTTCTGACTTTGTTCAAATTGAAGGG
TGCGATGTGCTGTTTGTTAATGCAACTGTAAGTGATTTGCCTAGTATTATACCT
GATTATATTGATATTAATCAGACTGTTCAAGACATATTAGAAAATTTAGACC
AAATTGGACTGTACCTGAGTTGACATTTGACATTTTTAACGCAACCTATTTAA
 
ACCTGACTGGTGAAATTGATGACTTAGAATTTAGGTCAGAAAAGCTACATAA
CACCACTGTAGAACTTGCCATTCTCATTGACAACATTAACAATACATTAGTCA
ATCTTGAATGGCTCAATAGAATTGAAACCTATGTAAAATGGCCTTGGTATGTG
TGGCTACTAATAGGCTTAGTAGTAATATTTTGCATACCATTACTGCTATTTTGC
TGTTGTAGTACAGGTTGCTGTGGATGCATAGGTTGTTTAGGAAGTTGTTGTCA
CTCTATATGTAGTAGAAGACAATTTGAAAATTACGAACCAATTGAAAAAGTG
CACGTCCATTAAGTCGCTTGCAAAGACAACGCCACATTTGGGAACGCGTCC
GATCCATCCTGAAATCTGGGCTCAACTTTGCTTCGACCATACCTGGCCCCGTA
GGAGTGGCAGCGACAGGGATCAAAGGCATCATTGAAGCCATTGGTTCCTTGT
GGGTTTGATTCCACCCACAGAAGCGTTGACGACGCAAAACGTCCTTAAAGCG
TTGACGACGCAAAACGTCCCCAAGCTCGTAGCACCGACCCTATACCCATCTCT
AGGGTCTTCAACCTCTTGGT
ORF2 from porcine circovirus 2 (PCV2) to be applied together with RNA2 
GTAAACAACCAATAACATCAGGGTATCCAAAGCAGCACGCCGCCGAA
GAGCGGCCATGACGTATCCAAGGAGGCGTTACCGGAGAAGACACCGCC
CCCGCAGCCATCTTGGCCAGATCCTCCGCCGCCCCTGGCTCGTCCACCCC
CGCCACCGTTACCGCTGGAGAAGGAAAAATGGCATCTTCAACACCCGCCTCT
CCCGCACCTTCGGATATACTGTCAAGCGAACCACAGTCAGAACGCCCTCCTG
GGCGGTGGACATGATGAGATTCAATATTAATGACTTTCTTCCCCCAGGAGGG
GGCTCAAACCCCCGCTCTGTGCCCTTTGAATACTACAGAATAAGAAAGGTTA
AGGTTGAATTCTGGCCCTGCTCCCCGATCACCCAGGGTGACAGGGGAGTGGG
CTCCAGTGCTGTTATTCTAGATGATAACTTTGTAACAAAGGCCACAGCCCTCA
CCTATGACCCCTATGTAAACTACTCCTCCCGCCATACCATAACCCAGCCCTTC
TCCTACCACTCCCGCTACTTTACCCCCAAACCTGTCCTAGATTCCACTATTGAT
TACTTCCAACCAAACAACAAAAGAAATCAGCTGTGGCTGAGACTACAAACTG
CTGGAAATGTAGACCACGTAGGCCTCGGCACTGCGTTCGAAAACAGTATATA
CGACCAGGAATACAATATCCGTGTAACCATGTATGTACAATTCAGAGAATTT
AATCTTAAAGACCCCCCACTTAACCCTTAAGTCGCTTGCAAAGACAACGCCAC
 
ATTTTGGGAACGCGTCCGATCCATCCTGAAATCTGGGCTCAACTTTGCTTCGA

CCATACCTGGCCCCGTAGGAGTGGCAGCGACAGGGATCAAAGGCATCATTGA

AGCCATTGGTTCCTTGTGGGTTTGATTCCACCCACAGAAGCGTTGACGACGCA

AAACGTCCTTAAAGCGTTGACGACGCAAAACGTCCCCAAGCTCGTAGCACCG

ACCCTATACCCATCTCTAGGGTCTTCAACCTCTTGGT
Fusion construct ORF2 from porcine circovirus 2 (PCV2)

GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG

CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG

GAGAAATCACTGACGTCTGGGCGGATATTACCATCAACCGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGACGTATCCAAGGAGGCGTTACCGGAGAAGAAGAC
ACCGCCCCCGCAGCCATCTTGGCCAGATCCTCCGCCGCCGCCCCTGGCTCGTC
CACCCCCGCCACCGTTACCGCTGGAGAAGGAAAAATGGCATCTTCAACACCC
GCCTCTCCCGCACCTTCGGATATACTGTCAAGCGAACCACAGTCAGAACGCCC
TCCTGGGCGGTGGACATGATGAGATTCAATATTAATGACTTTCTTCCCCCAGG
AGGGGGCTCAAACCCCCGCTCTGTGCCCTTTGAATACTACAGAATAAGAAAG
GTTAAGGTTGAATTCTGGCCCTGCTCCCCGATCACCCAGGGTGACAGGGGAG
TGGGCTCCAGTGCTGTTATTCTAGATGATAACTTTGTAACAAAGGCCACAGCC
CTCACCTATGACCCCTATGTAAACTACTCCTCCCGCCATACCATAACCCAGCC
CTTCTCCTACCACTCCCGCTACTTTACCCCCAAACCTGTCCTAGATTCCACTAT
TGATTACTTCCAACCAAACAACAAAAGAAATCAGCTGTGGCTGAGACTACAA
ACTGCTGGAAATGTAGACCACGTAGGCCTCGGCACTGCGTTCGAAAACAGTA
TATACGACCAGGAATACAATATCCGTGTAACCATGTATGTACAATTCAGAGA
ATTTAATCTTAAAGACCCCCCACTTAACCCTTAAGTCGCTTGCAAAGACAACG
CCACATTTTGGGAACGCGTCCGATCCATCCTGAAATCTGGGCTCAACTTTGCT
TCGACCATACCTGGCCCCGTAGGAGTGGCAGCGACAGGGATCAAAGGCATCA

TTGAAGCCATTGGTTCCTTGTGGGTTTGATTCCACCCACAGAAGCGTTGACGA
CGCAAAACGTCCTTAAAGCGTTGACGACGCAAAACGTCCCCAAGCTCGTAGC
ACCGACCCTATACCCATCTCTAGGGTCTTCAACCTCTTGGT

Examples for bi-segmented pharmacons cloned in the RNA2 of Nodamura virus (NoV) and replacing partially the capsid protein. The sequences are equipped at their 5' end with up to 55 nt derived from the 5' end of RNA2 followed by the gene of interest and the last 270 nt from the 3' end of RNA2 of NoV. At position 21 the T was replaced by G to mutate the natural occurring ATG start codon. Replication is facilitated in a bi-segmented fashion by RNA1.

The examples are taken from human and animal health.

Moreover, pharmacons in form of monocistronic replicons are created. Those replicons are designed as follows: RNA1 wildtype derived from NoV is directly fused at its 3'end with the first 55 nt derived from the 5' end of RNA2; the T in position 21 was replaced by G to mutate the natural occurring ATG start codon. This stretch of 55 nt is followed by the gene of interest and the terminal 270 nt from the 3' end of RNA2. All pharmacons (both RNA1 and RNA2) derived are capped with capO and to be applied together with RNA1 as replicase provided and RNA2 as GOI-provider, bi-segmented of mono-cistronic.

Cancer vaccine RNA-pharmacons
**NY-ESO-1**
**MART-1**
**p53**
RNA1 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
 
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
 
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
 ATGCCAGTGGT
**NY-ESO-1**
   Cancer/testis antigen 1B (CTAG1B)/NY-ESO-1 (multiple tumors): NM_001327.3 CDS
   *BNT111* (NY-ESO-1, tyrosinase, MAGE-A3, and *TPTE*)→ advanced melanoma (FDA fast track designation 2021)
**44-NYESO1-270** 
GTAAACAACCAATAACATCAGGGTATCCAAAGCAGCACGCCGCCATG
CAGGCCGAAGGCCGGGCACAGGGGTTCGACGGCGATGCTGATGGCCCA
GGAGGCCCTGGCATTCCTGATGGCCCAGGGGGCAATGCTGGCGGCCCAGGAG
AGGCGGGTGCCACGGGCGGCAGAGGTCCCCGGGGCGCAGGGGCAGCAAGGG
CCTCGGGGCCGGGAGGAGGCGCCCCGCGGGGTCCGCATGGCGGCGCGGCTTC
AGGGCTGAATGGATGCTGCAGATGCGGGGCCAGGGGGCCGGAGAGCCGCCT
GCTTGAGTTCTACCTCGCCATGCCTTTCGCGACACCCATGGAAGCAGAGCTGG
CCCGCAGGAGCCTGGCCCAGGATGCCCCACCGCTTCCCGTGCCAGGGGTGCT
TCTGAAGGAGTTCACTGTGTCCGGCAACATACTGACTATCCGACTGACTGCTG
CAGACCACCGCCAACTGCAGCTCTCCATCAGCTCCTGTCTCCAGCAGCTTTCC
CTGTTGATGTGGATCACGCAGTGCTTTCTGCCCGTGTTTTGGCTCAGCCTCCC
TCAGGGCAGAGGCGCTAAGTCGCTTGCAAAGACAACGCCACATTTGGGAAC
GCGTCCGATCCATCCTGAAATCTGGGCTCAACTTTGCTTCGACCATACCTGGC
CCCGTAGGAGTGGCAGCGACAGGGATCAAAGGCATCATTGAAGCCATTGGTT
CCTTGTGGGTTTGATTCCACCCACAGAAGCGTTGACGACGCAAAACGTCCTTA
AAGCGTTGACGACGCAAAACGTCCCCAAGCTCGTAGCACCGACCCTATACCC
ATCTCTAGGGTCTTCAACCTCTTGGT
Fusion construct **44-NYESO1-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
 
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
 
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
 
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCATGCAGGCCGAAGGCCGGGGCACAGGGGGTTCGACGGGCGATGCTGAT
GGCCCAGGAGGCCCTGGCATTCCTGATGGCCCAGGGGGCAATGCTGGCGGCC
CAGGAGAGGCGGGTGCCACGGGCGGCAGAGGTCCCCGGGGCGCAGGGGCAG
CAAGGGCCTCGGGGCCGGGAGGAGGCGCCCCGCGGGGTCCGCATGGCGGCG
CGGCTTCAGGGCTGAATGGATGCTGCAGATGCGGGGCCAGGGGGCCGGAGA
GCCGCCTGCTTGAGTTCTACCTCGCCATGCCTTTCGCGACACCCATGGAAGCA
GAGCTGGCCCGCAGGAGCCTGGCCCAGGATGCCCCACCGCTTCCCGTGCCAG
GGGTGCTTCTGAAGGAGTTCACTGTGTCCGGCAACATACTGACTATCCGACTG
ACTGCTGCAGACCACCGCCAACTGCAGCTCTCCATCAGCTCCTGTCTCCAGCA
GCTTTCCCTGTTGATGTGGATCACGCAGTGCTTTCTGCCCGTGTTTTGGCTCA
GCCTCCCTCAGGGCAGAGGCGCTAAGTCGCTTGCAAAGACAACGCCACATTT
TGGGAACGCGTCCGATCCATCCTGAAATCTGGGCTCAACTTTGCTTCGACCAT
ACCTGGCCCCGTAGGAGTGGCAGCGACAGGGATCAAAGGCATCATTGAAGCC
ATTGGTTCCTTGTGGGTTTGATTCCACCCACAGAAGCGTTGACGACGCAAAAC
GTCCTTAAAGCGTTGACGACGCAAAACGTCCCCAAGCTCGTAGCACCGACCC
TATACCCATCTCTAGGGTCTTCAACCTCTTGGT
**MART-1**
   Melanoma antigen recognized by T-cells 1
   Human differentiation antigen melan-A protein mRNA, complete cds
   GenBank: U06654.1
**44-MART1-270** 
GTAAACAACCAATAACATCAGGGTATCCAAAGCAGCACGCCGCCATG
CCAAGAGAAGATGCTCACTTCATCTATGGTTACCCCAAGAAGGGCACGGCC
ACTCTTACACCACGGCTGAAGAGGCCGCTGGGATCGGCATCCTGACAGTGAT
CCTGGGAGTCTTACTGCTCATCGGCTGTTGGTATTGTAGAAGACGAAATGGAT
 
ACAGAGCCTTGATGGATAAAAGTCTTCATGTTGGCACTCAATGTGCCTTAACA
AGAAGATGCCCACAAGAAGGGTTTGATCATCGGACAGCAAAGTGTCTCTTC
AAGAGAAAAACTGTGAACCTGTGGTTCCCAATGCTCCACCTGCTTATGAGAA
ACTCTCTGCAGAACAGTCACCACCACCTTATTCACCTTAAGTCGCTTGCAAAG
ACAACGCCACATTTTGGGAACGCGTCCGATCCATCCTGAAATCTGGGCTCAAC
TTTGCTTCGACCATACCTGGCCCCGTAGGAGTGGCAGCGACAGGGATCAAAG
GCATCATTGAAGCCATTGGTTCCTTGTGGGTTTGATTCCACCCACAGAAGCGT
TGACGACGCAAAACGTCCTTAAAGCGTTGACGACGCAAAACGTCCCCAAGCT
CGTAGCACCGACCCTATACCCATCTCTAGGGTCTTCAACCTCTTGGT
**Fusion construct 44-MART1-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
 
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
 
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCATGCCAAGAGAAGATGCTCACTTCATCTATGGTTACCCCAAGAAGGGGC
ACGGCCACTCTTACACCACGGCTGAAGAGGCCGCTGGGATCGGCATCCTGAC
AGTGATCCTGGGAGTCTTACTGCTCATCGGCTGTTGGTATTGTAGAAGACGAA
ATGGATACAGAGCCTTGATGGATAAAAGTCTTCATGTTGGCACTCAATGTGCC
TTAACAAGAAGATGCCCACAAGAAGGGTTTGATCATCGGGACAGCAAAGTGT
CTCTTCAAGAGAAAAACTGTGAACCTGTGGTTCCCAATGCTCCACCTGCTTAT
GAGAAACTCTCTGCAGAACAGTCACCACCACCTTATTCACCTTAAGTCGCTTG
CAAAGACAACGCCACATTTTGGGAACGCGTCCGATCCATCCTGAAATCTGGG
CTCAACTTTGCTTCGACCATACCTGGCCCCGTAGGAGTGGCAGCGACAGGGA
TCAAAGGCATCATTGAAGCCATTGGTTCCTTGTGGGTTTGATTCCACCCACAG
AAGCGTTGACGACGCAAAACGTCCTTAAAGCGTTGACGACGCAAAACGTCCC
CAAGCTCGTAGCACCGACCCTATACCCATCTCTAGGGTCTTCAACCTCTTGGT
**P53**
   Homo sapiens tumor protein p53, mRNA (cDNA clone MGC:646 IMAGE:3544714), complete cds
**44-p53-270** 
GTAAACAACCAATAACATCAGGGTATCCAAAGCAGCACGCCGCCATG
GAGGAGCCGCAGTCAGATCCTAGCGTCGAGCCCCCTCTGAGTCAGGAAACAT
TTTCAGACCTATGGAAACTACTTCCTGAAAACAACGTTCTGTCCCCCTTGCCG
TCCCAAGCAATGGATGATTTGATGCTGTCCCCGGACGATATTGAACAATGGTT
CACTGAAGACCCAGGTCCAGATGAAGCTCCCAGAATGCCAGAGGCTGCTCCC
CGCGTGGCCCCTGCACCAGCAGCTCCTACACCGGCGGCCCCTGCACCAGCCC
CCTCCTGGCCCCTGTCATCTTCTGTCCCTTCCCAGAAAACCTACCAGGGCAGC
TACGGTTTCCGTCTGGGCTTCTTGCATTCTGGGACAGCCAAGTCTGTGACTTG
CACGTACTCCCCTGCCCTCAACAAGATGTTTTGCCAACTGGCCAAGACCTGCC
CTGTGCAGCTGTGGGTTGATTCCACACCCCCGCCCGGCACCCGCGTCCGCGCC
ATGGCCATCTACAAGCAGTCACAGCACATGACGGAGGTTGTGAGGCGCTGCC
CCCACCATGAGCGCTGCTCAGATAGCGATGGTCTGGCCCCTCCTCAGCATCTT
ATCCGAGTGGAAGGAAATTTGCGTGTGGAGTATTTGGATGACAGAAACACTT
TTCGACATAGTGTGGTGGTGCCCTATGAGCCGCCTGAGGTTGGCTCTGACTGT
ACCACCATCCACTACAACTACATGTGTAACAGTTCCTGCATGGGCGGCATGA
ACCGGAGGCCCATCCTCACCATCATCACACTGGAAGACTCCAGTGGTAATCT
ACTGGGACGGAACAGCTTTGAGGTGCGTGTTTGTGCCTGTGCTGGGAGAGAC
CGGCGCACAGAGGAAGAGAATCTCCGCAAGAAAGGGGAGCCTCACCACGAG
CTGCCCCCAGGGAGCACTAAGCGAGCACTGCCCAACAACACCAGCTCCTCTC
CCCAGCCAAAGAAGAAACCACTGGATGGAGAATATTTCACCCTTCAGATCCG
TGGGCGTGAGCGCTTCGAGATGTTCCGAGAGCTGAATGAGGCCTTGGAACTC
AAGGATGCCCAGGCTGGGAAGGAGCCAGGGGGGAGCAGGGCTCACTCCAGC
CACCTGAAGTCCAAAAAGGGTCAGTCTACCTCCCGCCATAAAAAACTCATGT
TCAAGACAGAAGGGCCTGACTCAGACTGAGTCGCTTGCAAAGACAACGCCAC
 
ATTTTGGGAACGCGTCCGATCCATCCTGAAATCTGGGCTCAACTTTGCTTCGA
CCATACCTGGCCCCGTAGGAGTGGCAGCGACAGGGATCAAAGGCATCATTGA
AGCCATTGGTTCCTTGTGGGTTTGATTCCACCCACAGAAGCGTTGACGACGCA
AAACGTCCTTAAAGCGTTGACGACGCAAAACGTCCCCAAGCTCGTAGCACCG
ACCCTATACCCATCTCTAGGGTCTTCAACCTCTTGGT
Fusion construct **44-p53-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
 
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
 
GAGAAATCACTGACGTCTGGGCGGATATTACCATCAACCGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCATGGAGGAGCCGCAGTCAGATCCTAGCGTCGAGCCCCCTCTGAGTCAGG
AAACATTTTCAGACCTATGGAAACTACTTCCTGAAAACAACGTTCTGTCCCCC
TTGCCGTCCCAAGCAATGGATGATTTGATGCTGTCCCCGGACGATATTGAACA
ATGGTTCACTGAAGACCCAGGTCCAGATGAAGCTCCCAGAATGCCAGAGGCT
GCTCCCCGCGTGGCCCCTGCACCAGCAGCTCCTACACCGGCGGCCCCTGCACC
AGCCCCCTCCTGGCCCCTGTCATCTTCTGTCCCTTCCCAGAAAACCTACCAGG
GCAGCTACGGTTTCCGTCTGGGCTTCTTGCATTCTGGGACAGCCAAGTCTGTG
ACTTGCACGTACTCCCCTGCCCTCAACAAGATGTTTTGCCAACTGGCCAAGAC
CTGCCCTGTGCAGCTGTGGGTTGATTCCACACCCCCGCCCGGCACCCGCGTCC
GCGCCATGGCCATCTACAAGCAGTCACAGCACATGACGGAGGTTGTGAGGCG
CTGCCCCCACCATGAGCGCTGCTCAGATAGCGATGGTCTGGCCCCTCCTCAGC
ATCTTATCCGAGTGGAAGGAAATTTGCGTGTGGAGTATTTGGATGACAGAAA
CACTTTTCGACATAGTGTGGTGGTGCCCTATGAGCCGCCTGAGGTTGGCTCTG
ACTGTACCACCATCCACTACAACTACATGTGTAACAGTTCCTGCATGGGCGGC
ATGAACCGGAGGCCCATCCTCACCATCATCACACTGGAAGACTCCAGTGGTA
ATCTACTGGGACGGAACAGCTTTGAGGTGCGTGTTTGTGCCTGTGCTGGGAGA
 
GACCGGCGCACAGAGGAAGAGAATCTCCGCAAGAAAGGGGAGCCTCACCAC
GAGCTGCCCCCAGGGAGCACTAAGCGAGCACTGCCCAACAACACCAGCTCCT
CTCCCCAGCCAAAGAAGAAACCACTGGATGGAGAATATTTCACCCTTCAGAT
CCGTGGGCGTGAGCGCTTCGAGATGTTCCGAGAGCTGAATGAGGCCTTGGAA
CTCAAGGATGCCCAGGCTGGGAAGGAGCCAGGGGGGAGCAGGGCTCACTCC
AGCCACCTGAAGTCCAAAAAGGGTCAGTCTACCTCCCGCCATAAAAAACTCA
TGTTCAAGACAGAAGGGCCTGACTCAGACTGAGTCGCTTGCAAAGACAACGC
CACATTTTGGGAACGCGTCCGATCCATCCTGAAATCTGGGCTCAACTTTGCTT
CGACCATACCTGGCCCCGTAGGAGTGGCAGCGACAGGGATCAAAGGCATCAT
TGAAGCCATTGGTTCCTTGTGGGTTTGATTCCACCCACAGAAGCGTTGACGAC
GCAAAACGTCCTTAAAGCGTTGACGACGCAAAACGTCCCCAAGCTCGTAGCA
CCGACCCTATACCCATCTCTAGGGTCTTCAACCTCTTGGT
**19nt 5'UTR**
**NY-ESO-1**
**MART-1**
**p53**
**NY-ESO-1**
   Cancer/testis antigen 1B (CTAG1B)/NY-ESO-1 (multiple tumors): NM_001327.3 CDS
   *BNT111* (NY-ESO-1, tyrosinase, MAGE-A3, and *TPTE*)→ advanced melanoma (FDA fast track designation 2021)
**19-NYESO1-270** 
GTAAACAACCAATAACATCATGCAGGCCGAAGGCCGGGCACAGGGG
GTTCGACGGCGATGCTGATGGCCCAGGAGGCCCTGGCATTCCTGATGGCCC
AGGGGGCAATGCTGGCGGCCCAGGAGAGGCGGGTGCCACGGCGGCAGAGG
TCCCCGGGGCGCAGGGGCAGCAAGGGCCTCGGGGCCGGGAGGAGGCGCCCC
GCGGGGTCCGCATGGCGGCGCGGCTTCAGGGCTGAATGGATGCTGCAGATGC
GGGGCCAGGGGGCCGGAGAGCCGCCTGCTTGAGTTCTACCTCGCCATGCCTTT
 
CGCGACACCCATGGAAGCAGAGCTGGCCCGCAGGAGCCTGGCCCAGGATGCC
CCACCGCTTCCCGTGCCAGGGGTGCTTCTGAAGGAGTTCACTGTGTCCGGCAA
CATACTGACTATCCGACTGACTGCTGCAGACCACCGCCAACTGCAGCTCTCCA
TCAGCTCCTGTCTCCAGCAGCTTTCCCTGTTGATGTGGATCACGCAGTGCTTTC
TGCCCGTGTTTTGGCTCAGCCTCCCTCAGGGCAGAGGCGCTAAGTCGCTTGC
AAAGACAACGCCACATTTTGGGAACGCGTCCGATCCATCCTGAAATCTGGGC
TCAACTTTGCTTCGACCATACCTGGCCCCGTAGGAGTGGCAGCGACAGGGAT
CAAAGGCATCATTGAAGCCATTGGTTCCTTGTGGGTTTGATTCCACCCACAGA
AGCGTTGACGACGCAAAACGTCCTTAAAGCGTTGACGACGCAAAACGTCCCC
AAGCTCGTAGCACCGACCCTATACCCATCTCTAGGGTCTTCAACCTCTTGGT
Fusion construct **19-NYESO1-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
 
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
 
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCATGCAGGCCGAAGGCCGGGGCA
CAGGGGGTTCGACGGGCGATGCTGATGGCCCAGGAGGCCCTGGCATTCCTGA
TGGCCCAGGGGGCAATGCTGGCGGCCCAGGAGAGGCGGGTGCCACGGGCGG
CAGAGGTCCCCGGGGCGCAGGGGCAGCAAGGGCCTCGGGGCCGGGAGGAG
CGCCCCGCGGGGTCCGCATGGCGGCGCGGCTTCAGGGCTGAATGGATGCTGC
AGATGCGGGGCCAGGGGGCCGGAGAGCCGCCTGCTTGAGTTCTACCTCGCCA
TGCCTTTCGCGACACCCATGGAAGCAGAGCTGGCCCGCAGGAGCCTGGCCCA
GGATGCCCCACCGCTTCCCGTGCCAGGGGTGCTTCTGAAGGAGTTCACTGTGT
CCGGCAACATACTGACTATCCGACTGACTGCTGCAGACCACCGCCAACTGCA
GCTCTCCATCAGCTCCTGTCTCCAGCAGCTTTCCCTGTTGATGTGGATCACGC
AGTGCTTTCTGCCCGTGTTTTGGCTCAGCCTCCCTCAGGGCAGAGGCGCTAA
GTCGCTTGCAAAGACAACGCCACATTTGGGAACGCGTCCGATCCATCCTGA
 
AATCTGGGCTCAACTTTGCTTCGACCATACCTGGCCCCGTAGGAGTGGCAGCG
ACAGGGATCAAAGGCATCATTGAAGCCATTGGTTCCTTGTGGGTTTGATTCCA
CCCACAGAAGCGTTGACGACGCAAAACGTCCTTAAAGCGTTGACGACGCAAA
ACGTCCCCAAGCTCGTAGCACCGACCCTATACCCATCTCTAGGGTCTTCAACC
TCTTGGT
**MART-1**
   Melanoma antigen recognized by T-cells 1
   Human differentiation antigen melan-A protein mRNA, complete cds
   GenBank: U06654.1
**19-MART1-270** 
GTAAACAACCAATAACATCATGCCAAGAGAAGATGCTCACTTCATCTA
TGGTTACCCCAAGAAGGGCACGGCCACTCTTACACCACGGCTGAAGAGGCC
GCTGGGATCGGCATCCTGACAGTGATCCTGGGAGTCTTACTGCTCATCGGCTG
TTGGTATTGTAGAAGACGAAATGGATACAGAGCCTTGATGGATAAAAGTCTT
CATGTTGGCACTCAATGTGCCTTAACAAGAAGATGCCCACAAGAAGGGTTTG
ATCATCGGGACAGCAAAGTGTCTCTTCAAGAGAAAAACTGTGAACCTGTGGT
TCCCAATGCTCCACCTGCTTATGAGAAACTCTCTGCAGAACAGTCACCACCAC
CTTATTCACCTTAAGTCGCTTGCAAAGACAACGCCACATTTTGGGAACGCGTC
CGATCCATCCTGAAATCTGGGCTCAACTTTGCTTCGACCATACCTGGCCCCGT
AGGAGTGGCAGCGACAGGGATCAAAGGCATCATTGAAGCCATTGGTTCCTTG
TGGGTTTGATTCCACCCACAGAAGCGTTGACGACGCAAAACGTCCTTAAAGC
GTTGACGACGCAAAACGTCCCCAAGCTCGTAGCACCGACCCTATACCCATCTC
TAGGGTCTTCAACCTCTTGGT
**Fusion construct 19-MART1-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
 
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
 
CATTCCGTGATCAGGTGCTGCATGCAGAGCACAACCGGCATTGGTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
 
GCGTGGTAAATGAGTGATTCATCGTCCCATCTGACGAAACCCGAACTAGGCTT
ATGCCAGTGGTGTAAACAACCAATAACATCATGCCAAGAGAAGATGCTCACT
TCATCTATGGTTACCCCAAGAAGGGGCACGGCCACTCTTACACCACGGCTGA
AGAGGCCGCTGGGATCGGCATCCTGACAGTGATCCTGGGAGTCTTACTGCTC
ATCGGCTGTTGGTATTGTAGAAGACGAAATGGATACAGAGCCTTGATGGATA
AAAGTCTTCATGTTGGCACTCAATGTGCCTTAACAAGAAGATGCCCACAAGA
AGGGTTTGATCATCGGGACAGCAAAGTGTCTCTTCAAGAGAAAAACTGTGAA
CCTGTGGTTCCCAATGCTCCACCTGCTTATGAGAAACTCTCTGCAGAACAGTC
ACCACCACCTTATTCACCTTAAGTCGCTTGCAAAGACAACGCCACATTTGGG
AACGCGTCCGATCCATCCTGAAATCTGGGCTCAACTTTGCTTCGACCATACCT
GGCCCCGTAGGAGTGGCAGCGACAGGGATCAAAGGCATCATTGAAGCCATTG
GTTCCTTGTGGGTTTGATTCCACCCACAGAAGCGTTGACGACGCAAAACGTCC
TTAAAGCGTTGACGACGCAAAACGTCCCCAAGCTCGTAGCACCGACCCTATA
CCCATCTCTAGGGTCTTCAACCTCTTGGT
**P53**
   Homo sapiens tumor protein p53, mRNA (cDNA clone MGC:646 IMAGE:3544714), complete cds
**19-p53-270** 
GTAAACAACCAATAACATCATGGAGGAGCCGCAGTCAGATCCTAGCGT
CGAGCCCCCTCTGAGTCAGGAAACATTTTCAGACCTATGGAAACTACTTCCTG
AAAACAACGTTCTGTCCCCCTTGCCGTCCCAAGCAATGGATGATTTGATGCTG
TCCCCGGACGATATTGAACAATGGTTCACTGAAGACCCAGGTCCAGATGAAG
CTCCCAGAATGCCAGAGGCTGCTCCCCGCGTGGCCCCTGCACCAGCAGCTCCT
ACACCGGCGGCCCCTGCACCAGCCCCCTCCTGGCCCCTGTCATCTTCTGTCCC
TTCCCAGAAAACCTACCAGGGCAGCTACGGTTTCCGTCTGGGCTTCTTGCATT
CTGGGACAGCCAAGTCTGTGACTTGCACGTACTCCCCTGCCCTCAACAAGATG
TTTTGCCAACTGGCCAAGACCTGCCCTGTGCAGCTGTGGGTTGATTCCACACC
CCCGCCCGGCACCCGCGTCCGCGCCATGGCCATCTACAAGCAGTCACAGCAC
 
ATGACGGAGGTTGTGAGGCGCTGCCCCCACCATGAGCGCTGCTCAGATAGCG
ATGGTCTGGCCCCTCCTCAGCATCTTATCCGAGTGGAAGGAAATTGCGTGTG
GAGTATTTGGATGACAGAAACACTTTTCGACATAGTGTGGTGGTGCCCTATGA
GCCGCCTGAGGTTGGCTCTGACTGTACCACCATCCACTACAACTACATGTGTA
ACAGTTCCTGCATGGGCGGCATGAACCGGAGGCCCATCCTCACCATCATCAC
ACTGGAAGACTCCAGTGGTAATCTACTGGGACGGAACAGCTTTGAGGTGCGT
GTTTGTGCCTGTGCTGGGAGAGACCGGCGCACAGAGGAAGAGAATCTCCGCA
AGAAAGGGGAGCCTCACCACGAGCTGCCCCCAGGGAGCACTAAGCGAGCAC
TGCCCAACAACACCAGCTCCTCTCCCCAGCCAAAGAAGAAACCACTGGATGG
AGAATATTTCACCCTTCAGATCCGTGGGCGTGAGCGCTTCGAGATGTTCCGAG
AGCTGAATGAGGCCTTGGAACTCAAGGATGCCCAGGCTGGGAAGGAGCCAG
GGGGGAGCAGGGCTCACTCCAGCCACCTGAAGTCCAAAAAGGGTCAGTCTAC
CTCCCGCCATAAAAAACTCATGTTCAAGACAGAAGGGCCTGACTCAGACTGA
GTCGCTTGCAAAGACAACGCCACATTTTGGAACGCGTCCGATCCATCCTGA
AATCTGGGCTCAACTTTGCTTCGACCATACCTGGCCCCGTAGGAGTGGCAGCG
ACAGGGATCAAAGGCATCATTGAAGCCATTGGTTCCTTGTGGGTTTGATTCCA
CCCACAGAAGCGTTGACGACGCAAAACGTCCTTAAAGCGTTGACGACGCAAA
ACGTCCCCAAGCTCGTAGCACCGACCCTATACCCATCTCTAGGGTCTTCAACC
TCTTGGT
Fusion construct **19-p53-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
 
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
 
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCATGGAGGAGCCGCAGTCAGATC
CTAGCGTCGAGCCCCCTCTGAGTCAGGAAACATTTTCAGACCTATGGAAACTA
CTTCCTGAAAACAACGTTCTGTCCCCCTTGCCGTCCCAAGCAATGGATGATTT
 
GATGCTGTCCCCGGACGATATTGAACAATGGTTCACTGAAGACCCAGGTCCA
GATGAAGCTCCCAGAATGCCAGAGGCTGCTCCCCGCGTGGCCCCTGCACCAG
CAGCTCCTACACCGGCGGCCCCTGCACCAGCCCCCTCCTGGCCCCTGTCATCT
TCTGTCCCTTCCCAGAAAACCTACCAGGGCAGCTACGGTTTCCGTCTGGGCTT
CTTGCATTCTGGGACAGCCAAGTCTGTGACTTGCACGTACTCCCCTGCCCTCA
ACAAGATGTTTTGCCAACTGGCCAAGACCTGCCCTGTGCAGCTGTGGGTTGAT
TCCACACCCCCGCCCGGCACCCGCGTCCGCGCCATGGCCATCTACAAGCAGTC
ACAGCACATGACGGAGGTTGTGAGGCGCTGCCCCCACCATGAGCGCTGCTCA
GATAGCGATGGTCTGGCCCCTCCTCAGCATCTTATCCGAGTGGAAGGAAATTT
GCGTGTGGAGTATTTGGATGACAGAAACACTTTTCGACATAGTGTGGTGGTGC
CCTATGAGCCGCCTGAGGTTGGCTCTGACTGTACCACCATCCACTACAACTAC
ATGTGTAACAGTTCCTGCATGGGCGGCATGAACCGGAGGCCCATCCTCACCA
TCATCACACTGGAAGACTCCAGTGGTAATCTACTGGGACGGAACAGCTTTGA
GGTGCGTGTTTGTGCCTGTGCTGGGAGAGACCGGCGCACAGAGGAAGAGAAT
CTCCGCAAGAAAGGGGAGCCTCACCACGAGCTGCCCCCAGGGAGCACTAAGC
GAGCACTGCCCAACAACACCAGCTCCTCTCCCCAGCCAAAGAAGAAACCACT
GGATGGAGAATATTTCACCCTTCAGATCCGTGGGCGTGAGCGCTTCGAGATGT
TCCGAGAGCTGAATGAGGCCTTGGAACTCAAGGATGCCCAGGCTGGGAAGGA
GCCAGGGGGGAGCAGGGCTCACTCCAGCCACCTGAAGTCCAAAAAGGGTCA
GTCTACCTCCCGCCATAAAAAACTCATGTTCAAGACAGAAGGGCCTGACTCA
GACTGAGTCGCTTGCAAAGACAACGCCACATTTTGGGAACGCGTCCGATCCA
TCCTGAAATCTGGGCTCAACTTTGCTTCGACCATACCTGGCCCCGTAGGAGTG
GCAGCGACAGGGATCAAAGGCATCATTGAAGCCATTGGTTCCTTGTGGGTTT
GATTCCACCCACAGAAGCGTTGACGACGCAAAACGTCCTTAAAGCGTTGACG
ACGCAAAACGTCCCCAAGCTCGTAGCACCGACCCTATACCCATCTCTAGGGTC
TTCAACCTCTTGGT
**NY-ESO-1**
**MART-1**
**p53**
**MAGE-A1**
**MAGE-A3**
**MAGE-C2**
**gp100**
**Tyrosinase**
**HPV(16) E6 and E7**
**GM-CSF**
**CD40L**
RNA1 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
 
TCCATGGCCGCAATCGCCCCATCGCGCTTTGGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
 
ACGACGCGCGATCCAACAATACCATTGGCAACGGCTGCCATCGATCGCGTTG
AGGGCTACCTCGTCACCGACGGCCTGAGCCCGCTTACTGGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGT
**NY-ESO-1**
   Cancer/testis antigen 1B (CTAG1B)/NY-ESO-1 (multiple tumors): NM_001327.3 CDS
   *BNT111* (NY-ESO-1, tyrosinase, MAGE-A3, and *TPTE*)→ advanced melanoma (FDA fast track designation 2021)
**55-NYESO1-270** 
GTAAACAACCAATAACATCAGGGTATCCAAAGCAGCACGCCGCCGAA
GAGCGGCCATGCAGGCCGAAGGCCGGGCACAGGGGGTTCGACGGCGATG
CTGATGGCCCAGGAGGCCCTGGCATTCCTGATGGCCCAGGGGGCAATGCTGG
CGGCCCAGGAGAGGCGGGTGCCACGGGCGGCAGAGGTCCCCGGGGCGCAGG
 
GGCAGCAAGGGCCTCGGGGCCGGGAGGAGGCGCCCCGCGGGGTCCGCATGG

CGGCGCGGCTTCAGGGCTGAATGGATGCTGCAGATGCGGGGCCAGGGGGCCG

GAGAGCCGCCTGCTTGAGTTCTACCTCGCCATGCCTTTCGCGACACCCATGGA

AGCAGAGCTGGCCCGCAGGAGCCTGGCCCAGGATGCCCCACCGCTTCCCGTG

CCAGGGGTGCTTCTGAAGGAGTTCACTGTGTCCGGCAACATACTGACTATCCG

ACTGACTGCTGCAGACCACCGCCAACTGCAGCTCTCCATCAGCTCCTGTCTCC

AGCAGCTTTCCCTGTTGATGTGGATCACGCAGTGCTTTCTGCCCGTGTTTTGG

CTCAGCCTCCCTCAGGGCAGAGGCGCTAAGTCGCTTGCAAAGACAACGCCAC

ATTTTGGGAACGCGTCCGATCCATCCTGAAATCTGGGCTCAACTTTGCTTCGA

CCATACCTGGCCCCGTAGGAGTGGCAGCGACAGGGATCAAAGGCATCATTGA

AGCCATTGGTTCCTTGTGGGTTTGATTCCACCCACAGAAGCGTTGACGACGCA

AAACGTCCTTAAAGCGTTGACGACGCAAAACGTCCCCAAGCTCGTAGCACCG

ACCCTATACCCATCTCTAGGGTCTTCAACCTCTTGGT
Fusion construct **55-NYESO1-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
 
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
 
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGCAGGCCGAAGGCCGGGGCACAGGGGGTTCGACGG
GCGATGCTGATGGCCCAGGAGGCCCTGGCATTCCTGATGGCCCAGGGGGCAA
TGCTGGCGGCCCAGGAGAGGCGGGTGCCACGGGCGGCAGAGGTCCCGGGG
CGCAGGGGCAGCAAGGGCCTCGGGGCCGGGAGGAGGCGCCCGCGGGTCC
GCATGGCGGCGCGGCTTCAGGGCTGAATGGATGCTGCAGATGCGGGGCCAGG
GGGCCGGAGAGCCGCCTGCTTGAGTTCTACCTCGCCATGCCTTTCGCGACACC
CATGGAAGCAGAGCTGGCCCGCAGGAGCCTGGCCCAGGATGCCCCACCGCTT
CCCGTGCCAGGGGTGCTTCTGAAGGAGTTCACTGTGTCCGGCAACATACTGAC
 
TATCCGACTGACTGCTGCAGACCACCGCCAACTGCAGCTCTCCATCAGCTCCT
GTCTCCAGCAGCTTTCCCTGTTGATGTGGATCACGCAGTGCTTTCTGCCCGTGT
TTTTGGCTCAGCCTCCCTCAGGGCAGAGGCGCTAAGTCGCTTGCAAAGACAA
CGCCACATTTTGGGAACGCGTCCGATCCATCCTGAAATCTGGGCTCAACTTTG
CTTCGACCATACCTGGCCCCGTAGGAGTGGCAGCGACAGGGATCAAAGGCAT
CATTGAAGCCATTGGTTCCTTGTGGGTTTGATTCCACCCACAGAAGCGTTGAC
GACGCAAAACGTCCTTAAAGCGTTGACGACGCAAAACGTCCCCAAGCTCGTA
GCACCGACCCTATACCCATCTCTAGGGTCTTCAACCTCTTGGT
**MART-1**
   Melanoma antigen recognized by T-cells 1
   Human differentiation antigen melan-A protein mRNA, complete cds
   GenBank: U06654.1
**55-MART1-270** 
GTAAACAACCAATAACATCAGGGTATCCAAAGCAGCACGCCGCCGAA
GAGCGGCCATGCCAAGAGAAGATGCTCACTTCATCTATGGTTACCCCAAGAA
GGGGCACGGCCACTCTTACACCACGGCTGAAGAGGCCGCTGGGATCGGCATC
CTGACAGTGATCCTGGGAGTCTTACTGCTCATCGGCTGTTGGTATTGTAGAAG
ACGAAATGGATACAGAGCCTTGATGGATAAAAGTCTTCATGTTGGCACTCAA
TGTGCCTTAACAAGAAGATGCCCACAAGAAGGGTTTGATCATCGGGACAGCA
AAGTGTCTCTTCAAGAGAAAAACTGTGAACCTGTGGTTCCCAATGCTCCACCT
GCTTATGAGAAACTCTCTGCAGAACAGTCACCACCACCTTATTCACCTTAAGT
CGCTTGCAAAGACAACGCCACATTTTGGGAACGCGTCCGATCCATCCTGAAA
TCTGGGCTCAACTTTGCTTCGACCATACCTGGCCCCGTAGGAGTGGCAGCGAC
AGGGATCAAAGGCATCATTGAAGCCATTGGTTCCTTGTGGGTTTGATTCCACC
CACAGAAGCGTTGACGACGCAAAACGTCCTTAAAGCGTTGACGACGCAAAAC
GTCCCCAAGCTCGTAGCACCGACCCTATACCCATCTCTAGGGTCTTCAACCTC
TTGGT
**Fusion construct 55-MART1-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
 
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
 
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGCCAAGAGAAGATGCTCACTTCATCTATGGTTACCC
CAAGAAGGGGCACGGCCACTCTTACACCACGGCTGAAGAGGCCGCTGGGATC
GGCATCCTGACAGTGATCCTGGGAGTCTTACTGCTCATCGGCTGTTGGTATTG
TAGAAGACGAAATGGATACAGAGCCTTGATGGATAAAAGTCTTCATGTTGGC
ACTCAATGTGCCTTAACAAGAAGATGCCCACAAGAAGGGTTTGATCATCGGG
ACAGCAAAGTGTCTCTTCAAGAGAAAAACTGTGAACCTGTGGTTCCCAATGC
TCCACCTGCTTATGAGAAACTCTCTGCAGAACAGTCACCACCACCTTATTCAC
CTTAAGTCGCTTGCAAAGACAACGCCACATTTTGGAACGCGTCCGATCCATC
CTGAAATCTGGGCTCAACTTTGCTTCGACCATACCTGGCCCCGTAGGAGTGGC
AGCGACAGGGATCAAAGGCATCATTGAAGCCATTGGTTCCTTGTGGGTTTGAT
TCCACCCACAGAAGCGTTGACGACGCAAAACGTCCTTAAAGCGTTGACGACG
CAAAACGTCCCCAAGCTCGTAGCACCGACCCTATACCCATCTCTAGGGTCTTC
AACCTCTTGGT
**P53**
   Homo sapiens tumor protein p53, mRNA (cDNA clone MGC:646 IMAGE:3544714), complete cds
**55-p53-270** 
TAAACAACCAATAACATCAGGGTATCCAAAGCAGCACGCCGCCGAAG
AGCGGCCATGGAGGAGCCGCAGTCAGATCCTAGCGTCGAGCCCCCTCTGAGT
CAGGAAACATTTTCAGACCTATGGAAACTACTTCCTGAAAACAACGTTCTGTC
CCCCTTGCCGTCCCAAGCAATGGATGATTTGATGCTGTCCCCGGACGATATTG
 
AACAATGGTTCACTGAAGACCCAGGTCCAGATGAAGCTCCCAGAATGCCAGA
GGCTGCTCCCCGCGTGGCCCCTGCACCAGCAGCTCCTACACCGGCGGCCCCTG
CACCAGCCCCCTCCTGGCCCCTGTCATCTTCTGTCCCTTCCCAGAAAACCTAC
CAGGGCAGCTACGGTTTCCGTCTGGGCTTCTTGCATTCTGGGACAGCCAAGTC
TGTGACTTGCACGTACTCCCCTGCCCTCAACAAGATGTTTTGCCAACTGGCCA
AGACCTGCCCTGTGCAGCTGTGGGTTGATTCCACACCCCCGCCCGGCACCCGC
GTCCGCGCCATGGCCATCTACAAGCAGTCACAGCACATGACGGAGGTTGTGA
GGCGCTGCCCCCACCATGAGCGCTGCTCAGATAGCGATGGTCTGGCCCCTCCT
CAGCATCTTATCCGAGTGGAAGGAAATTTGCGTGTGGAGTATTTGGATGACA
GAAACACTTTTCGACATAGTGTGGTGGTGCCCTATGAGCCGCCTGAGGTTGGC
TCTGACTGTACCACCATCCACTACAACTACATGTGTAACAGTTCCTGCATGGG
CGGCATGAACCGGAGGCCCATCCTCACCATCATCACACTGGAAGACTCCAGT
GGTAATCTACTGGGACGGAACAGCTTTGAGGTGCGTGTTTGTGCCTGTGCTGG
GAGAGACCGGCGCACAGAGGAAGAGAATCTCCGCAAGAAAGGGGAGCCTCA
CCACGAGCTGCCCCCAGGGAGCACTAAGCGAGCACTGCCCAACAACACCAGC
TCCTCTCCCCAGCCAAAGAAGAAACCACTGGATGGAGAATATTTCACCCTTCA
GATCCGTGGGCGTGAGCGCTTCGAGATGTTCCGAGAGCTGAATGAGGCCTTG
GAACTCAAGGATGCCCAGGCTGGGAAGGAGCCAGGGGGGAGCAGGGCTCAC
TCCAGCCACCTGAAGTCCAAAAAGGGTCAGTCTACCTCCCGCCATAAAAAAC
TCATGTTCAAGACAGAAGGGCCTGACTCAGACTGAGTCGCTTGCAAAGACAA
CGCCACATTTTGGGAACGCGTCCGATCCATCCTGAAATCTGGGCTCAACTTTG
CTTCGACCATACCTGGCCCCGTAGGAGTGGCAGCGACAGGGATCAAAGGCAT
CATTGAAGCCATTGGTTCCTTGTGGGTTTGATTCCACCCACAGAAGCGTTGAC
GACGCAAAACGTCCTTAAAGCGTTGACGACGCAAAACGTCCCCAAGCTCGTA
GCACCGACCCTATACCCATCTCTAGGGTCTTCAACCTCTTGGT
Fusion construct **55-p53-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
 
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
 
TCATCGAAGCGTTCGTGAAGAACGAACCGACCATGAAGGCTGGCCGTATCAT
CTCGTCGTTCGCTGACATGCGGTTCCTACTGCGGTTTCCAGCTATACGCTGG
CATTCCGTGATCAGGTGCTGCATGCAGAGCACAACCGGCATTGGTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
 
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGGAGGAGCCGCAGTCAGATCCTAGCGTCGAGCCCCC
TCTGAGTCAGGAAACATTTTCAGACCTATGGAAACTACTTCCTGAAAACAAC
GTTCTGTCCCCCTTGCCGTCCCAAGCAATGGATGATTTGATGCTGTCCCCGGA
CGATATTGAACAATGGTTCACTGAAGACCCAGGTCCAGATGAAGCTCCCAGA
ATGCCAGAGGCTGCTCCCCGCGTGGCCCCTGCACCAGCAGCTCCTACACCGG
CGGCCCCTGCACCAGCCCCCTCCTGGCCCCTGTCATCTTCTGTCCCTTCCCAGA
AAACCTACCAGGGCAGCTACGGTTTCCGTCTGGGCTTCTTGCATTCTGGGACA
GCCAAGTCTGTGACTTGCACGTACTCCCCTGCCCTCAACAAGATGTTTTGCCA
ACTGGCCAAGACCTGCCCTGTGCAGCTGTGGGTTGATTCCACACCCCCGCCCG
GCACCCGCGTCCGCGCCATGGCCATCTACAAGCAGTCACAGCACATGACGGA
GGTTGTGAGGCGCTGCCCCCACCATGAGCGCTGCTCAGATAGCGATGGTCTG
GCCCCTCCTCAGCATCTTATCCGAGTGGAAGGAAATTTGCGTGTGGAGTATTT
GGATGACAGAAACACTTTTCGACATAGTGTGGTGGTGCCCTATGAGCCGCCT
GAGGTTGGCTCTGACTGTACCACCATCCACTACAACTACATGTGTAACAGTTC
CTGCATGGGCGGCATGAACCGGAGGCCCATCCTCACCATCATCACACTGGAA
GACTCCAGTGGTAATCTACTGGGACGGAACAGCTTTGAGGTGCGTGTTTGTGC
CTGTGCTGGGAGAGACCGGCGCACAGAGGAAGAGAATCTCCGCAAGAAAGG
GGAGCCTCACCACGAGCTGCCCCCAGGGAGCACTAAGCGAGCACTGCCCAAC
AACACCAGCTCCTCTCCCCAGCCAAAGAAGAAACCACTGGATGGAGAATATT
TCACCCTTCAGATCCGTGGGCGTGAGCGCTTCGAGATGTTCCGAGAGCTGAAT
GAGGCCTTGGAACTCAAGGATGCCCAGGCTGGGAAGGAGCCAGGGGGGAGC
AGGGCTCACTCCAGCCACCTGAAGTCCAAAAAGGGTCAGTCTACCTCCCGCC
ATAAAAAACTCATGTTCAAGACAGAAGGGCCTGACTCAGACTGAGTCGCTTG
CAAAGACAACGCCACATTTGGGAACGCGTCCGATCCATCCTGAAATCTGGG
 
CTCAACTTTGCTTCGACCATACCTGGCCCCGTAGGAGTGGCAGCGACAGGGA
TCAAAGGCATCATTGAAGCCATTGGTTCCTTGTGGGTTTGATTCCACCCACAG
AAGCGTTGACGACGCAAAACGTCCTTAAAGCGTTGACGACGCAAAACGTCCC
CAAGCTCGTAGCACCGACCCTATACCCATCTCTAGGGTCTTCAACCTCTTGGT
**MAGE-A1**
   Homo sapiens MAGE-1 protein (MAGEA1) mRNA, complete cds
   GenBank: AY148486.1
**55-MAGEA1-270** 
GTAAACAACCAATAACATCAGGGTATCCAAAGCAGCACGCCGCCGAA
GAGCGGCCATGTCTCTTGAGCAGAGGAGTCTGCACTGCAAGCCTGAGGAAGC
CCTTGAGGCCCAACAAGAGGCCCTGGGCCTGGTGTGTGTGCAGGCTGCCGCC
TCCTCCTCCTCTCCTCTGGTCCTGGGCACCCTGGAGGAGGTGCCCACTGCTGG
GTCAACAGATCCTCCCCAGAGTCCTCAGGGAGCCTCCGCCTTTCCCACTACCA
TCAACTTCACTCGACAGAGGCAACCCAGTGAGGGTTCCAGCAGCCGTGAAGA
GGAGGGGCCAAGCACCTCTTGTATCCTGGAGTCCTTGTTCCGAGCAGTAATCA
CTAAGAAGGTGGCTGATTTGGTTGGTTTTCTGCTCCTCAAATATCGAGCCAGG
GAGCCAGTCACAAAGGCAGAAATGCTGGAGAGTGTCATCAAAAATTACAAGC
ACTGTTTTCCTGAGATCTTCGGCAAAGCCTCTGAGTCCTTGCAGCTGGTCTTTG
GCATTGACGTGAAGGAAGCAGACCCCACCGGCCACTCCTATGTCCTTGTCACC
TGCCTAGGTCTCTCCTATGATGGCCTGCTGGGTGATAATCAGATCATGCCCAA
GACAGGCTTCCTGATAATTGTCCTGGTCATGATTGCAATGGAGGGCGGCCATG
CTCCTGAGGAGGAAATCTGGGAGGAGCTGAGTGTGATGGAGGTGTATGATGG
GAGGGAGCACAGTGCCTATGGGGAGCCCAGGAAGCTGCTCACCCAAGATTTG
GTGCAGGAAAAGTACCTGGAGTACCGGCAGGTGCCGGACAGTGATCCCGCAC
GCTATGAGTTCCTGTGGGGTCCAAGGGCCCTTGCTGAAACCAGCTATGTGAA
AGTCCTTGAGTATGTGATCAAGGTCAGTGCAAGAGTTCGCTTTTTCTTCCCAT
CCCTGCGTGAAGCAGCTTTGAGAGAGGAGGAAGAGGGAGTCTGAGTCGCTTG
CAAAGACAACGCCACATTTTGGGAACGCGTCCGATCCATCCTGAAATCTGGG
 
CTCAACTTTGCTTCGACCATACCTGGCCCCGTAGGAGTGGCAGCGACAGGGA
TCAAAGGCATCATTGAAGCCATTGGTTCCTTGTGGGTTTGATTCCACCCACAG
AAGCGTTGACGACGCAAAACGTCCTTAAAGCGTTGACGACGCAAAACGTCCC
CAAGCTCGTAGCACCGACCCTATACCCATCTCTAGGGTCTTCAACCTCTTGGT
**Fusion construct 55-MAGEA1-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
 
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
 
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGTCTCTTGAGCAGAGGAGTCTGCACTGCAAGCCTGA
GGAAGCCCTTGAGGCCCAACAAGAGGCCCTGGGCCTGGTGTGTGTGCAGGCT
GCCGCCTCCTCCTCCTCTCCTCTGGTCCTGGGCACCCTGGAGGAGGTGCCCAC
TGCTGGGTCAACAGATCCTCCCCAGAGTCCTCAGGGAGCCTCCGCCTTTCCCA
CTACCATCAACTTCACTCGACAGAGGCAACCCAGTGAGGGTTCCAGCAGCCG
TGAAGAGGAGGGGCCAAGCACCTCTTGTATCCTGGAGTCCTTGTTCCGAGCA
GTAATCACTAAGAAGGTGGCTGATTTGGTTGGTTTTCTGCTCCTCAAATATCG
AGCCAGGGAGCCAGTCACAAAGGCAGAAATGCTGGAGAGTGTCATCAAAAA
TTACAAGCACTGTTTTCCTGAGATCTTCGGCAAAGCCTCTGAGTCCTTGCAGC
TGGTCTTTGGCATTGACGTGAAGGAAGCAGACCCCACCGGCCACTCCTATGTC
CTTGTCACCTGCCTAGGTCTCTCCTATGATGGCCTGCTGGGTGATAATCAGAT
CATGCCCAAGACAGGCTTCCTGATAATTGTCCTGGTCATGATTGCAATGGAGG
GCGGCCATGCTCCTGAGGAGGAAATCTGGGAGGAGCTGAGTGTGATGGAGGT
GTATGATGGGAGGGAGCACAGTGCCTATGGGGAGCCCAGGAAGCTGCTCACC
CAAGATTTGGTGCAGGAAAAGTACCTGGAGTACCGGCAGGTGCCGGACAGTG
ATCCCGCACGCTATGAGTTCCTGTGGGGTCCAAGGGCCCTTGCTGAAACCAGC
TATGTGAAAGTCCTTGAGTATGTGATCAAGGTCAGTGCAAGAGTTCGCTTTTT
 
CTTCCCATCCCTGCGTGAAGCAGCTTTGAGAGAGGAGGAAGAGGGAGTCTGA
GTCGCTTGCAAAGACAACGCCACATTTTGGGAACGCGTCCGATCCATCCTGA
AATCTGGGCTCAACTTTGCTTCGACCATACCTGGCCCCGTAGGAGTGGCAGCG
ACAGGGATCAAAGGCATCATTGAAGCCATTGGTTCCTTGTGGGTTTGATTCCA
CCCACAGAAGCGTTGACGACGCAAAACGTCCTTAAAGCGTTGACGACGCAAA
ACGTCCCCAAGCTCGTAGCACCGACCCTATACCCATCTCTAGGGTCTTCAACC
TCTTGGT
**MAGE-A3 Melanoma-associated antigen 3**
   Homo sapiens MAGE family member A3 (MAGEA3), mRNA
   NCBI Reference Sequence: NM_005362.4
**55-MAGEA3-270** 
GTAAACAACCAATAACATCAGGGTATCCAAAGCAGCACGCCGCCGAA
GAGCGGCCATGCCTCTTGAGCAGAGGAGTCAGCACTGCAAGCCTGAAGAAGG
CCTTGAGGCCCGAGGAGAGGCCCTGGGCCTGGTGGGTGCGCAGGCTCCTGCT
ACTGAGGAGCAGGAGGCTGCCTCCTCCTCTTCTACTCTAGTTGAAGTCACCCT
GGGGGAGGTGCCTGCTGCCGAGTCACCAGATCCTCCCCAGAGTCCTCAGGGA
GCCTCCAGCCTCCCCACTACCATGAACTACCCTCTCTGGAGCCAATCCTATGA
GGACTCCAGCAACCAAGAAGAGGAGGGGCCAAGCACCTTCCCTGACCTGGAG
TCCGAGTTCCAAGCAGCACTCAGTAGGAAGGTGGCCGAGTTGGTTCATTTTCT
GCTCCTCAAGTATCGAGCCAGGGAGCCGGTCACAAAGGCAGAAATGCTGGGG
AGTGTCGTCGGAAATTGGCAGTATTTCTTTCCTGTGATCTTCAGCAAAGCTTC
CAGTTCCTTGCAGCTGGTCTTTGGCATCGAGCTGATGGAAGTGGACCCCATCG
GCCACTTGTACATCTTTGCCACCTGCCTGGGCCTCTCCTACGATGGCCTGCTG
GGTGACAATCAGATCATGCCCAAGGCAGGCCTCCTGATAATCGTCCTGGCCA
TAATCGCAAGAGAGGGCGACTGTGCCCCTGAGGAGAAAATCTGGGAGGAGCT
GAGTGTGTTAGAGGTGTTTGAGGGGAGGGAAGACAGTATCTTGGGGGATCCC
AAGAAGCTGCTCACCCAACATTTCGTGCAGGAAAACTACCTGGAGTACCGGC
AGGTCCCCGGCAGTGATCCTGCATGTTATGAATTCCTGTGGGGTCCAAGGGCC
 
CTCGTTGAAACCAGCTATGTGAAAGTCCTGCACCATATGGTAAAGATCAGTG
GAGGACCTCACATTTCCTACCCACCCCTGCATGAGTGGGTTTTGAGAGAGGG
GGAAGAGTGAGTCGCTTGCAAAGACAACGCCACATTTTGGGAACGCGTCCGA
TCCATCCTGAAATCTGGGCTCAACTTTGCTTCGACCATACCTGGCCCCGTAGG
AGTGGCAGCGACAGGGATCAAAGGCATCATTGAAGCCATTGGTTCCTTGTGG
GTTTGATTCCACCCACAGAAGCGTTGACGACGCAAAACGTCCTTAAAGCGTT
GACGACGCAAAACGTCCCCAAGCTCGTAGCACCGACCCTATACCCATCTCTA
GGGTCTTCAACCTCTTGGT
Fusion construct **55-MAGEA3-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
 
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
 
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGCCTCTTGAGCAGAGGAGTCAGCACTGCAAGCCTGA
AGAAGGCCTTGAGGCCCGAGGAGAGGCCCTGGGCCTGGTGGGTGCGCAGGCT
CCTGCTACTGAGGAGCAGGAGGCTGCCTCCTCCTCTTCTACTCTAGTTGAAGT
CACCCTGGGGGAGGTGCCTGCTGCCGAGTCACCAGATCCTCCCCAGAGTCCTC
AGGGAGCCTCCAGCCTCCCCACTACCATGAACTACCCTCTCTGGAGCCAATCC
TATGAGGACTCCAGCAACCAAGAAGAGGAGGGGCCAAGCACCTTCCCTGACC
TGGAGTCCGAGTTCCAAGCAGCACTCAGTAGGAAGGTGGCCGAGTTGGTTCA
TTTTCTGCTCCTCAAGTATCGAGCCAGGGAGCCGGTCACAAAGGCAGAAATG
CTGGGGAGTGTCGTCGGAAATTGGCAGTATTTCTTTCCTGTGATCTTCAGCAA
AGCTTCCAGTTCCTTGCAGCTGGTCTTTGGCATCGAGCTGATGGAAGTGGACC
CCATCGGCCACTTGTACATCTTTGCCACCTGCCTGGGCCTCTCCTACGATGGC
CTGCTGGGTGACAATCAGATCATGCCCAAGGCAGGCCTCCTGATAATCGTCCT
GGCCATAATCGCAAGAGAGGGCGACTGTGCCCCTGAGGAGAAAATCTGGGA
 
GGAGCTGAGTGTGTTAGAGGTGTTTGAGGGGAGGGAAGACAGTATCTTGGGG
GATCCCAAGAAGCTGCTCACCCAACATTTCGTGCAGGAAAACTACCTGGAGT
ACCGGCAGGTCCCCGGCAGTGATCCTGCATGTTATGAATTCCTGTGGGGTCCA
AGGGCCCTCGTTGAAACCAGCTATGTGAAAGTCCTGCACCATATGGTAAAGA
TCAGTGGAGGACCTCACATTTCCTACCCACCCCTGCATGAGTGGGTTTTGAGA
GAGGGGGAAGAGTGAGTCGCTTGCAAAGACAACGCCACATTTTGGGAACGCG
TCCGATCCATCCTGAAATCTGGGCTCAACTTTGCTTCGACCATACCTGGCCCC
GTAGGAGTGGCAGCGACAGGGATCAAAGGCATCATTGAAGCCATTGGTTCCT
TGTGGGTTTGATTCCACCCACAGAAGCGTTGACGACGCAAAACGTCCTTAAA
GCGTTGACGACGCAAAACGTCCCCAAGCTCGTAGCACCGACCCTATACCCAT
CTCTAGGGTCTTCAACCTCTTGGT
**MAGE-C2**
   Homo sapiens MAGE-C2 (MAGEC2) mRNA, complete cds
   GenBank: AF196482.1
**55-MAGEC2-270** 
GTAAACAACCAATAACATCAGGGTATCCAAAGCAGCACGCCGCCGAA
GAGCGGCCATGCCTCCCGTTCCAGGCGTTCCATTCCGCAACGTTGACAACGAC
TCCCCGACCTCAGTTGAGTTAGAAGACTGGGTAGATGCACAGCATCCCACAG
ATGAGGAAGAGGAGGAAGCCTCCTCCGCCTCTTCCACTTTGTACTTAGTATTT
TCCCCCTCTTCTTTCTCCACATCCTCTTCTCTGATTCTTGGTGGTCCTGAGGAG
GAGGAGGTGCCCTCTGGTGTGATACCAAATCTTACCGAGAGCATTCCCAGTA
GTCCTCCACAGGGTCCTCCACAGGGTCCTTCCCAGAGTCCTCTGAGCTCCTGC
TGCTCCTCTTTTTCATGGAGCTCATTCAGTGAGGAGTCCAGCAGCCAGAAAGG
GGAGGATACAGGCACCTGTCAGGGCCTGCCAGACAGTGAGTCCTCTTTCACA
TATACACTAGATGAAAAGGTGGCCGAGTTAGTGGAGTTCCTGCTCCTCAAAT
ACGAAGCAGAGGAGCCTGTAACAGAGGCAGAGATGCTGATGATTGTCATCAA
GTACAAAGATTACTTTCCTGTGATACTCAAGAGAGCCCGTGAGTTCATGGAGC
TTCTTTTTGGCCTTGCCCTGATAGAAGTGGGCCCTGACCACTTCTGTGTGTTTG
 
CAAACACAGTAGGCCTCACCGATGAGGGTAGTGATGATGAGGGCATGCCCGA
GAACAGCCTCCTGATTATTATTCTGAGTGTGATCTTCATAAAGGGCAACTGTG
CCTCTGAGGAGGTCATCTGGGAAGTGCTGAATGCAGTAGGGGTATATGCTGG
GAGGGAGCACTTCGTCTATGGGGAGCCTAGGGAGCTCCTCACTAAAGTTTGG
GTGCAGGGACATTACCTGGAGTATCGGGAGGTGCCCCACAGTTCTCCTCCATA
TTATGAATTCCTGTGGGGTCCAAGAGCCCATTCAGAAAGCATCAAGAAGAAA
GTACTAGAGTTTTTAGCCAAGCTGAACAACACTGTTCCTAGTTCCTTTCCATC
CTGGTACAAGGATGCTTTGAAAGATGTGGAAGAGAGAGTCCAGGCCACAATT
GATACCGCAGATGATGCCACTGTCATGGCCAGTGAAAGCCTCAGTGTCATGT
CCAGCAACGTCTCCTTTTCTGAGTGAGTCGCTTGCAAAGACAACGCCACATTT
TGGGAACGCGTCCGATCCATCCTGAAATCTGGGCTCAACTTTGCTTCGACCAT
ACCTGGCCCCGTAGGAGTGGCAGCGACAGGGATCAAAGGCATCATTGAAGCC
ATTGGTTCCTTGTGGGTTTGATTCCACCCACAGAAGCGTTGACGACGCAAAAC
GTCCTTAAAGCGTTGACGACGCAAAACGTCCCCAAGCTCGTAGCACCGACCC
TATACCCATCTCTAGGGTCTTCAACCTCTTGGT
Fusion construct **55-MAGEC2-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
 
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
 
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGCCTCCCGTTCCAGGCGTTCCATTCCGCAACGTTGAC
AACGACTCCCCGACCTCAGTTGAGTTAGAAGACTGGGTAGATGCACAGCATC
CCACAGATGAGGAAGAGGAGGAAGCCTCCTCCGCCTCTTCCACTTTGTACTTA
GTATTTTCCCCCTCTTCTTTCTCCACATCCTCTTCTCTGATTCTTGGTGGTCCTG
AGGAGGAGGAGGTGCCCTCTGGTGTGATACCAAATCTTACCGAGAGCATTCC
CAGTAGTCCTCCACAGGGTCCTCCACAGGGTCCTTCCCAGAGTCCTCTGAGCT
 
CCTGCTGCTCCTCTTTTTCATGGAGCTCATTCAGTGAGGAGTCCAGCAGCCAG
AAAGGGGAGGATACAGGCACCTGTCAGGGCCTGCCAGACAGTGAGTCCTCTT
TCACATATACACTAGATGAAAAGGTGGCCGAGTTAGTGGAGTTCCTGCTCCTC
AAATACGAAGCAGAGGAGCCTGTAACAGAGGCAGAGATGCTGATGATTGTCA
TCAAGTACAAAGATTACTTTCCTGTGATACTCAAGAGAGCCCGTGAGTTCATG
GAGCTTCTTTTTGGCCTTGCCCTGATAGAAGTGGGCCCTGACCACTTCTGTGT
GTTTGCAAACACAGTAGGCCTCACCGATGAGGGTAGTGATGATGAGGGCATG
CCCGAGAACAGCCTCCTGATTATTATTCTGAGTGTGATCTTCATAAAGGGCAA
CTGTGCCTCTGAGGAGGTCATCTGGGAAGTGCTGAATGCAGTAGGGGTATAT
GCTGGGAGGGAGCACTTCGTCTATGGGGAGCCTAGGGAGCTCCTCACTAAAG
TTTGGGTGCAGGGACATTACCTGGAGTATCGGGAGGTGCCCCACAGTTCTCCT
CCATATTATGAATTCCTGTGGGGTCCAAGAGCCCATTCAGAAAGCATCAAGA
AGAAAGTACTAGAGTTTTTAGCCAAGCTGAACAACACTGTTCCTAGTTCCTTT
CCATCCTGGTACAAGGATGCTTTGAAAGATGTGGAAGAGAGAGTCCAGGCCA
CAATTGATACCGCAGATGATGCCACTGTCATGGCCAGTGAAAGCCTCAGTGT
CATGTCCAGCAACGTCTCCTTTTCTGAGTGAGTCGCTTGCAAAGACAACGCCA
CATTTTGGGAACGCGTCCGATCCATCCTGAAATCTGGGCTCAACTTTGCTTCG
ACCATACCTGGCCCCGTAGGAGTGGCAGCGACAGGGATCAAAGGCATCATTG
AAGCCATTGGTTCCTTGTGGGTTTGATTCCACCCACAGAAGCGTTGACGACGC
AAAACGTCCTTAAAGCGTTGACGACGCAAAACGTCCCCAAGCTCGTAGCACC
GACCCTATACCCATCTCTAGGGTCTTCAACCTCTTGGT
**Melanocyte protein PMEL or Melanocytes lineage-specific antigen GP100**
   Human Pmel 17 mRNA, complete cds
   GenBank: M77348.1
**55-PMEL-270** 
GTAAACAACCAATAACATCAGGGTATCCAAAGCAGCACGCCGCCGAA
GAGCGGCCATGGATCTGGTGCTAAAAAGATGCCTTCTTCATTTGGCTGTGATA
GGTGCTTTGCTGGCTGTGGGGGCTACAAAAGTACCCAGAAACCAGGACTGGC
 
TTGGTGTCTCAAGGCAACTCAGAACCAAAGCCTGGAACAGGCAGCTGTATCC
AGAGTGGACAGAAGCCCAGAGACTTGACTGCTGGAGAGGTGGTCAAGTGTCC
CTCAAGGTCAGTAATGATGGGCCTACACTGATTGGTGCAAATGCCTCCTTCTC
TATTGCCTTGAACTTCCCTGGAAGCCAAAAGGTATTGCCAGATGGGCAGGTTA
TCTGGGTCAACAATACCATCATCAATGGGAGCCAGGTGTGGGGAGGACAGCC
AGTGTATCCCCAGGAAACTGACGATGCCTGCATCTTCCCTGATGGTGGACCTT
GCCCATCTGGCTCTTGGTCTCAGAAGAGAAGCTTTGTTTATGTCTGGAAGACC
TGGGGCCAATACTGGCAAGTTCTAGGGGGCCCAGTGTCTGGGCTGAGCATTG
GGACAGGCAGGGCAATGCTGGGCACACACACCATGGAAGTGACTGTCTACCA
TCGCCGGGGATCCCGGAGCTATGTGCCTCTTGCTCATTCCAGCTCAGCCTTCA
CCATTACTGACCAGGTGCCTTTCTCCGTGAGCGTGTCCCAGTTGCGGGCCTTG
GATGGAGGGAACAAGCACTTCCTGAGAAATCAGCCTCTGACCTTTGCCCTCC
AGCTCCATGACCCTAGTGGCTATCTGGCTGAAGCTGACCTCTCCTACACCTGG
GACTTTGGAGACAGTAGTGGAACCCTGATCTCTCGGGCACCTGTGGTCACTCA
TACTTACCTGGAGCCTGGCCCAGTCACTGCCCAGGTGGTCCTGCAGGCTGCCA
TTCCTCTCACCTCCTGTGGCTCCTCCCCAGTTCCAGGCACCACAGATGGGCAC
AGGCCAACTGCAGAGGCCCCTAACACCACAGCTGGCCAAGTGCCTACTACAG
AAGTTGTGGGTACTACACCTGGTCAGGCGCCAACTGCAGAGCCCTCTGGAAC
CACATCTGTGCAGGTGCCAACCACTGAAGTCATAAGCACTGCACCTGTGCAG
ATGCCAACTGCAGAGAGCACAGGTATGACACCTGAGAAGGTGCCAGTTTCAG
AGGTCATGGGTACCACACTGGCAGAGATGTCAACTCCAGAGGCTACAGGTAT
GACACCTGCAGAGGTATCAATTGTGGTGCTTTCTGGAACCACAGCTGCACAG
GTAACAACTACAGAGTGGGTGGAGACCACAGCTAGAGAGCTACCTATCCCTG
AGCCTGAAGGTCCAGATGCCAGCTCAATCATGTCTACGGAAAGTATTACAGG
TTCCCTGGGCCCCCTGCTGGATGGTACAGCCACCTTAAGGCTGGTGAAGAGA
CAAGTCCCCCTGGATTGTGTTCTGTATCGATATGGTTCCTTTTCCGTCACCCTG
GACATTGTCCAGGGTATTGAAAGTGCCGAGATCCTGCAGGCTGTGCCGTCCG
GTGAGGGGGATGCATTTGAGCTGACTGTGTCCTGCCAAGGCGGGCTGCCCAA
 
GGAAGCCTGCATGGAGATCTCATCGCCAGGGTGCCAGCCCCCTGCCCAGCGG
CTGTGCCAGCCTGTGCTACCCAGCCAGCCTGCCAGCTGGTTCTGCACCAGAT
ACTGAAGGGTGGCTCGGGGACATACTGCCTCAATGTGTCTCTGGCTGATACCA
ACAGCCTGGCAGTGGTCAGCACCCAGCTTATCATGCCTGTGCCTGGGATTCTT
CTCACAGGTCAAGAAGCAGGCCTTGGGCAGGTTCGGCTGATCGTGGGCATCT
TGCTGGTGTTGATGGCTGTGGTCCTTGCATCTCTGATATATAGGCGCAGACTT
ATGAAGCAAGACTTCTCCGTACCCCAGTTGCCACATAGCAGCAGTCACTGGCT
GCGTCTACCCCGCATCTTCTGCTCTTGTCCCATTGGTGAGAATAGCCCCCTCCT
CAGTGGGCAGCAGGTCTGAGTCGCTTGCAAAGACAACGCCACATTTGGGAA
CGCGTCCGATCCATCCTGAAATCTGGGCTCAACTTTGCTTCGACCATACCTGG
CCCCGTAGGAGTGGCAGCGACAGGGATCAAAGGCATCATTGAAGCCATTGGT
TCCTTGTGGGTTTGATTCCACCCACAGAAGCGTTGACGACGCAAAACGTCCTT
AAAGCGTTGACGACGCAAAACGTCCCCAAGCTCGTAGCACCGACCCTATACC
CATCTCTAGGGTCTTCAACCTCTTGGT
Fusion construct **55-PMEL-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
 
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
 
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGGATCTGGTGCTAAAAAGATGCCTTCTTCATTTGGCT
GTGATAGGTGCTTTGCTGGCTGTGGGGGCTACAAAAGTACCCAGAAACCAGG
ACTGGCTTGGTGTCTCAAGGCAACTCAGAACCAAAGCCTGGAACAGGCAGCT
GTATCCAGAGTGGACAGAAGCCCAGAGACTTGACTGCTGGAGAGGTGGTCAA
GTGTCCCTCAAGGTCAGTAATGATGGGCCTACACTGATTGGTGCAAATGCCTC
CTTCTCTATTGCCTTGAACTTCCCTGGAAGCCAAAAGGTATTGCCAGATGGGC
AGGTTATCTGGGTCAACAATACCATCATCAATGGGAGCCAGGTGTGGGGAGG
 
ACAGCCAGTGTATCCCCAGGAAACTGACGATGCCTGCATCTTCCCTGATGGTG
GACCTTGCCCATCTGGCTCTTGGTCTCAGAAGAGAAGCTTTGTTTATGTCTGG
AAGACCTGGGGCCAATACTGGCAAGTTCTAGGGGGCCCAGTGTCTGGGCTGA
GCATTGGGACAGGCAGGGCAATGCTGGGCACACACCATGGAAGTGACTGT
CTACCATCGCCGGGGATCCCGGAGCTATGTGCCTCTTGCTCATTCCAGCTCAG
CCTTCACCATTACTGACCAGGTGCCTTTCTCCGTGAGCGTGTCCCAGTTGCGG
GCCTTGGATGGAGGGAACAAGCACTTCCTGAGAAATCAGCCTCTGACCTTTG
CCCTCCAGCTCCATGACCCTAGTGGCTATCTGGCTGAAGCTGACCTCTCCTAC
ACCTGGGACTTTGGAGACAGTAGTGGAACCCTGATCTCTCGGGCACCTGTGGT
CACTCATACTTACCTGGAGCCTGGCCCAGTCACTGCCCAGGTGGTCCTGCAGG
CTGCCATTCCTCTCACCTCCTGTGGCTCCTCCCCAGTTCCAGGCACCACAGAT
GGGCACAGGCCAACTGCAGAGGCCCCTAACACCACAGCTGGCCAAGTGCCTA
CTACAGAAGTTGTGGGTACTACACCTGGTCAGGCGCCAACTGCAGAGCCCTC
TGGAACCACATCTGTGCAGGTGCCAACCACTGAAGTCATAAGCACTGCACCT
GTGCAGATGCCAACTGCAGAGAGCACAGGTATGACACCTGAGAAGGTGCCAG
TTTCAGAGGTCATGGGTACCACACTGGCAGAGATGTCAACTCCAGAGGCTAC
AGGTATGACACCTGCAGAGGTATCAATTGTGGTGCTTTCTGGAACCACAGCTG
CACAGGTAACAACTACAGAGTGGGTGGAGACCACAGCTAGAGAGCTACCTAT
CCCTGAGCCTGAAGGTCCAGATGCCAGCTCAATCATGTCTACGGAAAGTATT
ACAGGTTCCCTGGGCCCCCTGCTGGATGGTACAGCCACCTTAAGGCTGGTGA
AGAGACAAGTCCCCCTGGATTGTGTTCTGTATCGATATGGTTCCTTTTCCGTC
ACCCTGGACATTGTCCAGGGTATTGAAAGTGCCGAGATCCTGCAGGCTGTGC
CGTCCGGTGAGGGGGATGCATTTGAGCTGACTGTGTCCTGCCAAGGCGGGCT
GCCCAAGGAAGCCTGCATGGAGATCTCATCGCCAGGGTGCCAGCCCCCTGCC
CAGCGGCTGTGCCAGCCTGTGCTACCCAGCCCAGCCTGCCAGCTGGTTCTGCA
CCAGATACTGAAGGGTGGCTCGGGGACATACTGCCTCAATGTGTCTCTGGCTG
ATACCAACAGCCTGGCAGTGGTCAGCACCCAGCTTATCATGCCTGTGCCTGGG
ATTCTTCTCACAGGTCAAGAAGCAGGCCTTGGGCAGGTTCGGCTGATCGTGG
 
GCATCTTGCTGGTGTTGATGGCTGTGGTCCTTGCATCTCTGATATATAGGCGC
AGACTTATGAAGCAAGACTTCTCCGTACCCCAGTTGCCACATAGCAGCAGTC
ACTGGCTGCGTCTACCCCGCATCTTCTGCTCTTGTCCCATTGGTGAGAATAGC
CCCCTCCTCAGTGGGCAGCAGGTCTGAGTCGCTTGCAAAGACAACGCCACAT
TTTGGGAACGCGTCCGATCCATCCTGAAATCTGGGCTCAACTTTGCTTCGACC
ATACCTGGCCCCGTAGGAGTGGCAGCGACAGGGATCAAAGGCATCATTGAAG
CCATTGGTTCCTTGTGGGTTTGATTCCACCCACAGAAGCGTTGACGACGCAAA
ACGTCCTTAAAGCGTTGACGACGCAAAACGTCCCCAAGCTCGTAGCACCGAC
CCTATACCCATCTCTAGGGTCTTCAACCTCTTGGT
**Tyrosinase**
   Human tyrosinase (TYR) mRNA, complete cds
   GenBank: M27160.1
**55-Tyr-270** 
GTAAACAACCAATAACATCAGGGTATCCAAAGCAGCACGCCGCCGAA
GAGCGGCCATGCTCCTGGCTGTTTGTACTGCCTGCTGTGGAGTTCCAGACC
TCCGCTGGCCATTTCCCTAGAGCCTGTGTCTCCTCTAAGAACCTGATGGAGAA
GGAATGCTGTCCACCGTGGAGCGGGGACAGGAGTCCCTGTGGCCAGCTTTCA
GGCAGAGGTTCCTGTCAGAATATCCTTCTGTCCAATGCACCACTTGGGCCTCA
ATTTCCCTTCACAGGGGTGGATGACCGGGAGTCGTGGCCTTCCGTCTTTTATA
ATAGGACCTGCCAGTGCTCTGGCAACTTCATGGGATTCAACTGTGGAAACTGC
AAGTTTGGCTTTTGGGGACCAAACTGCACAGAGAGACGACTCTTGGTGAGAA
GAAACATCTTCGATTTGAGTGCCCCAGAGAAGGACAAATTTTTTGCCTACCTC
ACTTTAGCAAAGCATACCATCAGCTCAGACTATGTCATCCCCATAGGGACCTA
TGGCCAAATGAAAAATGGATCAACACCCATGTTTAACGACATCAATATTTAT
GACCTCTTTGTCTGGATGCATTATTATGTGTCAATGGATGCACTGCTTGGGGG
ATCTGAAATCTGGAGAGACATTGATTTTGCCCATGAAGCACCAGCTTTTCTGC
CTTGGCATAGACTCTTCTTGTTGCGGTGGAACAAGAAATCCAGAAGCTGAC
AGGAGATGAAAACTTCACTATTCCATATTGGGACTGGCGGGATGCAGAAAAG
 
TGTGACATTTGCACAGATGAGTACATGGGAGGTCAGCACCCCACAAATCCTA
ACTTACTCAGCCCAGCATCATTCTTCTCCTCTTGGCAGATTGTCTGTAGCCGAT
TGGAGGAGTACAACAGCCATCAGTCTTTATGCAATGGAACGCCCGAGGGACC
TTTACGGCGTAATCCTGGAAACCATGACAAATCCAGAACCCCAAGGCTCCCC
TCTTCAGCTGATGTAGAATTTTGCCTGAGTTTGACCCAATATGAATCTGGTTC
CATGGATAAAGCTGCCAATTTCAGCTTTAGAAATACACTGGAAGGATTTGCTA
GTCCACTTACTGGGATAGCGGATGCCTCTCAAAGCAGCATGCACAATGCCTTG
CACATCTATATGAATGGAACAATGTCCCAGGTACAGGGATCTGCCAACGATC
CTATCTTCCTTCTTCACCATGCATTTGTTGACAGTATTTTTGAGCAGTGGCTCC
GAAGGCACCGTCCTCTTCAAGAAGTTTATCCAGAAGCCAATGCACCCATTGG
ACATAACCGGGAATCCTACATGGTTCCTTTTATACCACTGTACAGAAATGGTG
ATTTCTTTATTTCATCCAAAGATCTGGGCTATGACTATAGCTATCTACAAGATT
CAGACCCAGACTCTTTTCAAGACTACATTAAGTCCTATTTGGAACAAGCGAGT
CGGATCTGGTCATGGCTCCTTGGGGCGGCGATGGTAGGGGCCGTCCTCACTGC
CCTGCTGGCAGGGCTTGTGAGCTTGCTGTGTCGTCACAAGAGAAAGCAGCTTC
CTGAAGAAAAGCAGCCACTCCTCATGGAGAAAGAGGATTACCACAGCTTGTA
TCAGAGCCATTTATAAGTCGCTTGCAAAGACAACGCCACATTTTGGGAACGC
GTCCGATCCATCCTGAAATCTGGGCTCAACTTTGCTTCGACCATACCTGGCCC
CGTAGGAGTGGCAGCGACAGGGATCAAAGGCATCATTGAAGCCATTGGTTCC
TTGTGGGTTTGATTCCACCCACAGAAGCGTTGACGACGCAAAACGTCCTTAAA
GCGTTGACGACGCAAAACGTCCCCAAGCTCGTAGCACCGACCCTATACCCAT
CTCTAGGGTCTTCAACCTCTTGGT
Fusion construct **55-Tyr-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
 
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
 
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
 
ATGCCAGTGGTGTAAACAACCAATAACATCAGGGTATCCAAAGCAGCACGCC
GCCGAAGAGCGGCCATGCTCCTGGCTGTTTTGTACTGCCTGCTGTGGAGTTTC
CAGACCTCCGCTGGCCATTTCCCTAGAGCCTGTGTCTCCTCTAAGAACCTGAT
GGAGAAGGAATGCTGTCCACCGTGGAGCGGGGACAGGAGTCCCTGTGGCCAG
CTTTCAGGCAGAGGTTCCTGTCAGAATATCCTTCTGTCCAATGCACCACTTGG
GCCTCAATTTCCCTTCACAGGGGTGGATGACCGGGAGTCGTGGCCTTCCGTCT
TTTATAATAGGACCTGCCAGTGCTCTGGCAACTTCATGGGATTCAACTGTGGA
AACTGCAAGTTTGGCTTTTGGGGACCAAACTGCACAGAGAGACGACTCTTGG
TGAGAAGAAACATCTTCGATTTGAGTGCCCCAGAGAAGGACAAATTTTTTGC
CTACCTCACTTTAGCAAAGCATACCATCAGCTCAGACTATGTCATCCCCATAG
GGACCTATGGCCAAATGAAAAATGGATCAACACCCATGTTTAACGACATCAA
TATTTATGACCTCTTTGTCTGGATGCATTATTATGTGTCAATGGATGCACTGCT
TGGGGGATCTGAAATCTGGAGAGACATTGATTTTGCCCATGAAGCACCAGCT
TTTCTGCCTTGGCATAGACTCTTCTTGTTGCGGTGGAACAAGAAATCCAGAA
GCTGACAGGAGATGAAAACTTCACTATTCCATATTGGGACTGGCGGGATGCA
GAAAAGTGTGACATTTGCACAGATGAGTACATGGGAGGTCAGCACCCCACAA
ATCCTAACTTACTCAGCCCAGCATCATTCTTCTCCTCTTGGCAGATTGTCTGTA
GCCGATTGGAGGAGTACAACAGCCATCAGTCTTTATGCAATGGAACGCCCGA
GGGACCTTTACGGCGTAATCCTGGAAACCATGACAAATCCAGAACCCCAAGG
CTCCCCTCTTCAGCTGATGTAGAATTTTGCCTGAGTTTGACCCAATATGAATCT
GGTTCCATGGATAAAGCTGCCAATTTCAGCTTTAGAAATACACTGGAAGGATT
TGCTAGTCCACTTACTGGGATAGCGGATGCCTCTCAAAGCAGCATGCACAAT
GCCTTGCACATCTATATGAATGGAACAATGTCCCAGGTACAGGGATCTGCCA
ACGATCCTATCTTCCTTCTTCACCATGCATTTGTTGACAGTATTTTTGAGCAGT
GGCTCCGAAGGCACCGTCCTCTTCAAGAAGTTTATCCAGAAGCCAATGCACC
CATTGGACATAACCGGGAATCCTACATGGTTCCTTTTATACCACTGTACAGAA
ATGGTGATTTCTTTATTTCATCCAAAGATCTGGGCTATGACTATAGCTATCTAC
AAGATTCAGACCCAGACTCTTTTCAAGACTACATTAAGTCCTATTTGGAACAA
 
GCGAGTCGGATCTGGTCATGGCTCCTTGGGGCGGCGATGGTAGGGGCCGTCC
TCACTGCCCTGCTGGCAGGGCTTGTGAGCTTGCTGTGTCGTCACAAGAGAAAG
CAGCTTCCTGAAGAAAAGCAGCCACTCCTCATGGAGAAAGAGGATTACCACA
GCTTGTATCAGAGCCATTTATAAGTCGCTTGCAAAGACAACGCCACATTTTGG
GAACGCGTCCGATCCATCCTGAAATCTGGGCTCAACTTTGCTTCGACCATACC
TGGCCCCGTAGGAGTGGCAGCGACAGGGATCAAAGGCATCATTGAAGCCATT
GGTTCCTTGTGGGTTTGATTCCACCCACAGAAGCGTTGACGACGCAAAACGTC
CTTAAAGCGTTGACGACGCAAAACGTCCCCAAGCTCGTAGCACCGACCCTAT
ACCCATCTCTAGGGTCTTCAACCTCTTGGT
**E7**
   Human papillomavirus type 16 isolate b00441 E6 (E6) and E7 (E7) genes, complete cds
   GenBank: EU869318.1
**55-E7-270** 
GTAAACAACCAATAACATCAGGGTATCCAAAGCAGCACGCCGCCGAA
GAGCGGCCATGCATGGAGATACACCTACATTGCATGAATATATGTTAGATTTG
CAACCAGAGACAACTGATCTCTACTGTTATGAGCAATTAAATGACAGCTCAG
AGGAGGAGGATGAAATAGATGGTCCAGCTGGACAAGCAGAACCGGACAGAG
CCCATTACAATATTGTAACCTTTTGTTGCAAGTGTGACTCTACGCTTCGGTTGT
GCGTACAAAGCACACACGTAGACATTCGTACTTTGGAAGACCTGTTAATGGG
CACACTAGGAATTGTGTGCCCCATCTGTTCTCAGAAACCATAAGTCGCTTGCA
AAGACAACGCCACATTTTGGGAACGCGTCCGATCCATCCTGAAATCTGGGCT
CAACTTTGCTTCGACCATACCTGGCCCCGTAGGAGTGGCAGCGACAGGGATC
AAAGGCATCATTGAAGCCATTGGTTCCTTGTGGGTTTGATTCCACCCACAGAA
GCGTTGACGACGCAAAACGTCCTTAAAGCGTTGACGACGCAAAACGTCCCCA
AGCTCGTAGCACCGACCCTATACCCATCTCTAGGGTCTTCAACCTCTTGGT
**Fusion construct 55-E7-270** GTATTGAATCCAAAACTCAAAATGCTGAACTACGAGACAATCATCAAC 
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
 
ACCCTCGCCATCCAACAGGTGTGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTCCAGCTATACGCTGG
CATTCCGTGATCAGGTGCTGCATGCAGAGCACAACCGGCATTGGTTTGCCCG
GGTTTGACCCCCGAGCAGATCGCCACAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
 
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGCATGGAGATACACCTACATTGCATGAATATATGTT
AGATTTGCAACCAGAGACAACTGATCTCTACTGTTATGAGCAATTAAATGAC
AGCTCAGAGGAGGAGGATGAAATAGATGGTCCAGCTGGACAAGCAGAACCG
GACAGAGCCCATTACAATATTGTAACCTTTTGTTGCAAGTGTGACTCTACGCT
TCGGTTGTGCGTACAAAGCACACACGTAGACATTCGTACTTTGGAAGACCTGT
TAATGGGCACACTAGGAATTGTGTGCCCCATCTGTTCTCAGAAACCATAAGTC
GCTTGCAAAGACAACGCCACATTTTGGGAACGCGTCCGATCCATCCTGAAAT
CTGGGCTCAACTTTGCTTCGACCATACCTGGCCCCGTAGGAGTGGCAGCGACA
GGGATCAAAGGCATCATTGAAGCCATTGGTTCCTTGTGGGTTTGATTCCACCC
ACAGAAGCGTTGACGACGCAAAACGTCCTTAAAGCGTTGACGACGCAAAACG
TCCCCAAGCTCGTAGCACCGACCCTATACCCATCTCTAGGGTCTTCAACCTCT
TGGT
**E6**
   Human papillomavirus type 16 isolate NEA-074 E6 (E6)
**55-E6-270** 
GTAAACAACCAATAACATCAGGGTATCCAAAGCAGCACGCCGCCGAA
GAGCGGCCATGTTTCAGGACCCACAGGAGCGACCCAGAAAGTTACCACAGTT
ATGCACAGAGCTGCAAACAACTATACATGATATAATATTAGAATGTGTGTAC
TGCAAGCAACAGTTACTGCGACGTGAGGTATATGACTTTGCTTTTCGGGATTT
ATGCATAGTATATAGAGATGGGAATCCATATGCTGTATGTGATAAATGTTTAA
AGTTTTATTCTAAAATTAGTGAGTATAGACATTATTGTTATAGTTTGTATGGA
ACAACATTAGAACAGCAATACAACAAACCGTTGTGTGATTTGTTAATTAGGT
GTATTAACTGTCAAAAGCCACTGTGTCCTGAAGAAAAGCAAAGACATCTGGA
 
CAAAAAGCAAAGATTCCATAATATAAGGGGTCGGTGGACCGGTCGATGTATG
TCTTGTTGCAGATCATCAAGAACACGTAGAGAAACCCAGCTGTAAGTCGCTT
GCAAAGACAACGCCACATTTTGGGAACGCGTCCGATCCATCCTGAAATCTGG
GCTCAACTTTGCTTCGACCATACCTGGCCCCGTAGGAGTGGCAGCGACAGGG
ATCAAAGGCATCATTGAAGCCATTGGTTCCTTGTGGGTTTGATTCCACCCACA
GAAGCGTTGACGACGCAAAACGTCCTTAAAGCGTTGACGACGCAAAACGTCC
CCAAGCTCGTAGCACCGACCCTATACCCATCTCTAGGGTCTTCAACCTCTTGG
T
Fusion construct **55-E6-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
 
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
 
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGTTTCAGGACCCACAGGAGCGACCCAGAAAGTTACC
ACAGTTATGCACAGAGCTGCAAACAACTATACATGATATAATATTAGAATGT
GTGTACTGCAAGCAACAGTTACTGCGACGTGAGGTATATGACTTTGCTTTTCG
GGATTTATGCATAGTATATAGAGATGGGAATCCATATGCTGTATGTGATAAAT
GTTTAAAGTTTTATTCTAAAATTAGTGAGTATAGACATTATTGTTATAGTTTGT
ATGGAACAACATTAGAACAGCAATACAACAAACCGTTGTGTGATTTGTTAAT
TAGGTGTATTAACTGTCAAAAGCCACTGTGTCCTGAAGAAAAGCAAAGACAT
CTGGACAAAAAGCAAAGATTCCATAATATAAGGGGTCGGTGGACCGGTCGAT
GTATGTCTTGTTGCAGATCATCAAGAACACGTAGAGAAACCCAGCTGTAAGT
CGCTTGCAAAGACAACGCCACATTTGGGAACGCGTCCGATCCATCCTGAAA
TCTGGGCTCAACTTTGCTTCGACCATACCTGGCCCCGTAGGAGTGGCAGCGAC
AGGGATCAAAGGCATCATTGAAGCCATTGGTTCCTTGTGGGTTTGATTCCACC
CACAGAAGCGTTGACGACGCAAAACGTCCTTAAAGCGTTGACGACGCAAAAC
 
GTCCCCAAGCTCGTAGCACCGACCCTATACCCATCTCTAGGGTCTTCAACCTC
TTGGT
**GM-CSF**
   Human granulocyte-macrophage colony stimulating factor (GM-CSF) mRNA
   GenBank: M11220.1
**55-GMCSF-270** 
GTAAACAACCAATAACATCAGGGTATCCAAAGCAGCACGCCGCCGAA
GAGCGGCCATGTGGCTGCAGAGCCTGCTGCTCTTGGGCACTGTGGCCTGCAG
CATCTCTGCACCCGCCCGCTCGCCCAGCCCCAGCACGCAGCCCTGGGAGCAT
GTGAATGCCATCCAGGAGGCCCGGCGTCTCCTGAACCTGAGTAGAGACACTG
CTGCTGAGATGAATGAAACAGTAGAAGTCATCTCAGAAATGTTTGACCTCCA
GGAGCCGACCTGCCTACAGACCCGCCTGGAGCTGTACAAGCAGGGCCTGCGG
GGCAGCCTCACCAAGCTCAAGGGCCCCTTGACCATGATGGCCAGCCACTACA
AGCAGCACTGCCCTCCAACCCCGGAAACTTCCTGTGCAACCCAGATTATCACC
TTTGAAAGTTTCAAAGAGAACCTGAAGGACTTTCTGCTTGTCATCCCCTTTGA
CTGCTGGGAGCCAGTCCAGGAGTGAGTCGCTTGCAAAGACAACGCCACATTT
TGGGAACGCGTCCGATCCATCCTGAAATCTGGGCTCAACTTTGCTTCGACCAT
ACCTGGCCCCGTAGGAGTGGCAGCGACAGGGATCAAAGGCATCATTGAAGCC
ATTGGTTCCTTGTGGGTTTGATTCCACCCACAGAAGCGTTGACGACGCAAAAC
GTCCTTAAAGCGTTGACGACGCAAAACGTCCCCAAGCTCGTAGCACCGACCC
TATACCCATCTCTAGGGTCTTCAACCTCTTGGT
**Fusion construct 55-GMCSF-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
 
AGAACACGCGCGGAAAGCGGATAATGGGCATCCGATCTCCGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
 
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
 
GCCGAAGAGCGGCCATGTGGCTGCAGAGCCTGCTGCTCTTGGGCACTGTGGC
CTGCAGCATCTCTGCACCCGCCCGCTCGCCCAGCCCCAGCACGCAGCCCTGGG
AGCATGTGAATGCCATCCAGGAGGCCCGGCGTCTCCTGAACCTGAGTAGAGA
CACTGCTGCTGAGATGAATGAAACAGTAGAAGTCATCTCAGAAATGTTTGAC
CTCCAGGAGCCGACCTGCCTACAGACCCGCCTGGAGCTGTACAAGCAGGGCC
TGCGGGGCAGCCTCACCAAGCTCAAGGGCCCCTTGACCATGATGGCCAGCCA
CTACAAGCAGCACTGCCCTCCAACCCCGGAAACTTCCTGTGCAACCCAGATTA
TCACCTTTGAAAGTTTCAAAGAGAACCTGAAGGACTTTCTGCTTGTCATCCCC
TTTGACTGCTGGGAGCCAGTCCAGGAGTGAGTCGCTTGCAAAGACAACGCCA
CATTTTGGGAACGCGTCCGATCCATCCTGAAATCTGGGCTCAACTTTGCTTCG
ACCATACCTGGCCCCGTAGGAGTGGCAGCGACAGGGATCAAAGGCATCATTG
AAGCCATTGGTTCCTTGTGGGTTTGATTCCACCCACAGAAGCGTTGACGACGC
AAAACGTCCTTAAAGCGTTGACGACGCAAAACGTCCCCAAGCTCGTAGCACC
GACCCTATACCCATCTCTAGGGTCTTCAACCTCTTGGT
**CD40 ligand**
   Homo sapiens CD40 ligand (CD40LG), mRNA
   NCBI Reference Sequence: NM_000074.3
**55-CD40lig-270** 
GTAAACAACCAATAACATCAGGGTATCCAAAGCAGCACGCCGCCGAA
GAGCGGCCATGATCGAAACATACAACCAAACTTCTCCCCGATCTGCGGCCAC
TGGACTGCCCATCAGCATGAAAATTTTATGTATTTACTTACTGTTTTTCTTAT
CACCCAGATGATTGGGTCAGCACTTTTTGCTGTGTATCTTCATAGAAGGTTGG
ACAAGATAGAAGATGAAAGGAATCTTCATGAAGATTTTGTATTCATGAAAAC
GATACAGAGATGCAACACAGGAGAAAGATCCTTATCCTTACTGAACTGTGAG
GAGATTAAAAGCCAGTTTGAAGGCTTTGTGAAGGATATAATGTTAAACAAAG
AGGAGACGAAGAAAGAAAACAGCTTTGAAATGCAAAAAGGTGATCAGAATC
CTCAAATTGCGGCACATGTCATAAGTGAGGCCAGCAGTAAAACAACATCTGT
GTTACAGTGGGCTGAAAAAGGATACTACACCATGAGCAACAACTTGGTAACC
 
CTGGAAAATGGGAAACAGCTGACCGTTAAAAGACAAGGACTCTATTATATCT
ATGCCCAAGTCACCTTCTGTTCCAATCGGGAAGCTTCGAGTCAAGCTCCATTT
ATAGCCAGCCTCTGCCTAAAGTCCCCGGTAGATTCGAGAGAATCTTACTCAG
AGCTGCAAATACCCACAGTTCCGCCAAACCTTGCGGGCAACAATCCATTCACT
TGGGAGGAGTATTTGAATTGCAACCAGGTGCTTCGGTGTTTGTCAATGTGACT
GATCCAAGCCAAGTGAGCCATGGCACTGGCTTCACGTCCTTTGGCTTACTCAA
ACTCTGAGTCGCTTGCAAAGACAACGCCACATTTTGGAACGCGTCCGATCC
ATCCTGAAATCTGGGCTCAACTTTGCTTCGACCATACCTGGCCCCGTAGGAGT
GGCAGCGACAGGGATCAAAGGCATCATTGAAGCCATTGGTTCCTTGTGGGTT
TGATTCCACCCACAGAAGCGTTGACGACGCAAAACGTCCTTAAAGCGTTGAC
GACGCAAAACGTCCCCAAGCTCGTAGCACCGACCCTATACCCATCTCTAGGG
TCTTCAACCTCTTGGT
Fusion construct **55-CD401ig-270** 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
 
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
 
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGTGTAAACAACCAATAACATCAGGGTATCCAAAGCAGCACGCC
GCCGAAGAGCGGCCATGATCGAAACATACAACCAAACTTCTCCCCGATCTGC
GGCCACTGGACTGCCCATCAGCATGAAAATTTTATGTATTTACTTACTGTTTT
TCTTATCACCCAGATGATTGGGTCAGCACTTTTGCTGTGTATCTTCATAGAA
GGTTGGACAAGATAGAAGATGAAAGGAATCTTCATGAAGATTTGTATTCAT
GAAAACGATACAGAGATGCAACACAGGAGAAAGATCCTTATCCTTACTGAAC
TGTGAGGAGATTAAAAGCCAGTTTGAAGGCTTTGTGAAGGATATAATGTTAA
ACAAAGAGGAGACGAAGAAAGAAAACAGCTTTGAATGCAAAAAGGTGATC
AGAATCCTCAAATTGCGGCACATGTCATAAGTGAGGCCAGCAGTAAAACAAC
ATCTGTGTTACAGTGGGCTGAAAAAGGATACTACACCATGAGCAACAACTTG
 
GTAACCCTGGAAAATGGAAACAGCTGACCGTTAAAAGACAAGGACTCTATT
ATATCTATGCCCAAGTCACCTTCTGTTCCAATCGGAAGCTTCGAGTCAAGCT
CCATTTATAGCCAGCCTCTGCCTAAAGTCCCCCGGTAGATTCGAGAGAATCTT
ACTCAGAGCTGCAAATACCCACAGTTCCGCCAAACCTTGCGGGCAACAATCC
ATTCACTTGGGAGGAGTATTTGAATTGCAACCAGGTGCTTCGGTGTTTGTCAA
TGTGACTGATCCAAGCCAAGTGAGCCATGGCACTGGCTTCACGTCCTTTGGCT
TACTCAAACTCTGAGTCGCTTGCAAAGACAACGCCACATTTGGGAACGCGTC
CGATCCATCCTGAAATCTGGGCTCAACTTTGCTTCGACCATACCTGGCCCCGT
AGGAGTGGCAGCGACAGGGATCAAAGGCATCATTGAAGCCATTGGTTCCTTG
TGGGTTTGATTCCACCCACAGAAGCGTTGACGACGCAAAACGTCCTTAAAGC
GTTGACGACGCAAAACGTCCCCAAGCTCGTAGCACCGACCCTATACCCATCTC
TAGGGTCTTCAACCTCTTGGT
RNA1 and RNA2 sequences for transfection
**RNA1 (viral RNA construct)**

The RNA1 corresponds to the sequence published in Virology 305 (2), 436-451 (2003) by Johnson K.L. et al. and comprises a capO structure at its 5' end, followed by a 5' UTR, the region encoding the RNA-depending RNA-polymerase (RdRp) as well as protein B1 and protein B2. A 3'UTR is located at its 3'end. Notably, the molecule does not contain a poly A tail. 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
 
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTCCGGATCACGAACAATCAAGTC
ACGTTTGATGTTAGTGGCGGTGGATCTTGGTCCCATGAAGTTTGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCCGCG
 
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGT
 **RNA2 (viral RNA construct)**

GTAAACAACCAATAACATCATGGTATCCAAAGCAGCACGCCGCCGAA
GAGCGGCCCCTCGACAACAACAACGACAACAGTCGAACCGTGCATCAAACCA

GCCTCGTCGGAGGAGAGCTCGCCGTACTCGAAGACAGCAGAGAATGGCAGCA
ACAAACAACATGCTGAAAATGAGCGCTCCCGGACTCGATTTCTGAAATGTG
CCTTTGCTTCGCCGGATTTTCCACCGATCCCGGCAAAGGTATTCCAGACAAA
TTTCAGGGTCTCGTTTTACCGAAGAAACATTGTCTGACCCAGTCGATAACGTT
TACTCCGGGGAAACAGACTATGCTGTTGGTTGCACCTATTCCTGGAATTGCTT
GCCTGAAGGCAGAAGCGAATGTTGGCGCATCCTTTTCAGGTGTTCCTCTTGCC
TCAGTTGAATTTCCAGGGTTCGACCAGCTGTTTGGCACGTCAGCAACTGACAC
CGCAGCTAATGTCACTGCTTTCCGGTATGCGTCAATGGCAGCCGGTGTTTACC
CCACGAGCAATCTAATGCAATTTGCTGGGTCAATACAGGTGTACAAGATACC
TCTAAAACAGGTTCTTAACTCCTACTCTCAGACCGTAGCGACCGTACCACCTA
CCAACTTGGCTCAGAACACAATTGCAATAGATGGACTAGAAGCGTTAGATGC
ACTACCAAATAACAACTACTCCGGTTCTTTATCGAGGGTTGTTATTCACAAT
CGGTGTGCAATGAACCTGAGTTTGAGTTCCATCCCATCATGGAGGGTTACGCG
TCCGTTCCACCCGCGAACGTAACCAATGCTCAAGCTAGCATGTTCACCAATCT
AACCTTCTCAGGCGCAGCATATACCGGTCTTGGCGACATGGACGCAATTGCG
ATCCTTGTGACTACGCCCACCGGTGCCGTCAACACGGCCGTGCTGAAGGTCTG
GGCCTGCGTAGAGTATCGTCCGAATCCAAATTCCACCCTCTATGAGTTCGCTC
GTGAGTCACCAGCAAACGACGAATACGCGCTCGCCGCCTATAGGAAAATTGC
CAGAGATATTCCGATTGCCGTCGCTTGCAAAGACAACGCCACATTTTGGGAA
CGCGTCCGATCCATCCTGAAATCTGGGCTCAACTTTGCTTCGACCATACCTGG
CCCCGTAGGAGTGGCAGCGACAGGGATCAAAGGCATCATTGAAGCCATTGGT
TCCTTGTGGGTTTGATTCCACCCACAGAAGCGTTGACGACGCAAAACGTCCTT
AAAGCGTTGACGACGCAAAACGTCCCCAAGCTCGTAGCACCGACCCTATACC
CATCTCTAGGGTCTTCAACCTCTTGGT

The table describes the length of the deleted sequence fragments from RNA2 for the generation of different constructs with various 5' and 3' end; the gene of interest is cloned instead of the deleted fragment in the RNA2 vector and RNA is generated by IVT thereof. The full-length RNA is 1336 nucleotides in length. For the 5' 52CC construct 2 Cs were introduced in the sequence to generate a Kozak sequence; in consequence, the length of the RNA2 deleted fragment is the same as for the 52nt vector.

| Delta RNA2 | 5' 3nt | 5' 19nt | 5' 44nt | 5' 52nt | 5' 52CCnt | 5' 55 nt |
|---|---|---|---|---|---|---|
| 3' 270nt | 1063 | 1047 | 1022 | 1014 | 1014 | 1011 |
| 3' 236nt | 1097 | 1082 | 1056 | 1048 | 1048 | 1045 |
| 3' 100nt | 1233 | 1217 | 1192 | 1184 | 1184 | 1181 |
| 3' 50nt | 1283 | 1267 | 1242 | 1234 | 1234 | 1231 |
| 3' 25nt | 1308 | 1292 | 1267 | 1259 | 1259 | 1256 |

For the expression of a gene of interest using the NoV replicon system, the RNA2 published in Virology 305 (2), 436-451 (2003) by Johnson K.L. et al. was modified and is composed as follows; it comprises a capO or a cap1 structure at its 5' end. The natural 5' UTR comprises 19 nucleotides (nt) followed by a start codon of the NoV capsid gene. We generated and tested different 5' UTRs of different length: 3 nt, 19 nt, 44nt, and 52nt, whereby the constructs with 44 and 52 non translated nucleotides at their 5' end showed highest translation level of the gene of interest. For the generation of those constructs, the thymine (T) (uracil, U, respectively) in position 21 was mutated to a guanin (G) to destroy the ATG-startcodon. Inserting the start codon of the GOI directly after nucleotide 44, the translation efficacy benefits from a natural occurring Kozak sequence. In another construct 2 cytosins were added after nucleotide 52 for the generation of a Kozak sequence.
5'UTR 3 (RNA construct)
GTA
5'UTR 19 (RNA construct)
GTAAACAACCAATAACATC
5'UTR 44 (RNA construct)
GTAAACAACCAATAACATCAGGGTATCCAAAGCAGCACGCCGCC
5'UTR 52 (RNA construct) 
GTAAACAACCAATAACATCAGGGTATCCAAAGCAGCACGCCGCCGAA
GAGCG
5'UTR 52CC (RNA construct) 
GTAAACAACCAATAACATCAGGGTATCCAAAGCAGCACGCCGCCGAA
GAGCGCC
5'UTR 55 (RNA construct)

GTAAACAACCAATAACATCAGGGTATCCAAAGCAGCACGCCGCCGAA
GAGCGGCC

For the identification of optimal length of the 3' end derived from NoV RNA2 to get highest expression levels of the gene of interest, different deletion of the capsid gene were generated and replaced by a gene of interest; to measure the expression level, the firefly luciferase gene was used as gene of interest. In one construct, the start codon of the gene of interest was placed after nucleotide 52; the stop codon of the firefly luciferase gene was followed directly by the 3' terminal 1284 nucleotides from NoV RNA2; this construct is containing the entire sequence of NoV RNA2 without deletion.

### 3'UTR 1284 nt (RNA construct)

GCCCCTCGACAACAACAACGACAACAGTCGAACCGTGCATCAAACCA
GCCTCGTCGGAGGAGAGCTCGCCGTACTCGAAGACAGCAGAGAATGGCAGCA
ACAAACAACATGCTGAAAATGAGCGCTCCCGGACTCGATTTCTGAAATGTG
CCTTTGCTTCGCCGGATTTTTCCACCGATCCCGGCAAAGGTATTCCAGACAAA
TTTCAGGGTCTCGTTTTACCGAAGAAACATTGTCTGACCCAGTCGATAACGTT
TACTCCGGGGAAACAGACTATGCTGTTGGTTGCACCTATTCCTGGAATTGCTT
GCCTGAAGGCAGAAGCGAATGTTGGCGCATCCTTTTCAGGTGTTCCTCTTGCC
TCAGTTGAATTTCCAGGGTTCGACCAGCTGTTTGGCACGTCAGCAACTGACAC
CGCAGCTAATGTCACTGCTTTCCGGTATGCGTCAATGGCAGCCGGTGTTTACC
CCACGAGCAATCTAATGCAATTTGCTGGGTCAATACAGGTGTACAAGATACC
TCTAAAACAGGTTCTTAACTCCTACTCTCAGACCGTAGCGACCGTACCACCTA
CCAACTTGGCTCAGAACACAATTGCAATAGATGGACTAGAAGCGTTAGATGC
ACTACCAAATAACAACTACTCCGGTTCTTTTATCGAGGGTTGTTATTCACAAT
CGGTGTGCAATGAACCTGAGTTTGAGTTCCATCCCATCATGGAGGGTTACGCG
TCCGTTCCACCCGCGAACGTAACCAATGCTCAAGCTAGCATGTTCACCAATCT

AACCTTCTCAGGCGCAGCATATACCGGTCTTGGCGACATGGACGCAATTGCG
ATCCTTGTGACTACGCCCACCGGTGCCGTCAACACGGCCGTGCTGAAGGTCTG
GGCCTGCGTAGAGTATCGTCCGAATCCAAATTCCACCCTCTATGAGTTCGCTC
GTGAGTCACCAGCAAACGACGAATACGCGCTCGCCGCCTATAGGAAAATTGC
CAGAGATATTCCGATTGCC

In another construct the last 914 nucleotides from NoV RNA2 were cloned directly after the stop codon of the firefly luciferase gene, generating a deletion of only 318 nucleotides in NoV RNA2.

### 3'UTR 914 nt (RNA construct)

AATTTCCAGGGTTCGACCAGCTGTTTGGCACGTCAGCAACTGACACCG
CAGCTAATGTCACTGCTTTCCGGTATGCGTCAATGGCAGCCGGTGTTTACCCC
ACGAGCAATCTAATGCAATTTGCTGGGTCAATACAGGTGTACAAGATACCTCT
AAAACAGGTTCTTAACTCCTACTCTCAGACCGTAGCGACCGTACCACCTACCA
ACTTGGCTCAGAACACAATTGCAATAGATGGACTAGAAGCGTTAGATGCACT
ACCAAATAACAACTACTCCGGTTCTTTATCGAGGGTTGTTATTCACAATCGG
TGTGCAATGAACCTGAGTTTGAGTTCCATCCCATCATGGAGGGTTACGCGTCC
GTTCCACCCGCGAACGTAACCAATGCTCAAGCTAGCATGTTCACCAATCTAAC
CTTCTCAGGCGCAGCATATACCGGTCTTGGCGACATGGACGCAATTGCGATCC
TTGTGACTACGCCCACCGGTGCCGTCAACACGGCCGTGCTGAAGGTCTGGGC
CTGCGTAGAGTATCGTCCGAATCCAAATTCCACCCTCTATGAGTTCGCTCGTG
AGTCACCAGCAAACGACGAATACGCGCTCGCCGCCTATAGGAAAATTGCCAG
AGATATTCCGATTGCCGTCGCTTGCAAAGACAACGCCACATTTTGGGAACGC
GTCCGATCCATCCTGAAATCTGGGCTCAACTTTGCTTCGACCATACCTGGCCC
CGTAGGAGTGGCAGCGACAGGGATCAAAGGCATCATTGAAGCCATTGGTTCC
TTGTGGGTTTGATTCCACCCACAGAAGCGTTGACGACGCAAAACGTCCTTAAA
GCGTTGACGACGCAAAACGTCCCCAAGCTCGTAGCACCGACCCTATACCCAT
CTCTAGGGTCTTCAACCTCTTGGT

### 3'UTR 270 nt (RNA construct)

In another construct, the last 270 nucleotides from NoV RNA2 were directly attached after the stop codon of the firefly luciferase, generating a deletion of 1014 nucleotides in NoV RNA2.

GTCGCTTGCAAAGACAACGCCACATTTTGGAACGCGTCCGATCCATC
CTGAAATCTGGGCTCAACTTTGCTTCGACCATACCTGGCCCGTAGGAGTGGC
AGCGACAGGGATCAAAGGCATCATTGAAGCCATTGGTTCCTTGTGGGTTTGAT
TCCACCCACAGAAGCGTTGACGACGCAAAACGTCCTTAAAGCGTTGACGACG
CAAAACGTCCCCAAGCTCGTAGCACCGACCCTATACCCATCTCTAGGGTCTTC
AACCTCTTGGT

In another construct, the last 236 nucleotides from NoV RNA2 were directly attached after the stop codon of the firefly luciferase, generating a deletion of 1048 nucleotides in NoV RNA2.

### 3'UTR 236 nt (RNA construct)

GCGTCCGATCCATCCTGAAATCTGGGCTCAACTTTGCTTCGACCATACC
TGGCCCCGTAGGAGTGGCAGCGACAGGGATCAAAGGCATCATTGAAGCCATT
GGTTCCTTGTGGGTTTGATTCCACCCACAGAAGCGTTGACGACGCAAAACGTC
CTTAAAGCGTTGACGACGCAAAACGTCCCCAAGCTCGTAGCACCGACCCTAT
ACCCATCTCTAGGGTCTTCAACCTCTTGGT

In another construct, the last 100 nucleotides from NoV RNA2 were directly attached after the stop codon of the firefly luciferase generating a deletion of 1184 nucleotides in NoV RNA2.

### 3' UTR 100 nt (RNA construct)

TTGACGACGCAAAACGTCCTTAAAGCGTTGACGACGCAAAACGTCCCC
AAGCTCGTAGCACCGACCCTATACCCATCTCTAGGGTCTTCAACCTCTTGGT

In another construct, the last 50 nucleotides from NoV RNA2 were directly attached after the stop codon of the firefly luciferase, generating a deletion of 1234 nucleotides in NoV RNA2.

### 3' UTR 50 nt (RNA construct)

GCTCGTAGCACCGACCCTATACCCATCTCTAGGGTCTTCAACCTCTTGG
T

In another construct, the last 25 nucleotides from NoV RNA2 were directly attached after the stop codon of the firefly luciferase, generating a deletion of 1259 nucleotides in NoV RNA2.

### 3' UTR 25 nt (RNA construct)

### TCTCTAGGGTCTTCAACCTCTTGGT

Example:
Here is an example with the first 44 nucleotides from NoV RNA2, followed by the firefly luciferase gene, followed by the last 270 nucleotides from NoV RNA2.

### 44_Fluc_270 (RNA construct)

GTAAACAACCAATAACATCAGGGTATCCAAAGCAGCACGCCGCCATG
GAGGACGCCAAGAACATCAAGAAGGGCCCGCCCCTTCTACCCCTGGAGG
ACGGCACCGCCGGCGAGCAGCTGCACAAGGCCATGAAGCGGTACGCCCTGGT
GCCCGGCACCATCGCCTTCACCGACGCCCACATCGAGGTGGACATCACCTAC
GCCGAGTACTTCGAGATGAGCGTGCGGCTGGCCGAGGCCATGAAGCGGTACG
GCCTGAACACCAACCACCGGATCGTGGTGTGCAGCGAGAACAGCCTGCAGTT
CTTCATGCCCGTGCTGGGCGCCCTGTTCATCGGCGTGGCCGTGGCCCCCGCCA
ACGACATCTACAACGAGCGGGAGCTGCTGAACAGCATGGGCATCAGCCAGCC
CACCGTGGTGTTCGTGAGCAAGAAGGGCCTGCAGAAGATCCTGAACGTGCAG
AAGAAGCTGCCCATCATCCAGAAGATCATCATCATGGACAGCAAGACCGACT
ACCAGGGCTTCCAGAGCATGTACACCTTCGTGACCAGCCACCTGCCCCCGGC
TTCAACGAGTACGACTTCGTGCCCGAGAGCTTCGACCGGGACAAGACCATCG
CCCTGATCATGAACAGCAGCGGCAGCACCGGCCTGCCCAAGGGCGTGGCCCT
GCCCCACCGGACCGCCTGCGTGCGGTTCAGCCACGCCCGGGACCCCATCTTCG
GCAACCAGATCATCCCCGACACCGCCATCCTGAGCGTGGTGCCCTTCCACCAC
GGCTTCGGCATGTTCACCACCCTGGGCTACCTGATCTGCGGCTTCCGGGTGGT

GCTGATGTACCGGTTCGAGGAGGAGCTGTTCCTGCGGAGCCTGCAGGACTAC
AAGATCCAGAGCGCCCTGCTGGTGCCCACCCTGTTCAGCTTCTTCGCCAAGAG
CACCCTGATCGACAAGTACGACCTGAGCAACCTGCACGAGATCGCCAGCGGC
GGCGCCCCCCTGAGCAAGGAGGTGGGCGAGGCCGTGGCCAAGCGGTTCCACC
TGCCCGGCATCCGGCAGGGCTACGGCCTGACCGAGACCACCAGCGCCATCCT
GATCACCCCCGAGGGCGACGACAAGCCCGGCGCCGTGGGCAAGGTGGTGCCC
TTCTTCGAGGCCAAGGTGGTGGACCTGGACACCGGCAAGACCCTGGGCGTGA
ACCAGCGGGGCGAGCTGTGCGTGCGGGGCCCCATGATCATGAGCGGCTACGT
GAACAACCCCGAGGCCACCAACGCCCTGATCGACAAGGACGGCTGGCTGCAC
AGCGGCGACATCGCCTACTGGGACGAGGACGAGCACTTCTTCATCGTGGACC
GGCTGAAGAGCCTGATCAAGTACAAGGGCTACCAGGTGGCCCCCGCCGAGCT
GGAGAGCATCCTGCTGCAGCACCCCAACATCTTCGACGCCGGCGTGGCCGGC
CTGCCCGACGACGACGCCGGCGAGCTGCCCGCCGCCGTGGTGGTGCTGGAGC
ACGGCAAGACCATGACCGAGAAGGAGATCGTGGACTACGTGGCCAGCCAGG
TGACCACCGCCAAGAAGCTGCGGGGCGGCGTGGTGTTCGTGGACGAGGTGCC
CAAGGGCCTGACCGGCAAGCTGGACGCCCGGAAGATCCGGGAGATCCTGATC
AAGGCCAAGAAGGGCGGCAAGATCGCCGTGTGAGTCGCTTGCAAAGACAAC
GCCACATTTTGGGAACGCGTCCGATCCATCCTGAAATCTGGGCTCAACTTTGC
TTCGACCATACCTGGCCCCGTAGGAGTGGCAGCGACAGGGATCAAAGGCATC
ATTGAAGCCATTGGTTCCTTGTGGGTTTGATTCCACCCACAGAAGCGTTGACG
ACGCAAAACGTCCTTAAAGCGTTGACGACGCAAAACGTCCCCAAGCTCGTAG
CACCGACCCTATACCCATCTCTAGGGTCTTCAACCTCTTGGT

In another example the first 52 nucleotides from NoV RNA2 are followed by the firefly luciferase gene, followed by the last 270 nucleotides from NoV RNA2.

### 52_Fluc_270 (RNA construct)

GTAAACAACCAATAACATCAGGGTATCCAAAGCAGCACGCCGCCGAA
GAGCGATGGAGGACGCCAAGAACATCAAGAAGGGCCCGCCCCCTTCTACCC
CCTGGAGGACGGCACCGCCGGCGAGCAGCTGCACAAGGCCATGAAGCGGTA

CGCCCTGGTGCCCGGCACCATCGCCTTCACCGACGCCCACATCGAGGTGGAC
ATCACCTACGCCGAGTACTTCGAGATGAGCGTGCGGCTGGCCGAGGCCATGA
AGCGGTACGGCCTGAACACCAACCACCGGATCGTGGTGCAGCGAGAACAG
CCTGCAGTTCTTCATGCCCGTGCTGGGCGCCCTGTTCATCGGCGTGGCCGTGG
CCCCCGCCAACGACATCTACAACGAGCGGGAGCTGCTGAACAGCATGGGCAT
CAGCCAGCCCACCGTGGTGTTCGTGAGCAAGAAGGGCCTGCAGAAGATCCTG
AACGTGCAGAAGAAGCTGCCCATCATCCAGAAGATCATCATCATGGACAGCA
AGACCGACTACCAGGGCTTCCAGAGCATGTACACCTTCGTGACCAGCCACCT
GCCCCCCGGCTTCAACGAGTACGACTTCGTGCCCGAGAGCTTCGACCGGGAC
AAGACCATCGCCCTGATCATGAACAGCAGCGGCAGCACCGGCCTGCCCAAGG
GCGTGGCCCTGCCCCACCGGACCGCCTGCGTGCGGTTCAGCCACGCCCGGGA
CCCCATCTTCGGCAACCAGATCATCCCCGACACCGCCATCCTGAGCGTGGTGC
CCTTCCACCACGGCTTCGGCATGTTCACCACCCTGGGCTACCTGATCTGCGGC
TTCCGGGTGGTGCTGATGTACCGGTTCGAGGAGGAGCTGTTCCTGCGGAGCCT
GCAGGACTACAAGATCCAGAGCGCCCTGCTGGTGCCCACCCTGTTCAGCTTCT
TCGCCAAGAGCACCCTGATCGACAAGTACGACCTGAGCAACCTGCACGAGAT
CGCCAGCGGCGGCGCCCCCCTGAGCAAGGAGGTGGGCGAGGCCGTGGCCAA
GCGGTTCCACCTGCCCGGCATCCGGCAGGGCTACGGCCTGACCGAGACCACC
AGCGCCATCCTGATCACCCCCGAGGGCGACGACAAGCCCGGCGCCGTGGGCA
AGGTGGTGCCCTTCTTCGAGGCCAAGGTGGTGGACCTGGACACCGGCAAGAC
CCTGGGCGTGAACCAGCGGGGCGAGCTGTGCGTGCGGGGCCCCATGATCATG
AGCGGCTACGTGAACAACCCCGAGGCCACCAACGCCCTGATCGACAAGGACG
GCTGGCTGCACAGCGGCGACATCGCCTACTGGGACGAGGACGAGCACTTCTT
CATCGTGGACCGGCTGAAGAGCCTGATCAAGTACAAGGGCTACCAGGTGGCC
CCCGCCGAGCTGGAGAGCATCCTGCTGCAGCACCCCAACATCTTCGACGCCG
GCGTGGCCGGCCTGCCCGACGACGACGCCGGCGAGCTGCCCGCCGCCGTGGT
GGTGCTGGAGCACGGCAAGACCATGACCGAGAAGGAGATCGTGGACTACGT
GGCCAGCCAGGTGACCACCGCCAAGAAGCTGCGGGGCGGCGTGGTGTTCGTG

GACGAGGTGCCCAAGGGCCTGACCGGCAAGCTGGACGCCCGGAAGATCCGG
GAGATCCTGATCAAGGCCAAGAAGGGCGGCAAGATCGCCGTGTGAGTCGCTT
GCAAAGACAACGCCACATTTTGGGAACGCGTCCGATCCATCCTGAAATCTGG
GCTCAACTTTGCTTCGACCATACCTGGCCCCGTAGGAGTGGCAGCGACAGGG
ATCAAAGGCATCATTGAAGCCATTGGTTCCTTGTGGGTTTGATTCCACCCACA
GAAGCGTTGACGACGCAAAACGTCCTTAAAGCGTTGACGACGCAAAACGTCC
CCAAGCTCGTAGCACCGACCCTATACCCATCTCTAGGGTCTTCAACCTCTTGG
T

For the replication of the NoV genome (also containing a gene of interest), the correct 5' and 3' ends of both, RNA1 and RNA2 are mandatory. To produce RNA1 and RNA2 with their precise 5' and 3' ends via in vitro transcription (IVT) reaction, the DNA containing the respective information is cloned under the control of a T7 or SP6 promoter. Both, T7 and SP6 promoter initiate transcription within the promoter and strongly prefer initiation with G residues. The optimized starting sequence for the T7 promoter is GGG. However, the starting sequence of the RNA1 and RNA2 is in both cases GTA. This is a sequence the T7 polymerase cannot process very well, and the enzyme starts to "stutter" adding one or more Gs to the 5' end of the RNA 1 and RNA2, respectively, lowering the replication level significantly. This problem can be side-stepped by using a SP6 promoter, which has different requirements for its optimal 5' starting sequence {https://doi.org/10.1038/s42003-020-01167-x}. Alternatively, a self-cleaving hammerhead (HH) sequence can be used for the generation of the precise 5'end. Part of the HH ribozyme structure incorporates the target sequence. Thus, different HH sequences for RNA1 and RNA2 must be designed for the different target sequence.

### Hammerhead sequences for the generation of defined 5' RNA1 ends: (RNA construct)

TTTGGATTCAATACCTGATGAGGCCGAAAGGCCGAAACTCCGTAAGGA
GTC

Hammerhead sequences for the generation of defined 5' RNA2 ends: (RNA construct)

TATTGGTTGTTTACCTGATGAGGCCGAAAGGCCGAAACTCCGTAAGGA
GTC

Using the HH sequence allows to use of an optimized +1 to +3 nucleotide sequence for the IVT reaction and thus the optimization of RNA yields in the IVT reaction, since this sequence is cleaving itself from the RNA. A typical sequence can look like:
T7 or SP6 promoter followed by an optimized +1 to +3 nucleotide sequence (GGG for T7 and GAA for SP6 promoter), followed by a short spacer sequence, followed by the HH sequence for either RNA1 or RNA2, followed by the 5' end of RNA1 or RNA2, respectively.

### Example T7 promoter for generation of RNA1: (DNA vector construct)

AATACGACTCACTATAGGATAATTTGGATTCAATACCTGATGAGGCC
GAAAGGCCGAAACTCCGTAAGGAGTC

### Example T7 promoter for generation of RNA2: (DNA vector construct)

TAATACGACTCACTATAGGGATAATTATTGGTTGTTTACCTGATGAGGC
CGAAAGGCCGAAACTCCGTAAGGAGTC

The correct formation of the 3' end of RNA1 and RNA2 are essential for RNA replication. This can be achieved by using a self-cleaving HDV ribozyme (Rz) sequence. To improve the self-cleavage activity of the ribozyme site, a T7 termination sequence can be added after the Rz in case the T7 promoter is used. Different versions of Rz exist and here are 2 examples for a Rz sequence and a T7 termination sequence.

### Example 1 (RNA construct)

GGCCGGCATGGTCCCAGCCTCCTCGCTGGCGCCGGCTGGGCAACACCA
TTGCACTCCGGTGGCGAATGGGACT

example 2 (RNA construct)

GGCCGGCATGGTCCCAGCCTCCTCGCTGGCGCCGGCTGGGCAACATTC
CGAGGGGACCGTCCCCTCGGTAATGGCGAATGGGAC

### T7 terminator sequence for improving the ribozyme cleavage: (RNA construct)

TAGAGCGGCCGCCACCGCTGAGCAATAACTAGCATAACCCCTTGGGGC
CTCTAAACGGGTCTTGAGGGGTTTTTTGCTGAAAGGAGGAACTATATCCGGAT

Taken together, here are some examples for plasmids which can be used in an IVT reaction to generate RNA1 and RNA2 expressing a gene of interest:
RNA1 (DNA vector construct)
RNA1 controlled by T7 promoter, followed by an optimized promoter recognition site, hammerhead sequence, 5'UTR, coding sequence of RNA1, HDV ribozyme site, and a T7 termination site: 
TAATACGACTCACTATAGGGATAATTTGGATTCAATACCTGATGAGGC
CGAAAGGCCGAAACTCCGTAAGGAGTCGTATTGAATCCAAAACTCAAAATGC
TGAACTACGAGACAATCATCAACGGCGCATCGAGCGCTCTGAACATCGTTTC
GCGTGCGTTAGGATACCGCGTGCCACTAGCCAAATCGCTGGCGCTGGTCGCG
GGGTCCTGCGTGGTGTACAAAATAATCGTGCATCGACGCACGCTCGTGGCGTT
CCTGGTAATCGGACCATACGCCACGGTGGTGCAGCACCGTCTGCCGATGGCC
CTTCAGAGGGCCATCATTGAATATACACGAGAAGACCGTGAGATCAGCCTGT
TTCCGCAAAATTCCATCGTTTCCGCAGAACACGCGCGGAAAGCGGATAATGG
GCATCCGATCTCCGGGGGAACGCGTGATGTCGCGAGGGAGACTATTTCCCTT
GCCATAAGGGCCGCTGGTTTTCGTCATTACGAAATCAGCCCCGCGAGGCAAT
CACCAGCTGAGGCGGCAAGCCACCAACATTATGCCGCCGCTGACCTCGTGAG
AGCGGCTACTGAAGATAAGATCCAAGACGGTGATGTGGTAGTTGCCATCGAC
ATCGATTACTACCTGCGTGACATTGACCGCTACCTGGGTCGTGGTGTCCCGTT
CATGGCTTACACCTTCAATCCTGTTGAAGTAGCTGGCCGTGACGGTGACTCCT
TTTTCCGGATCACGAACAATCAAGTCACGTTTGATGTTAGTGGCGGTGGATCT
TGGTCCCATGAAGTTTGGGACTGGTGCGCGTTTGGTGAGTTCATCGAGACCCG
AGACGCGAGCTGGCTTGCTTGGTTCGCCCGGGCGGTTGGACTCACCAAGTCG
CAGATCCACAAAGTTCACTACTGCCGTCCATGGCCGCAATCGCCCCATCGCGC
TTTGGTGTGGTGTCTGCCTGTAGCAAGCTACTGGCGCTTCACTTTCATTCCGAC
GGACCTGCATACGCGCACGCTTCGGCGTGTGCGTTATCAGGACACGTCCCGG
 
CCCGGTTGGAATTCCATCGTCTCGACCGGGTCCGAAGGCCTTAATATCAGCCT
TGGTCGCGAAGGAGCTGATCATTGCGTGACGATTCCAAAGGTGCACTACGAC
ATGCTTATGGGTTTGTCGAGTGCGCAGTCGTTGTCGTCCCGCATGATCGGGCT
CAAGTACACTGATCCTAGTGTACTCGCGACGGTTGCCCAATACTATCAGGGCA
AGAATGTTGAAGTTGCCGACGCTGACAGGATCGGCCGCGCCATAAATCCCAA
GGTCCACTGGCCAGCGCACGTCGAAGTTGACGAGGCGGAGGTTAGTGCTCGG
GTGTACGCCAGCCCGTTGGTATCTGACGAAAATATGATGCCTATGATCAAGC
GCTGGGAGACGCTGTCGTTGTCGCTGGACCGCCGGGTTACATTCCAACGTAAT
CCGAAGGTTCCTGGAAAACGGCTCAGGGCTTATGCCATTGAGTTCGTTGACTT
GGTTGTGCCTGAGCGTGGTGTCGGAGTCCCCTATTCATTGGAGGACACCGCCG
CCATGCTGGACAAACCAAGCCAGACCCTCGCCATCCAACAGGTGTGGAGAC
TGTCGACATGCCCCCAAGAAGGCTCATCGAAGCGTTCGTGAAGAACGAACCG
ACCATGAAGGCTGGCCGTATCATCTCGTCGTTCGCTGACATGCGGTTCCTACT
GCGGTTTTCCAGCTATACGCTGGCATTCCGTGATCAGGTGCTGCATGCAGAGC
ACAACCGGCATTGGTTTTGCCCGGGTTTGACCCCCGAGCAGATCGCCACAAA
AGTGGTTGATTACGTGTCCGGTGTTGAAGAACCATCGGAGGGAGACTTTTCCA
ACTTTGATGGCACGGTTAGTGAGTGGCTACAACGCCACGTCATGAACGCCGT
CTACCTGCGTTATTTCAATCACCGAGCGCAGCGAGACCTCAGGTCGTATACCG
ACATGCTGGTCTCATGCCCCGCGAGGGCGAAGCGATTCGGTTTTGCTTATGAC
GCGGGTGTCGGCGTTAAGAGCGGGTCGCCAACAACTTGCGACCTGAATACCG
TGTGCAATGGTTTCCTCCAATATTGCTCCATTCGAATGACACACCCAGAGCTG
ACACCAATCGATGCTTTCCGGCTCATCGGTCTCGCGTTTGGGGACGATTCCCT
CTTCGAGCGACGTTTCGCTAAGAACTATGCGAAGGTTTCCGCCGAGGTGGGG
ATGGTCCTCAAAATCGAGCGATTCGACCCGGCACAAGGCATCACTTTCCTCGC
CCGTGTTTATCCCGACCCCTACACGTCGACCACAAGTTTCCAGGACCCTTTGC
GTACCTGGAGGAAGCTCCACTTGACGACGCGCGATCCAACAATACCATTGGC
AACGGCTGCCATCGATCGCGTTGAGGGCTACCTCGTCACCGACGGCCTGAGC
CCGCTTACTGGCGCGTATTGTCGCATGGTTAAGCGGGTTTACGAGGCCGGCGG
 
AGCCGAGGATGCCGCCAAGAGGAGGTCGCGAAAATCCCATTCCCGCGAAAA
GCCGTATTGGTTGACTGTTGGAGGCGCTTGGCCCCAAGATGTCAAGGACGTTG
ATCTTATGTTCCAGTGTGCGGCCGCACGTACCGGAGTAGACCTCGAGACACTT
CGGTCTCTGGATCAGCGTCTAGGAGAAATCACTGACGTCTGGGCGGATATTA
CCATCAACCGGGATAATGAACCAAACCCCTACAAGGATACACTGGACTTGGA
GGGCCCGGCTGATGGCCGGGTGGACGATCGTGTATTTCAGAATGACAAACAT
GTCATGCGCTTACGAGCTAATCAAGTCACTTCCAGCCAAGCTGGAGCAGCTG
GCTCAGGAGACGCAAGCAACGATCCAAACGCTCATGATCGCGGATCCCAACG
TCAACAAGGATCTGCGAGCGTTCTGCGAGTTCCTGACCGTGCAGCACCAGCG
GGCGTATCGAGCGACGAACAGCCTGCTCATCAAACCGCGAGTCGCAGCAGCG
CTTCGCGGGGAGGAGCTGGACCTGGGCGAGGCGGACGTCGCCGCCCGGGTCC
GCCAGCTAAAACAACAGCTGGCGGAGCTCGAGATGGAAATCAAGCCAGGGC
ACCAACAAGTGGCCCAAGTAAGCGGCAGGCGGAAGGCCGCAGCCGCAGCTC
CCGTGGCCCAGCTGGGTCGCGTGGGCGTGGTAAATGAGTGATTCATCGTCCC
ATCTGACGAAACCCGAACTAGGCTTATGCCAGTGGTGGCCGGCATGGTCCCA
GCCTCCTCGCTGGCGCCGGCTGGGCAACATTCCGAGGGGACCGTCCCCTCGGT
AATGGCGAATGGGACTAGAGCGGCCGCCACCGCTGAGCAATAACTAGCATAA
CCCCTTGGGGCCTCTAAACGGGTCTTGAGGGGTTTTTTGCTGAAAGGAGGAAC
TATATCCGGA
RNA2 encoding HA codon optimized sequence (DNA vector construct) 
TAATACGACTCACTATAGGGATAATTTGGATTCAATACCTGATGAGGC
CGAAAGGCCGAAACTCCGTAAGGAGTCGTAAACAACCAATAACATCAGGGTA
TCCAAAGCAGCACGCCGCCATGAAAGCTATTCTGACTTTCCTGCTGTACACTT
TCACTGCTGCTAACGCTGACACTCTGTGCATTGGATACCACGCTAACAACAGC
ACTGAAACTGTTGACACTGTTCTGGAAAAAAACGTTACTGTTACTCACAGCGT
TAACCTGCTGGAAGACAAACACAACGAAAAACTGTGCAAACTGAGCGGAGTT
GCTCCTCTGCACCTGGGAAAATGCAACATTGCTGGATGGATTCTGGGAAACC
CTGAATGCGAAAGCCTGAGCACTGCTAGAAGCTGGAGCTACATTGTTGAAAC
 
TAGCAGCAGCGACAACGGAACTTGCTACCCTGGAGACTTCATTGACTACGAA
GAACTGAGAGAACAACTGAGCAGCGTTAGCAGCTTCGAAAGATTCGAAATTT
TCCCTAAAACTAGCAGCTGGCCTAACCACGACAGCAACAAAGGAGTTACTGC
TGCTTGCCCTCACGCTGGAGCTAAAAGCTTCTACAAAAACCTGATTTGGCTGG
TTAAACAAAGAAACAGCTACCCTAAAATTAGCAAAAGCTACATTAACGACAA
AGGAAAAGAAGTTCTGGTTCTGTGGGGAATTCACCACCCTCCTACTATTGCTG
ACCAACAAAGCCTGTACCAAAACGCTGACACTTACGTTTTCGTTGGAACTAGC
AGATACAGCAAAAAATTCAAACCTGAAATTGCTATTAGACCTAAAGTTAGAA
ACCAAGAAGGAAGAATGAACTACTACTGGACTCTGGTTGAACCTGGAGACAA
AATTACTTTCGAAGCTACTGGAAACCTGGTTGTTCCTAGATACGCTTTCGCTA
TGGAAAGAAACGCTGGAAGCGGAATTATTATTAGCGACAGCCCTATTCACGA
CTGCAACACTACTTGCCAAACTCCTAAAGGAGCTATTAACACTAGCCTGCCTT
TCCAAAACATTCACCCTATTACTATTGGAAAATGCCCTAAATACGTTAAAAGC
ACTAAACTGAGACTGGCTACTGGACTGAGAAACGTTCCTAGCATTCAAAGCA
GAGGACTGTTCGGAGCTATTGCTGGATTCATTGAAGGAGGATGGACTGGAAT
GGTTGACGGATGGTACGGATACCACCACCAAAACGAACAAGGAACTGGATAC
GCTGCTGACCTGAAAAGCACTCAAAACGCTATTGACGAAATTACTAACAAAG
TTAACAGCGTTATTGAAAAAATGAACACTCAATTCACTGCTGTTGGAAAAGA
ATTCAACCACCTGGAAAAAAGAATTGAAAACCTGAACAAAAAAGTTGACGAC
GGATTCCTGGACATTTGGACTTACAACGCTGAACTGCTGGTTCTGCTGGAAAA
CGAAAGAACTCTGGACTACCACGACAGCAACGTTAAAAACCTGTACGAAAAA
GTTAGAAGCCAACTGAAAAACAACGCTAAAGAAATTGGAAACGGATGCTTCG
AATTCTACCACAAATGCGACAACACTTGCATGGAAAGCGTTAAAAACGGAAC
TTACGAATACCCTAAATACAGCGAAGAAGCTAAACTGAACAGAGAAAAAATT
GAAGGAGTTAAACTGGAAAGCACTAGAATTTACCAAATTCTGGCTATTTACA
GCACTGTTGCTAGCAGCCTGGTTCTGGTTATTAGCCTGGGAGCTATTAGCTTC
TGGATGTGCAGCAACGGAAGCCTGCAATGCAGAATTTGCATTTAGGTCGCTT
GCAAAGACAACGCCACATTTTGGGAACGCGTCCGATCCATCCTGAAATCTGG
 
GCTCAACTTTGCTTCGACCATACCTGGCCCCGTAGGAGTGGCAGCGACAGGG
ATCAAAGGCATCATTGAAGCCATTGGTTCCTTGTGGGTTTGATTCCACCCACA
GAAGCGTTGACGACGCAAAACGTCCTTAAAGCGTTGACGACGCAAAACGTCC
CCAAGCTCGTAGCACCGACCCTATACCCATCTCTAGGGTCTTCAACCTCTTGG
TGGCCGGCATGGTCCCAGCCTCCTCGCTGGCGCCGGCTGGGCAACATTCCGA
GGGGACCGTCCCCTCGGTAATGGCGAATGGGACTAGAGCGGCCGCCACCGCT
GAGCAATAACTAGCATAACCCCTTGGGGCCTCTAAACGGGTCTTGAGGGGTT
TTTTGCTGAAAGGAGGAACTATATCCGGAT
RNA2 encoding firefly luciferase (DNA vector construct) 
TAATACGACTCACTATAGGGATAATTTGGATTCAATACCTGATGAGGC
CGAAAGGCCGAAACTCCGTAAGGAGTCGTAAACAACCAATAACATCAGGGTA
TCCAAAGCAGCACGCCGCCATGGAGGACGCCAAGAACATCAAGAAGGGCCC
CGCCCCCTTCTACCCCCTGGAGGACGGCACCGCCGGCGAGCAGCTGCACAAG
GCCATGAAGCGGTACGCCCTGGTGCCCGGCACCATCGCCTTCACCGACGCCC
ACATCGAGGTGGACATCACCTACGCCGAGTACTTCGAGATGAGCGTGCGGCT
GGCCGAGGCCATGAAGCGGTACGGCCTGAACACCAACCACCGGATCGTGGTG
TGCAGCGAGAACAGCCTGCAGTTCTTCATGCCCGTGCTGGGCGCCCTGTTCAT
CGGCGTGGCCGTGGCCCCCGCCAACGACATCTACAACGAGCGGGAGCTGCTG
AACAGCATGGGCATCAGCCAGCCCACCGTGGTGTTCGTGAGCAAGAAGGGCC
TGCAGAAGATCCTGAACGTGCAGAAGAAGCTGCCCATCATCCAGAAGATCAT
CATCATGGACAGCAAGACCGACTACCAGGGCTTCCAGAGCATGTACACCTTC
GTGACCAGCCACCTGCCCCCCGGCTTCAACGAGTACGACTTCGTGCCCGAGA
GCTTCGACCGGGACAAGACCATCGCCCTGATCATGAACAGCAGCGGCAGCAC
CGGCCTGCCCAAGGGCGTGGCCCTGCCCCACCGGACCGCCTGCGTGCGGTTC
AGCCACGCCCGGGACCCCATCTTCGGCAACCAGATCATCCCCGACACCGCCA
TCCTGAGCGTGGTGCCCTTCCACCACGGCTTCGGCATGTTCACCACCCTGGGC
TACCTGATCTGCGGCTTCCGGGTGGTGCTGATGTACCGGTTCGAGGAGGAGCT
GTTCCTGCGGAGCCTGCAGGACTACAAGATCCAGAGCGCCCTGCTGGTGCCC
 
ACCCTGTTCAGCTTCTTCGCCAAGAGCACCCTGATCGACAAGTACGACCTGAG
CAACCTGCACGAGATCGCCAGCGGCGCCCCCTGAGCAAGGAGGTGGGC
GAGGCCGTGGCCAAGCGGTTCCACCTGCCCGGCATCCGGCAGGGCTACGGCC
TGACCGAGACCACCAGCGCCATCCTGATCACCCCCGAGGGCGACGACAAGCC
CGGCGCCGTGGGCAAGGTGGTGCCCTTCTTCGAGGCCAAGGTGGTGGACCTG
GACACCGGCAAGACCCTGGGCGTGAACCAGCGGGGCGAGCTGTGCGTGCGG
GGCCCCATGATCATGAGCGGCTACGTGAACAACCCCGAGGCCACCAACGCCC
TGATCGACAAGGACGGCTGGCTGCACAGCGGCGACATCGCCTACTGGGACGA
GGACGAGCACTTCTTCATCGTGGACCGGCTGAAGAGCCTGATCAAGTACAAG
GGCTACCAGGTGGCCCCCGCCGAGCTGGAGAGCATCCTGCTGCAGCACCCCA
ACATCTTCGACGCCGGCGTGGCCGGCCTGCCCGACGACGACGCCGGCGAGCT
GCCCGCCGCCGTGGTGGTGCTGGAGCACGGCAAGACCATGACCGAGAAGGA
GATCGTGGACTACGTGGCCAGCCAGGTGACCACCGCCAAGAAGCTGCGGGGC
GGCGTGGTGTTCGTGGACGAGGTGCCCAAGGGCCTGACCGGCAAGCTGGACG
CCCGGAAGATCCGGGAGATCCTGATCAAGGCCAAGAAGGGCGGCAAGATCG
CCGTGTGAGTCGCTTGCAAAGACAACGCCACATTTGGGAACGCGTCCGATC
CATCCTGAAATCTGGGCTCAACTTTGCTTCGACCATACCTGGCCCGTAGGAG
TGGCAGCGACAGGGATCAAAGGCATCATTGAAGCCATTGGTTCCTTGTGGGT
TTGATTCCACCCACAGAAGCGTTGACGACGCAAAACGTCCTTAAAGCGTTGA
CGACGCAAAACGTCCCCAAGCTCGTAGCACCGACCCTATACCCATCTCTAGG
GTCTTCAACCTCTTGGTGGCCGGCATGGTCCCAGCCTCCTCGCTGGCGCCGGC
TGGGCAACATTCCGAGGGGACCGTCCCCTCGGTAATGGCGAATGGACTAGA
GCGGCCGCCACCGCTGAGCAATAACTAGCATAACCCCTTGGGGCCTCTAAAC
GGGTCTTGAGGGGTTTTTTGCTGAAAGGAGGAACTATATCCGGAT
RNA2 H5 St. Jude (DNA vector construct) 
TAATACGACTCACTATAGGGATAATTTGGATTCAATACCTGATGAGGC
CGAAAGGCCGAAACTCCGTAAGGAGTCGTAAACAACCAATAACATCAGGGTA
TCCAAAGCAGCACGCCGCCATGGAGAAAATAGTGCTTCTTCTTGCAATAGTC
 
AGTCTTGTTAAAAGTGATCAGATTTGCATTGGTTACCATGCAAACAACTCGAC
AGAGCAGGTTGACACAATAATGGAAAAGAACGTCACTGTTACACACGCCCAA
GACATACTGGAAAAGACACACAACGGGAAGCTCTGCGATCTAGATGGAGTGA
AGCCTCTAATTTTAAGAGATTGTAGTGTAGCTGGATGGCTCCTCGGAACCCA
ATGTGTGACGAATTCCTCAATGTGCCGGAATGGTCTTACATAGTGGAGAAGA
TCAATCCAGCCAATGACCTCTGTTACCCAGGGAATTTCAACGACTATGAAGA
ACTGAAACACCTATTGAGCAGAATAAACCATTTTGAGAAAATTCAGATCATC
CCCAAAAGTTCTTGGTCAGATCATGAAGCCTCATCAGGGGTGAGCTCAGCAT
GTCCATACCAGGGAAGGTCCTCCTTTTTTAGAAATGTGGTATGGCTTATCAAA
AAGAACAATGCATACCCAACAATAAAGAGAAGTTACAATAATACCAACCAA
GAAGATCTTTTGGTACTGTGGGGGATTCACCATCCAAATGATGCGGCAGAGC
AGACAAGGCTCTATCAAAACCCAACCACCTATATTTCCGTTGGGACATCAAC
ACTAAACCAGAGATTGGTACCAAAAATAGCTACTAGATCCAAGGTAAACGGG
CAAAGTGGAAGGATGGAGTTCTTTTGGACAATTTTAAAACCGAATGATGCAA
TAAACTTTGAGAGTAATGGAAATTTCATTGCTCCAGAAAATGCATACAAAATT
GTCAAGAAAGGGGACTCAACAATTATGAAAAGTGAATTGGAATATGGTAACT
GCAACACCAAGTGTCAAACTCCAATAGGGGCGATAAACTCTAGTATGCCATT
CCACAACATCCACCCTCTCACCATCGGGGAATGCCCCAAATATGTGAAATCA
AACAGATTAGTCCTTGCGACTGGGCTCAGAAATAGCCCTCAAATTGAAACTA
GAGGATTATTTGGAGCTATAGCAGGTTTTATAGAGGGAGGATGGCAGGGAAT
GGTAGATGGTTGGTATGGGTACCACCATAGCAACGAGCAGGGGAGTGGGTAC
GCTGCAGACAAAGAATCCACTCAAAAGGCAATAGATGGAGTCACCAATAAG
GTCAACTCGATCATTGACAAAATGAACACTCAGTTTGAGGCCGTTGGAAGGG
AATTTAATAACTTAGAAAGGAGAATAGAAAATTTAAACAAGAAGATGGAAG
ACGGATTCCTAGATGTCTGGACTTATAATGCTGAACTTCTGGTTCTCATGGAA
AATGAGAGAACTCTAGACTTTCATGACTCAAATGTCAAGAACCTTTACGACA
AGGTCCGACTACAGCTTAGGGATAATGCAAAGGAGCTTGGTAACGGTTGTTT
CGAGTTCTATCACAGATGTGATAATGAATGTATGGAAAGTGTAAGAAACGGA
 
ACGTATGACTACCCGCAGTATTCAGAAGAAGCAAGATTAAAAAGAGAGGAA
ATAAGTGGAGTAAAATTGGAATCAATAGGAACTTACCAAATACTGTCAATTT
ATTCAACAGTGGCGAGCTCCCTAGCACTGGCAATCATGGTGGCTGGTCTATCT
TTGTGGATGTGCTCCAATGGATCGTTACAATGCAGAATTTGCATTTAAGTCGC
TTGCAAAGACAACGCCACATTTTGGGAACGCGTCCGATCCATCCTGAAATCTG
GGCTCAACTTTGCTTCGACCATACCTGGCCCCGTAGGAGTGGCAGCGACAGG
GATCAAAGGCATCATTGAAGCCATTGGTTCCTTGTGGGTTTGATTCCACCCAC
AGAAGCGTTGACGACGCAAAACGTCCTTAAAGCGTTGACGACGCAAAACGTC
CCCAAGCTCGTAGCACCGACCCTATACCCATCTCTAGGGTCTTCAACCTCTTG
GTGGCCGGCATGGTCCCAGCCTCCTCGCTGGCGCCGGCTGGGCAACATTCCG
AGGGGACCGTCCCCTCGGTAATGGCGAATGGGACTAGAGCGGCCGCCACCGC
TGAGCAATAACTAGCATAACCCCTTGGGGCCTCTAAACGGGTCTTGAGGGGT
TTTTTGCTGAAAGGAGGAACTATATCCGGAT
RNA2 PEDV S
EXAMPLE RNA2_PEDV-spike (DNA vector construct) 
TAATACGACTCACTATAGGGATAATTTGGATTCAATACCTGATGAGGC
CGAAAGGCCGAAACTCCGTAAGGAGTCGTAAACAACCAATAACATCAGGGTA
TCCAAAGCAGCACGCCGCCATGAAGTCTTTAACCTACTTCTGGTTGTTCTTAC
CAGTACTTTCAACATTTAGCCTACCACAAGATGTCGCCAGGTGCTCAGCTAAC
ACTAATTTTAGGCGGTTCTTCTCAAAATTTAATGTTCAGGCGCCTGCAGTTGTT
GTACTCGGCGGTTATCTACCTACTGGTGAAAACCAGGGTGCAACTTGGTACTG
TGCTGGCCAACATCCAACTGCTAGTGGCGTTCATGGTATCTTTCTTAGCCATA
TTAGAGGTGGTCATGGCTTTGAGATTGGCATTTCGCAAGAGCCTTTTGACCCT
AGTGGTTACCAGCTTTATTTACATAAGGCTACTAACGGTAACACTAATGCTAC
TGCGCGACTGCGTATTTGCCAGTTTCCTAACATTAAAACATTGGGCCCCACTG
CTAATAATGATGTTACAACAGGTCGTAACTGCCTATTTAACAAAGCCATCCCA
GCTCATATGAGTGAACACAGTGTTGTCGGCGTAACATGGGATAATGATCGTG
TCACTGTCTTTTCTGACAAGATCTATCATTTTTATTTTAAAAATGATTGGTCCC
 
GTGTTGCGACAAAGTGTTACAACAGTGGAGGTTGTGCTATGCAATATGTTTAC
AAACCCACCTACTACATGCTTAATGTTACTAGTGCTGGTGAGGATGGCATTTC
TTATCAACCCTGTACAGCTAATTGCGTTGGTTATGCTGCCAATGTATTTGCTAC
TGAGCCCAATGGCCACATACCAGAAGGTTTAGTTTTAATAATTGGTTTCTTT
TGTCCAATGATTCCACTTTGGTGCATGGTAAGGTGGTTTCCAACCAACCTTTG
TTGGTTAATTGTCTTTTGGCCATTCCTAAGATTTATGGACTAGGCCAATTTTTC
TCATTCAATCAAACTATGGATGGCGTTTGTAATGGAGCTGCTGTGCAGCGTGC
ACCAGAGGCTCTGAGGTTTAATATTAATGACACCTCTGCCATTCTTGCTGAAG
GCTCAATTGTACTTCATACTGCTTTAGGAACAAATTTTCTTTTGTTTGCAGTA
ATTCCTCAGATCCTCACTTAGCCACCTTCGCCATACCTCTGGGTGCTACCCAG
TTACCCTATTATTGTTTTTTAAAGTGGATACTTTCAACTCTACTGTTTATAAA
TTCTTGGCTGTTTACCTCCTACCGTCAGGGAAATTGTCATCACCAAGTATGG
TGATGTTTATGTCAATGGGTTTGGCTACTTGCATCTTGGTTTGTTGGATGCTGT
CACAATTAATTTCACTAGTCATGGCACTGACGATGACGTTTCTGGTTTTTGGA
CCATAGCATCGACTAATTTTGTTGATGCACTTATTGAAGTTCAAGGAACTGCC
ATTCAGCGTATTCTTTATTGTGATGATCCTGTTAGCCAACTCAAGTGTTCTCAG
GTTGCTTTTGACCTTGACGATGGTTTTTACCCTATCTCCTCTACAAACCTCCTG
AGTCATGAACAGCCAACTTCTTTTGTTACTTTGCCATCATTTAATGATCATTCT
TTTGTTAATATTACTGTCTCTGCTGCTTTTGGTGGTCATAGTGGTGCCAACCTT
ATTGCATCTGACACTACTATCAATGGGTTTAGTTCTTTCTGTGTTGACACTAGA
CAATTTACCATTTCACTGTTTTATAACGTTACAAACAGTTATGGTTATGTGTCT
AAATCACAGGACAGTAATTGTCCTTTTACCTTGCAATCTGTTAATGATTACCT
GTCTTTTAGCAAATTTTGTGTTTCTACCAGCCTTTTGGCTAGTGCCTGTACCAT
AGATCTTTTTGGTTACCCTGAGTTTGGTAGTGGTGTCAAGTTCACGTCCCTTTA
CTTTCAATTCACAAAGGGTGAGTTGATTACTGGCACGCCTAAACCACTTGAAG
GTGTTACGGACGTTTCTTTTATGACTCTGGATGTGTGTACCAAGTATACTATCT
ATGGCTTTAAAGGTGAGGGTATCATTACCCTTACAAATTCTAGCTTTTTGGCA
GGTGTTCATTATACATCTGATTCTGGACAGTTGTTAGCTTTTAAGAATGTCACT
 
AGTGGTGCTGTTTATTCTGTTACGCCGTGTTCTTTTCAGAGCAGGCTGCATAT
GTTGATGATGATATAGTGGGTGTTATTTCTAGTTTGTCTAACTCCACTTTTAAC
AGTACTAGGGAGTTGCCTGGTTTCTTCTACCATTCTAATGATGGCTCTAATTGT
ACAGAGCCTGTGTTGGTGTATAGTAACATAGGTGTTTGTAAATCTGGCAGTAT
TGGCTATGTCCCATCTCAGTCTGGCCAAGTCAAGATTACACCCACGGTTACTG
GGAATATTAGTATTCCCACCAACTTTAGTATGAGTATTAGGACAGAATATTTA
CAGCTTTACAACACACCTGTTAGTGTTGATTGTGCTACATATGTTTGTAATGG
TAACTCTCGTTGTAAACAATTACTCACCCAGTACACTTCAGCATGTAAGACCA
TAGAGTCAGCATTACAACTCAGCGCTAGGCTTGAGTCTGCTGAAGTCAACTCT
ATGCTTACTATTTCTGAAGAGGCTCTACAGTTAGCTACCATCAGTTCGTTTAA
TGGTGATGGATATAATTTTACCAACGTGCTGGGTGTTTCCGTGTATGATCCTG
CAAGTGGCAGGGTGGTACAAAAAGGTCTTTATTGAAGACCTGCTTTTTAAT
AAAGTGGTTACTAATGGTCTTGGTACTGTTGATGAAGACTATAAGCGCTGTTC
TAATGGCCGCTCTGTGGCAGATTTAGTCTGTGCGCAGTATTACTCTGGTGTCA
TGGTACTACCTGGTGTTGTTGACGCTGAGAAGCTTCATATGTATAGTGCGTCT
CTCATCGGTGGTATGGTGCTAGGAGGTTTTACTGCAGCAGCGGCATTGCCTTT
TAGCTATGCTGTTCAAGCGAGACTTAATTATCTTGCTCTACAGACGGATGTTC
TACAGCGGAACCAGCAATTGCTTGCTGAGTCTTTTAATTCTGCTATTGGTAAT
ATAACTTCAGCCTTTGAGAGTGTTAAAGAGGCTATTAGTCAAACTTCTAAGGG
TTTGAACACTGTGGCTCATGCGCTTACTAAGGTCCAAGAGGTTGTTAATTCGC
AGGGTGCAGCTTTGACCCAACTTACCGTACAGCTGCAACACAACTTCCAAGC
CATTTCTAGTTCTATTGATGACATTTACTCCCGACTTGACATTCTTTCAGCCGA
TGCTCAGGTAGACCGTCTCATCACCGGCAGATTATCAGCACTTAATGCTTTTG
TTGCTCAAACCCTTACTAAGTATACTGAGGTTCAGGCTAGTAGGAAACTAGCA
CAGCAAAAGGTTAATGAGTGCGTTAAATCGCAATCTCAGCGTTATGGTTTTG
TGGTGGTGATGGCGAGCACATTTTCTCACTGGTACAGGCCGCACCTCAAGGCC
TGCTGTTTTTACACACAGTACTTGTACCGGGTGACTTTGTAAATGTTATTGCCA
TCGCTGGCTTATGTGTTAACGATGAAATTGCCTTGACTCTACGTGAGCCTGGC
 
TTACTCTTGTTTACGCATGAACTTCAAGATACTGCGACGGAATATTTTGTTTCA
TCGCGACGTATGTATGAACCTAGAAAACCTACCGTTGGTGATTTTGTTCAAAT
TGAGAGTTGTGTGGTCACCTATGTCAATTTGACTAGAGACCAACTACCAGAA
GTAATCCCAGATTACATCGATGTTAACAAAACATTTGATGAGATTTTAACTTC
TCTGCCCAATAGAACTGGTCCAAGTCTTTCTCTAGATGTTTTTAATGCCACTTA
TCTTAATCTCACTGGTGAAATTGCAGATTTAGAGCAGCGTTCAGAGTCTCTCC
GTAATACTACAGAAGCGCTCCAAAGTCTTATATATAATATCAACAACACACT
AGTTGACCTTGAGTGGCTCAACCGAGTTGAGACATATATCAAGTGGCCGTGG
TGGGTTTGGTTGATTATTTTATTGTTCTCATCTTTGTTGTGTCATTACTAGTGT
TCTGCTGCATTTCCACGGGTTGTTGTGGGTGCTGCGGCTGCTGTGGTGCTTGTT
TTTCAGGTTGTTGTAGGGGTCCTAGACTTCAACCTTACGAAGCTTTTGAAAAG
GTCCACGTGCAGTGAGTCGCTTGCAAAGACAACGCCACATTTGGGAACGCG
TCCGATCCATCCTGAAATCTGGGCTCAACTTTGCTTCGACCATACCTGGCCCC
GTAGGAGTGGCAGCGACAGGGATCAAAGGCATCATTGAAGCCATTGGTTCCT
TGTGGGTTTGATTCCACCCACAGAAGCGTTGACGACGCAAAACGTCCTTAAA
GCGTTGACGACGCAAAACGTCCCCAAGCTCGTAGCACCGACCCTATACCCAT
CTCTAGGGTCTTCAACCTCTTGGTGGCCGGCATGGTCCCAGCCTCCTCGCTGG
CGCCGGCTGGGCAACATTCCGAGGGGACCGTCCCCTCGGTAATGGCGAATGG
GACTAGAGCGGCCGCCACCGCTGAGCAATAACTAGCATAACCCCTTGGGGCC
TCTAAACGGGTCTTGAGGGGTTTTTTGCTGAAAGGAGGAACTATATCCGGAT
EXAMPLE RNA-ORF2d (DNA vector construct) 
TAATACGACTCACTATAGGGATAATTTGGATTCAATACCTGATGAGGC
CGAAAGGCCGAAACTCCGTAAGGAGTCGTAAACAACCAATAACATCAGGGTA
TCCAAAGCAGCACGCCGCCATGACGTATCCAAGGAGGCGTTTCCGCAGACGA
AGACACCGCCCCCGCAGCCATCTTGGCCAGATCCTCCGCCGCCGCCCCTGGCT
CGTCCACCCCCGCCACCGTTACCGCTGGAGAAGGAAAAATGGCATCTTCAAC
ACCCGCCTCTCCCGCACCATCGGTTATACTGTCAAGAAAACCACAGTCAGAA
CGCCCTCCTGGAATGTGGACATGATGAGATTTAATATTAATGATTTCTTCCC
 
CCAGGAGGGGGCTCAAACCCCCTCACTGTGCCCTTTGAATACTACAGAATAA
GGAAGGTTAAGGTTGAATTCTGGCCCTGCTCCCCAATCACCCAGGGTGACAG
GGGAGTGGGCTCCACTGCTGTTATTCTAGATGATAACTTTGTAACAAAGGCCA
ATGCCCTAACCTATGACCCCTATGTAAACTACTCCTCCCGCCATACCATAACC
CAGCCCTTCTCCTACCACTCCCGGTACTTTACCCCGAAACCTGTCCTTGATGG
GACAATCGATTACTTCCAACCCAATAACAAAAGAAATCAACTCTGGCTGAGA
CTACAAACTACTGGAAATGTAGACCATGTAGGCCTCGGCACTGCGTTCGAAA
ACAGTATATACGACCAGGACTACAATATCCGTATAACCATGTATGTACAATTC
AGAGAATTTAATCTTAAAGACCCCCCACTTAACCCTAAGTGAGTCGCTTGCAA
AGACAACGCCACATTTTGGGAACGCGTCCGATCCATCCTGAAATCTGGGCTC
AACTTTGCTTCGACCATACCTGGCCCCGTAGGAGTGGCAGCGACAGGGATCA
AAGGCATCATTGAAGCCATTGGTTCCTTGTGGGTTTGATTCCACCCACAGAAG
CGTTGACGACGCAAAACGTCCTTAAAGCGTTGACGACGCAAAACGTCCCCAA
GCTCGTAGCACCGACCCTATACCCATCTCTAGGGTCTTCAACCTCTTGGTGGC
CGGCATGGTCCCAGCCTCCTCGCTGGCGCCGGCTGGGCAACATTCCGAGGGG
ACCGTCCCCTCGGTAATGGCGAATGGGACTAGAGCGGCCGCCACCGCTGAGC
AATAACTAGCATAACCCCTTGGGGCCTCTAAACGGGTCTTGAGGGGTTTTTG
CTGAAAGGAGGAACTATATCCGGAT
EXAMPLE RNA2_HA09 (DNA vector construct)
   strain="A/California/04/2009"
   serotype="H1N1"
Gene Bank accession number: MN371616 
TAATACGACTCACTATAGGGATAATTTGGATTCAATACCTGATGAGGC
CGAAAGGCCGAAACTCCGTAAGGAGTCGTAAACAACCAATAACATCAGGGTA
TCCAAAGCAGCACGCCGCCATGAAGGCAATACTAGTAGTTCTGCTATATACA
TTTGCAACCGCAAATGCAGACACATTATGTATAGGTTATCATGCGAACAATTC
AACAGACACTGTAGACACAGTACTAGAAAAGAATGTAACAGTAACACACTCT
GTTAACCTTCTAGAAGACAAGCATAACGGGAAACTATGCAAACTAAGAGGGG
 
TAGCCCCATTGCATTTGGGTAAATGTAACATTGCTGGCTGGATCCTGGGAAAT
CCAGAGTGTGAATCACTCTCCACAGCAAGCTCATGGTCCTACATTGTGGAAAC
ACCTAGTTCAGACAATGGAACGTGTTACCCAGGAGATTTCATCGATTATGAG
GAGCTAAGAGAGCAATTGAGCTCAGTGTCATCATTTGAAAGGTTTGAGATAT
TCCCCAAGACAAGTTCATGGCCCAATCATGACTCGAACAAAGGTGTAACGGC
AGCATGTCCTCATGCTGGAGCAAAAAGCTTCTACAAAAATTTAATATGGCTA
GTTAAAAAAGGAAATTCATACCCAAAGCTCAGCAAATCCTACATTAATGATA
AAGGGAAAGAAGTCCTCGTGCTATGGGGCATTCACCATCCAYCTACTAGTGC
TGACCAACAAAGTMTCTATCAGAATGCAGATACATATGTTTTTGTGGGGTCAT
CAAGATACAGCAAGAAGTTCAAGCCGGAAATAGCAATAAGACCCAAAGTGA
GGGATCAAGAAGGGAGAATGAACTATTACTGGACACTAGTAGAGCCGGGAG
ACAAAATAACATTCGAAGCAACTGGAAATCTAGTGGTACCGAGATATGCATT
CGCAATGGAAAGAAATGCTGGATCTGGTATTATCATTTCAGATACACCAGTCC
ACGATTGCAATACAACTTGTCAAACACCCAAGGGTGCTATAAACACCAGCCT
CCCATTTCAGAATATACATCCGATCACAATTGGAAAATGTCCAAAATATGTAA
AAAGCACAAAATTGAGACTGGCCACAGGATTGAGGAATATCCCGTCTATTCA
ATCTAGAGGCCTATTTGGGGCCATTGCCGGTTTCATTGAAGGGGGGTGGACA
GGGATGGTAGATGGATGGTACGGTTATCACCATCAAAATGAGCAGGGGTCAG
GATATGCAGCCGACCTGAAGAGCACACAGAATGCCATTGACGAGATTACTAA
CAAAGTAAATTCTGTTATTGAAAAGATGAATACACAGTTCACAGCAGTAGGT
AAAGAGTTCAACCACCTGGAAAAAAGAATAGAGAATTTAAATAAAAAAGTT
GATGATGGTTTCCTGGACATTTGGACTTACAATGCCGAACTGTTGGTTCTATT
GGAAAATGAAAGAACTTTGGACTACCACGATTCAAATGTGAAGAACTTATAT
GAAAAGGTAAGAAGCCAGCTAAAAAACAATGCCAAGGAAATTGGAAACGGC
TGCTTTGAATTTTACCACAAATGCGATAACACGTGCATGGAAAGTGTCAAAA
ATGGGACTTATGACTACCCAAAATACTCAGAGGAAGCAAAATTAAACAGAGA
AGAAATAGATGGGGTAAAGCTGGAATCAACAAGGATTTACCAGATTTTGGCG
ATCTATTCAACTGTCGCCAGTTCATTGGTACTGGTAGTCTCCCTGGGGGCAAT
 
CAGTTTCTGGATGTGCTCTAATGGGTCTCTACAGTGTAGAATATGTATTTAAG
TCGCTTGCAAAGACAACGCCACATTTGGGAACGCGTCCGATCCATCCTGAA
ATCTGGGCTCAACTTTGCTTCGACCATACCTGGCCCGTAGGAGTGGCAGCGA
CAGGGATCAAAGGCATCATTGAAGCCATTGGTTCCTTGTGGGTTTGATTCCAC
CCACAGAAGCGTTGACGACGCAAAACGTCCTTAAAGCGTTGACGACGCAAAA
CGTCCCCAAGCTCGTAGCACCGACCCTATACCCATCTCTAGGGTCTTCAACCT
CTTGGTGGCCGGCATGGTCCCAGCCTCCTCGCTGGCGCCGGCTGGGCAACATT
CCGAGGGGACCGTCCCCTCGGTAATGGCGAATGGGACTAGAGCGGCCGCCAC
CGCTGAGCAATAACTAGCATAACCCCTTGGGGCCTCTAAACGGGTCTTGAGG
GGTTTTTTGCTGAAAGGAGGAACTATATCCGGAT
Replication system for FHV (Flock House virus)
RNA1 FHV (DNA vector construct) 
TAATACGACTCACTATAGGGATAATTTGGATTCAATACCTGATGAGGC
CGAAAGGCCGAAACTCCGTAAGGAGTCGTAAACAACCAATAACATCAGGGTA
TCCAAAGCAGCACGCCGCCGGTTTCGTTTTCGAACAAACAAACAGCGAACCC
TAAAACAATGACCCTAAAAGTTATTCTTGGAGACCACCAGATCACCCGGACT
GAATTGCTCGTCGGGCTTGCAACCGTATCTGGGTGCGGTGCCATAGCGTACTG
CATATCCAAGTTCTGGGGCTATGGGGCAATTGCGCCCTATCCCCAGAGTGGA
GGGAACCGAGTTACACGCGCATTGCAACGGGCTGTCATTGACAAAACGAAGA
CCCCGATAGGAACACGTTTCTATCCGCTTGACAGCCTGCGTACCGTAACGCCT
AAGCGTGCCATTAATAACGGGCATGCCGTTTCAGGAGCCGTACGTGATGCCG
CTCGTCGTTTAATCGACGAGGCTATAACATCTGTAGGAGGATCTAAATTTGAA
GTTAACCCGAACCCAAATTCTAGCACTGGACTGCGGAAACATTTCCACTTCGC
CGTCGGTGATTTGGCGCAAGACTTTCGCAATGATACACCAGCGGAAGACGCC
TTCATCGTAGGTGTTGATGTTGATTATTATGTCACCGAGCCAGATGTGCTGTT
AGAGCACATGCGTCCAGTGGTTTTACACACCTTCAACCCGAAGAAAGTGAGC
GGCTTTGATGCTGACTCACCATTCACCATCAAGAACAATCTGGTTGAATACAA
GGTTAGTGGAGGTGCTGCGTGGGTCCATCCAGTTTGGGATTGGTGCGAAGCT
 
GGTGAGTTCATCGCCAGCCGAGTCCGAACAAGTTGGAAGGAATGGGTTTTAC
AACTGCCATTGAGAATAGTTGGTTTAGAAAAGGTCGGTTATCACAAAATCCA
CCATTGTAGACCCTGGACTGATTGTCCAGATCGATCACTCGTCTACACAATAC
CGCAGTATACTGTTTGGCGGTTCAATTGGATCGACACCGAGCTACACGTACGC
AAACTAAAACGGATTGAATACCAGGATGAAACTAAACCTGGTTGGAACAGAT
TGGAGTATGTCACCGACAAGAACCAACTAATGGTTTCCATCGGTAGAGAAGG
AGAGCACGCTCAGATCACCATCGAGAAAGAAAAGTTGGATATGCTCTCGGGA
TTATCCGCCACACAATCTGTCAACGCTAGGCTTATCGGTATGGGGCACAAGG
ACCCACAGTACACATCTATGATTGTTCAGTATTACACCGGCAAGCGAGTTGTG
TCACCAATTAGTCCAACTGTGTATAAACCTACAATGCCACGCGTCCATTGGCC
AGTAACCAGTGACGCAGATGTACCAGAAGTGAGCGCACGCCAATACACCTTG
CCTATCGTGAGTGACTGTATGATGATGCCAATGATTAAACGCTGGGAGACAA
TGTCTGAATCAATTGAACGCAGGGTAACCTTTGTCGCCAACGACAAGAAACC
AAGCGACAGAATTGCGAGGATCGCCGACACTTTCGTACGGTTGATGAACGGG
CCTTTCAACAACCTTGACCCATTGTCAATCGAGGAAACGATTAAGCGTCTAAA
CAAGCCGTCCCAACAACTACAACTTAGGGCGGTTTTCGAGATGATCGGAGTT
GAGCCGCGTCAATTGATTGAGTCATTCAACAAAAACGAACCGGGAATGAAAT
CTAGTCGGATAATATCCGGCTTTCCTGACATCCTTTTCATCCTGAAGGTCTCCA
GATACACATTAGCGTACTCGGATATCGTTCTACATGCCGAGCATAATCAACAT
TGGTATTATCCTGGAAGGAACCCAACTGAGATTGCCGATGGGGTTTGTGAATT
TGTCAGTGACTGTGACGCTGAAGTCATAGAAACGGATTTTTCCAACCTCGATG
GCCGGGTTTCTGGCTGGATGCAACGAAATATCGCTCAAAAGGCGATGGTTCA
AGCATTCCGGCCAGAATACAGAGATGAGATCATTTCATTCATGGACACGATA
ATCAATTGTCCAGCCAAAGCTAAACGCTTTGGTTTCCGATATGAGCCTGGAGT
TGGTGTTAAAAGTGGTAGTCCAACAACCACGCCGCATAACACGCAATATAAT
GCATGTGTCGAATTTACAGCTCTCACCATTGAGCACCCTTATGCCGAACCAGA
AGAACTGTTCCGATTAATCGGTCCAAAGTGCGGTGATGATGGTCTGTCGCGG
GCCATCATTCAAAATGCCATCAACCGTGCTGCCAAATGTTATGGCTTGGAGCT
 
CAAAGTGGAACGATACAACCCAGAGATAGGTCTTTGCTTTCTCTCACGTGTTT
TTGTGGACCCGCTCGCAACGACGACAACACTACAAGACCCACTGCGTACACT
GCGAAAACTGCACCTCACAGCAAGAGATCCTACGATACCACTAGCTGACGCG
GCTTGCGACCGCGTCGAAGGTTATCTTTGTACCGATGCGCTTACTCCGTTGAT
ATCGGATTACTGCAAAATGGTACTACGGTTATACGGCCGACTGCCTCCACA
GAGGAAGTCAGGAACCAACGTAGAAGCCGGAATAAGGAGAAACCCTACTGG
TTAACGTGCGATGGATCATGGCCACAGCATCCGCAAGACGTCCATTTGATGA
AGCAGGTTTTAATCAAGCGTACCAGAATTGACGAAGATCAGGTCGATGCACT
CATTGGGCGTTTTGCCGCAATGAAGGATGTCTGGGAGAAGATTACACGTGAC
AGCGAGGAGAGCGCCGCTGCGTGTACGTTTGATGAAGACGGCGTTATGCCGG
GCTCCGTGGACGAATCGTTACCTAAGCTTAACGATGCCAAGCAAACTCGCGC
TAATCCAGGAACTTCCCGACCGCATTCAAACGGCGGTAGAAGCAGCAATGGG
AATGAGCTATCAGGACGCACCGAACAACGTGCGCAGGGACCTCGACAACCTG
CATGCTTGCCTAAACAAGGCAAAGCTAACGGTAAGACGAATGGTAACATCAC
TGCTGGAGAAACCCAGCGTGGTGGCATACCTCGAAGGAAGGGCCCCGGAGG
AGGCAAAACCAACACTCGAAGAACGCCTCCGAAAGCTAGAGCTCAGCCACA
GCCTTCCAACAACCGGAAGTGACCCCCCACCCGCTAAATCGTAGGTGACTCCT
AGGAGCACCCACACACGTTCTAGCCCGAAAGGGCGGAGGTGTCGCTTGCAAA
GACAACGCCACATTTTGGGAACGCGTCCGATCCATCCTGAAATCTGGGCTCA
ACTTTGCTTCGACCATACCTGGCCCCGTAGGAGTGGCAGCGACAGGGATCAA
AGGCATCATTGAAGCCATTGGTTCCTTGTGGGTTTGATTCCACCCACAGAAGC
GTTGACGACGCAAAACGTCCTTAAAGCGTTGACGACGCAAAACGTCCCCAAG
CTCGTAGCACCGACCCTATACCCATCTCTAGGGTCTTCAACCTCTTGGTGGCC
GGCATGGTCCCAGCCTCCTCGCTGGCGCCGGCTGGGCAACATTCCGAGGGGA
CCGTCCCCTCGGTAATGGCGAATGGGACTAGAGCGGCCGCCACCGCTGAGCA
ATAACTAGCATAACCCCTTGGGGCCTCTAAACGGGTCTTGAGGGGTTTTTTGC
TGAAAGGAGGAACTATATCCGGAT
RNA2 FHV (DNA vector construct) 
TAATACGACTCACTATAGGGATAATTTGGATTCAATACCTGATGAGGC
CGAAAGGCCGAAACTCCGTAAGGAGTCGTAAACAACCAATAACATCAGGGTA
TCCAAAGCAGCACGCCGCCGTAAACAATTCCAAGTTCAAAATGGTTAACAAC
ATCAAACCGAAACGACAACGATCCCAACGCGTCGTCGTAACAACAACCCAAA
CAGCGCCTATTCCACAGCAAAACGTACCACGTAATGGTAGACGTCGACGCAA
TCGCGCGGCGGAATCGCCGTCGTGGACGCGGCATGAATATGGCGGCGCTT
TCCCGGCTTAGTCAGCCTGGGTTGGCGTTTCTCAAGTGTGCCTTCGCACCACC
TGATTTCAACACCGATCCCGGAAAAGGAATACCAGATAGATTTGAAGGCAAA
GTGGTCAGCCGAAAGGATGTCCTCAATCAATCTATCAGTTTCGAATCCGGAA
AGGACACTTTTATACTTATCGCACCTACCCCTGGAGTCGCTTACTGGAGTGCC
AGTGTTGATGCTGGTAGTTTTCCTACTAGCACGACTACGTTTACACCGACCAA
TTATCCGGGCTTTACATCGATGTTCGGAACTACTGCAACATCTAGGTCCGATC
AAGTGTCCTCCTTCAGGTACGCTTCCATGAACGTGGGCATTTACCCAACGTCG
AACTTGATGCAGTTTGCCGGAAGCATAACTGTTTGGAAATGTCCTATAAAGCT
GACCACTGCACAATTCCCGGTTTCTACAGTGCCAGCCACCTCTTCACTAGTAC
ACAGTCTTGTTGGTTTAGATGGTGTATTAGCGGTGGACCAGACAATTTCTCA
GAATCATTCATTAAAGGAGTATTCTCTCAATCGGCTTGTAATGAGCCCGATTT
TGAGTTCAGCGACATATTGGAGGGTATCCAAATATTGCCACCATTGAATGTAG
ATATAGGTTCAACGGGCCAACCGTTTACCTTGTCCGCAGGAAACGAAAACAC
CAGTGGCATTGTTGGCTGGGGTAATATGGACACCATTGTCATCCGTGTCTCTG
CCCCCACTGGCGCAGTCAACTCCGCCATACTCAAGGCATGGTCTTGCATTGAG
TACAGACCCAATCCTAATGCTATGCTATATCAATTCGGCCATGACTCGCCACC
TCTTGATGAGGTAGCACTTCAAGAATACCGTACGGTTGCCAGATCTTTGCCGG
TTGCTGTCATCGCAGCGCAAAACGCATCAATGTGGGAGAGAGTTAAAGCTAT
CATCAAGTCCTCCTTAGCTGCTGCAAGCAACATTCCTGGCCCTATCGGTGTCG
CCGCAAGCGGCATTAGTGGACTGTCGGCCTTATTTGAAGGTTTTGGATTTAG
ACTACCAACCGGATGCCAACCTAACTGGGCAAATATCCGAACGATCGGACAT
TGGGCCACAATAAGCCCAACTTGGTTGATGAGCGAAACAATGAGCCCCCTTA
 
GCTCGAATCCGGGCCTGTCGCTTGCAAAGACAACGCCACATTTTGGGAACGC
GTCCGATCCATCCTGAAATCTGGGCTCAACTTTGCTTCGACCATACCTGGCCC
CGTAGGAGTGGCAGCGACAGGGATCAAAGGCATCATTGAAGCCATTGGTTCC
TTGTGGGTTTGATTCCACCCACAGAAGCGTTGACGACGCAAAACGTCCTTAAA
GCGTTGACGACGCAAAACGTCCCCAAGCTCGTAGCACCGACCCTATACCCAT
CTCTAGGGTCTTCAACCTCTTGGTGGCCGGCATGGTCCCAGCCTCCTCGCTGG
CGCCGGCTGGGCAACATTCCGAGGGGACCGTCCCCTCGGTAATGGCGAATGG
GACTAGAGCGGCCGCCACCGCTGAGCAATAACTAGCATAACCCCTTGGGGCC
TCTAAACGGGTCTTGAGGGGTTTTTTGCTGAAAGGAGGAACTATATCCGGAT
**EXAMPLE pRNA1** (DNA vector construct) this is simply the cDNA sequence of the RNA1 replicon including T7 promoter and ribozyme site
   1-18 ... T7 promoter
   19-3222 ... saRNA1
   3223-3295 ... HDV ribozyme 
TAATACGACTCACTATAGGTATTGAATCCAAAACTCAAAATGCTGAAC
TACGAGACAATCATCAACGGCGCATCGAGCGCTCTGAACATCGTTTCGCGTG
CGTTAGGATACCGCGTGCCACTAGCCAAATCGCTGGCGCTGGTCGCGGGTC
CTGCGTGGTGTACAAAATAATCGTGCATCGACGCACGCTCGTGGCGTTCCTGG
TAATCGGACCATACGCCACGGTGGTGCAGCACCGTCTGCCGATGGCCCTTCA
GAGGGCCATCATTGAATATACACGAGAAGACCGTGAGATCAGCCTGTTTCCG
CAAAATTCCATCGTTTCCGCAGAACACGCGCGGAAAGCGGATAATGGGCATC
CGATCTCCGGGGGAACGCGTGATGTCGCGAGGGAGACTATTTCCCTTGCCAT
AAGGGCCGCTGGTTTTCGTCATTACGAAATCAGCCCCGCGAGGCAATCACCA
GCTGAGGCGGCAAGCCACCAACATTATGCCGCCGCTGACCTCGTGAGAGCGG
CTACTGAAGATAAGATCCAAGACGGTGATGTGGTAGTTGCCATCGACATCGA
TTACTACCTGCGTGACATTGACCGCTACCTGGGTCGTGGTGTCCCGTTCATGG
CTTACACCTTCAATCCTGTTGAAGTAGCTGGCCGTGACGGTGACTCCTTTTTCC
GGATCACGAACAATCAAGTCACGTTTGATGTTAGTGGCGGTGGATCTTGGTCC
 
CATGAAGTTTGGGACTGGTGCGCGTTTGGTGAGTTCATCGAGACCCGAGACG
CGAGCTGGCTTGCTTGGTTCGCCCGGGCGGTTGGACTCACCAAGTCGCAGATC
CACAAAGTTCACTACTGCCGTCCATGGCCGCAATCGCCCCATCGCGCTTTGGT
GTGGTGTCTGCCTGTAGCAAGCTACTGGCGCTTCACTTTCATTCCGACGGACC
TGCATACGCGCACGCTTCGGCGTGTGCGTTATCAGGACACGTCCCGGCCCGGT
TGGAATTCCATCGTCTCGACCGGGTCCGAAGGCCTTAATATCAGCCTTGGTCG
CGAAGGAGCTGATCATTGCGTGACGATTCCAAAGGTGCACTACGACATGCTT
ATGGGTTTGTCGAGTGCGCAGTCGTTGTCGTCCCGCATGATCGGGCTCAAGTA
CACTGATCCTAGTGTACTCGCGACGGTTGCCCAATACTATCAGGGCAAGAAT
GTTGAAGTTGCCGACGCTGACAGGATCGGCCGCGCCATAAATCCCAAGGTCC
ACTGGCCAGCGCACGTCGAAGTTGACGAGGCGGAGGTTAGTGCTCGGGTGTA
CGCCAGCCCGTTGGTATCTGACGAAAATATGATGCCTATGATCAAGCGCTGG
GAGACGCTGTCGTTGTCGCTGGACCGCCGGGTTACATTCCAACGTAATCCGAA
GGTTCCTGGAAAACGGCTCAGGGCTTATGCCATTGAGTTCGTTGACTTGGTTG
TGCCTGAGCGTGGTGTCGGAGTCCCCTATTCATTGGAGGACACCGCCGCCATG
CTGGACAAACCAAGCCAGACCCTCGCCATCCAACAGGTGTGGGAGACTGTCG
ACATGCCCCCAAGAAGGCTCATCGAAGCGTTCGTGAAGAACGAACCGACCAT
GAAGGCTGGCCGTATCATCTCGTCGTTCGCTGACATGCGGTTCCTACTGCGGT
TTTCCAGCTATACGCTGGCATTCCGTGATCAGGTGCTGCATGCAGAGCACAAC
CGGCATTGGTTTTGCCCGGGTTTGACCCCCGAGCAGATCGCCACAAAAGTGGT
TGATTACGTGTCCGGTGTTGAAGAACCATCGGAGGGAGACTTTTCCAACTTTG
ATGGCACGGTTAGTGAGTGGCTACAACGCCACGTCATGAACGCCGTCTACCT
GCGTTATTTCAATCACCGAGCGCAGCGAGACCTCAGGTCGTATACCGACATG
CTGGTCTCATGCCCCGCGAGGGCGAAGCGATTCGGTTTTGCTTATGACGCGGG
TGTCGGCGTTAAGAGCGGGTCGCCAACAACTTGCGACCTGAATACCGTGTGC
AATGGTTTCCTCCAATATTGCTCCATTCGAATGACACACCCAGAGCTGACACC
AATCGATGCTTTCCGGCTCATCGGTCTCGCGTTTGGGGACGATTCCCTCTTCG
AGCGACGTTTCGCTAAGAACTATGCGAAGGTTTCCGCCGAGGTGGGGATGGT
 
CCTCAAAATCGAGCGATTCGACCCGGCACAAGGCATCACTTTCCTCGCCCGTG
TTTATCCCGACCCCTACACGTCGACCACAAGTTTCCAGGACCCTTTGCGTACC
TGGAGGAAGCTCCACTTGACGACGCGCGATCCAACAATACCATTGGCAACGG
CTGCCATCGATCGCGTTGAGGGCTACCTCGTCACCGACGGCCTGAGCCCGCTT
ACTGGCGCGTATTGTCGCATGGTTAAGCGGGTTTACGAGGCCGGCGGAGCCG
AGGATGCCGCCAAGAGGAGGTCGCGAAAATCCCATTCCCGCGAAAAGCCGTA
TTGGTTGACTGTTGGAGGCGCTTGGCCCCAAGATGTCAAGGACGTTGATCTTA
TGTTCCAGTGTGCGGCCGCACGTACCGGAGTAGACCTCGAGACACTTCGGTCT
CTGGATCAGCGTCTAGGAGAAATCACTGACGTCTGGGCGGATATTACCATCA
ACCGGGATAATGAACCAAACCCCTACAAGGATACACTGGACTTGGAGGGCCC
GGCTGATGGCCGGGTGGACGATCGTGTATTTCAGAATGACAAACATGTCATG
CGCTTACGAGCTAATCAAGTCACTTCCAGCCAAGCTGGAGCAGCTGGCTCAG
GAGACGCAAGCAACGATCCAAACGCTCATGATCGCGGATCCCAACGTCAACA
AGGATCTGCGAGCGTTCTGCGAGTTCCTGACCGTGCAGCACCAGCGGGCGTA
TCGAGCGACGAACAGCCTGCTCATCAAACCGCGAGTCGCAGCAGCGCTTCGC
GGGGAGGAGCTGGACCTGGGCGAGGCGGACGTCGCCGCCCGGGTCCGCCAG
CTAAAACAACAGCTGGCGGAGCTCGAGATGGAAATCAAGCCAGGGCACCAA
CAAGTGGCCCAAGTAAGCGGCAGGCGGAAGGCCGCAGCCGCAGCTCCCGTG
GCCCAGCTGGGTCGCGTGGGCGTGGTAAATGAGTGATTCATCGTCCCATCTGA
CGAAACCCGAACTAGGCTTATGCCAGTGGTGGCCGGCATGGTCCCAGCCTCC
TCGCTGGCGCCGGCTGGGCAACACCATTGCACTCCGGTGGCGAATGGGACT
Coding sequence of Ide Strep Suis including signal peptide and transmembrane domain (DNA vector construct) 
ATGAACATTCAAGAACGATTTTCTTTGAGAAAATCCGCGGTTGGCTTG
GTCTCAGTCTCTTTGCTATGTGCTATTTATACATCCACTGTTGCTGCCGATACA
GTTGTTACAGGAGTGAATGAAATAATTGAAGAATCACAAGTCAAGGATGAGG
TATCTATTGAATCAGAAAAAAATGAATCCCTAGATGGTTCTAATATTGAAATT
GTAGAGGAAATAGCAGACAACATCCCATCACCTGTTATCGCTGAAGGGGAAG
 
TAGCGGTAGAGATGAAAGTTGACAGAGGACCGAGAATGTAGTTTCTAGAAA
TGATACAGAAGTTACGACGAGCGAGCAAAATCAGATAGAGGTTACTGAGACA
AAAGAAATTTTGAATCAGACCAGTTATCAAACGGAGAGTGGCGAGCAACGAC
AAATTATATGGGCCCATGGAATTACTCCTCCTGCAATGGAACAAAGCGGTGG
TTTTGTAAAGGAAAAGTATGGAGACTATTTAAACTATACAGCGCCATTTGAG
GCTGGAAAAGGCTACTATGATACCAATAAGAGTCTGAATGCTTCATTTATTGA
CCTTAATCTTTGTTTTGCAGCTGTGTCTTCAAACATGGTACATTGGTGGTTGGA
ACAGAATAGTTCCTATGTTGAGCGATATCTCAAAGAAAAAAGGGTACAGTA
AATGTTGAAGAAAACTACGCAATAACGGACTTACGGCGCTATATTAATTCATT
CCAAAATCAACAAAATAGTCGAGTTTTTGATATGTTCAAAACTTACTATGGTT
ATCGTACAAATGGTTTTGTATCAGATGCCTTGGTTGACTTGTTTATTAACGGA
TATAAACCTAAGGCACAGGGCGGTGTCAATCTGGAAGATAGCCAGTTAGTAC
CAGATAGTAGGGGTGGCTTTTTCTACGACGTTTTCAAAGAGAAAAACTGAC
AAATCGAATTTTTAGTGGTAGTTATGAGCGGTTTGGTGAGGATGTTCGAACTG
TTTTGGAAAGCAAAGGATTACTCGGCTTAACTTATAGAACATTAGGTTATGCA
ACGCATATTGTGACGGTATGGGGTGCTGAGTACGACAATCAAGGTAAGATTA
AGGCTGTCTATATCACAGATTCTGATGATCAACAAGAACAAATTGGTTTGAA
GCGTATGGGAATCACTCGTGATGCTTCCGGAAATCCACGTTTGAATAATCATA
TGAAAAATAATTCAGCTGGAGCGCTTTTGGATTATGTCCATACAATCCGTCTG
GGTCAAGACTTATGGGAAGAATATTTCAATCCGCTTGCAAAAGCCAAAGAAA
CAGCTAGTCAGACATTAGCCGATACAAAGAAGGCGTTGGATTTGTCTATTCA
AGGACAATCTGAATTGCCAGAATCAATGCGACTGATTTATCTTGAAAAACTA
AATAATCTCTATAATCAAGGAATTCTATCTATTCAAAAGGCAGAAAGTTCTGA
GATGCTAAGTGGTGCATTGGAAAATGGTTTAAATAGTTTAAAGAGTTTAGATT
TTCCTATTTCAGAAGTTGGAAATGCTTTGGCACCAGATTTACCAGTAGGTGAT
CGCTCAACGGTTTCAGATGTTGATTCTCTATCATCTCAAGAAACAAGTTCCAC
AAATTTGGAAGCAGACACAGAGAATGCAGGTATTATTGCAGATGGTACCAAT
CAATTGCATTTTCCAGTGGAGGCCCAAACGACATCTTCAGTAGAGGCTGAGG
 
GAGATAATGTTTTTGAACAAGAGGCAGATACATTACCAATAATTATTGAAAA
CAAGGATGAATTTGGTTCAGAACTATCAAGAAACATGCAAACGTCAGAAACG
GATTCGCTAGTAGTAGCTGTTGAAGAAGATGTGAAAAATGATGAGGTAGCCC
AAGTTGAAGAGCTTCTTGAATCAGAAAAAGTTGAAAATCAGAGTTCGGAACT
TCTGTCAGACACCCTAATCGTAGAGAGTGCAAATGACAAAGAAGAAGATAGA
GTGGAGGCGGTTGTTTCTGAACAACCAGACTCAATACCACATCAAAATGTAG
AAATCTCTCTTGTAGAACCAACGAATGTCGAAACTGAAACTGTGGTCACTCCT
ATTAATGATGCAGCTACTCCTCATGGTTCCCCGACGTATATAGATAATTCCGT
AACTGAATCTGTAGCTACTCCACTTGAAAAAGACTCCATTCAAGCCGGGGAG
ACAGAGATTGCAGAACCAACCTCGAGCGAATCAACGAATGTCGAAACTGAAA
CTGTGGTCACTCCTGTTAATGATGTAGCTACTCCTCATGGTTCCCCGACGTAT
ATAGATAATTCCGTAACTGAATCTGTAGCTACTCCACTTGAAAAAGACTCCAT
TCAAGCCGGAGAGACAGAAATTGCAGAACCAACCTCGAGCGAATCAACGAA
TGTCGAAACTGAAACTGTGGTCACTCCTGTTAATGATGTAGCTACTCCTCATG
GTTCCCCGACGTATATAGATAATTCCGTAACTGAATCTGTAGCTACTCCACTT
GAAAAAGACTCCATTCAAGCCGGGGAGACAGAGATTGCAGAACCAACCTCG
AGCGAATCAACTAGTGTTGAAGCTGAACTTGTCGACAATTCTGAAATTCATGC
AGCTACCTCTTCAGTTACTCCCTGTGGCTCCTCGGCATATGCAGATGGTTCCA
CAACTGAATCTGTAGCCACTCCGCTTGAAAAAGACTCCATTCAGACTGGAAA
TACAGAAATTGCAGAACCAACCTCGAGCAAATCAACTAATGTAGAAGCTGCA
TCTGTCGACAATTCTGAAATTCATGCAGATGCCTCTCTAACTGCTGTTTCATCA
GTTAATCTGGATAATCCAGTGATTGAACCAGTAGCTATCTCCCTTATCGGTTC
TAAGAGGGACACGAATGCAGAAGTAGAAGTTTCTTCATTATCGAAAAGAGAG
GTTAGAAAAACAAATACTGACGGGCTAATCTCTGTTCAATCAAAAGTTATTA
AGAAAGAATTGCTAGAATCAAGCTTAGCAGAAGCAGGGTCTCCATTGCTAGA
AGCCACCATTGCTCAGTCTTCAAACTCAAATAGTACTGAGATAGGTATGAGCT
ATCAGAATACTGTGTTATTAGAGTCTAATAATACAGAGCGTCAGGTGTCTAAA
GCAGAAATTGTTATGGAACACAAGGAGACAGAGTTAGTTGAAACGGTTTCAT
 
CTGCTTCTGAGCCTGTAGTGCTCGTAGAAAATATCTCACAAACCTCAAATAAT
ACTATTGAATCTGGTAAGAATATGGGAGTTCAATCTCAAGCAGGTGCAAAAC
AAATTTTAGGCGTAGAACAATCTTCGAAAGTAAGTACACCTACTTCAAGACA
GATTATGGGAGTCGGTCTATTGACTCTTGTTCTTGGTAGTGCTTTAGGTTTGTT
AAAGAAAAGACGTAAGTAA
**EXAMPLE pRNA2** (DNA vector construct)
   1-18 ... T7 promoter
   19-1354 ... taRNA2
   1355-1427 ... HDV ribozyme 
TAATACGACTCACTATAGGTAAACAACCAATAACATCATGGTATCCAA
AGCAGCACGCCGCCGAAGAGCGGCCCCTCGACAACAACAACGACAACAGTC
GAACCGTGCATCAAACCAGCCTCGTCGGAGGAGAGCTCGCCGTACTCGAAGA
CAGCAGAGAATGGCAGCAACAAACAACATGCTGAAAATGAGCGCTCCCGGA
CTCGATTTTCTGAAATGTGCCTTTGCTTCGCCGGATTTTCCACCGATCCCGGC
AAAGGTATTCCAGACAAATTTCAGGGTCTCGTTTACCGAAGAAACATTGTCT
GACCCAGTCGATAACGTTTACTCCGGGAAACAGACTATGCTGTTGGTTGCAC
CTATTCCTGGAATTGCTTGCCTGAAGGCAGAAGCGAATGTTGGCGCATCCTTT
TCAGGTGTTCCTCTTGCCTCAGTTGAATTTCCAGGGTTCGACCAGCTGTTTGGC
ACGTCAGCAACTGACACCGCAGCTAATGTCACTGCTTTCCGGTATGCGTCAAT
GGCAGCCGGTGTTTACCCCACGAGCAATCTAATGCAATTTGCTGGGTCAATAC
AGGTGTACAAGATACCTCTAAAACAGGTTCTTAACTCCTACTCTCAGACCGTA
GCGACCGTACCACCTACCAACTTGGCTCAGAACACAATTGCAATAGATGGAC
TAGAAGCGTTAGATGCACTACCAAATAACAACTACTCCGGTTCTTTTATCGAG
GGTTGTTATTCACAATCGGTGTGCAATGAACCTGAGTTTGAGTTCCATCCCAT
CATGGAGGGTTACGCGTCCGTTCCACCCGCGAACGTAACCAATGCTCAAGCT
AGCATGTTCACCAATCTAACCTTCTCAGGCGCAGCATATACCGGTCTTGGCGA
CATGGACGCAATTGCGATCCTTGTGACTACGCCCACCGGTGCCGTCAACACG
GCCGTGCTGAAGGTCTGGGCCTGCGTAGAGTATCGTCCGAATCCAAATTCCAC
 
CCTCTATGAGTTCGCTCGTGAGTCACCAGCAAACGACGAATACGCGCTCGCC
GCCTATAGGAAAATTGCCAGAGATATTCCGATTGCCGTCGCTTGCAAAGACA
ACGCCACATTTGGGAACGCGTCCGATCCATCCTGAAATCTGGGCTCAACTTT
GCTTCGACCATACCTGGCCCCGTAGGAGTGGCAGCGACAGGGATCAAAGGCA
TCATTGAAGCCATTGGTTCCTTGTGGGTTTGATTCCACCCACAGAAGCGTTGA
CGACGCAAAACGTCCTTAAAGCGTTGACGACGCAAAACGTCCCCAAGCTCGT
AGCACCGACCCTATACCCATCTCTAGGGTCTTCAACCTCTTGGTGGCCGGCAT
GGTCCCAGCCTCCTCGCTGGCGCCGGCTGGGCAACACCATTGCACTCCGGTGG
CGAATGGGACT
EXAMPLE pRNA2_HA09 (DNA vector construct)
   1-18 ... T7 promoter
   19-70 ... 5'UTR, mutated start codon (ATG → **AGG**), 5' cds. of capsid
   71-1771 coding sequence HA
   strain="A/California/04/2009"
   serotype="H1N1"
   Gene Bank accession number: MN371616
   1772-2041 ... 3' cds. From NoV capsid (153 nt), 3'UTR
   2042-2114 ... Hepatitis Delta virus ribozyme 
TAATACGACTCACTATAGTAAACAACCAATAACATCAGGGTATCCAAA
GCAGCACGCCGCCGAAGAGCGATGAAGGCAATACTAGTAGTTCTGCTATATA
CATTTGCAACCGCAAATGCAGACACATTATGTATAGGTTATCATGCGAACAAT
TCAACAGACACTGTAGACACAGTACTAGAAAAGAATGTAACAGTAACACACT
CTGTTAACCTTCTAGAAGACAAGCATAACGGGAAACTATGCAAACTAAGAGG
GGTAGCCCCATTGCATTTGGGTAAATGTAACATTGCTGGCTGGATCCTGGGAA
ATCCAGAGTGTGAATCACTCTCCACAGCAAGCTCATGGTCCTACATTGTGGAA
ACACCTAGTTCAGACAATGGAACGTGTTACCCAGGAGATTTCATCGATTATGA
GGAGCTAAGAGAGCAATTGAGCTCAGTGTCATCATTTGAAAGGTTTGAGATA
TTCCCCAAGACAAGTTCATGGCCCAATCATGACTCGAACAAAGGTGTAACGG
 
CAGCATGTCCTCATGCTGGAGCAAAAGCTTCTACAAAATTTAATATGGCTA
GTTAAAAAGGAAATTCATACCCAAAGCTCAGCAAATCCTACATTAATGATA
AAGGGAAAGAAGTCCTCGTGCTATGGGGCATTCACCATCCAYCTACTAGTGC
TGACCAACAAAGTMTCTATCAGAATGCAGATACATATGTTTTTGTGGGGTCAT
CAAGATACAGCAAGAAGTTCAAGCCGGAAATAGCAATAAGACCCAAAGTGA
GGGATCAAGAAGGGAGAATGAACTATTACTGGACACTAGTAGAGCCGGGAG
ACAAAATAACATTCGAAGCAACTGGAAATCTAGTGGTACCGAGATATGCATT
CGCAATGGAAAGAAATGCTGGATCTGGTATTATCATTTCAGATACACCAGTCC
ACGATTGCAATACAACTTGTCAAACACCCAAGGGTGCTATAAACACCAGCCT
CCCATTTCAGAATATACATCCGATCACAATTGGAAAATGTCCAAAATATGTAA
AAAGCACAAAATTGAGACTGGCCACAGGATTGAGGAATATCCCGTCTATTCA
ATCTAGAGGCCTATTTGGGGCCATTGCCGGTTTCATTGAAGGGGGGTGGACA
GGGATGGTAGATGGATGGTACGGTTATCACCATCAAAATGAGCAGGGGTCAG
GATATGCAGCCGACCTGAAGAGCACACAGAATGCCATTGACGAGATTACTAA
CAAAGTAAATTCTGTTATTGAAAAGATGAATACACAGTTCACAGCAGTAGGT
AAAGAGTTCAACCACCTGGAAAAAAGAATAGAGAATTTAAATAAAAAAGTT
GATGATGGTTTCCTGGACATTTGGACTTACAATGCCGAACTGTTGGTTCTATT
GGAAAATGAAAGAACTTTGGACTACCACGATTCAAATGTGAAGAACTTATAT
GAAAAGGTAAGAAGCCAGCTAAAAAACAATGCCAAGGAAATTGGAAACGGC
TGCTTTGAATTTTACCACAAATGCGATAACACGTGCATGGAAAGTGTCAAAA
ATGGGACTTATGACTACCCAAAATACTCAGAGGAAGCAAAATTAAACAGAGA
AGAAATAGATGGGGTAAAGCTGGAATCAACAAGGATTTACCAGATTTTGGCG
ATCTATTCAACTGTCGCCAGTTCATTGGTACTGGTAGTCTCCCTGGGGGCAAT
CAGTTTCTGGATGTGCTCTAATGGGTCTCTACAGTGTAGAATATGTATTTAAG
TCGCTTGCAAAGACAACGCCACATTTGGGAACGCGTCCGATCCATCCTGAA
ATCTGGGCTCAACTTTGCTTCGACCATACCTGGCCCCGTAGGAGTGGCAGCGA
CAGGGATCAAAGGCATCATTGAAGCCATTGGTTCCTTGTGGGTTTGATTCCAC
CCACAGAAGCGTTGACGACGCAAAACGTCCTTAAAGCGTTGACGACGCAAAA
 
CGTCCCCAAGCTCGTAGCACCGACCCTATACCCATCTCTAGGGTCTTCAACCT

CTTGGTGGCCGGCATGGTCCCAGCCTCCTCGCTGGCGCCGGCTGGGCAACACC

ATTGCACTCCGGTGGCGAATGGGACT
EXAMPLE pRNA-ORF2d (DNA vector construct)
   1-18 ... T7 promoter
   19-70 ... 5'UTR, mutated start codon, 5'cds of capsid from NoV
   71-775 ... cds. ORF2d from porcine circovirus type 2 (PCV2) strain GDYZ (accession number JX519293)
   776-1045 ... 3'end cds. from NoV capsid (153 nt) and 3'UTR from NoV
   1046-1118 ... Hepatitis Delta virus ribozyme 
TAATACGACTCACTATAGTAAACAACCAATAACATCAGGGTATCCAAA
GCAGCACGCCGCCGAAGAGCGATGACGTATCCAAGGAGGCGTTTCCGCAGAC
GAAGACACCGCCCCCGCAGCCATCTTGGCCAGATCCTCCGCCGCCGCCCCTG
GCTCGTCCACCCCCGCCACCGTTACCGCTGGAGAAGGAAAAATGGCATCTTC
AACACCCGCCTCTCCCGCACCATCGGTTATACTGTCAAGAAAACCACAGTCA
GAACGCCCTCCTGGAATGTGGACATGATGAGATTTAATATTAATGATTTCTT
CCCCCAGGAGGGGGCTCAAACCCCCTCACTGTGCCCTTTGAATACTACAGAAT
AAGGAAGGTTAAGGTTGAATTCTGGCCCTGCTCCCCAATCACCCAGGGTGAC
AGGGGAGTGGGCTCCACTGCTGTTATTCTAGATGATAACTTTGTAACAAAGGC
CAATGCCCTAACCTATGACCCCTATGTAAACTACTCCTCCCGCCATACCATAA
CCCAGCCCTTCTCCTACCACTCCCGGTACTTTACCCCGAAACCTGTCCTTGATG
GGACAATCGATTACTTCCAACCCAATAACAAAAGAAATCAACTCTGGCTGAG
ACTACAAACTACTGGAAATGTAGACCATGTAGGCCTCGGCACTGCGTTCGAA
AACAGTATATACGACCAGGACTACAATATCCGTATAACCATGTATGTACAATT
CAGAGAATTTAATCTTAAAGACCCCCCACTTAACCCTAAGTGAGTCGCTTGCA
AAGACAACGCCACATTTTGGGAACGCGTCCGATCCATCCTGAAATCTGGGCT
CAACTTTGCTTCGACCATACCTGGCCCCGTAGGAGTGGCAGCGACAGGGATC
AAAGGCATCATTGAAGCCATTGGTTCCTTGTGGGTTTGATTCCACCCACAGAA
 
GCGTTGACGACGCAAAACGTCCTTAAAGCGTTGACGACGCAAAACGTCCCCA

AGCTCGTAGCACCGACCCTATACCCATCTCTAGGGTCTTCAACCTCTTGGTGG

CCGGCATGGTCCCAGCCTCCTCGCTGGCGCCGGCTGGGCAACACCATTGCACT

CCGGTGGCGAATGGGACT
EXAMPLE pRNA2_PEDV-spike (DNA vector construct) 
TAATACGACTCACTATAGTAAACAACCAATAACATCAGGGTATCCAAA
GCAGCACGCCGCCGAAGAGCGATGAAGTCTTTAACCTACTTCTGGTTGTTCTT
ACCAGTACTTTCAACATTTAGCCTACCACAAGATGTCGCCAGGTGCTCAGCTA
ACACTAATTTTAGGCGGTTCTTCTCAAAATTTAATGTTCAGGCGCCTGCAGTT
GTTGTACTCGGCGGTTATCTACCTACTGGTGAAAACCAGGGTGCAACTTGGTA
CTGTGCTGGCCAACATCCAACTGCTAGTGGCGTTCATGGTATCTTTCTTAGCC
ATATTAGAGGTGGTCATGGCTTTGAGATTGGCATTTCGCAAGAGCCTTTTGAC
CCTAGTGGTTACCAGCTTTATTTACATAAGGCTACTAACGGTAACACTAATGC
TACTGCGCGACTGCGTATTTGCCAGTTTCCTAACATTAAAACATTGGGCCCCA
CTGCTAATAATGATGTTACAACAGGTCGTAACTGCCTATTTAACAAAGCCATC
CCAGCTCATATGAGTGAACACAGTGTTGTCGGCGTAACATGGGATAATGATC
GTGTCACTGTCTTTTCTGACAAGATCTATCATTTTTATTTTAAAAATGATTGGT
CCCGTGTTGCGACAAAGTGTTACAACAGTGGAGGTTGTGCTATGCAATATGTT
TACAAACCCACCTACTACATGCTTAATGTTACTAGTGCTGGTGAGGATGGCAT
TTCTTATCAACCCTGTACAGCTAATTGCGTTGGTTATGCTGCCAATGTATTTGC
TACTGAGCCCAATGGCCACATACCAGAAGGTTTTAGTTTTAATAATTGGTTTC
TTTTGTCCAATGATTCCACTTTGGTGCATGGTAAGGTGGTTTCCAACCAACCTT
TGTTGGTTAATTGTCTTTTGGCCATTCCTAAGATTTATGGACTAGGCCAATTTT
TCTCATTCAATCAAACTATGGATGGCGTTTGTAATGGAGCTGCTGTGCAGCGT
GCACCAGAGGCTCTGAGGTTTAATATTAATGACACCTCTGCCATTCTTGCTGA
AGGCTCAATTGTACTTCATACTGCTTTAGGAACAAATTTTTCTTTTGTTTGCAG
TAATTCCTCAGATCCTCACTTAGCCACCTTCGCCATACCTCTGGGTGCTACCC
AGTTACCCTATTATTGTTTTTTTAAAGTGGATACTTTCAACTCTACTGTTTATA
 
AATTCTTGGCTGTTTACCTCCTACCGTCAGGGAAATTGTCATCACCAAGTAT
GGTGATGTTTATGTCAATGGGTTTGGCTACTTGCATCTTGGTTTGTTGGATGCT
GTCACAATTAATTTCACTAGTCATGGCACTGACGATGACGTTTCTGGTTTTTG
GACCATAGCATCGACTAATTTTGTTGATGCACTTATTGAAGTTCAAGGAACTG
CCATTCAGCGTATTCTTTATTGTGATGATCCTGTTAGCCAACTCAAGTGTTCTC
AGGTTGCTTTTGACCTTGACGATGGTTTTTACCCTATCTCCTCTACAAACCTCC
TGAGTCATGAACAGCCAACTTCTTTTGTTACTTTGCCATCATTTAATGATCATT
CTTTTGTTAATATTACTGTCTCTGCTGCTTTTGGTGGTCATAGTGGTGCCAACC
TTATTGCATCTGACACTACTATCAATGGGTTTAGTTCTTTCTGTGTTGACACTA
GACAATTTACCATTTCACTGTTTTATAACGTTACAAACAGTTATGGTTATGTGT
CTAAATCACAGGACAGTAATTGTCCTTTTACCTTGCAATCTGTTAATGATTAC
CTGTCTTTTAGCAAATTTTGTGTTTCTACCAGCCTTTTGGCTAGTGCCTGTACC
ATAGATCTTTTTGGTTACCCTGAGTTTGGTAGTGGTGTCAAGTTCACGTCCCTT
TACTTTCAATTCACAAAGGGTGAGTTGATTACTGGCACGCCTAAACCACTTGA
AGGTGTTACGGACGTTTCTTTTATGACTCTGGATGTGTGTACCAAGTATACTA
TCTATGGCTTTAAAGGTGAGGGTATCATTACCCTTACAAATTCTAGCTTTTTG
GCAGGTGTTCATTATACATCTGATTCTGGACAGTTGTTAGCTTTTAAGAATGT
CACTAGTGGTGCTGTTTATTCTGTTACGCCGTGTTCTTTTTCAGAGCAGGCTGC
ATATGTTGATGATGATATAGTGGGTGTTATTTCTAGTTTGTCTAACTCCACTTT
TAACAGTACTAGGGAGTTGCCTGGTTTCTTCTACCATTCTAATGATGGCTCTA
ATTGTACAGAGCCTGTGTTGGTGTATAGTAACATAGGTGTTTGTAAATCTGGC
AGTATTGGCTATGTCCCATCTCAGTCTGGCCAAGTCAAGATTACACCCACGGT
TACTGGGAATATTAGTATTCCCACCAACTTTAGTATGAGTATTAGGACAGAAT
ATTTACAGCTTTACAACACACCTGTTAGTGTTGATTGTGCTACATATGTTTGTA
ATGGTAACTCTCGTTGTAAACAATTACTCACCCAGTACACTTCAGCATGTAAG
ACCATAGAGTCAGCATTACAACTCAGCGCTAGGCTTGAGTCTGCTGAAGTCA
ACTCTATGCTTACTATTTCTGAAGAGGCTCTACAGTTAGCTACCATCAGTTCG
TTTAATGGTGATGGATATAATTTTACCAACGTGCTGGGTGTTTCCGTGTATGA
 
TCCTGCAAGTGGCAGGGTGGTACAAAAAGGTCTTTATTGAAGACCTGCTTT
TTAATAAAGTGGTTACTAATGGTCTTGGTACTGTTGATGAAGACTATAAGCGC
TGTTCTAATGGCCGCTCTGTGGCAGATTTAGTCTGTGCGCAGTATTACTCTGG
TGTCATGGTACTACCTGGTGTTGTTGACGCTGAGAAGCTTCATATGTATAGTG
CGTCTCTCATCGGTGGTATGGTGCTAGGAGGTTTTACTGCAGCAGCGGCATTG
CCTTTTAGCTATGCTGTTCAAGCGAGACTTAATTATCTTGCTCTACAGACGGA
TGTTCTACAGCGGAACCAGCAATTGCTTGCTGAGTCTTTTAATTCTGCTATTG
GTAATATAACTTCAGCCTTTGAGAGTGTTAAAGAGGCTATTAGTCAAACTTCT
AAGGGTTTGAACACTGTGGCTCATGCGCTTACTAAGGTCCAAGAGGTTGTTAA
TTCGCAGGGTGCAGCTTTGACCCAACTTACCGTACAGCTGCAACACAACTTCC
AAGCCATTTCTAGTTCTATTGATGACATTTACTCCCGACTTGACATTCTTTCAG
CCGATGCTCAGGTAGACCGTCTCATCACCGGCAGATTATCAGCACTTAATGCT
TTTGTTGCTCAAACCCTTACTAAGTATACTGAGGTTCAGGCTAGTAGGAAACT
AGCACAGCAAAAGGTTAATGAGTGCGTTAAATCGCAATCTCAGCGTTATGGT
TTTTGTGGTGGTGATGGCGAGCACATTTTCTCACTGGTACAGGCCGCACCTCA
AGGCCTGCTGTTTTTACACACAGTACTTGTACCGGGTGACTTTGTAAATGTTA
TTGCCATCGCTGGCTTATGTGTTAACGATGAAATTGCCTTGACTCTACGTGAG
CCTGGCTTACTCTTGTTTACGCATGAACTTCAAGATACTGCGACGGAATATTT
TGTTTCATCGCGACGTATGTATGAACCTAGAAAACCTACCGTTGGTGATTTTG
TTCAAATTGAGAGTTGTGTGGTCACCTATGTCAATTTGACTAGAGACCAACTA
CCAGAAGTAATCCCAGATTACATCGATGTTAACAAAACATTTGATGAGATTTT
AACTTCTCTGCCCAATAGAACTGGTCCAAGTCTTTCTCTAGATGTTTTTAATGC
CACTTATCTTAATCTCACTGGTGAAATTGCAGATTTAGAGCAGCGTTCAGAGT
CTCTCCGTAATACTACAGAAGCGCTCCAAAGTCTTATATATAATATCAACAAC
ACACTAGTTGACCTTGAGTGGCTCAACCGAGTTGAGACATATATCAAGTGGC
CGTGGTGGGTTTGGTTGATTATTTTTATTGTTCTCATCTTTGTTGTGTCATTACT
AGTGTTCTGCTGCATTTCCACGGGTTGTTGTGGGTGCTGCGGCTGCTGTGGTG
CTTGTTTTTCAGGTTGTTGTAGGGGTCCTAGACTTCAACCTTACGAAGCTTTTG
 
AAAAGGTCCACGTGCAGTGAGTCGCTTGCAAAGACAACGCCACATTTTGGGA
ACGCGTCCGATCCATCCTGAAATCTGGGCTCAACTTTGCTTCGACCATACCTG
GCCCCGTAGGAGTGGCAGCGACAGGGATCAAAGGCATCATTGAAGCCATTGG
TTCCTTGTGGGTTTGATTCCACCCACAGAAGCGTTGACGACGCAAAACGTCCT
TAAAGCGTTGACGACGCAAAACGTCCCCAAGCTCGTAGCACCGACCCTATAC
CCATCTCTAGGGTCTTCAACCTCTTGGTGGCCGGCATGGTCCCAGCCTCCTCG
CTGGCGCCGGCTGGGCAACACCATTGCACTCCGGTGGCGAATGGGACT
EXAMPLE pRNA2_HA5_St_Jude (DNA vector construct) 
TAATACGACTCACTATAGTAAACAACCAATAACATCAGGGTATCCAAA
GCAGCACGCCGCCGAAGAGCGATGGAGAAAATAGTGCTTCTTCTTGCAATAG
TCAGTCTTGTTAAAAGTGATCAGATTTGCATTGGTTACCATGCAAACAACTCG
ACAGAGCAGGTTGACACAATAATGGAAAAGAACGTCACTGTTACACACGCCC
AAGACATACTGGAAAAGACACACAACGGGAAGCTCTGCGATCTAGATGGAGT
GAAGCCTCTAATTTTAAGAGATTGTAGTGTAGCTGGATGGCTCCTCGGGAACC
CAATGTGTGACGAATTCCTCAATGTGCCGGAATGGTCTTACATAGTGGAGAA
GATCAATCCAGCCAATGACCTCTGTTACCCAGGGAATTTCAACGACTATGAA
GAACTGAAACACCTATTGAGCAGAATAAACCATTTTGAGAAAATTCAGATCA
TCCCCAAAAGTTCTTGGTCAGATCATGAAGCCTCATCAGGGGTGAGCTCAGC
ATGTCCATACCAGGGAAGGTCCTCCTTTTTAGAAATGTGGTATGGCTTATCA
AAAAGAACAATGCATACCCAACAATAAAGAGAAGTTACAATAATACCAACC
AAGAAGATCTTTTGGTACTGTGGGGATTCACCATCCAAATGATGCGGCAGA
GCAGACAAGGCTCTATCAAAACCCAACCACCTATATTTCCGTTGGGACATCA
ACACTAAACCAGAGATTGGTACCAAAAATAGCTACTAGATCCAAGGTAAACG
GGCAAAGTGGAAGGATGGAGTTCTTTTGGACAATTTTAAAACCGAATGATGC
AATAAACTTTGAGAGTAATGGAAATTTCATTGCTCCAGAAAATGCATACAAA
ATTGTCAAGAAAGGGGACTCAACAATTATGAAAAGTGAATTGGAATATGGTA
ACTGCAACACCAAGTGTCAAACTCCAATAGGGGCGATAAACTCTAGTATGCC
ATTCCACAACATCCACCCTCTCACCATCGGGGAATGCCCCAAATATGTGAAAT
 
CAAACAGATTAGTCCTTGCGACTGGGCTCAGAAATAGCCCTCAAATTGAAAC
TAGAGGATTATTTGGAGCTATAGCAGGTTTATAGAGGGAGGATGGCAGGGA
ATGGTAGATGGTTGGTATGGGTACCACCATAGCAACGAGCAGGGGAGTGGGT
ACGCTGCAGACAAAGAATCCACTCAAAGGCAATAGATGGAGTCACCAATAA
GGTCAACTCGATCATTGACAAAATGAACACTCAGTTTGAGGCCGTTGGAAGG
GAATTTAATAACTTAGAAAGGAGAATAGAAAATTTAAACAAGAAGATGGAA
GACGGATTCCTAGATGTCTGGACTTATAATGCTGAACTTCTGGTTCTCATGGA
AAATGAGAGAACTCTAGACTTTCATGACTCAAATGTCAAGAACCTTTACGAC
AAGGTCCGACTACAGCTTAGGGATAATGCAAAGGAGCTTGGTAACGGTTGTT
TCGAGTTCTATCACAGATGTGATAATGAATGTATGGAAAGTGTAAGAAACGG
AACGTATGACTACCCGCAGTATTCAGAAGAAGCAAGATTAAAAGAGAGGA
AATAAGTGGAGTAAAATTGGAATCAATAGGAACTTACCAAATACTGTCAATT
TATTCAACAGTGGCGAGCTCCCTAGCACTGGCAATCATGGTGGCTGGTCTATC
TTTGTGGATGTGCTCCAATGGATCGTTACAATGCAGAATTTGCATTTAAGTCG
CTTGCAAAGACAACGCCACATTTTGGGAACGCGTCCGATCCATCCTGAAATCT
GGGCTCAACTTTGCTTCGACCATACCTGGCCCCGTAGGAGTGGCAGCGACAG
GGATCAAAGGCATCATTGAAGCCATTGGTTCCTTGTGGGTTTGATTCCACCCA
CAGAAGCGTTGACGACGCAAAACGTCCTTAAAGCGTTGACGACGCAAAACGT
CCCCAAGCTCGTAGCACCGACCCTATACCCATCTCTAGGGTCTTCAACCTCTT
GGTGGCCGGCATGGTCCCAGCCTCCTCGCTGGCGCCGGCTGGGCAACACCAT
TGCACTCCGGTGGCGAATGGGACT
EXAMPLE pRNA2_Ssuis_ide_mut (DNA vector construct)
   T7
   5' UTR (original start codon deleted)
   Murine Ig-Kappa leader sequence
   Strep suis Ide from strain P1/7 (mutated N-glycosylation deleted)
   3' UTR
   HDV ribozyme 
TAATACGACTCACTATAGTAAACAACCAATAACATCAGGGTATCCAAA
GCAGCACGCCGCCGAAGAGCGATGGAGACAGACACACTCCTGCTATGGGTAC
TGCTGCTCTGGGTTCCAGGTTCCACTGGTGACAACATTCAAGAACGATTTTCT
TTGAGAAAATCCGCGGTTGGCTTGGTCTCAGTCTCTTTGCTATGTGCTATTTAT
ACATCCACTGTTGCTGCCGATACAGTTGTTACAGGAGTGAATGAAATAATTGA
AGAATCACAAGTCAAGGATGAGGTATCTATTGAATCAGAAAAAAATGAATCC
CTAGATGGTTCTAATATTGAAATTGTAGAGGAAATAGCAGACAACATCCCAT
CACCTGTTATCGCTGAAGGGGAAGTAGCGGTAGAGATGAAAGTTGACAGAGG
GACCGAGAATGTAGTTTCTAGAAATGATACAGAAGTTACGACGAGCGAGCAA
AATCAGATAGAGGTTACTGAGACAAAAGAAATTTTGAATCAGgCCAGTTATC
AAACGGAGAGTGGCGAGCAACGACAAATTATATGGCCCATGGAATTACTCC
TCCTGCAATGGAACAAAGCGGTGGTTTGTAAAGGAAAAGTATGGAGACTAT
TTAAACTATgCAGCGCCATTTGAGGCTGGAAAAGGCTACTATGATACCAATAA
GgcTCTGAATGCTgCATTTATTGACCTTAATCTTTGTTTTGCAGCTGTGTCTTCA
AACATGGTACATTGGTGGTTGGAACAGAATAGTTCCTATGTTGAGCGATATCT
CAAAGAAAAAAAGGGTACAGTAAATGTTGAAGAAAACTACGCAATAACGGA
CTTACGGCGCTATATTAATTCATTCCAAAATCAACAAAATAGTCGAGTTTTTG
ATATGTTCAAAACTTACTATGGTTATCGTACAAATGGTTTTGTATCAGATGCC
TTGGTTGACTTGTTTATTAACGGATATAAACCTAAGGCACAGGGCGGTGTCAA
TCTGGAAGATAGCCAGTTAGTACCAGATAGTAGGGGTGGCTTTTTCTACGACG
TTTTCAAAGAGAAAAAACTGACAAATCGAATTTTTAGTGGTAGTTATGAGCG
GTTTGGTGAGGATGTTCGAACTGTTTTGGAAAGCAAAGGATTACTCGGCTTAA
CTTATAGAACATTAGGTTATGCAACGCATATTGTGACGGTATGGGGTGCTGAG
TACGACAATCAAGGTAAGATTAAGGCTGTCTATATCACAGATTCTGATGATCA
ACAAGAACAAATTGGTTTGAAGCGTATGGGAATCACTCGTGATGCTTCCGGA
AATCCACGTTTGAATAATCATATGAAAAATAATgCAGCTGGAGCGCTTTTGGA
TTATGTCCATACAATCCGTCTGGGTCAAGACTTATGGGAAGAATATTTCAATC
CGCTTGCAAAAGCCAAAGAAACAGCTAGTCAGACATTAGCCGATACAAAGAA
 
GGCGTTGGATTTGTCTATTCAAGGACAATCTGAATTGCCAGAATCAATGCGAC
TGATTTATCTTGAAAAACTAAATAATCTCTATAATCAAGGAATTCTATCTATT
CAAAAGGCAGAAAGTTCTGAGATGCTAAGTGGTGCATTGGAAAATGGTTTAA
ATAGTTTAAAGAGTTTAGATTTTCCTATTTCAGAAGTTGGAAATGCTTTGGCA
CCAGATTTACCAGTAGGTGATCGCTCAACGGTTTCAGATGTTGATTCTCTATC
ATCTCAAGAAACAAGTTCCACAAATTTGGAAGCAGACACAGAGAATGCAGGT
ATTATTGCAGATGGTACCAATCAATTGCATTTTCCAGTGGAGGCCCAAACGAC
ATCTTCAGTAGAGGCTGAGGGAGATAATGTTTTTGAACAAGAGGCAGATACA
TTACCAATAATTATTGAAAACAAGGATGAATTTGGTTCAGAACTATCAAGAA
ACATGCAAACGTCAGAAACGGATTCGCTAGTAGTAGCTGTTGAAGAAGATGT
GAAAAATGATGAGGTAGCCCAAGTTGAAGAGCTTCTTGAATCAGAAAAAGTT
GAAAATCAGAGTTCGGAACTTCTGTCAGACACCCTAATCGTAGAGAGTGCAA
ATGACAAAGAAGATAGAGTGGAGGCGGTTGTTTCTGAACAACCAGACTC
AATACCACATCAAAATGTAGAAATCTCTCTTGTAGAACCAACGAATGTCGAA
ACTGAAACTGTGGTCACTCCTATTAATGATGCAGCTACTCCTCATGGTTCCCC
GACGTATATAGATAATTCCGTAACTGAATCTGTAGCTACTCCACTTGAAAAAG
ACTCCATTCAAGCCGGGGAGACAGAGATTGCAGAACCAACCTCGAGCGAATC
AACGAATGTCGAAACTGAAACTGTGGTCACTCCTGTTAATGATGTAGCTACTC
CTCATGGTTCCCCGACGTATATAGATAATTCCGTAACTGAATCTGTAGCTACT
CCACTTGAAAAAGACTCCATTCAAGCCGGAGAGACAGAAATTGCAGAACCAA
CCTCGAGCGAATCAACGAATGTCGAAACTGAAACTGTGGTCACTCCTGTTAAT
GATGTAGCTACTCCTCATGGTTCCCCGACGTATATAGATAATTCCGTAACTGA
ATCTGTAGCTACTCCACTTGAAAAAGACTCCATTCAAGCCGGGGAGACAGAG
ATTGCAGAACCAACCTCGAGCGAATCAACTAGTGTTGAAGCTGAACTTGTCG
ACAATTCTGAAATTCATGCAGCTACCTCTTCAGTTACTCCCTGTGGCTCCTCG
GCATATGCAGATGGTTCCACAACTGAATCTGTAGCCACTCCGCTTGAAAAAG
ACTCCATTCAGACTGGAAATACAGAAATTGCAGAACCAACCTCGAGCAAATC
AACTAATGTAGAAGCTGCATCTGTCGACAATTCTGAAATTCATGCAGATGCCT
 
CTCTAACTGCTGTTTCATCAGTTAATCTGGATAATCCAGTGATTGAACCAGTA
GCTATCTCCCTTATCGGTTCTAAGAGGACACGAATGCAGAAGTAGAAGTTTC
TTCATTATCGAAAAGAGAGGTTAGAAAAACAAATACTGACGGCTAATCTCT
GTTCAATCAAAAGTTATTAAGAAAGAATTGCTAGAATCAAGCTTAGCAGAAG
CAGGGTCTCCATTGCTAGAAGCCACCATTGCTCAGTCTTCAAACTCAAATAGT
ACTGAGATAGGTATGAGCTATCAGAATACTGTGTTATTAGAGTCTAATAATAC
AGAGCGTCAGGTGTCTAAAGCAGAAATTGTTATGGAACACAAGGAGACAGA
GTTAGTTGAAACGGTTTCATCTGCTTCTGAGCCTGTAGTGCTCGTAGAAAATA
TCTCACAAACCTCAAATAAGTCGCTTGCAAAGACAACGCCACATTTTGGGAA
CGCGTCCGATCCATCCTGAAATCTGGGCTCAACTTTGCTTCGACCATACCTGG
CCCCGTAGGAGTGGCAGCGACAGGGATCAAAGGCATCATTGAAGCCATTGGT
TCCTTGTGGGTTTGATTCCACCCACAGAAGCGTTGACGACGCAAAACGTCCTT
AAAGCGTTGACGACGCAAAACGTCCCCAAGCTCGTAGCACCGACCCTATACC
CATCTCTAGGGTCTTCAACCTCTTGGTGGCCGGCATGGTCCCAGCCTCCTCGC
TGGCGCCGGCTGGGCAACACCATTGCACTCCGGTGGCGAATGGGACT
EXAMPLE pRNA1-FHV (DNA vector construct) 
TAATACGACTCACTATAGGTTTCGTTTTCGAACAAACAAACAGCGAAC
CCTAAAACAATGACCCTAAAAGTTATTCTTGGAGACCACCAGATCACCCGGA
CTGAATTGCTCGTCGGGCTTGCAACCGTATCTGGGTGCGGTGCCATAGCGTAC
TGCATATCCAAGTTCTGGGGCTATGGGGCAATTGCGCCCTATCCCCAGAGTGG
AGGGAACCGAGTTACACGCGCATTGCAACGGGCTGTCATTGACAAAACGAAG
ACCCCGATAGGAACACGTTTCTATCCGCTTGACAGCCTGCGTACCGTAACGCC
TAAGCGTGCCATTAATAACGGGCATGCCGTTTCAGGAGCCGTACGTGATGCC
GCTCGTCGTTTAATCGACGAGGCTATAACATCTGTAGGAGGATCTAAATTTGA
AGTTAACCCGAACCCAAATTCTAGCACTGGACTGCGGAAACATTTCCACTTCG
CCGTCGGTGATTTGGCGCAAGACTTTCGCAATGATACACCAGCGGAAGACGC
CTTCATCGTAGGTGTTGATGTTGATTATTATGTCACCGAGCCAGATGTGCTGT
TAGAGCACATGCGTCCAGTGGTTTTACACACCTTCAACCCGAAGAAAGTGAG
 
CGGCTTTGATGCTGACTCACCATTCACCATCAAGAACAATCTGGTTGAATACA
AGGTTAGTGGAGGTGCTGCGTGGGTCCATCCAGTTTGGGATTGGTGCGAAGC
TGGTGAGTTCATCGCCAGCCGAGTCCGAACAAGTTGGAAGGAATGGGTTTA
CAACTGCCATTGAGAATAGTTGGTTTAGAAAAGGTCGGTTATCACAAAATCC
ACCATTGTAGACCCTGGACTGATTGTCCAGATCGATCACTCGTCTACACAATA
CCGCAGTATACTGTTTGGCGGTTCAATTGGATCGACACCGAGCTACACGTACG
CAAACTAAAACGGATTGAATACCAGGATGAAACTAAACCTGGTTGGAACAGA
TTGGAGTATGTCACCGACAAGAACCAACTAATGGTTTCCATCGGTAGAGAAG
GAGAGCACGCTCAGATCACCATCGAGAAAGAAAAGTTGGATATGCTCTCGGG
ATTATCCGCCACACAATCTGTCAACGCTAGGCTTATCGGTATGGGGCACAAG
GACCCACAGTACACATCTATGATTGTTCAGTATTACACCGGCAAGCGAGTTGT
GTCACCAATTAGTCCAACTGTGTATAAACCTACAATGCCACGCGTCCATTGGC
CAGTAACCAGTGACGCAGATGTACCAGAAGTGAGCGCACGCCAATACACCTT
GCCTATCGTGAGTGACTGTATGATGATGCCAATGATTAAACGCTGGGAGACA
ATGTCTGAATCAATTGAACGCAGGGTAACCTTTGTCGCCAACGACAAGAAAC
CAAGCGACAGAATTGCGAGGATCGCCGACACTTTCGTACGGTTGATGAACGG
GCCTTTCAACAACCTTGACCCATTGTCAATCGAGGAAACGATTAAGCGTCTAA
ACAAGCCGTCCCAACAACTACAACTTAGGGCGGTTTTCGAGATGATCGGAGT
TGAGCCGCGTCAATTGATTGAGTCATTCAACAAAAACGAACCGGGAATGAAA
TCTAGTCGGATAATATCCGGCTTTCCTGACATCCTTTTCATCCTGAAGGTCTCC
AGATACACATTAGCGTACTCGGATATCGTTCTACATGCCGAGCATAATCAACA
TTGGTATTATCCTGGAAGGAACCCAACTGAGATTGCCGATGGGGTTTGTGAAT
TTGTCAGTGACTGTGACGCTGAAGTCATAGAAACGGATTTTTCCAACCTCGAT
GGCCGGGTTTCTGGCTGGATGCAACGAAATATCGCTCAAAAGGCGATGGTTC
AAGCATTCCGGCCAGAATACAGAGATGAGATCATTTCATTCATGGACACGAT
AATCAATTGTCCAGCCAAAGCTAAACGCTTTGGTTTCCGATATGAGCCTGGAG
TTGGTGTTAAAAGTGGTAGTCCAACAACCACGCCGCATAACACGCAATATAA
TGCATGTGTCGAATTTACAGCTCTCACCATTGAGCACCCTTATGCCGAACCAG
 
AAGAACTGTTCCGATTAATCGGTCCAAAGTGCGGTGATGATGGTCTGTCGCG
GGCCATCATTCAAAATGCCATCAACCGTGCTGCCAAATGTTATGGCTTGGAGC
TCAAAGTGGAACGATACAACCCAGAGATAGGTCTTTGCTTTCTCTCACGTGTT
TTTGTGGACCCGCTCGCAACGACGACAACACTACAAGACCCACTGCGTACAC
TGCGAAAACTGCACCTCACAGCAAGAGATCCTACGATACCACTAGCTGACGC
GGCTTGCGACCGCGTCGAAGGTTATCTTTGTACCGATGCGCTTACTCCGTTGA
TATCGGATTACTGCAAAATGGTACTACGGTTATACGGGCCGACTGCCTCCACA
GAGGAAGTCAGGAACCAACGTAGAAGCCGGAATAAGGAGAAACCCTACTGG
TTAACGTGCGATGGATCATGGCCACAGCATCCGCAAGACGTCCATTTGATGA
AGCAGGTTTTAATCAAGCGTACCAGAATTGACGAAGATCAGGTCGATGCACT
CATTGGGCGTTTTGCCGCAATGAAGGATGTCTGGGAGAAGATTACACGTGAC
AGCGAGGAGAGCGCCGCTGCGTGTACGTTTGATGAAGACGGCGTTATGCCGG
GCTCCGTGGACGAATCGTTACCTAAGCTTAACGATGCCAAGCAAACTCGCGC
TAATCCAGGAACTTCCCGACCGCATTCAAACGGCGGTAGAAGCAGCAATGGG
AATGAGCTATCAGGACGCACCGAACAACGTGCGCAGGGACCTCGACAACCTG
CATGCTTGCCTAAACAAGGCAAAGCTAACGGTAAGACGAATGGTAACATCAC
TGCTGGAGAAACCCAGCGTGGTGGCATACCTCGAAGGAAGGGCCCCGGAGG
AGGCAAAACCAACACTCGAAGAACGCCTCCGAAAGCTAGAGCTCAGCCACA
GCCTTCCAACAACCGGAAGTGACCCCCCACCCGCTAAATCGTAGGTGACTCCT
AGGAGCACCCACACACGTTCTAGCCCGAAAGGGCGGAGGTGGCCGGCATGGT
CCCAGCCTCCTCGCTGGCGCCGGCTGGGCAACACCATTGCACTCCGGTGGCG
AATGGGACT
EXAMPLE pRNA2_FHV (DNA vector construct)

TAATACGACTCACTATAGTAAACAACCAATAACATCATGGTATCCAAA
GCAGCACGCCGCCGAAGAGCGGCCCCTCGACAACAACAACGACAACAGTCG
AACCGTGCATCAAACCAGCCTCGTCGGAGGAGAGCTCGCCGTACTCGAAGAC
AGCAGAGAATGGCAGCAACAAACAACATGCTGAAAATGAGCGCTCCCGGAC
TCGATTTTCTGAAATGTGCCTTTGCTTCGCCGGATTTTTCCACCGATCCCGGCA

AAGGTATTCCAGACAAATTTCAGGGTCTCGTTTTACCGAAGAAACATTGTCTG
ACCCAGTCGATAACGTTTACTCCGGGGAAACAGACTATGCTGTTGGTTGCACC
TATTCCTGGAATTGCTTGCCTGAAGGCAGAAGCGAATGTTGGCGCATCCTTTT
CAGGTGTTCCTCTTGCCTCAGTTGAATTTCCAGGGTTCGACCAGCTGTTTGGC
ACGTCAGCAACTGACACCGCAGCTAATGTCACTGCTTTCCGGTATGCGTCAAT
GGCAGCCGGTGTTTACCCCACGAGCAATCTAATGCAATTTGCTGGGTCAATAC
AGGTGTACAAGATACCTCTAAAACAGGTTCTTAACTCCTACTCTCAGACCGTA
GCGACCGTACCACCTACCAACTTGGCTCAGAACACAATTGCAATAGATGGAC
TAGAAGCGTTAGATGCACTACCAAATAACAACTACTCCGGTTCTTTTATCGAG
GGTTGTTATTCACAATCGGTGTGCAATGAACCTGAGTTTGAGTTCCATCCCAT
CATGGAGGGTTACGCGTCCGTTCCACCCGCGAACGTAACCAATGCTCAAGCT
AGCATGTTCACCAATCTAACCTTCTCAGGCGCAGCATATACCGGTCTTGGCGA
CATGGACGCAATTGCGATCCTTGTGACTACGCCCACCGGTGCCGTCAACACG
GCCGTGCTGAAGGTCTGGGCCTGCGTAGAGTATCGTCCGAATCCAAATTCCAC
CCTCTATGAGTTCGCTCGTGAGTCACCAGCAAACGACGAATACGCGCTCGCC
GCCTATAGGAAAATTGCCAGAGATATTCCGATTGCCGTCGCTTGCAAAGACA
ACGCCACATTTTGGGAACGCGTCCGATCCATCCTGAAATCTGGGCTCAACTTT
GCTTCGACCATACCTGGCCCCGTAGGAGTGGCAGCGACAGGGATCAAAGGCA
TCATTGAAGCCATTGGTTCCTTGTGGGTTTGATTCCACCCACAGAAGCGTTGA
CGACGCAAAACGTCCTTAAAGCGTTGACGACGCAAAACGTCCCCAAGCTCGT
AGCACCGACCCTATACCCATCTCTAGGGTCTTCAACCTCTTGGTGGCCGGCAT
GGTCCCAGCCTCCTCGCTGGCGCCGGCTGGGCAACACCATTGCACTCCGGTGG
CGAATGGGACT

### List of quasispecies NoV sequences

Published RNA1 NoV quasispecies variant sequences identified as PN0448 taken from AF174533 and NC_002690
ORIGIN GTATTGAATCCAAAACTCAAAATGCTGAACTACGAGACAATCATCAAC 
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAATGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCACGAACAATCAAGTC
ACGTTTGATGTTAGTGGCGGTGGATCTTGGTCCCATGAAGTTTGGGACTGGTG
CGCGTTTGGTGAGTTCATCGAGACCCGAGACGCGAGCTGGCTTGCTTGGTTCG
CCCGGGCGGTTGGACTCACCAAGTCGCAGATCCACAAAGTTCACTACTGCCG
TCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGCGCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
 
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTCCAGCTATACGCTGG
CATTCCGTGATCAGGTGCTGCATGCAGAGCACAACCGGCATTGGTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGTTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTCGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
 
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCTGGGTCGCGTGG
GCGTGGTAAATGAGTGATTCATCGTCCCATCTGACGAAACCCGAACTAGGCTT
ATGCCAGTGGT
Novel RNA1 NoV quasispecies variant sequence PN0448 with nucleotide polymorphisms identified at positions 336, 664, 702, 726, 732, 777, 837, 1218, 1219, 1956, 2589, 3129, 3179 are underlined. Nucleotide polymorphisms resulting in a coding change in the replicase are indicated in bold at positions **664** and **1219.**
RNA1 
GTATTGAATCCAAAACTCAAAATGCTGAACTACGAGACAATCATCAAC
GGCGCATCGAGCGCTCTGAACATCGTTTCGCGTGCGTTAGGATACCGCGTGCC
ACTAGCCAAATCGCTGGCGCTGGTCGCGGGTCCTGCGTGGTGTACAAAATA
ATCGTGCATCGACGCACGCTCGTGGCGTTCCTGGTAATCGGACCATACGCCAC
GGTGGTGCAGCACCGTCTGCCGATGGCCCTTCAGAGGGCCATCATTGAATAT
ACACGAGAAGACCGTGAGATCAGCCTGTTTCCGCAAAATTCCATCGTTTCCGC
AGAACACGCGCGGAAAGCGGATAACGGGCATCCGATCTCCGGGGGAACGCG
TGATGTCGCGAGGGAGACTATTTCCCTTGCCATAAGGGCCGCTGGTTTTCGTC
ATTACGAAATCAGCCCCGCGAGGCAATCACCAGCTGAGGCGGCAAGCCACCA
ACATTATGCCGCCGCTGACCTCGTGAGAGCGGCTACTGAAGATAAGATCCAA
GACGGTGATGTGGTAGTTGCCATCGACATCGATTACTACCTGCGTGACATTGA
CCGCTACCTGGGTCGTGGTGTCCCGTTCATGGCTTACACCTTCAATCCTGTTG
AAGTAGCTGGCCGTGACGGTGACTCCTTTTTCCGGATCGCGAACAATCAAGTC
ACGTTTGATGTTAGTGGCGGTGGGTCTTGGTCCCATGAAGTTTGGGATTGGTG
TGCGTTTGGTGAGTTCATCGAGACCCGAGACGCGAGCTGGCTTGCCTGGTTCG
CCCGGGCGGTTGGACTCACCAAGTCGCAGATCCACAAAGTTCACTACTGCCG
CCCATGGCCGCAATCGCCCCATCGCGCTTTGGTGTGGTGTCTGCCTGTAGCAA
GCTACTGGCGCTTCACTTTCATTCCGACGGACCTGCATACGCGCACGCTTCGG
 
CGTGTGCGTTATCAGGACACGTCCCGGCCCGGTTGGAATTCCATCGTCTCGAC
CGGGTCCGAAGGCCTTAATATCAGCCTTGGTCGCGAAGGAGCTGATCATTGC
GTGACGATTCCAAAGGTGCACTACGACATGCTTATGGGTTTGTCGAGTGCGCA
GTCGTTGTCGTCCCGCATGATCGGGCTCAAGTACACTGATCCTAGTGTACTCG
CGACGGTTGCCCAATACTATCAGGGCAAGAATGTTGAAGTTGCCGACGCTGA
CAGGATCGGCCGACCCATAAATCCCAAGGTCCACTGGCCAGCGCACGTCGAA
GTTGACGAGGCGGAGGTTAGTGCTCGGGTGTACGCCAGCCCGTTGGTATCTG
ACGAAAATATGATGCCTATGATCAAGCGCTGGGAGACGCTGTCGTTGTCGCT
GGACCGCCGGGTTACATTCCAACGTAATCCGAAGGTTCCTGGAAAACGGCTC
AGGGCTTATGCCATTGAGTTCGTTGACTTGGTTGTGCCTGAGCGTGGTGTCGG
AGTCCCCTATTCATTGGAGGACACCGCCGCCATGCTGGACAAACCAAGCCAG
ACCCTCGCCATCCAACAGGTGTGGGAGACTGTCGACATGCCCCCAAGAAGGC
TCATCGAAGCGTTCGTGAAGAACGAACCGACCATGAAGGCTGGCCGTATCAT
CTCGTCGTTCGCTGACATGCGGTTCCTACTGCGGTTTTCCAGCTATACGCTGG
CATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGT
TGAAGAACCATCGGAGGGAGACTTTTCCAACTTTGATGGCACGGTTAGTGAG
TGGCTACAACGCCACGTCATGAACGCCGTCTACCTGCGTTATTTCAATCACCG
AGCGCAGCGAGACCTCAGGTCGTATACCGACATGCTGGTCTCATGCCCCGCG
AGGGCGAAGCGATTCGGGTTTGCTTATGACGCGGGTGTCGGCGTTAAGAGCG
GGTCGCCAACAACTTGCGACCTGAATACCGTGTGCAATGGTTTCCTCCAATAT
TGCTCCATTCGAATGACACACCCAGAGCTGACACCAATCGATGCTTTCCGGCT
CATCGGTCTCGCGTTTGGGGACGATTCCCTCTTCGAGCGACGTTTCGCTAAGA
ACTATGCGAAGGTTTCCGCCGAGGTGGGGATGGTCCTCAAAATCGAGCGATT
CGACCCGGCACAAGGCATCACTTTCCTCGCCCGTGTTTATCCCGACCCCTACA
CGTCGACCACAAGTTTCCAGGACCCTTTGCGTACCTGGAGGAAGCTCCACTTG
ACGACGCGCGATCCAACAATACCATTGGCAACGGCTGCCATCGATCGCGTTG
AGGGCTACCTCGTCACCGACGGCCTGAGCCCGCTTACTGGCGCGTATTGTCGC
 
ATGGTTAAGCGGGTTTACGAGGCCGGCGGAGCCGAGGATGCCGCCAAGAGG
AGGTCGCGAAAATCCCATTCCCGCGAAAAGCCGTATTGGTTGACTGTTGGAG
GCGCTTGGCCCCAAGATGTCAAGGACGTTGATCTTATGTTCCAGTGTGCGGCC
GCACGTACCGGAGTAGACCTTGAGACACTTCGGTCTCTGGATCAGCGTCTAG
GAGAAATCACTGACGTCTGGGCGGATATTACCATCAACCGGGATAATGAACC
AAACCCCTACAAGGATACACTGGACTTGGAGGGCCCGGCTGATGGCCGGGTG
GACGATCGTGTATTTCAGAATGACAAACATGTCATGCGCTTACGAGCTAATCA
AGTCACTTCCAGCCAAGCTGGAGCAGCTGGCTCAGGAGACGCAAGCAACGAT
CCAAACGCTCATGATCGCGGATCCCAACGTCAACAAGGATCTGCGAGCGTTC
TGCGAGTTCCTGACCGTGCAGCACCAGCGGGCGTATCGAGCGACGAACAGCC
TGCTCATCAAACCGCGAGTCGCAGCAGCGCTTCGCGGGGAGGAGCTGGACCT
GGGCGAGGCGGACGTCGCCGCCCGGGTCCGCCAGCTAAAACAACAGCTGGCG
GAGCTCGAGATGGAAATCAAGCCAGGGCACCAACAAGTGGCCCAAGTAAGC
GGCAGGCGGAAGGCCGCAGCCGCAGCTCCCGTGGCCCAGCAGGGTCGCGTGG
GCGTGGTAAATGAGTGATTCATCGTCCCATCTGACGAATCCCGAACTAGGCTT
ATGCCAGTGGT
>rf 1 ORIGINALLY DETERMINED SEQUENCE (accession code Q9IMM4) 
MLNYETIINGASSALNIVSRALGYRVPLAKSLALVAGSCVVYKIIVHRRTL
VAFLVIGPYATVVQHRLPMALQRAIIEYTREDREISLFPQNSIVSAEHARKADNGH
PISGGTRDVARETISLAIRAAGFRHYEISPARQSPAEAASHQHYAAADLVRAATED
KIQDGDVVVAIDIDYYLRDIDRYLGRGVPFMAYTFNPVEVAGRDGDSFFRITNNQ
VTFDVSGGGSWSHEVWDWCAFGEFIETRDASWLAWFARAVGLTKSQIHKVHYC
RPWPQSPHRALVWCLPVASYWRFTFIPTDLHTRTLRRVRYQDTSRPGWNSIVSTG
SEGLNISLGREGADHCVTIPKVHYDMLMGLSSAQSLSSRMIGLKYTDPSVLATVA
QYYQGKNVEVADADRIGRAINPKVHWPAHVEVDEAEVSARVYASPLVSDENM
MPMIKRWETLSLSLDRRVTFQRNPKVPGKRLRAYAIEFVDLVVPERGVGVPYSLE
DTAAMLDKPSQTLAIQQVWETVDMPPRRLIEAFVKNEPTMKAGRIISSFADMRFL
LRFSSYTLAFRDQVLHAEHNRHWFCPGLTPEQIATKVVDYVSGVEEPSEGDFSNF
 
DGTVSEWLQRHVMNAVYLRYFNHRAQRDLRSYTDMLVSCPARAKRFGFAYDA
GVGVKSGSPTTCDLNTVCNGFLQYCSIRMTHPELTPIDAFRLIGLAFGDDSLFERR
FAKNYAKVSAEVGMVLKIERFDPAQGITFLARVYPDPYTSTTSFQDPLRTWRKLH
LTTRDPTIPLATAAIDRVEGYLVTDGLSPLTGAYCRMVKRVYEAGGAEDAAKRR
SRKSHSREKPYWLTVGGAWPQDVKDVDLMFQCAAARTGVDLETLRSLDQRLGE
ITDVWADITINRDNEPNPYKDTLDLEGPADGRVDDRVFQNDKHVMRLRANQVTS
SQAGAAGSGDASNDPNAHDRGSQRQQGSASVLRVPDRAAPAGVSSDEQPAHQT
ASRSSASRGGAGPGRGGRRRPGPPAKTTAGGARDGNQARAPTSGPSKRQAEGRS
RSSRGPAGSRGRGK
>rf 2 Novel quasispecies variant replicase sequence with coding changes T215A and A400P. 
MLNYETIINGASSALNIVSRALGYRVPLAKSLALVAGSCVVYKIIVHRRTL
VAFLVIGPYATVVQHRLPMALQRAIIEYTREDREISLFPQNSIVSAEHARKADNGH
PISGGTRDVARETISLAIRAAGFRHYEISPARQSPAEAASHQHYAAADLVRAATED
KIQDGDVVVAIDIDYYLRDIDRYLGRGVPFMAYTFNPVEVAGRDGDSFFRIANNQ
VTFDVSGGGSWSHEVWDWCAFGEFIETRDASWLAWFARAVGLTKSQIHKVHYC
RPWPQSPHRALVWCLPVASYWRFTFIPTDLHTRTLRRVRYQDTSRPGWNSIVSTG
SEGLNISLGREGADHCVTIPKVHYDMLMGLSSAQSLSSRMIGLKYTDPSVLATVA
QYYQGKNVEVADADRIGRPINPKVHWPAHVEVDEAEVSARVYASPLVSDENMM
PMIKRWETLSLSLDRRVTFQRNPKVPGKRLRAYAIEFVDLVVPERGVGVPYSLED
TAAMLDKPSQTLAIQQVWETVDMPPRRLIEAFVKNEPTMKAGRIISSFADMRFLL
RFSSYTLAFRDQVLHAEHNRHWFCPGLTPEQIATKVVDYVSGVEEPSEGDFSNFD
GTVSEWLQRHVMNAVYLRYFNHRAQRDLRSYTDMLVSCPARAKRFGFAYDAG
VGVKSGSPTTCDLNTVCNGFLQYCSIRMTHPELTPIDAFRLIGLAFGDDSLFERRF
AKNYAKVSAEVGMVLKIERFDPAQGITFLARVYPDPYTSTTSFQDPLRTWRKLHL
TTRDPTIPLATAAIDRVEGYLVTDGLSPLTGAYCRMVKRVYEAGGAEDAAKRRS
RKSHSREKPYWLTVGGAWPQDVKDVDLMFQCAAARTGVDLETLRSLDQRLGEI
TDVWADITINRDNEPNPYKDTLDLEGPADGRVDDRVFQNDKHVMRLRANQVTS
 
SQAGAAGSGDASNDPNAHDRGSQRQQGSASVLRVPDRAAPAGVSSDEQPAHQT
ASRSSASRGGAGPGRGGRRRPGPPAKTTAGGARDGNQARAPTSGPSKRQAEGRS
RSSRGPAGSRGRGK
RNA2 original 
GTAAACAACCAATAACATCATGGTATCCAAAGCAGCACGCCGCCGAA
GAGCGGCCCCTCGACAACAACAACGACAACAGTCGAACCGTGCATCAAACCA
GCCTCGTCGGAGGAGAGCTCGCCGTACTCGAAGACAGCAGAGAATGGCAGCA
ACAAACAACATGCTGAAAATGAGCGCTCCCGGACTCGATTTCTGAAATGTG
CCTTTGCTTCGCCGGATTTTCCACCGATCCCGGCAAAGGTATTCCAGACAAA
TTTCAGGGTCTCGTTTACCGAAGAAACATTGTCTGACCCAGTCGATAACGTT
TACTCCGGGGAAACAGACTATGCTGTTGGTTGCACCTATTCCTGGAATTGCTT
GCCTGAAGGCAGAAGCGAATGTTGGCGCATCCTTTTCAGGTGTTCCTCTTGCC
TCAGTTGAATTTCCAGGGTTCGACCAGCTGTTTGGCACGTCAGCAACTGACAC
CGCAGCTAATGTCACTGCTTTCCGGTATGCGTCAATGGCAGCCGGTGTTTACC
CCACGAGCAATCTAATGCAATTTGCTGGGTCAATACAGGTGTACAAGATACC
TCTAAAACAGGTTCTTAACTCCTACTCTCAGACCGTAGCGACCGTACCACCTA
CCAACTTGGCTCAGAACACAATTGCAATAGATGGACTAGAAGCGTTAGATGC
ACTACCAAATAACAACTACTCCGGTTCTTTATCGAGGGTTGTTATTCACAAT
CGGTGTGCAATGAACCTGAGTTTGAGTTCCATCCCATCATGGAGGGTTACGCG
TCCGTTCCACCCGCGAACGTAACCAATGCTCAAGCTAGCATGTTCACCAATCT
AACCTTCTCAGGCGCAGCATATACCGGTCTTGGCGACATGGACGCAATTGCG
ATCCTTGTGACTACGCCCACCGGTGCCGTCAACACGGCCGTGCTGAAGGTCTG
GGCCTGCGTAGAGTATCGTCCGAATCCAAATTCCACCCTCTATGAGTTCGCTC
GTGAGTCACCAGCAAACGACGAATACGCGCTCGCCGCCTATAGGAAAATTGC
CAGAGATATTCCGATTGCCGTCGCTTGCAAAGACAACGCCACATTTGGGAA
CGCGTCCGATCCATCCTGAAATCTGGGCTCAACTTTGCTTCGACCATACCTGG
CCCCGTAGGAGTGGCAGCGACAGGGATCAAAGGCATCATTGAAGCCATTGGT
TCCTTGTGGGTTTGATTCCACCCACAGAAGCGTTGACGACGCAAAACGTCCTT
 
AAAGCGTTGACGACGCAAAACGTCCCCAAGCTCGTAGCACCGACCCTATACC
CATCTCTAGGGTCTTCAACCTCTTGGT
RNA2 with nt-polymorphism at position 238 relative to AF 174534 and NC_002691

GTAAACAACCAATAACATCATGGTATCCAAAGCAGCACGCCGCCGAA
GAGCGGCCCCTCGACAACAACAACGACAACAGTCGAACCGTGCATCAAACCA
GCCTCGTCGGAGGAGAGCTCGCCGTACTCGAAGACAGCAGAGAATGGCAGCA
ACAAACAACATGCTGAAAATGAGCGCTCCCGGACTCGATTTTCTGAAATGTG
CCTTTGCTTCGCCGGATTTTTCCACCGATCCCGGCAAAGGTATTCCAGACAAA
TTTCAGGGTCTCGTTTTACCGAAGAAACATTGTCTGACCCAGTCGATAACGTT
TACTCCGGGGAAACAGACGATGCTGTTGGTTGCACCTATTCCTGGAATTGCTT
GCCTGAAGGCAGAAGCGAATGTTGGCGCATCCTTTTCAGGTGTTCCTCTTGCC
TCAGTTGAATTTCCAGGGTTCGACCAGCTGTTTGGCACGTCAGCAACTGACAC
CGCAGCTAATGTCACTGCTTTCCGGTATGCGTCAATGGCAGCCGGTGTTTACC
CCACGAGCAATCTAATGCAATTTGCTGGGTCAATACAGGTGTACAAGATACC
TCTAAAACAGGTTCTTAACTCCTACTCTCAGACCGTAGCGACCGTACCACCTA
CCAACTTGGCTCAGAACACAATTGCAATAGATGGACTAGAAGCGTTAGATGC
ACTACCAAATAACAACTACTCCGGTTCTTTATCGAGGGTTGTTATTCACAAT
CGGTGTGCAATGAACCTGAGTTTGAGTTCCATCCCATCATGGAGGGTTACGCG
TCCGTTCCACCCGCGAACGTAACCAATGCTCAAGCTAGCATGTTCACCAATCT
AACCTTCTCAGGCGCAGCATATACCGGTCTTGGCGACATGGACGCAATTGCG
ATCCTTGTGACTACGCCCACCGGTGCCGTCAACACGGCCGTGCTGAAGGTCTG
GGCCTGCGTAGAGTATCGTCCGAATCCAAATTCCACCCTCTATGAGTTCGCTC
GTGAGTCACCAGCAAACGACGAATACGCGCTCGCCGCCTATAGGAAAATTGC
CAGAGATATTCCGATTGCCGTCGCTTGCAAAGACAACGCCACATTTGGGAA
CGCGTCCGATCCATCCTGAAATCTGGGCTCAACTTTGCTTCGACCATACCTGG
CCCCGTAGGAGTGGCAGCGACAGGGATCAAAGGCATCATTGAAGCCATTGGT
TCCTTGTGGGTTTGATTCCACCCACAGAAGCGTTGACGACGCAAAACGTCCTT
AAAGCGTTGACGACGCAAAACGTCCCCAAGCTCGTAGCACCGACCCTATACC

CATCTCTAGGGTCTTCAACCTCTTGGT

### Further embodiments

1. A self-replicating ribonucleic acid (RepRNA)-vaccine pharmacon comprising ribonucleic acid encoding a gene-of-interest (GOI) and ribonucleic acid encoding at least a functional part of an RNA-dependent RNA polymerase derived from a quasispecies variant nucleic acid sequence of any of the *Nodaviridae*
2. The pharmacon according to embodiment 1 for use in a vaccine.
3. The pharmacon according to embodiment 1 or 2 comprising at least the first five (5) nucleotides located downstream of a first start codon ATG of a 5' conserved sequence element (CSE) flanking the RNA2 of any of the Alphanodaviridae, preferably of any of the Alphanodaviridae generally capable of replicating in cells derived from a warm-blooded animal.
4. The pharmacon according to anyone of embodiments 1 to 3 comprising a nucleic acid encoding at least a part of a first conserved sequence element (CSE) 5' flanking said RNA2, said 5' CSE comprising at least the first 3 nucleotides, preferably at least the first 19 nucleotides, more preferably at least the first 44 nucleotides, most preferably at least the first 52 nucleotides of a 5' conserved sequence element (CSE) flanking the RNA2 of a Nodamuravirus.
5. The pharmacon according to anyone of embodiments 1 to 4, provided with a ribonucleic acid encoding a functional RNA-dependent RNA polymerase comprising at least one nucleotide polymorphism selected from the group of nucleotide polymorphisms identified at positions 336, 664, 702, 726, 732, 777, 837, 1218, 1219, 1956, 2589, 3129, 3179, relative to quasispecies variant sequences obtainable from the nucleic acid sequences with accession numbers AF174533 or NC_002690.
6. The pharmacon according to anyone of embodiments 1 to 5, provided with a ribonucleic acid encoding a functional RNA-dependent RNA polymerase comprising at least one nucleotide polymorphism selected from the group of nucleotide polymorphism identified at positions 664 and 1219, relative to quasispecies variant sequences obtainable from the nucleic acid sequences with accession numbers AF174533 or NC_002690.
7. The pharmacon according to anyone of embodiments 1 to 6, comprising a nucleic acid encoding at least a part of a second CSE 3' flanking said RNA2, said 3' CSE comprising at least the last 50 nucleotides, preferably at least the last 100 nucleotides, more preferably at least the last 176 nucleotides, more preferably at least the last 236 nucleotides, most preferably at least the last 270 nucleotides of 3' conserved sequence element (CSE) flanking the RNA2 of a virus selected from the *Alphanodaviridae.*
8. The pharmacon according to anyone of embodiment 1 to 7, wherein said ribonucleic acid encoding a functional RNA-dependent RNA polymerase is fused with a nucleic acid encoding a conserved sequence element (CSE) flanking the RNA2 of a virus selected from the *Alphanodaviridae.*
9. The pharmacon according to any of embodiments 1 to 8, further comprising a nucleic acid derived from a wild-type RNA1 and/or RNA2 quasispecies variant of a virus selected from the *Alphanodaviridae.*
10. The pharmacon according to any of embodiments 1 to 9, wherein said RNA1 is a wild-type RNA1 of a virus selected from a quasispecies variant of the Nodamura virus (NoV).
11. A self-replicating ribonucleic acid (RepRNA) comprising a ribonucleic acid encoding a gene-of-interest (GOI) and a ribonucleic acid encoding at least a functional part of an RNA-dependent RNA polymerase derived from a quasispecies variant nucleic acid sequence of any of the *Alphanodaviridae* and comprising at least the first five (5) nucleotides located downstream of a first start codon ATG of a 5' conserved sequence element (CSE) flanking the RNA2 of any of the Alphanodaviridae, preferably of any of the Alphanodaviridae general capable of replicating in cells derived from a warm-blooded animal.
12. The RepRNA of embodiment 11 comprising a nucleic acid encoding at least a part of a first conserved sequence element (CSE) 5' flanking said RNA2, said 5' CSE comprising at least the first 3 nucleotides, preferably at least the first 19 nucleotides, more preferably at least the first 44 nucleotides, most preferably at least the first 52 nucleotides of a 5' conserved sequence element (CSE) flanking the RNA2 of a Nodamuravirus.
13. The RepRNA according to embodiment 11 or 12, provided with a ribonucleic acid encoding a functional RNA-dependent RNA polymerase comprising at least one nucleotide polymorphism selected from the group of nucleotide polymorphisms identified at positions 336, 664, 702, 726, 732, 777, 837, 1218, 1219, 1956, 2589, 3129, 3179, relative to quasispecies variant sequences obtainable from the nucleic acid sequences with accession numbers AF174533 or NC_002690, preferably provided with a ribonucleic acid encoding a functional RNA-dependent RNA polymerase comprising at least one nucleotide polymorphism selected from the group of nucleotide polymorphism identified at positions 664 and 1219, relative to quasispecies variant sequences obtainable from the nucleic acid sequences with accession numbers AF174533 or NC_002690.
14. The RepRNA according to anyone of embodiments 11 to 13 additionally comprising a nucleic acid encoding at least a part of a first conserved sequence element (CSE) 5' flanking said GOI, said 5' CSE comprising at least the first 3 nucleotides, preferably at least the first 19 nucleotides, more preferably at least the first 44 nucleotides, most preferably at least the first 52 nucleotides of a 5' conserved sequence element (CSE) flanking the RNA2 of a virus selected from the *Nodaviridae,* and/or comprising a nucleic acid encoding at least a part of a second CSE 3' flanking said RNA2, said 3' CSE comprising at least the last 50 nucleotides, preferably at least the last 100 nucleotides, more preferably at least the last 176 nucleotides, more preferably at least the last 236 nucleotides, most preferably at least the last 270 nucleotides of 3' conserved sequence element (CSE) flanking the RNA2 of a virus selected from the *Nodaviridae.*
15. The RepRNA according to anyone of embodiment 11 to 14, wherein said ribonucleic acid encoding a functional RNA-dependent RNA polymerase is fused with a nucleic acid encoding a conserved sequence element (CSE) flanking the RNA2 of a virus selected from the *Nodaviridae.*
16. The RepRNA according to anyone of embodiments 11 to 15, further comprising a nucleic acid derived from a wild-type RNA1 and/or RNA2 quasispecies variant of a virus selected from the *Nodaviridae.*
17. The RepRNA according to anyone of embodiments 11 to 16, wherein said RNA1 is a wild-type RNA1 of a virus selected from a quasispecies variant of the Nodamura virus (NoV).
18. The RepRNA according to anyone of embodiments 11 to 17 wherein RNA2 is additionally provided with a nucleic acid encoding an antigen, such as a vaccine antigen.
19. The RepRNA according to embodiment 18 for use in the production of a RepRNA.
20. The RepRNA according to embodiment 18 or 19 for use in the production of an RNA-pharmacon.
21. A ribonucleic acid according to anyone of embodiments 1 to 20 for use in the preparation of a formulation suitable for transfection.
22. A transfection formulation comprising ribonucleic acid according to anyone of embodiments 1 to 21.
23. A transfectant comprising ribonucleic acid according to anyone of embodiments 1 to 22.
24. A method for producing a protein in a subject comprising administering ribonucleic acid according to anyone of embodiments 1 to 21 or a formulation according to embodiment 22 to the subject.
25. A DNA vector encoding a ribonucleic acid according to anyone of embodiments 1 to 20.

## Claims

1. A ribonucleic acid (RNA)-pharmacon comprising at least a functional part of a trans-amplifying RNA (taRNA) replication element of an Alphanoda virus, preferably wherein said replication element is derived from an Alphanoda virus generally capable of replicating in cells derived from a warm-blooded animal.

2. The pharmacon of claim 1 comprising at least a native-length 5'-untranslated region (UTR) replication-element derived from the RNA2 of said virus, wherein said replication element is 3'-linked to a ribonucleic acid encoding an open-reading-frame of a gene-of-interest (GOI).

3. The pharmacon according to claim 2 wherein said replication-element is derived at by destruction of a first native start codon ATG in said RNA2, preferably by replacing T with G.

4. The pharmacon according to claim 3, wherein said replication-element is further 3'-extended with at least the first five (5) native nucleotides located downstream of a first start codon ATG between a 5' untranslated region and conserved sequence element (5'-UTR/CSE) flanking said RNA2.

5. The pharmacon according to anyone of claims 1 to 4 wherein said ribonucleic acid encoding said GOI is 3'-linked to a ribonucleic acid derived from at least the last 50, nucleotides of a 3'-UTR/CSE flanking said RNA2.

6. The pharmacon of anyone of claims 1-5 additionally encoding at least a functional part of a self-amplifying RNA (saRNA) replication element of an Alphanoda virus.

7. The pharmacon of claim 6 wherein the saRNA replication element is obtainable from a ribonucleic acid derived from the RNA1 of a quasispecies variant of said virus.

8. The pharmacon of claim 6 or 7 wherein the taRNA element as well as the saRNA element are located on a single (monocistronic) ribonucleic acid molecule.

9. The pharmacon according to anyone of claims 1 to 8 wherein said ribonucleic acid is capped.

10. The pharmacon of anyone of claims 1 to 9 for use in a vaccine.

11. A self-replicating bi-segmented or monocistronic ribonucleic acid (RepRNA) encoding a pharmacon according to anyone of claims 1 - 10.

12. A DNA-vector encoding a RepRNA according to claim 11.

13. A transfection formulation comprising a ribonucleic acid according to anyone of claims 1 to 11.

14. A transfectant comprising ribonucleic acid according to anyone of claims 1 to 11.

15. A method for producing a protein in a subject comprising administering ribonucleic acid according to anyone of claims 1 to 11 or a formulation according to claim 13 to the subj ect.
